# EUROPEAN PATENT APPLICATION

(11) **EP 4 851 148 A1**
(43) Date of publication of application: **09.12.2026**
(21) Application number: 25747789.3
(22) Date of filing: 24.01.2025
(51) Int. Cl.: C07D 401/14, C07D 401/04, C07D 487/08, C07D 471/08, C07D 405/14, A61K 31/501, A61K 31/517, A61P 35/00, A61P 31/00, A61P 29/00, A61P 25/00

(54) **NOVEL SUBSTITUTED ISOINDOLINONE SKELETON-BASED COMPOUNDS, BIFUNCTIONAL PROTEIN DEGRADERS CONTAINING SAME, AND USES THEREOF**

(30) Priority: 04.02.2024 CN 202410160525
(71) Applicant: Gluetacs Therapeutics (Shanghai) Co., Ltd., Shanghai 201306 (CN)
(72) Inventor: YANG, Xiaobao, Shanghai 201306 (CN); SUN, Renhong, Shanghai 201306 (CN); REN, Chaowei, Shanghai 201306 (CN); ZHAO, Baoyin, Shanghai 201306 (CN); LI, Yan, Shanghai 201306 (CN); ZHOU, Hailong, Shanghai 201306 (CN); YANG, Sen, Shanghai 201306 (CN); DENG, Meigui, Shanghai 201306 (CN); JIANG, Suping, Shanghai 201306 (CN); REN, Yinbo, Shanghai 201306 (CN)
(74) Representative: J A Kemp LLP
(86) International application number: PCT/CN2025/074554
(87) International publication number: WO 2025/162221

(57) **Abstract**

The present disclosure relates to compounds of Formula (I) and Formula (II), or a salt, enantiomer, stereoisomer, solvate, isotopically enriched analogue, prodrug, or polymorph thereof, and the use thereof. The present disclosure also relates to pharmaceutical compositions comprising, as an active ingredient, a compound of Formula (I) or Formula (II), or a salt, enantiomer, stereoisomer, solvate, isotopically enriched analogue, prodrug, or polymorph thereof, and the use thereof.

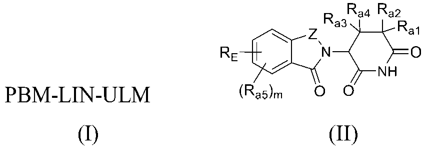

## Description

### Technical Field

The present disclosure relates to compounds of Formula (I), and novel substituted isoindolinone-based skeleton compounds of Formula (II), and uses thereof. The novel substituted isoindolinone-based skeleton compounds of Formula (II) of the present disclosure are capable of binding to Cereblon (CRBN) E3 ubiquitin ligase, and are useful for the preparation of compounds of Formula (I). The compounds of Formula (I) of the present disclosure, which are designed based on the compounds of Formula (II) and various target protein ligands, can exhibit a broad range of pharmacological activities through the degradation/inhibition of various different classes or families of pathogenic proteins.

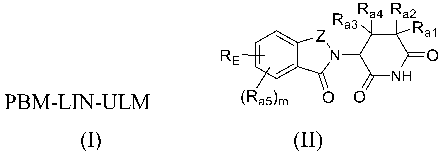

### Background

Bifunctional proteolysis-targeting drugs consist of three moieties; one end of which is a ligand warhead that can bind to a specific target protein, and the other end is a small molecule ligand of E3 ubiquitin ligase, both of which are connected together through linkers of different lengths and categories located in the middle. The bifunctional proteolysis-targeting small molecules can utilize the binding effects of the ligands at both ends to simultaneously bind to a specific target protein and E3 ubiquitin ligase, thereby recruiting E3 ubiquitin ligase to the vicinity of the specific target protein and enabling E3 ubiquitin ligase to ubiquitinate the target protein. The polyubiquitinated target protein will be recognized and degraded by proteasomes in vivo. This bifunctional protein degrader can fundamentally eliminate disease progression caused by aberrant expression of driver genes, as well as drug resistance resulting from acquired mutations in driver genes.

Over 600 distinct E3 ubiquitin ligases are involved in the design of proteolysis-targeting drugs, each exhibiting distinct cellular expression profiles and being classifiable into multiple categories based on structural elements essential for their E3 functional activity. To date, however, only a few of these ligases have been successfully employed in preclinical and clinical studies of degraders. Among them, Cereblon (CRBN) E3 ubiquitin ligase is one of the most widely used E3 ubiquitin ligases. The known ligands for CRBN-type E3 ubiquitin ligase (CRBN ligands) include thalidomide and its analogs pomalidomide and lenalidomide, all of which possess a phthalimide backbone. CRBN-based E3 ubiquitin ligase ligands themselves can also serve as molecular glues to induce the degradation of specific proteins. CRBN forms a functional E3 ubiquitin ligase complex with damaged DNA-binding protein 1 (DDB1) and Cullin 4A, in which CRBN acts as a substrate receptor. Upon binding to CRBN, molecular glue degraders such as thalidomide induce CRBN to recognize neo-substrates, leading to their ubiquitination and subsequent degradation by the proteasome. CRBN ligands can not only function as molecular glue degraders to induce the degradation of various substrate proteins, but also serve as a component of bifunctional protein degradation drugs for the effective degradation of specific pathogenic proteins. To date, the structural novelty of the discovered candidate CRBN ligands has been rather limited, and the development of more CRBN ligands with diverse skeletons has become a research hotspot in this field.

Therefore, there is an urgent need for a series of novel CRBN E3 ubiquitin ligase ligands that can serve as effective molecular glue degraders or can be further used to synthesize corresponding bifunctional protein degraders, for use in the treatment and/or prevention of a disease or disorder mediated by or associated with a target protein.

### Summary of Invention

In view of the above, an object of the present disclosure is to provide novel small molecule ligands for E3 ubiquitin ligases, bifunctional protein degraders designed based on such novel E3 ubiquitin ligase ligands and various target protein ligands, as well as their applications and usage methods. The advantage of the compounds of the present disclosure lies in their broad pharmacological activity through the degradation/inhibition of various different classes or families of pathogenic proteins.

To achieve the above and other related objects, in one aspect, the present disclosure provides compounds having a novel substituted isoindolinone scaffold, which compounds are capable of acting as molecular glues binding to the CRBN E3 ubiquitin ligase. The compounds of the present invention, which are based on the novel substituted isoindolinone scaffold, exhibit CRBN binding activity and antitumor activity, and can further be applied in the development of bifunctional small-molecule protein degraders.

In some embodiments, the compounds having a novel substituted isoindolinone scaffold of the present disclosure may be the compound of Formula (II): 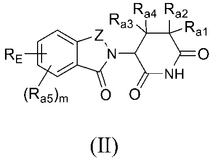 or salts, stereoisomers (including enantiomers), solvates, isotopically enriched analogs, prodrugs, or polymorphs thereof,
wherein Z represents C(O) or CH₂;
Rₐ₁, Rₐ₂, Rₐ₃, and Rₐ₄ are identical or different and each independently represents hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
m represents an integer of 0, 1, 2, or 3;
R_{E} represents: 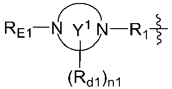
   wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
   R₁ represents N(R_{w}), where R_{w} represents hydrogen or C₁₋₃ alkyl, and R_{E1} represents hydrogen; or
   R₁ represents a bond, and R_{E1} represents NH₂; or
R_{E} represents: 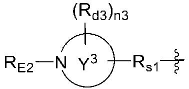
   wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20; and
   wherein Rₛ₁ represents 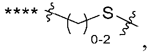 symbol **** indicates the point of attachment to ring Y³; and R_{E2} represents NH₂;
provided that the following compounds are excluded:
   3-(1-oxo-5-(piperazin-1-ylamino)isoindolin-2-yl)piperidine-2,6-dione.

In another aspect, the bifunctional protein degraders designed based on such novel E3 ubiquitin ligase ligands and various target protein ligands of the present disclosure may be the compound of Formula (I):

PBM-LIN-ULM (I)

or salts, stereoisomers (including enantiomers), solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 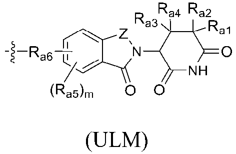
wherein Z represents C(O) or CH₂;
Rₐ₁, Rₐ₂, Rₐ₃, and Rₐ₄ are identical or different and each independently represents hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
m represents an integer of 0, 1, 2, or 3;
Rₐ₆ represents: 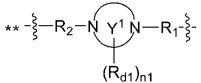
   wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
   R₁ represents N(R_{w}), where R_{w} represents hydrogen or C₁₋₃ alkyl, and R₂ represents a bond, or R₂ represents CH₂, C(O), **-NHC(O)-, or **-N(CH₃)C(O)-, or R₂ represents a structure of the following formula: 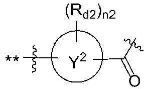 wherein ring Y² represents cycloalkylene, or heterocyclylene containing at least one nitrogen atom, and (R_{d2})ₙ₂ indicates that ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n2 represents an integer of 0-20;
      or
   R₁ represents a bond, and R₂ represents N(R_{w}), where R_{w} represents hydrogen or C₁₋₃ alkyl, or R₂ represents **C(O)N(R_{w}), where R_{w} represents hydrogen or C₁₋₃ alkyl, or R₂ represents **-CH=N-; or
Rₐ₆ represents: 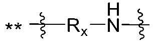 wherein Rₓ represents NH or **-CH=N-; or
Rₐ₆ represents: 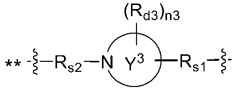
   wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20; and
   wherein Rₛ₁ represents 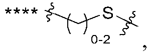 symbol **** indicates the point of attachment to ring Y³; and Rₛ₂ represents N(R_{w}) or **C(O)N(R_{w}), where R_{w} represents hydrogen or C₁₋₃ alkyl (e.g., methyl, ethyl, or propyl);
   symbol ** in each of the above formulae indicates the point of attachment to LIN; and
LIN represents a bond, or a structure of the following formula: 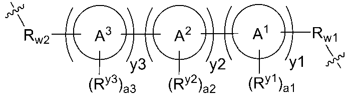
wherein R_{w2} is linked to PBM, and R_{w1} is linked to Rₐ₆;
R_{w2} represents a bond, C(O), C(O)NH, or NHC(O);
each ring A¹ is identical or different and each independently represents heterocyclylene, cycloalkylene, arylene, or heteroarylene, y1 represents an integer of 0, 1, 2, or 3, and (R^{y1})ₐ₁ indicates that each ring A¹ is independently optionally substituted with a1 R^{y1} substituents, wherein each R^{y1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a1 represents an integer of 0-10;
each ring A² is identical or different and each independently represents heterocyclylene, cycloalkylene, arylene, or heteroarylene, y2 represents an integer of 0, 1, 2, or 3, and (R^{y2})ₐ₂ indicates that each ring A² is independently optionally substituted with a2 R^{y2} substituents, wherein each R^{y2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a2 represents an integer of 0-10;
each ring A³ is identical or different and each independently represents heterocyclylene, cycloalkylene, arylene, or heteroarylene, y3 represents an integer of 0, 1, 2, or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a3 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof.

In a further aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient, for use as a medicament.

In a further aspect, the present disclosure provides the compound of Formula (I) or salts (e.g., pharmaceutically acceptable salts), stereoisomers (including enantiomers and diastereomers), solvates, isotopically enriched analogs, prodrugs, or polymorphs thereof, or the pharmaceutical composition comprising the same as active ingredient for use in the treatment and/or prevention of a disease or disorder selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure provides the use of the compound of Formula (I) or pharmaceutically acceptable salts, stereoisomers (including enantiomers and diastereomers), solvates, isotopically enriched analogs, prodrugs, or polymorphs thereof, or the pharmaceutical composition as described herein, for the manufacture of a medicament for the prevention and/or treatment of a disease or disorder selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure provides a method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof, or the pharmaceutical composition comprising the same, wherein the disease or disorder comprises: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure also provides a use of the compound of Formula (II) or salts, stereoisomers (including enantiomers), solvates, isotopically enriched analogs, prodrugs, or polymorphs thereof for the preparation of the compound of Formula (I) or salts, enantiomers, stereoisomers, solvates, isotopically enriched analogs, prodrugs, or polymorphs thereof of the present disclosure.

In a further aspect, the present disclosure also provides a method for preparing the compound of Formula (I) or salts, enantiomers, stereoisomers, solvates, isotopically enriched analogs, prodrugs, or polymorphs thereof of the present disclosure by using the compound of Formula (II) or salts, stereoisomers (including enantiomers), solvates, isotopically enriched analogs, prodrugs, or polymorphs thereof.

In a further aspect, the present disclosure also provides a use of 3-(1-oxo-5-(piperazin-1-ylamino)isoindolin-2-yl)piperidine-2,6-dione or salts, stereoisomers (including enantiomers), solvates, isotopically enriched analogs, prodrugs, or polymorphs thereof for the preparation of the compound of Formula (I) or salts, enantiomers, stereoisomers, solvates, isotopically enriched analogs, prodrugs, or polymorphs thereof of the present disclosure.

In a further aspect, the present disclosure also provides a method for preparing the compound of Formula (I) or salts, enantiomers, stereoisomers, solvates, isotopically enriched analogs, prodrugs, or polymorphs thereof of the present disclosure by using 3-(1-oxo-5-(piperazin-1-ylamino)isoindolin-2-yl)piperidine-2,6-dione or salts, stereoisomers (including enantiomers), solvates, isotopically enriched analogs, prodrugs, or polymorphs thereof.

In a further aspect, the present disclosure provides a pharmaceutical composition comprising the compound of Formula (II) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (II) or a pharmaceutically acceptable salt, or the pharmaceutical composition comprising the same.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient, for use as a medicament.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use in the prevention and/or treatment of a disease or disorder associated with cereblon protein. The disease or disorder associated with cereblon protein includes: tumors, cancers, and autoimmune diseases.

In a further aspect, the present disclosure provides the use of the compound of Formula (II) or pharmaceutically acceptable salts, stereoisomers (including enantiomers, diastereomers), solvates, isotopically enriched analogs, prodrugs, or polymorphs thereof, or the pharmaceutical composition of the present disclosure, for the manufacture of a medicament for the prevention or treatment of a disease or disorder associated with cereblon protein. In some embodiments, the disease or disorder associated with cereblon protein includes: tumors, cancers, and autoimmune diseases.

In a further aspect, the present disclosure provides a method for treating or preventing a disease or disorder associated with cereblon protein in a subject, comprising administering to the subject a therapeutically effective amount of the compound of Formula (II) or salts, enantiomers, stereoisomers, solvates, isotopically enriched analogs, prodrugs, or polymorphs thereof, or the pharmaceutical composition comprising the same. In some embodiments, the disease or disorder associated with cereblon protein includes: tumors, cancers, and autoimmune diseases.

In a further aspect, the present disclosure provides the use of the compound of Formula (II) or pharmaceutically acceptable salts, stereoisomers (including enantiomers, diastereomers), solvates, isotopically enriched analogs, prodrugs, or polymorphs thereof for the manufacture of a cereblon protein (CRBN) binder.

In a further aspect, the present disclosure provides 3-(1-oxo-5-(piperazin-1-ylamino)isoindolin-2-yl)piperidine-2,6-dione or salts, stereoisomers (including enantiomers), solvates, isotopically enriched analogs, prodrugs, or polymorphs thereof, or the pharmaceutical composition comprising the same as active ingredient for use in the prevention and/or treatment of a disease or disorder associated with cereblon protein. In some embodiments, the disease or disorder associated with cereblon protein includes: tumors, cancers, and autoimmune diseases.

In a further aspect, the present disclosure provides the use of 3-(1-oxo-5-(piperazin-1-ylamino)isoindolin-2-yl)piperidine-2,6-dione or pharmaceutically acceptable salts, stereoisomers (including enantiomers, diastereomers), solvates, isotopically enriched analogs, prodrugs, or polymorphs thereof for the manufacture of a medicament for the prevention or treatment of a disease or disorder associated with cereblon protein. In some embodiments, the disease or disorder associated with cereblon protein includes: tumors, cancers, and autoimmune diseases.

In a further aspect, the present disclosure provides a method for treating or preventing a disease or disorder associated with cereblon protein in a subject, comprising administering to the subject a therapeutically effective amount of 3-(1-oxo-5-(piperazin-1-ylamino)isoindolin-2-yl)piperidine-2,6-dione or salts, enantiomers, stereoisomers, solvates, isotopically enriched analogs, prodrugs, or polymorphs thereof, or the pharmaceutical composition comprising the same. In some embodiments, the disease or disorder associated with cereblon protein includes: tumors, cancers, and autoimmune diseases.

In a further aspect, the present disclosure provides the use of 3-(1-oxo-5-(piperazin-1-ylamino)isoindolin-2-yl)piperidine-2,6-dione or pharmaceutically acceptable salts, stereoisomers (including enantiomers, diastereomers), solvates, isotopically enriched analogs, prodrugs, or polymorphs thereof for the manufacture of a cereblon protein (CRBN) binder.

In a further aspect, the present disclosure provides a method for preparing the compound of Formula (I), comprising using a compound of Formula (M1) and a compound of Formula (M2) as starting materials to obtain the compound of Formula (I): 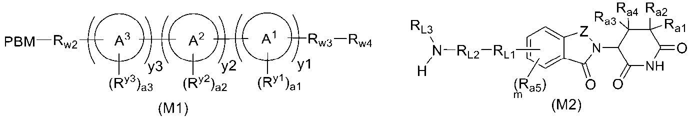 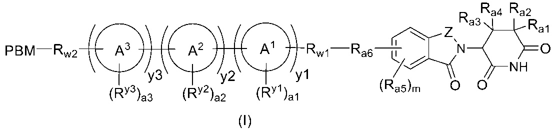
wherein PBM, Rₐ₁, Rₐ₂, Rₐ₃, Rₐ₄, (Rₐ₅)ₘ, Z, R_{w2}, ring A¹, ring A², ring A³, y1, y2, y3, (R^{y1})ₐ₁, (R^{y2})ₐ₂, and (R^{y3})ₐ₃ are as defined in the compounds of formula (I) of the present disclosure and the respective sub-embodiments thereof;
R_{w3} represents a bond or an optionally substituted linear or branched C₁₋₁₉ alkylene, wherein any one or more methylene groups in the main carbon chain skeleton of the linear or branched C₁₋₁₉ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₁₉ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and wherein one or more hydrogens of said linear or branched C₁₋₁₉ alkylene are optionally replaced by a substituent selected from the group consisting of deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
R_{w4} represents CHO, COOH, or 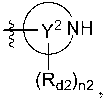 wherein ring Y² and (R_{d2})ₙ₂ are as defined in the compounds of formula (I) of the present disclosure and the respective sub-embodiments thereof;
wherein (1) when R_{w4} represents CHO or COOH,
   (a1) R_{L1} represents N(R_{w}), and R_{L2} and R_{L3}, together with the nitrogen atom to which they are attached, form 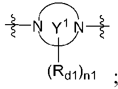 or
   (a2) R_{L1} and R_{L2} together form 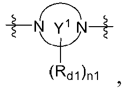 and R_{L3} represents H or C₁₋₃ alkyl; or
   (a3) R_{L1} represents NH, R_{L2} represents a bond, and R_{L3} represents H; or
   (a4) R_{L1} represents 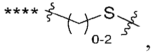 wherein the symbol **** indicates the point of attachment to R_{L2}, and R_{L2} represents 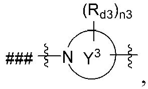 wherein the symbol ### indicates the point of attachment to N, and ring Y³ and (R_{d3})ₙ₃ are as defined in the compounds of formula (I) of the present disclosure and the respective sub-embodiments thereof, and R_{L3} represents H or C₁₋₃ alkyl; or
wherein (2) when R_{w4} represents 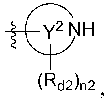 R_{L1} represents N(R_{w}), and R_{L2} and R_{L3}, together with the nitrogen atom to which they are attached, form 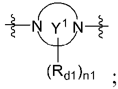 and
wherein each of the above R_{w}, ring Y¹, and (R_{d1})ₙ₁ is as defined in the compounds of formula (I) of the present disclosure and the respective sub-embodiments thereof.

In a further aspect, the present disclosure provides a method for preparing the compound of Formula (II), comprising using a compound of Formula (M3) and a compound of Formula (M4) as starting materials to obtain the compound of Formula (II): 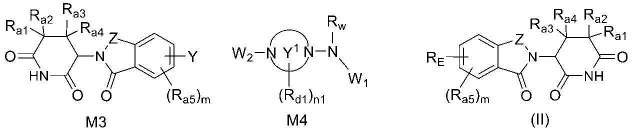
wherein Rₐ₁, Rₐ₂, Rₐ₃, Rₐ₄, (Rₐ₅)ₘ, Z, ring Y¹, R_{w}, and (R_{d1})ₙ₁ are as defined in the compounds of formula (II) of the present disclosure and the respective sub-embodiments thereof;
Y represents F or Br;
W₁ represents H, and W₂ represents an amino protecting group; or
W₁ represents an amino protecting group, and W₂ represents H; and
R_{E} represents: 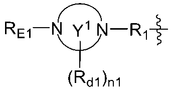
   wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
   R₁ represents N(R_{w}), where R_{w} represents hydrogen or C₁₋₃ alkyl, and R_{E1} represents hydrogen; or
   R₁ represents a bond, and R_{E1} represents NH₂.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The compound of Formula (I) of the present disclosure, or salts (including pharmaceutically acceptable salts), stereoisomers (including enantiomers), solvates, isotopically enriched analogs, prodrugs, or polymorphs thereof, can bind to the target protein, recruit the target protein to an E3 ubiquitin ligase for ubiquitination, and subsequently lead to its degradation. The compound of Formula (I) of the present disclosure can specifically target particular classes or families of target proteins, and exhibit a broad spectrum of pharmacological activities by degrading/inhibiting diverse classes or families of target proteins.

PBM-LIN-ULM (I)

wherein PBM, LIN, and ULM are as defined in the compounds of Formula (I) above.

In some embodiments, the PBM may be a small molecule ligand that binds to a specific target protein, including but not limited to, epidermal growth factor receptor (EGFR), Cyclin-dependent kinase 4/6 (CDK4/6), breakpoint cluster region (BCR)-Abelson leukemia virus (ABL) (BCR-ABL) tyrosine kinase, Androgen receptor (AR), or Interleukin-1 receptor-associated kinase 1/2/3/4 (IRAK1/2/3/4).

In some embodiments, said PBM in the compound of Formula (I) comprises a structure selected from the following formula: 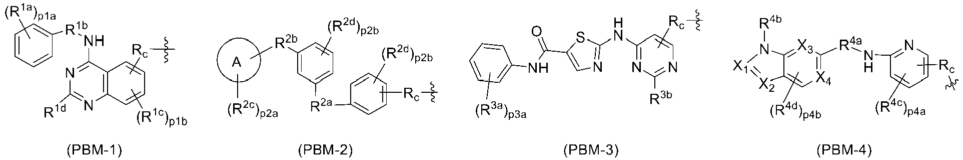 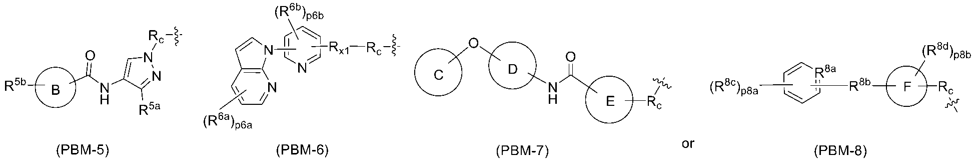
wherein (R^{1a})ₚ₁ₐ in Formula (PBM-1) indicates that the benzene ring to which it is attached is optionally substituted with p1a R^{1a} groups, wherein each R^{1a} is identical or different and each independently represents deuterium, halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
p1a represents an integer of 0, 1, 2, 3, 4, or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen;
(R^{1c})_{p1b} indicates that the quinazoline ring in Formula (PBM-1) is optionally substituted with p1b R^{1c} groups, wherein each R^{1c} is identical or different and each independently represents deuterium, halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R^{1e}, where R^{1e} represents C₁₋₁₀ alkyl, deuterated C₁₋₁₀ alkyl, or C₂₋₁₀ alkenyl, for example, R^{1c} represents methyl, methoxy, 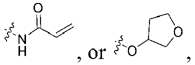 such as 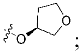
p1b represents an integer of 1, 2, or 3;
R^{1d} represents hydrogen, deuterium, or C₁₋₃ alkyl;
R^{2a} in Formula (PBM-2) represents -NHC(O)- or -C(O)NH-;
R^{2b} represents -NH-, -N(C₁₋₆ alkyl)-, or ethynylene;
ring A in Formula (PBM-2) represents a 5- to 20-membered monocyclic, bicyclic, or polycyclic heteroaryl group containing 1 to 4 heteroatoms selected from N, O, and S, and (R^{2c})ₚ₂ₐ indicates that the ring A is optionally substituted with p2a R^{2c}, wherein each R^{2c} is identical or different and each independently represents optionally substituted 5- to 15-membered heteroaryl, deuterium, halogen, hydroxy, cyano, amino, NO₂, C₁₋₆ alkyl, halogenated C₁₋₆ alkyl, deuterated C₁₋₆ alkyl, C₁₋₆ alkoxy, or halogenated C₁₋₆ alkoxy;
p2a represents an integer of 0, 1, 2, or 3;
each (R^{2d})_{p2b} in Formula (PBM-2) independently indicates that the benzene ring to which it is attached is independently optionally substituted with p2b R^{2d}, wherein each R^{2d} is identical or different and each independently represents deuterium, halogen, hydroxy, cyano, amino, NO₂, C₁₋₆ alkoxy, halogenated C₁₋₆ alkoxy, C₁₋₆ alkyl, deuterated C₁₋₆ alkyl, or halogenated C₁₋₆ alkyl;
each p2b is identical or different and each independently represents an integer of 0, 1, 2, 3, or 4;
(R^{3a})ₚ₃ₐ in Formula (PBM-3) indicates that the benzene ring to which it is attached is substituted with p3a R^{3a}, wherein each R^{3a} is identical or different and each independently represents deuterium, halogen, hydroxy, cyano, amino, NO₂, C₁₋₆ alkoxy, halogenated C₁₋₆ alkoxy, C₁₋₆ alkyl, deuterated C₁₋₆ alkyl, or halogenated C₁₋₆ alkyl;
p3a represents an integer of 1, 2, 3, 4, or 5;
R^{3b} represents halogen, C₁₋₆ alkyl, deuterated C₁₋₆ alkyl, or halogenated C₁₋₆ alkyl;
X₁ in Formula (PBM-4) represents CR^{4e} or a fragment 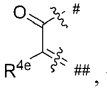 wherein symbol # indicates the point of attachment to N atom adjacent to X₁, symbol ## indicates the point of attachment to X₂, and each R^{4e} independently represents deuterium, hydrogen, halogen, C₁₋₆ alkyl, deuterated C₁₋₆ alkyl, halogenated C₁₋₆ alkyl, -C(O)-C₁₋₆ alkyl, -C(O)-deuterated C₁₋₆ alkyl, or -C(O)NR^{4f}R^{4g}, where R^{4f} and R^{4g} are identical or different and each independently represents hydrogen, C₁₋₆ alkyl, or deuterated C₁₋₆ alkyl, and R^{4f} and R^{4g} are not both hydrogen at the same time;
X₂ in Formula (PBM-4) represents N or CR^{4h}, wherein R^{4h} represents hydrogen, deuterium, halogen, C₁₋₆ alkyl, deuterated C₁₋₆ alkyl, or halogenated C₁₋₆ alkyl;
X₃ and X₄ each independently represent CH or N;
R^{4a} represents a bond, or optionally substituted heteroarylene (e.g., heteroarylene substituted with halogen or deuterium);
R^{4b} represents hydrogen, C₁₋₆ alkyl, halogenated C₁₋₆ alkyl, deuterated C₁₋₆ alkyl, or optionally substituted C₃₋₁₂ cycloalkyl;
(R^{4c})ₚ₄ₐ in Formula (PBM-4) indicates that the pyridine ring to which it is attached is optionally substituted with p4a R^{4c}, wherein each R^{4c} is identical or different and each independently represents deuterium, halogen, C₁₋₆ alkyl, deuterated C₁₋₆ alkyl, halogenated C₁₋₆ alkyl, C₁₋₆ alkoxy, or halogenated C₁₋₆ alkoxy, and p4a represents an integer of 0, 1, 2, or 3;
(R^{4d})_{p4b} in Formula (PBM-4) indicates that the nitrogen-containing bicyclic heteroaryl ring to which it is attached is optionally substituted with p4b R^{4d}, wherein each R^{4d} is identical or different and each independently represents deuterium, halogen, C₁₋₆ alkyl, deuterated C₁₋₆ alkyl, halogenated C₁₋₆ alkyl, C₁₋₆ alkoxy, or halogenated C₁₋₆ alkoxy, and p4b represents an integer of 0 or 1;
ring B in Formula (PBM-5) represents a 5- to 20-membered monocyclic, bicyclic, or polycyclic heteroarylene group containing 1 to 4 heteroatoms selected from N, O, and S;
R^{5b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{5a} represents hydrogen, halogen, C₁₋₆ alkyl, deuterated C₁₋₆ alkyl, halogenated C₁₋₆ alkyl, C₁₋₆ alkoxy, or halogenated C₁₋₆ alkoxy;
Rₓ₁ in Formula (PBM-6) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{6a})ₚ₆ₐ indicates that the 1H-pyrrolo[2,3-b]pyridine ring to which it is attached is optionally substituted with p6a R^{6a}, wherein each R^{6a} independently represents cyano, halogen, hydroxy, amino, nitro, C₁₋₆ alkyl, deuterated C₁₋₆ alkyl, halogenated C₁₋₆ alkyl, C₁₋₆ alkoxy, or halogenated C₁₋₆ alkoxy, and p6a represents an integer of 0, 1, 2, 3, 4, or 5;
(R^{6b})_{p6b} indicates that the pyridine ring to which it is attached is optionally substituted with p6b R^{6b}, wherein each R^{6b} independently represents cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy, or NR^{6c}R^{6d}, where R^{6c} and R^{6d} are identical or different and each independently represents hydrogen, C₁₋₆ alkyl, deuterated C₁₋₆ alkyl, halogenated C₁₋₆ alkyl, optionally substituted C₃₋₁₅ cycloalkyl, or optionally substituted 4- to 15-membered heterocyclyl, and p6b represents an integer of 0, 1, 2, or 3;
ring C in Formula (PBM-7) represents C₆₋₁₀ aryl or a 5- to 20-membered heteroaryl (e.g., a 5- to 20-membered heteroaryl comprising one or two 5- to 7-membered rings and 1 to 4 heteroatoms selected from N, O, and S), and said ring C is optionally substituted with one or more R^{7a}, and each R^{7a} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkenyl, or C₂₋₆ alkynyl;
ring D in Formula (PBM-7) represents C₃₋₁₅ cycloalkyl, or 4- to 20-membered heterocyclyl (e.g., a 4- to 20-membered heterocyclyl comprising 1 to 4 heteroatoms selected from N, O, and S), wherein said ring D is optionally substituted with one or more R^{7b}, and each R^{7b} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, or halogenated C₁₋₆ alkoxy;
ring E in Formula (PBM-7) represents a 5- to 20-membered heteroaryl (e.g., a 5- to 20-membered monocyclic or bicyclic heteroaryl comprising 1 to 4 heteroatoms selected from N, O, and S), wherein said ring E is optionally substituted with one or more R^{7c}, and each R^{7c} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, or halogenated C₁₋₆ alkoxy;
R^{8a} in Formula (PBM-8) represents N or CH;
R^{8b} represents NHC(O), C(O)NH, NHS(O)₂, or S(O)₂NH;
(R^{8c})ₚ₈ₐ indicates that the 6-membered ring containing R^{8a} to which it is attached is substituted with p8a R^{8c}, wherein each R^{8c} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p8a represents an integer of 0, 1, 2, 3, or 4;
ring F represents 5- to 15-membered heteroarylene, and (R^{8d})_{p8b} indicates that the ring F to which it is attached is optionally substituted with p8b R^{8d}, and each R^{8d} is identical or different and each independently represents halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy, or optionally substituted C₃₋₁₅ cycloalkyl, and p8b represents an integer of 0, 1, 2, or 3; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -Re-C(O)O-*, -R_{f}-OC(O)-*, -C(O)O-*, -OC(O)-*, -R_{f}-, -R_{f}-N(R_{d})-*, -O-R_{f}-N(R_{d})-*, 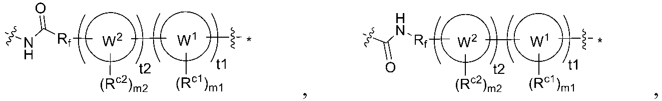 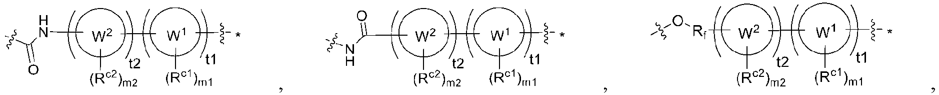 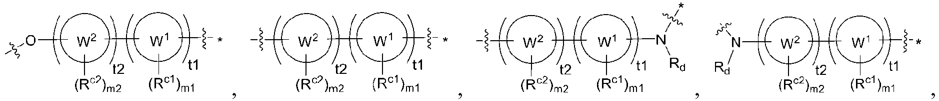 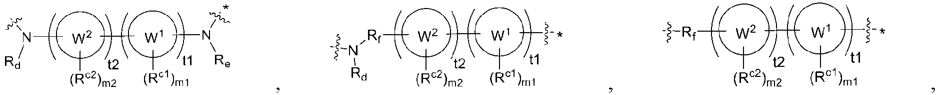 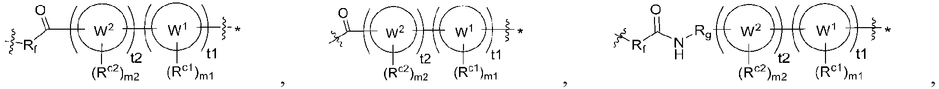 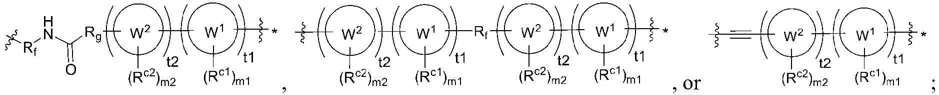
   wherein each ring W¹ is identical or different and each independently represents nitrogen-containing heterocyclylene, cycloalkylene, arylene, or heteroarylene, t1 represents an integer of 0, 1, or 2, and (R^{c1})ₘ₂ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, R^{c4} represents halogen, R^{c5}R^{c6}N-, hydroxy, carboxyl, ethynyl, or vinyl, wherein R^{c5} and R^{c6} are identical or different and each independently represents hydrogen or C₁₋₃ alkyl, and m1 represents an integer of 0-20;
   each ring W² is identical or different and each independently represents nitrogen-containing heterocyclylene, cycloalkylene, arylene, or heteroarylene, t2 represents an integer of 0 or 1, and (R^{c2})ₘ₃ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represents hydrogen or C₁₋₃ alkyl, and m2 represents an integer of 0-20; and
   R_{d} and Rₑ each independently represent hydrogen or C₁₋₆ alkyl; R_{f} and R_{g} each independently represent optionally substituted C₁₋₆ alkylene or optionally substituted C₂₋₆ alkenylene; and symbol * indicates the point of attachment to LIN.

In the embodiments of the present disclosure, the number of substituents is not theoretically limited in any way, or is automatically limited by the size of the building units (i.e., the total number of replaceable hydrogen atoms in the building units), or as clearly defined herein.

In the embodiments of the present disclosure, each R_{c} is as defined herein.

In some embodiments, PBM represents a small molecule ligand of EGFR.

In some embodiments, PBM represents a structure of the following Formula (PBM-1): 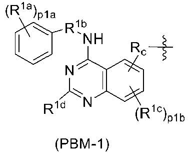
wherein (R^{1a})ₚ₁ₐ in Formula (PBM-1) indicates that the benzene ring to which it is attached is optionally substituted with p1a R^{1a} groups, wherein each R^{1a} is identical or different and each independently represents deuterium, halogen (e.g., fluorine, chlorine, bromine, or iodine), hydroxy, NH₂, NO₂, cyano, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-);
p1a represents an integer of 0, 1, 2, 3, 4, or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen (e.g., methylene or ethylene);
(R^{1c})_{p1b} indicates that the quinazoline ring in Formula (PBM-1) is optionally substituted with p1b R^{1c} groups, wherein each R^{1c} is identical or different and each independently represents deuterium, halogen, NO₂, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₁₀ alkyl (e.g., C₁₋₆ alkyl, or C₁₋₄ alkyl, such as methyl, ethyl, propyl, isopropyl, butyl, sec-butyl, tert-butyl, pentyl, or hexyl), deuterated C₁₋₁₀ alkyl (e.g., deuterated C₁₋₆ alkyl, or deuterated C₁₋₄ alkyl, such as CD₃, CD₃CD₂-, CD₃CD₂CD₂- etc.), C₁₋₁₀ alkoxy (e.g., C₁₋₆ alkoxy, or C₁₋₄ alkoxy, such as methoxy, ethoxy, propoxy, isopropoxy, n-butoxy, sec-butoxy, or tert-butoxy), halogenated C₁₋₁₀ alkoxy (e.g., halogenated C₁₋₆ alkoxy, or halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), -O-optionally substituted heterocyclyl (e.g., -O-optionally substituted 4- to 20-membered heterocyclyl), or -NHC(O)R^{1e}, where R^{1e} represents C₁₋₁₀ alkyl (e.g., C₁₋₈ alkyl, C₁₋₆ alkyl, or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.), or C₂₋₁₀ alkenyl (e.g., C₂₋₆ alkenyl, or C₂₋₄ alkenyl, such as vinyl, propenyl, butenyl, or pentenyl), such as R^{1c} represents methyl, methoxy, 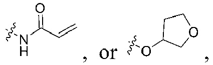 such as 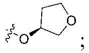
p1b represents an integer of 1, 2, or 3; and
R^{1d} represents hydrogen, deuterium, or C₁₋₃ alkyl (e.g., methyl, ethyl, or propyl).

In some embodiments, PBM represents the following non-limiting examples of the structure of Formula (PBM-1), i.e., the structures of Formula (PBM-1-1A), Formula (PBM-1-1B), Formula (PBM-1-1C), or Formula (PBM-1-1D): 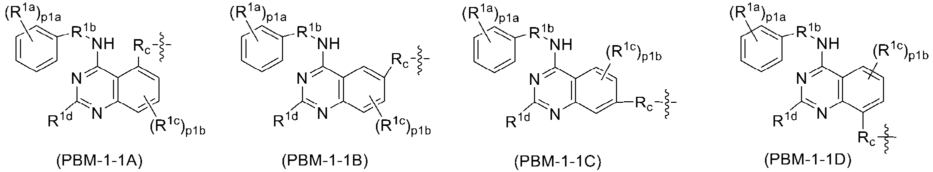 wherein (R^{1a})ₚ₁ₐ, R^{1b}, (R^{1c})_{p1b}, R^{1d}, and R_{c} are as defined in the respective embodiments of Formula (PBM-1) herein and the respective sub-embodiments thereof.

In some embodiments, (R^{1c})_{p1b} indicates that each quinazoline ring in Formula (PBM-1), Formula (PBM-1-1A), Formula (PBM-1-1B), Formula (PBM-1-1C), or Formula (PBM-1-1D) is independently substituted with p1b R^{1c}, wherein p1b represents an integer of 1, 2, or 3, and R^{1c} represents -O-optionally substituted heterocyclyl. The heterocyclyl group is a 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the heterocyclyl is optionally substituted with a substituent selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, and any combination thereof. In some embodiments, p1b represents an integer of 1.

In some embodiments, said R^{1c} represents:
fluorine, chlorine, bromine, or iodine;
methyl, ethyl, propyl, isopropyl, butyl, sec-butyl, tert-butyl, pentyl, hexyl, heptyl, or octyl;
methoxy, ethoxy, propoxy, isopropoxy, n-butoxy, sec-butoxy, or tert-butoxy;
-NHC(O)-C₂₋₁₀ alkenyl, such as -NHC(O)-vinyl (i.e., 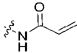 ), -NHC(O)-propenyl, or -NHC(O)-butenyl; or

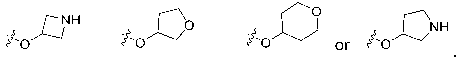

In some embodiments, R^{1b} as described herein represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen. In some embodiments, R^{1b} represents a bond, or methylene or ethylene, each of which is optionally substituted by a substituent selected from the group consisting of C₁₋₃ alkyl, deuterated C₁₋₃ alkyl, halogenated C₁₋₃ alkyl, and halogen. In some embodiments, R^{1b} represents a bond.

In some embodiments, PBM represents a structure of Formula (PBM-1-1), Formula (PBM-1-2), Formula (PBM-1-3), Formula (PBM-1-4), or Formula (PBM-1-5):

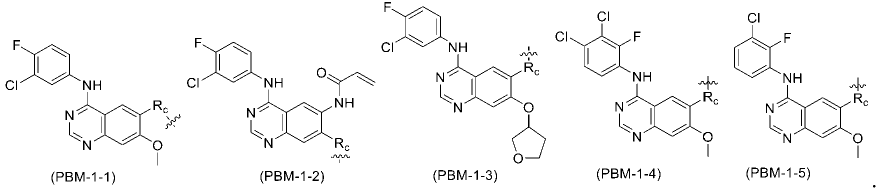

In some embodiments, PBM represents a small molecule ligand of BCR-ABL.

In some embodiments, PBM represents a structure of Formula (PBM-2): 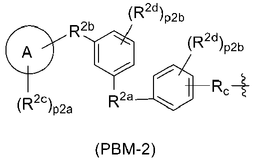
wherein R^{2a} represents -NHC(O)- or -C(O)NH-;
R^{2b} represents -NH-, -N(C₁₋₆ alkyl)-, or ethynylene;
ring A in Formula (PBM-2) represents a 5- to 20-membered monocyclic, bicyclic, or polycyclic heteroaryl group containing 1 to 4 heteroatoms selected from N, O, and S, and (R^{2c})ₚ₂ₐ indicates that the ring A is optionally substituted with p2a R^{2c}, wherein each R^{2c} is identical or different and each independently represents optionally substituted 5- to 15-membered heteroaryl, deuterium, halogen (e.g., fluorine, chlorine, bromine, or iodine), hydroxy, cyano, amino, NO₂, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-);
p2a represents an integer of 0, 1, 2, or 3;
each (R^{2d})_{p2b} in Formula (PBM-2) independently indicates that the benzene ring to which it is attached is independently optionally substituted with p2b R^{2d}, wherein each R^{2d} is identical or different and each independently represents deuterium, halogen (e.g., fluorine, chlorine, bromine, or iodine), hydroxy, cyano, amino, NO₂, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-); and
each p2b is identical or different and each independently represents an integer of 0, 1, 2, 3, or 4 .

In some embodiments, ring A in Formula (PBM-2) represents a 5- to 20-membered monocyclic, bicyclic, or polycyclic heteroaryl group containing 1 to 4 heteroatoms selected from N, O, and S. In a sub-embodiment, examples of 5- to 20-membered monocyclic, bicyclic, or polycyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with p2a R^{2c}, wherein p2a represents an integer of 0, 1, 2, or 3, and each R^{2c} is identical or different and each independently represents: optionally substituted 5- to 15-membered heteroaryl, deuterium, halogen (e.g., fluorine, chlorine, bromine, or iodine), hydroxy, cyano, amino, NO₂, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, R^{2c} in Formula (PBM-2) represents optionally substituted 5- to 15-membered heteroaryl. In some embodiments, the 5- to 15-membered heteroaryl is 5- to 15-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with one or more (e.g., 1-6, such as 1, 2, 3, 4, 5, or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, R^{2b} in Formula (PBM-2) represents -NH-.

In some embodiments, PBM represents the following non-limiting examples of the structure of Formula (PBM-2), i.e., the structure of Formula (PBM-2-1A): 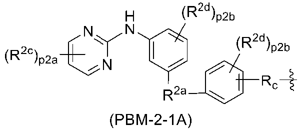
wherein R^{2a} represents -NHC(O)- or -C(O)NH-;
(R^{2c})ₚ₂ₐ indicates that the pyrimidinyl group to which it is attached is optionally substituted with p2a R^{2c} substituents, wherein each R^{2c} is identical or different and each independently represents optionally substituted 5- to 15-membered heteroaryl, deuterium, halogen (e.g., fluorine, chlorine, bromine, or iodine), hydroxy, cyano, amino, NO₂, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-);
p2a represents an integer of 0, 1, 2, or 3;
each (R^{2d})_{p2b} in Formula (PBM-2-1A) independently indicates that the benzene ring to which it is attached is independently optionally substituted with p2b R^{2d}, wherein each R^{2d} is identical or different and each independently represents deuterium, halogen (e.g., fluorine, chlorine, bromine, or iodine), hydroxy, cyano, amino, NO₂, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-); and
each p2b is identical or different and each independently represents an integer of 0, 1, 2, 3, or 4.

In some embodiments, R^{2c} in Formula (PBM-2-1A) represents optionally substituted 5- to 15-membered heteroaryl. In some embodiments, the 5- to 15-membered heteroaryl is 5- to 15-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with one or more (e.g., 1-6, such as 1, 2, 3, 4, 5, or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the structure of Formula (PBM-2-1):

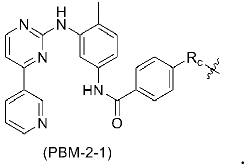

In some embodiments, PBM represents the structure of Formula (PBM-3): 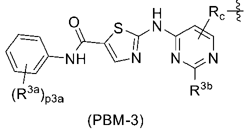
wherein (R^{3a})ₚ₃ₐ in Formula (PBM-3) indicates that the benzene ring to which it is attached is substituted with p3a R^{3a} , where each R^{3a} is identical or different and each independently represents deuterium, halogen, hydroxy, cyano, amino, NO₂, C₁₋₆ alkoxy, halogenated C₁₋₆ alkoxy, C₁₋₆ alkyl, deuterated C₁₋₆ alkyl, or halogenated C₁₋₆ alkyl;
p3a represents an integer of 1, 2, 3, 4, or 5;
R^{3b} represents halogen, C₁₋₆ alkyl, deuterated C₁₋₆ alkyl, or halogenated C₁₋₆ alkyl.

In some embodiments, each R^{3a} independently represents deuterium, halogen (e.g., fluorine, chlorine, bromine, or iodine), hydroxy, cyano, amino, NO₂, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, R^{3b} represents halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, PBM represents the structure of Formula (PBM-3-1):

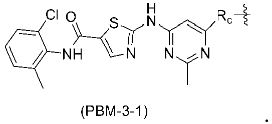

In some embodiments, PBM represents a small molecule ligand of CDK4/6.

In some embodiments, PBM represents the structure of Formula (PBM-4): 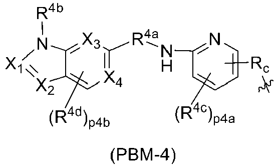
wherein X₁ in Formula (PBM-4) represents CR^{4e} or a fragment 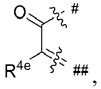 wherein symbol # indicates the point of attachment to N atom adjacent to X₁, symbol ## indicates the point of attachment to X₂, and each R^{4e} independently represents deuterium, hydrogen, halogen, C₁₋₆ alkyl, deuterated C₁₋₆ alkyl, halogenated C₁₋₆ alkyl, -C(O)-C₁₋₆ alkyl, -C(O)-deuterated C₁₋₆ alkyl, or -C(O)NR^{4f}R^{4g}, where R^{4f} and R^{4g} are identical or different and each independently represents hydrogen, C₁₋₆ alkyl, or deuterated C₁₋₆ alkyl, and R^{4f} and R^{4g} are not both hydrogen at the same time;
X₂ in Formula (PBM-4) represents N or CR^{4h}, wherein R^{4h} represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-);
X₃ and X₄ each independently represent CH or N;
R^{4a} represents a bond, or optionally substituted heteroarylene (e.g., optionally substituted 5- to 20-membered monocyclic, bicyclic, or polycyclic aromatic ring group containing one or more (e.g., from 1 to 6, or from 1 to 5, or from 1 to 4, or from 1 to 3) heteroatoms independently selected from the group consisting of oxygen, nitrogen, and sulfur);
R^{4b} represents hydrogen, C₁₋₆ alkyl, halogenated C₁₋₆ alkyl, deuterated C₁₋₆ alkyl, or optionally substituted C₃₋₁₂ cycloalkyl;
(R^{4c})ₚ₄ₐ in Formula (PBM-4) indicates that the pyridine ring to which it is attached is optionally substituted with p4a R^{4c}, wherein each R^{4c} is identical or different and each independently represents deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and p4a represents an integer of 0, 1, 2, or 3; and
(R^{4d})_{p4b} in Formula (PBM-4) indicates that the nitrogen-containing bicyclic heteroaryl ring to which it is attached is optionally substituted with p4b R^{4d}, wherein each R^{4d} is identical or different and each independently represents deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and p4b represents an integer of 0 or 1.

In some embodiments, X₁ represents CR^{4e}, wherein R^{4e} represents deuterium, hydrogen, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), -C(O)-C₁₋₆ alkyl (e.g., -C(O)-C₁₋₅ alkyl, or -C(O)-C₁₋₃ alkyl, such as -C(O)-CH₃, -C(O)-CH₂CH₃), -C(O)-deuterated C₁₋₆ alkyl (e.g., -C(O)-deuterated C₁₋₅ alkyl, or -C(O)-deuterated C₁₋₃ alkyl, such as -C(O)-CD₃), or -C(O)NR^{4f}R^{4g}, where R^{4f} and R^{4g} are identical or different and each independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{4f} and R^{4g} are not both hydrogen at the same time.

In some embodiments, X₁ represents a fragment 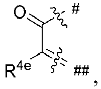 wherein symbol # indicates the point of attachment to N atom adjacent to X₁, symbol ## indicates the point of attachment to X₂, and R^{4e} represents deuterium, hydrogen, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), -C(O)-C₁₋₆ alkyl, -C(O)-deuterated C₁₋₆ alkyl, or -C(O)NR^{4f}R^{4g}, where R^{4f} and R^{4g} are identical or different and each independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, R^{4a} represents a bond, or optionally substituted heteroarylene (e.g., optionally substituted 5- to 20-membered monocyclic, bicyclic, or polycyclic aromatic ring group containing one or more (e.g., from 1 to 6, or from 1 to 5, or from 1 to 4, or from 1 to 3) heteroatoms independently selected from the group consisting of oxygen, nitrogen, and sulfur), wherein the heteroarylene is optionally substituted with one or more (e.g., 1-4, such as 1, 2, 3, or 4) substituents each independently selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, R^{4a} represents optionally substituted heteroarylene, such as optionally substituted 5- to 14-membered monocyclic, bicyclic, or polycyclic heteroarylene group containing one or more (e.g., from 1 to 6, or from 1 to 5, or from 1 to 4, or from 1 to 3) heteroatoms independently selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. The heteroarylene is optionally substituted with one or more (e.g., 1-4, such as 1, 2, 3, or 4) substituents selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, R^{4b} represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or optionally substituted C₃₋₁₂ cycloalkyl (e.g., cyclopropyl, cyclobutyl, cyclopentyl, cyclopentenyl, cyclohexyl, cyclohexenyl, or cycloheptyl, which are optionally substituted with a substituent selected from the group consisting of e.g., halogen, C₁₋₆ alkyl, halogenated C₁₋₆ alkyl, and deuterated C₁₋₆ alkyl).

In some embodiments, PBM represents the following non-limiting examples of the structure of Formula (PBM-4), i.e., the structures of Formula (PBM-4-1A), Formula (PBM-4-1B), Formula (PBM-4-1C), or Formula (PBM-4-1D): 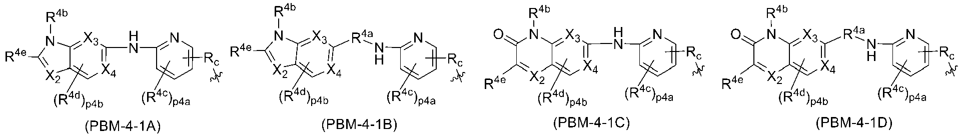
wherein each R^{4e} independently represents deuterium, hydrogen, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), -C(O)-C₁₋₆ alkyl (e.g., -C(O)-C₁₋₅ alkyl, or -C(O)-C₁₋₃ alkyl, such as -C(O)-CH₃, -C(O)-CH₂CH₃), -C(O)-deuterated C₁₋₆ alkyl (e.g., -C(O)-deuterated C₁₋₅ alkyl, or -C(O)-deuterated C₁₋₃ alkyl, such as -C(O)-CD₃), or -C(O)NR^{4f}R^{4g}, where R^{4f} and R^{4g} are identical or different and each independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{4f} and R^{4g} are not both hydrogen at the same time;
each X₂ independently represents N or CR^{4h}, wherein R^{4h} represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-);
X₃ and X₄ each independently represent CH or N;
R^{4a} in Formula (PBM-4-1B) and Formula (PBM-4-1D) independently represents optionally substituted heteroarylene (e.g., optionally substituted 5- to 20-membered monocyclic, bicyclic, or polycyclic aromatic ring group containing one or more (e.g., from 1 to 6, or from 1 to 5, or from 1 to 4, or from 1 to 3) heteroatoms independently selected from the group consisting of oxygen, nitrogen, and sulfur), wherein the heteroarylene is optionally substituted with one or more (e.g., 1-4, such as 1, 2, 3, or 4) substituents independently selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and any combination thereof;
each R^{4b} independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or optionally substituted C₃₋₁₂ cycloalkyl (e.g., cyclopropyl, cyclobutyl, cyclopentyl, cyclopentenyl, cyclohexyl, cyclohexenyl, or cycloheptyl, which are optionally substituted with a substituent selected from the group consisting of e.g., halogen, C₁₋₆ alkyl, halogenated C₁₋₆ alkyl, and deuterated C₁₋₆ alkyl);
each (R^{4c})ₚ₄ₐ independently indicates that the pyridine ring to which it is attached is optionally substituted with p4a R^{4c}, wherein each R^{4c} is identical or different and each independently represents deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), and p4a represents an integer of 0, 1, 2, or 3; and
each (R^{4d})_{p4b} independently indicates that the nitrogen-containing bicyclic heteroaryl ring to which it is attached is optionally substituted with p4b R^{4d} , wherein each R^{4d} is identical or different and each independently represents deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), and p4b represents an integer of 0 or 1.

In some embodiments, R^{4a} in Formula (PBM-4-1B) and Formula (PBM-4-1D) each independently represents optionally substituted heteroarylene, such as optionally substituted 5- to 14-membered monocyclic, bicyclic, or polycyclic heteroarylene group containing one or more (e.g., from 1 to 6, or from 1 to 5, or from 1 to 4, or from 1 to 3) heteroatoms independently selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. The heteroarylene is optionally substituted with one or more (e.g., 1-4, such as 1, 2, 3, or 4) substituents selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the following structures of Formula (PBM-4-1) or Formula (PBM-4-2):

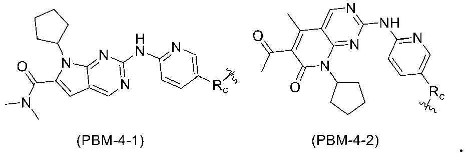

In some embodiments, PBM represents a small molecule ligand of Interleukin-1 receptor-associated kinase (IRAK1, IRAK2, IRAK-3, and IRAK4).

In some embodiments, PBM represents the structure of Formula (PBM-5): 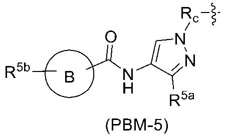
wherein ring B in Formula (PBM-5) represents a 5- to 20-membered monocyclic, bicyclic, or polycyclic heteroarylene group containing 1 to 4 heteroatoms selected from N, O, and S;
R^{5b} represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-); and
R^{5a} represents hydrogen, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, ring B in Formula (PBM-5) represents a 5- to 20-membered monocyclic, bicyclic, or polycyclic heteroarylene group containing 1 to 4 heteroatoms selected from N, O, and S. In some embodiments, the 5- to 20-membered monocyclic, bicyclic, or polycyclic heteroarylene is 5- to 20-membered divalent monocyclic, bicyclic, or polycyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of the 5- to 20-membered monocyclic, bicyclic, or polycyclic heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, and imidazo[1,2-b]pyridazinylene. 5- to 20-membered monocyclic, bicyclic, or polycyclic heteroarylene is optionally substituted with one or more (e.g., 1-3, such as 1, 2, or 3) substituents, wherein each substituent independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, R^{5b} in Formula (PBM-5) represents optionally substituted 5- to 15-membered heteroaryl. In some embodiments, the 5- to 15-membered heteroaryl is 5- to 15-membered monocyclic, bicyclic, or polycyclic aromatic ring group containing 1-4 (e.g., 1-4, 1-3, 1-2, or 1) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl represented by R^{5b} is optionally substituted with one or more (e.g., 1-3, such as 1, 2, or 3) substituents optionally selected from the group consisting of: -N(R^{5c})-C₁₋₆ alkylene-optionally substituted C₃₋₈ cycloalkyl, -N(R^{5c})-optionally halogenated C₁₋₆ alkyl, halogen (e.g., fluorine, chlorine, bromine, or iodine), hydroxy, cyano, amino, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), wherein each R^{5c} independently represents hydrogen or C₁₋₃ alkyl. In some embodiments, the substituent on the heteroaryl group represented by R^{5b} is -N(R^{5c})-C₁₋₆ alkylene-optionally substituted C₃₋₈ cycloalkyl, wherein R^{5c} represents hydrogen or C₁₋₃ alkyl (e.g., methyl). In some embodiments, examples of C₁₋₆ alkylene include, but are not limited to, C₁₋₃ alkylene. In some embodiments, examples of C₃₋₈ cycloalkyl include, but are not limited to, C₃₋₆ cycloalkyl. In some embodiments, representative examples of C₃₋₈ cycloalkyl include, but are not limited to, cyclopropyl, cyclobutyl, cyclopentyl, cyclopentenyl, cyclohexyl, cyclohexenyl, cycloheptyl, and cyclooctyl. C₃₋₈ cycloalkyl is optionally substituted with one or more (e.g., 1-3, such as 1, 2, or 3) substituents independently selected from the group consisting of: C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, and cyano. In some embodiments, the substituent on the heteroaryl group represented by R^{5b} is -N(R^{5c})-optionally halogenated C₁₋₆ alkyl, wherein R^{5c} represents hydrogen or C₁₋₃ alkyl (e.g., methyl). In some embodiments, examples of optionally halogenated C₁₋₆ alkyl include, but are not limited to, optionally halogenated C₁₋₄ alkyl, optionally halogenated C₁₋₃ alkyl, and optionally halogenated C₁₋₂ alkyl. In some embodiments, representative examples of optionally halogenated C₁₋₆ alkyl include, but are not limited to, -CH₂-CF₃.

In some embodiments, R^{5b} in Formula (PBM-5) represents optionally substituted 5- to 15-membered heterocyclyl. In some sub-embodiments, "5- to 15-membered heterocyclyl" refers to a 5- to 15-membered saturated or partially unsaturated (i.e., containing one or more double bonds, but not having a fully conjugated π-electron system) monocyclic, bicyclic, tricyclic, or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl is optionally substituted with one or more (e.g., 1-5, 1-4, 1-3, 1-2, or 1) substituents independently selected from the group consisting of deuterium, hydroxyl, amino, mercapto, nitro, halogen (e.g., fluorine, chlorine, bromine, or iodine), cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₃₋₆ cycloalkyl, C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the following structure of Formula (PBM-5-1), Formula (PBM-5-2), Formula (PBM-5-3), or Formula (PBM-5-4):

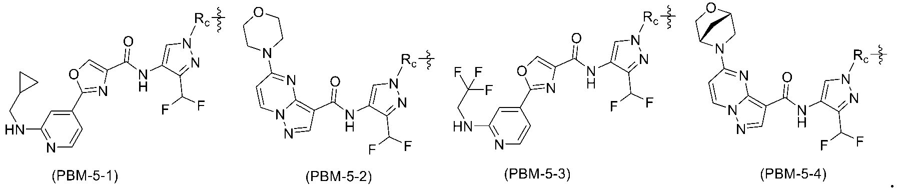

In some embodiments, PBM represents a structure of Formula (PBM-6): 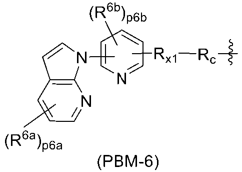
wherein Rₓ₁ in Formula (PBM-6) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{6a})ₚ₆ₐ indicates that the 1H-pyrrolo[2,3-b]pyridine ring to which it is attached is optionally substituted with p6a R^{6a} , wherein each R^{6a} independently represents cyano, halogen (e.g., fluorine, chlorine, bromine, or iodine), hydroxy, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and p6a represents an integer of 0, 1, 2, 3, 4, or 5;
(R^{6b})_{p6b} indicates that the pyridine ring to which it is attached is optionally substituted with p6b R^{6b} , wherein each R^{6b} independently represents cyano, halogen (e.g., fluorine, chlorine, bromine, or iodine), hydroxy, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or NR^{6c}R^{6d}, where R^{6c} and R^{6d} are identical or different and each independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), optionally substituted C₃₋₁₅ cycloalkyl, or optionally substituted 4- to 15-membered heterocyclyl, and p6b represents an integer of 0, 1, 2, or 3.

In some embodiments, Rₓ₁ in Formula (PBM-6) represents a bond.

In some embodiments, Rₓ₁ in Formula (PBM-6) represents C(O).

In some embodiments, Rₓ₁ in Formula (PBM-6) represents -C(O)NH-Rₓ₂-C(O)-***, wherein symbol *** indicates the point of attachment to R_{c}, and Rₓ₂ represents optionally substituted C₃₋₁₅ cycloalkyl. In some sub-embodiments, examples of optionally substituted C₃₋₁₅ cycloalkyl include, but are not limited to, optionally substituted C₃₋₁₁ cycloalkyl and optionally substituted C₃₋₈ cycloalkyl, such as optionally substituted cyclopropyl, optionally substituted cyclobutyl, optionally substituted cyclopentyl, optionally substituted cyclohexyl, optionally substituted cycloheptyl, or optionally substituted cyclooctyl. In some embodiments, C₃₋₁₅ cycloalkyl is optionally substituted with one or more (e.g., 1-3, 1, 2, or 3) substituents independently selected from the group consisting of: deuterium, hydroxyl, amino, mercapto, nitro, halogen (e.g., fluorine, chlorine, bromine, or iodine), cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, (R^{6a})ₚ₆ₐ indicates that the 1H-pyrrolo[2,3-b]pyridine ring to which it is attached is optionally substituted with p6a R^{6a}, wherein each R^{6a} independently represents cyano, halogen (e.g., fluorine, chlorine, bromine, or iodine), hydroxy, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CHO-, CF₃CF₂-O-, CF₃CHF-O-, CHF₂CF₂-O-, CHF₂CHF-O-, CF₃CH₂-O-, or CH₂ClCH₂-O-), and p6a represents an integer of 0, 1, 2, 3, 4, or 5.

In some embodiments, (R^{6a})ₚ₆ₐ indicates that the 1H-pyrrolo[2,3-b]pyridine ring to which it is attached in Formula (PBM-6) is optionally substituted with p6a R^{6a}, wherein p6a represents an integer of 1, and R^{6a} is cyano.

In some embodiments, (R^{6b})_{p6b} indicates that the pyridine ring to which it is attached is optionally substituted with p6b R^{6b}, wherein each R^{6b} independently represents cyano, halogen (e.g., fluorine, chlorine, bromine, or iodine), hydroxy, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or NR^{6c}R^{6d}, where R^{6c} and R^{6d} are identical or different and each independently represents hydrogen, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), optionally substituted C₃₋₁₅ cycloalkyl (e.g., optionally substituted C₃₋₁₂ cycloalkyl, and optionally substituted C₃₋₈ cycloalkyl), or optionally substituted 4- to 15-membered heterocyclyl (e.g., optionally substituted 4- to 12-membered heterocyclyl, or optionally substituted 4- to 10-membered heterocyclyl), and p6b represents an integer of 0, 1, 2, or 3. In some sub-embodiments, examples of optionally substituted C₃₋₁₅ cycloalkyl include, but are not limited to, optionally substituted cyclopropyl, optionally substituted cyclobutyl, optionally substituted cyclopentyl, optionally substituted cyclohexyl, optionally substituted cycloheptyl, or optionally substituted cyclooctyl. In some sub-embodiments, examples of optionally substituted 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl, azacyclooctyl, or diazacyclooctyl. In some sub-embodiments, the substituents of optionally substituted C₃₋₁₅ cycloalkyl and optionally substituted 4- to 15-membered heterocyclyl are each independently optionally selected from the group consisting of: deuterium, hydroxyl, amino, mercapto, nitro, halogen (e.g., fluorine, chlorine, bromine, or iodine), cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, (R^{6b})_{p6b} in Formula (PBM-6) indicates that the pyridine ring to which it is attached is optionally substituted with p6b R^{6b}, wherein each p6b represents an integer of 1, and R^{6b} represents NR^{6c}R^{6d}, where R^{6c} represents hydrogen, and R^{6d} represents C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl).

In some embodiments, PBM represents the following structure of Formula (PBM-6-1), Formula (PBM-6-2), Formula (PBM-6-3), or Formula (PBM-6-4):

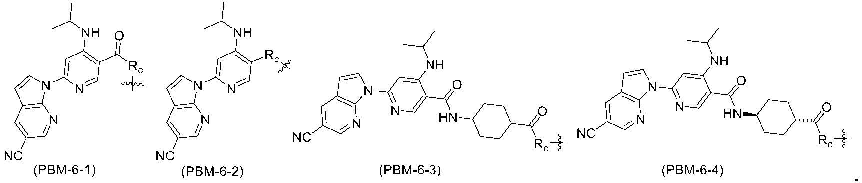

In some embodiments, PBM represents the structure of Formula (PBM-8): 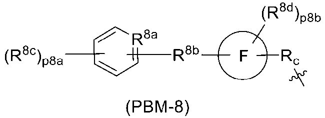
wherein R^{8a} in Formula (PBM-8) represents N or CH;
R^{8b} represents NHC(O), C(O)NH, NHS(O)₂, or S(O)₂NH;
(R^{8c})ₚ₈ₐ indicates that the 6-membered ring containing R^{8a} to which it is attached is substituted with p8a R^{8c}, wherein each R^{8c} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and p8a represents an integer of 0, 1, 2, 3, or 4; and
ring F represents 5- to 15-membered heteroarylene, and (R^{8d})_{p8b} indicates that the ring F to which it is attached is optionally substituted with p8b R^{8d}, and each R^{8d} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), hydroxy-substituted C₁₋₆ alkyl, amino-substituted C₁₋₆ alkyl, C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or optionally substituted C₃₋₁₅ cycloalkyl, and p8b represents an integer of 0, 1, 2, or 3.

In some embodiments, R^{8a} represents N. In some embodiments, R^{8a} represents CH.

In some embodiments, (R^{8c})ₚ₈ₐ indicates that the 6-membered ring containing R^{8a} to which it is attached is substituted with p8a R^{8c}, wherein each R^{8c} is identical or different and each independently represents halogen (e.g., fluorine, chlorine, bromine, and iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), p8a represents an integer of 0, 1, 2, 3, or 4, and the aromatic ring containing R^{8a} is pyridine ring or benzene ring. In some embodiments, the aromatic ring containing R^{8a} is pyridine ring, R^{8c} represents F₃C-, and p8a represents an integer of 1.

In some embodiments, ring F represents 5- to 15-membered heteroarylene, such as 5- to 12-membered heteroarylene, or 5- to 10-membered heteroarylene. In some embodiments, examples of ring F include, but are not limited to, benzimidazolylene, benzothiazolylene, 2H-indazolylene, benzoxazolylene, isoindolylene, benzofuranylene, isobenzofuranylene, benzothienylene, benzisoxazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, imidazo[1,2-b]pyridazinylene, or 6,7-dihydro-5H-pyrrolo[1,2-c]imidazolylene. Ring F is optionally substituted with p8b R^{8d} , wherein each R^{8d} is identical or different and each independently represents halogen (e.g., fluorine, chlorine, bromine, and iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), hydroxy-substituted C₁₋₆ alkyl (e.g., hydroxy-substituted C₁₋₄ alkyl, such as hydroxypropyl, and hydroxyisopropyl), amino-substituted C₁₋₆ alkyl (e.g., amino-substituted C₁₋₄ alkyl, such as aminopropyl, aminoisopropyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or optionally substituted C₃₋₁₅ cycloalkyl, and p8b represents an integer of 0, 1, 2, or 3. In some embodiments, R^{8d} represents hydroxypropyl or hydroxyisopropyl, and p8b represents an integer of 1. In some embodiments, R^{8d} represents optionally substituted C₃₋₁₅ cycloalkyl. Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally substituted with a substituent selected from the group consisting of halogen (e.g., fluorine, chlorine, bromine, or iodine), hydroxy, mercapto, cyano, amino, nitro, C₁₋₆ alkyl (e.g., C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the following structure of Formula (PBM-8-1), Formula (PBM-8-2), Formula (PBM-8-3), Formula (PBM-8-4), Formula (PBM-8-5), Formula (PBM-8-6), Formula (PBM-8-7), Formula (PBM-8-8), Formula (PBM-8-9), Formula (PBM-8-10), Formula (PBM-8-11), Formula (PBM-8-12), or Formula (PBM-8-13):

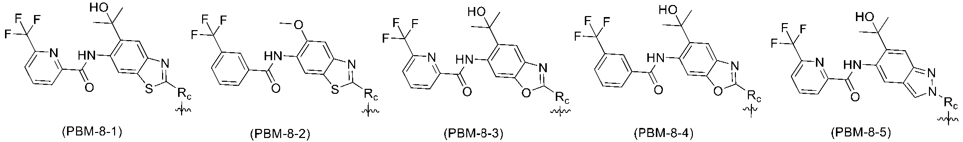

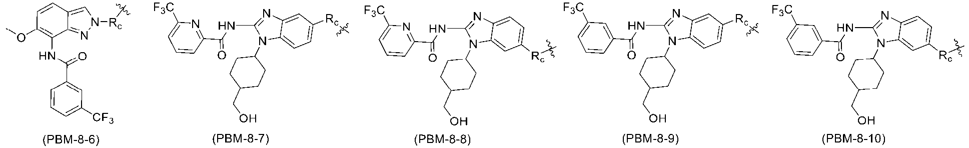

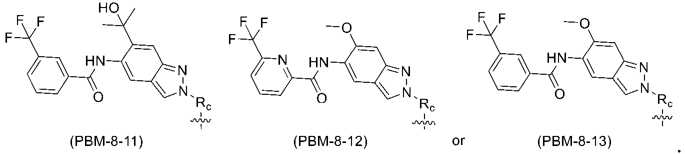

In some embodiments, PBM represents a small molecule ligand of AR.

In some embodiments, PBM represents the structure of Formula (PBM-7): 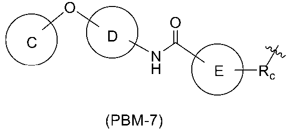
wherein ring C represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring C is optionally substituted with one or more (e.g., 1-10, 1-8, or 1-6, such as 1, 2, 3, 4, 5, 6, 7, 8, 9, or 10) R^{7a}, wherein each R^{7a} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₂₋₆ alkenyl (e.g., vinyl, propenyl, or butenyl), or C₂₋₆ alkynyl (e.g., ethynyl, propynyl, or butynyl);
ring D represents C₃₋₁₅ cycloalkyl or 4- to 20-membered heterocyclyl (e.g., 4- to 20-membered heterocyclyl containing 1 to 4 heteroatoms selected from N, O and S), wherein the ring D is optionally substituted with one or more (e.g., 1-10, 1-8, or 1-6, such as 1, 2, 3, 4, 5, 6, 7, 8, 9, or 10) R^{7b}, wherein each R^{7b} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-); and
ring E represents 5- to 20-membered heteroaryl (e.g., 5- to 20-membered monocyclic or bicyclic heteroaryl containing 1 to 4 heteroatoms selected from N, O and S), wherein the ring E is optionally substituted with one or more (e.g., 1-10, 1-8, or 1-6, such as 1, 2, 3, 4, 5, 6, 7, 8, 9, or 10) R^{7c}, wherein each R^{7c} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the structure of Formula (PBM-7-1A): 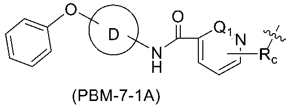
wherein the benzene ring in Formula (PBM-7-1A) is optionally substituted with one or more (e.g., 1-10, 1-8, or 1-6, such as 1, 2, 3, 4, 5, 6, 7, 8, 9, or 10) R^{7a}, and each R^{7a} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₂₋₆ alkenyl (e.g., vinyl, propenyl, or butenyl), or C₂₋₆ alkynyl (e.g., ethynyl, propynyl or butynyl);
ring D represents C₃₋₁₅ cycloalkyl or 4- to 20-membered heterocyclyl (e.g., 4- to 20-membered heterocyclyl containing 1 to 4 heteroatoms selected from N, O and S), wherein the ring D is optionally substituted with one or more (e.g., 1-10, 1-8, or 1-6, such as 1, 2, 3, 4, 5, 6, 7, 8, 9, or 10) R^{7b} , and each R^{7b} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-);
Q₁ represents N or CH; and
the heteroaryl group containing Q₁ and a nitrogen atom in Formula (PBM-7-1A) is optionally substituted with one or more (e.g., 1-3, such as 1, 2, or 3) R^{7c} , and each R^{7c} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, ring D in Formula (PBM-7) and Formula (PBM-7-1A) each independently represents C₃₋₁₅ cycloalkyl or 4- to 20-membered heterocyclyl (e.g., 4- to 20-membered heterocyclyl containing 1 to 4 heteroatoms selected from N, O, and S). Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of deuterium, halogen (e.g., fluorine, chlorine, bromine, or iodine), hydroxy, cyano, amino, nitro, C₁₋₆ alkyl (e.g., C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-). Examples of 4- to 20-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). The optional substituents of 4- to 20-membered heterocyclyl are optionally selected from the group consisting of deuterium, halogen (e.g., fluorine, chlorine, bromine, or iodine), hydroxy, cyano, amino, nitro, C₁₋₆ alkyl (e.g., C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In some embodiments, PBM represents the following structure of Formula (PBM-7-1), Formula (PBM-7-2), Formula (PBM-7-3), Formula (PBM-7-4), Formula (PBM-7-5), Formula (PBM-7-6), Formula (PBM-7-7), or Formula (PBM-7-8):

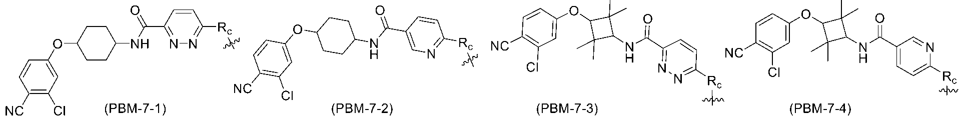

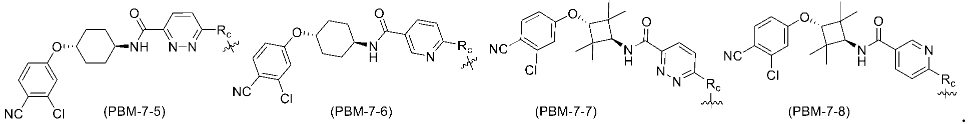

In some embodiments, in Formula (PBM-1), Formula (PBM-1-1A), Formula (PBM-1-1B), Formula (PBM-1-1C), Formula (PBM-1-1D), Formula (PBM-1-1), Formula (PBM-1-2), Formula (PBM-1-3), Formula (PBM-1-4), Formula (PBM-1-5), Formula (PBM-2), Formula (PBM-2-1A), Formula (PBM-2-1), Formula (PBM-3), Formula (PBM-3-1), Formula (PBM-4), Formula (PBM-4-1A), Formula (PBM-4-1B), Formula (PBM-4-1C), Formula (PBM-4-1D), Formula (PBM-4-1), Formula (PBM-4-2), Formula (PBM-5), Formula (PBM-5-1), Formula (PBM-5-2), Formula (PBM-5-3), Formula (PBM-5-4), Formula (PBM-6), Formula (PBM-6-1), Formula (PBM-6-2), Formula (PBM-6-3), Formula (PBM-6-4), Formula (PBM-7), Formula (PBM-7-1A), Formula (PBM-7-1), Formula (PBM-7-2), Formula (PBM-7-3), Formula (PBM-7-4), Formula (PBM-7-5), Formula (PBM-7-6), Formula (PBM-7-7), Formula (PBM-7-8), Formula (PBM-8), Formula (PBM-8-1), Formula (PBM-8-2), Formula (PBM-8-3), Formula (PBM-8-4), Formula (PBM-8-5), Formula (PBM-8-6), Formula (PBM-8-7), Formula (PBM-8-8), Formula (PBM-8-9), Formula (PBM-8-10), Formula (PBM-8-11), Formula (PBM-8-12), and Formula (PBM-8-13) of PBM, each R_{c} independently represents a bond, -O-, -NHC(O)-*, -C(O)NH-*, -C(O)O-*, -OC(O)-*, or -NH-.

In some embodiments, in Formula (PBM-1), Formula (PBM-1-1A), Formula (PBM-1-1B), Formula (PBM-1-1C), Formula (PBM-1-1D), Formula (PBM-1-1), Formula (PBM-1-2), Formula (PBM-1-3), Formula (PBM-1-4), Formula (PBM-1-5), Formula (PBM-2), Formula (PBM-2-1A), Formula (PBM-2-1), Formula (PBM-3), Formula (PBM-3-1), Formula (PBM-4), Formula (PBM-4-1A), Formula (PBM-4-1B), Formula (PBM-4-1C), Formula (PBM-4-1D), Formula (PBM-4-1), Formula (PBM-4-2), Formula (PBM-5), Formula (PBM-5-1), Formula (PBM-5-2), Formula (PBM-5-3), Formula (PBM-5-4), Formula (PBM-6), Formula (PBM-6-1), Formula (PBM-6-2), Formula (PBM-6-3), Formula (PBM-6-4), Formula (PBM-7), Formula (PBM-7-1A), Formula (PBM-7-1), Formula (PBM-7-2), Formula (PBM-7-3), Formula (PBM-7-4), Formula (PBM-7-5), Formula (PBM-7-6), Formula (PBM-7-7), Formula (PBM-7-8), Formula (PBM-8), Formula (PBM-8-1), Formula (PBM-8-2), Formula (PBM-8-3), Formula (PBM-8-4), Formula (PBM-8-5), Formula (PBM-8-6), Formula (PBM-8-7), Formula (PBM-8-8), Formula (PBM-8-9), Formula (PBM-8-10), Formula (PBM-8-11), Formula (PBM-8-12), and Formula (PBM-8-13) of PBM, each R_{c} independently represents a structure of the following formula:
-N(R_{d})-, -N(R_{d})-R_{f}-N(Rₑ)-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 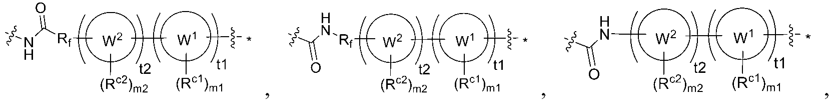 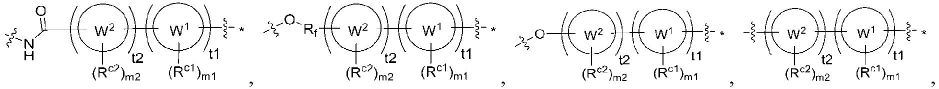 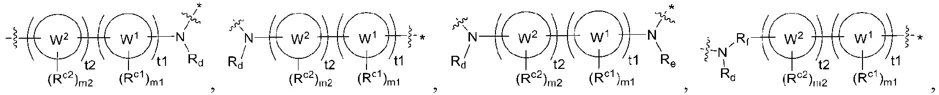 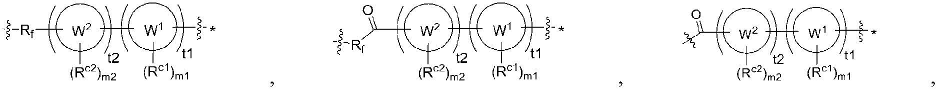 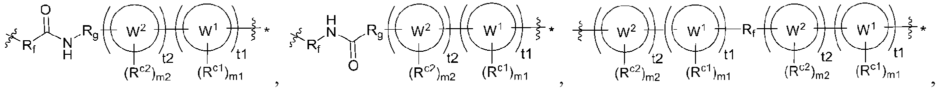 or 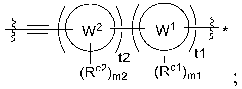
wherein each ring W¹ is identical or different and each independently represents 4- to 20-membered nitrogen-containing heterocyclylene, C₃₋₂₀ cycloalkylene, C₅₋₂₀ arylene, or 5- to 20-membered heteroarylene, t1 represents an integer of 0, 1, or 2, and (R^{c1})ₘ₁ indicates that each ring W¹ is independently optionally substituted with m1 R^{c1} groups, wherein each R^{c1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are identical or different and each independently represents hydrogen or C₁₋₃ alkyl, and m1 represents an integer of 0-20;
each ring W² is identical or different and each independently represents 4- to 20-membered nitrogen-containing heterocyclylene, C₃₋₂₀ cycloalkylene, C₅₋₂₀ arylene, or 5- to 20-membered heteroarylene, t2 represents an integer of 0 or 1, and (R^{c2})ₘ₃ indicates that each ring W² is independently optionally substituted with m2 R^{c2} groups, wherein each R^{c2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represents hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents optionally selected from the group consisting of: deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and
symbol * indicates the point of attachment to LIN.

In some embodiments, R_{d} and Rₑ each independently represent hydrogen or C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl).

In some embodiments, R_{f} and R_{g} each independently represent C₁₋₆ alkylene (e.g., C₁₋₅ alkylene, C₁₋₄ alkylene, C₁₋₃ alkylene, or C₁₋₂ alkylene, such as methylene, ethylene, propylene, isopropylene, butylene, sec-butylene, tert-butylene, n-pentylene, or hexylene), and C₂₋₆ alkenylene (e.g., C₂₋₄ alkenylene, or C₂₋₃ alkenylene, such as vinylene or propenylene). The C₁₋₆ alkylene and C₂₋₆ alkenylene are optionally substituted with one or more (e.g., 1-5, such as 1, 2, 3, 4, or 5) substituents independently selected from the group consisting of deuterium, halogen, hydroxy, mercapto, cyano, amino, nitro, C₁₋₆ alkyl, halogenated C₁₋₃ alkyl, deuterated C₁₋₆ alkyl, C₁₋₆ alkoxy, and halogenated C₁₋₆ alkoxy.

In some embodiments, t1 represents an integer of 0.

In some embodiments, t1 represents an integer of 1.

In some embodiments, t1 represents an integer of 2.

In some embodiments, each ring W¹ is identical or different and each independently represents 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, C₃₋₂₀ cycloalkylene (e.g., C₃₋₁₅ cycloalkylene or C₃₋₁₁ cycloalkylene), C₅₋₂₀ arylene (e.g., C₆₋₂₀ arylene, C₅₋₁₅ arylene, or C₆₋₁₅ arylene), or 5- to 20-membered (e.g., 5- to 15-membered, 5- to 10-membered, 5- to 9-membered, 5- to 8-membered, 5- to 7-membered, or 5- to 6-membered) heteroarylene. In some embodiments, 4- to 20-membered nitrogen-containing heterocyclylene is 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxo-8-azaspiro[4.5]decanylene, and 3-methyl-2-oxo-8-azaspiro[4.5]decanylene. In some embodiments, examples of the C₃₋₂₀ cycloalkylene (e.g., C₃₋₁₅ cycloalkylene) include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene and naphthylene. In some embodiments, 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Each ring W¹ is independently optionally substituted with m1 R^{c1} groups, wherein each R^{c1} is independently deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c4}-R^{c3}-, wherein R^{c3} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more (e.g., 1-5, such as 1, 2, 3, 4, or 5) substituents independently selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), and halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-). m1 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, each ring W¹ is identical or different and each independently represents the following optionally substituted groups: 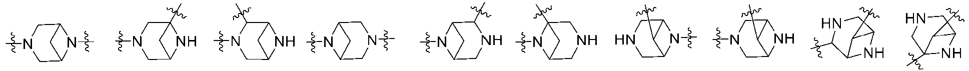 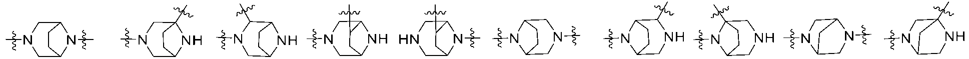 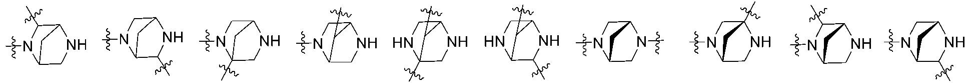 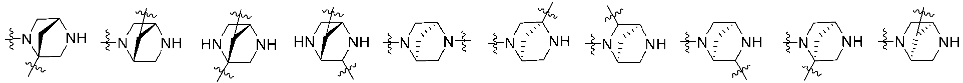 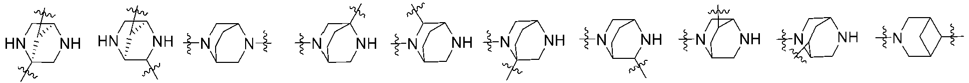 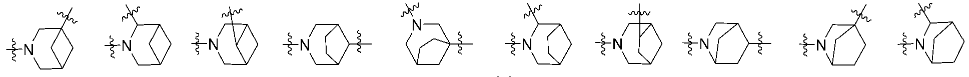 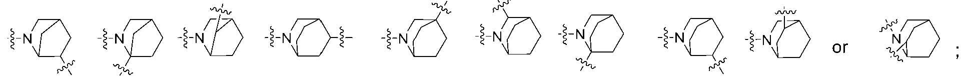 each ring W¹ is independently optionally substituted with m1 R^{c1} groups, wherein each R^{c1} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c4}-R^{c3}-, wherein R^{c3} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), and R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), or halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-). m1 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, each ring W² is identical or different and each independently represents 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, C₃₋₂₀ cycloalkylene (e.g., C₃₋₁₅ cycloalkylene or C₃₋₁₁ cycloalkylene), C₅₋₂₀ arylene (e.g., C₆₋₂₀ arylene, C₅₋₁₅ arylene, or C₆₋₁₅ arylene), or 5- to 20-membered (e.g., 5- to 15-membered, 5- to 10-membered, 5- to 9-membered, 5- to 8-membered, 5- to 7-membered, or 5- to 6-membered) heteroarylene. In some embodiments, 4- to 20-membered nitrogen-containing heterocyclylene is 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxo-8-azaspiro[4.5]decanylene, and 3-methyl-2-oxo-8-azaspiro[4.5]decanylene. In some embodiments, examples of the C₃₋₂₀ cycloalkylene (e.g., C₃₋₁₅ cycloalkylene) include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene and naphthylene. In some embodiments, 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Each ring W² is independently optionally substituted with m2 R^{c2} groups, wherein each R^{c2} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c8}-R^{c7}-, wherein R^{c7} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more (e.g., 1-5, such as 1, 2, 3, 4, or 5) substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy. m2 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, each ring W² is identical or different and each independently represents: 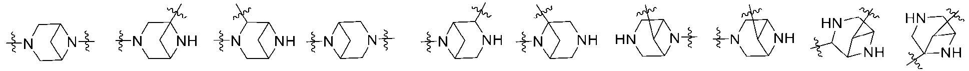 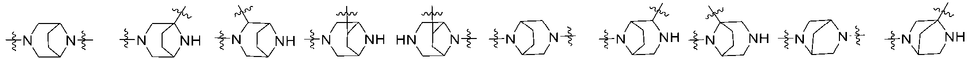 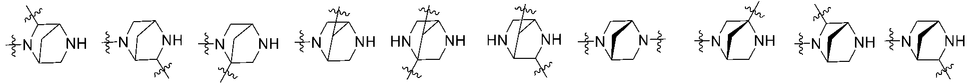 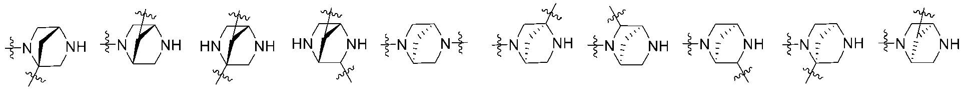 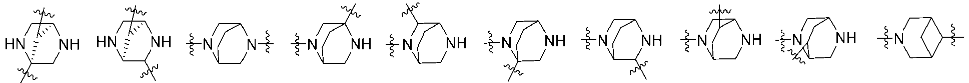 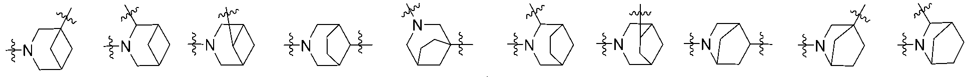 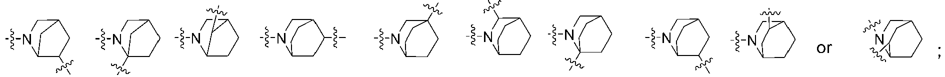 each ring W² is independently optionally substituted with m2 R^{c2} groups, wherein each R^{c2} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c8}-R^{c7}-, wherein R^{c7} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more (e.g., 1-5, such as 1, 2, 3, 4, or 5) substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy. m2 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, t2 represents an integer of 0.

In some embodiments, t2 represents an integer of 1.

In some embodiments, the moiety 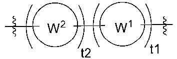 in each of the general formulae of R_{c} represents the following groups: 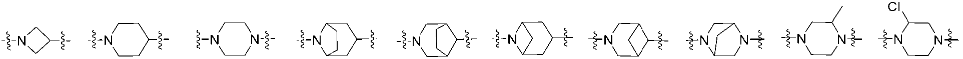 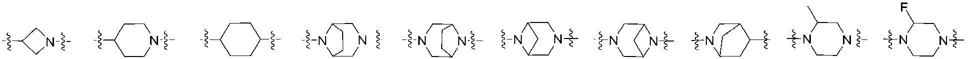 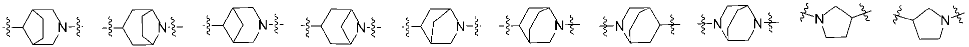 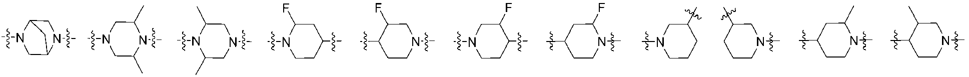 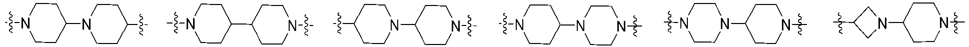 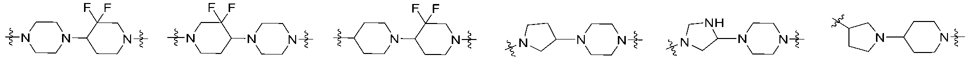 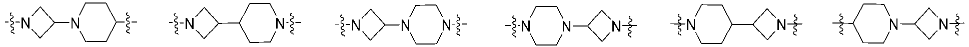 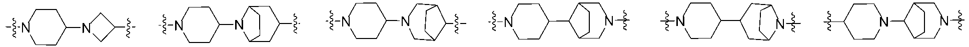 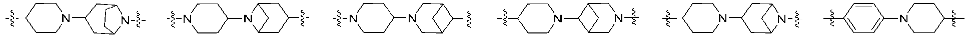 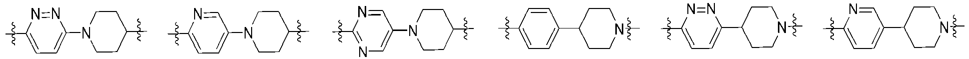 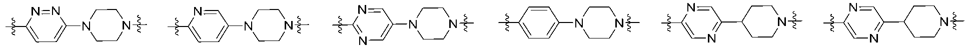 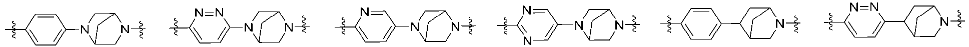 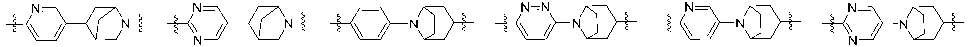 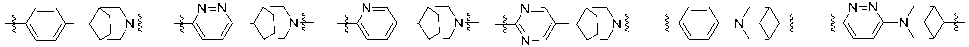 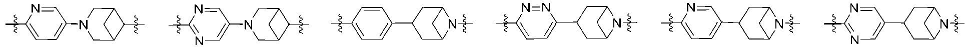 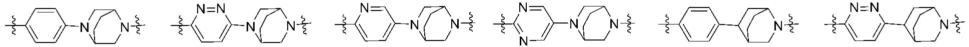 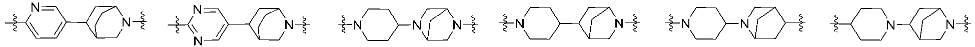 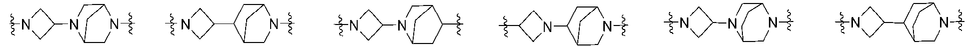 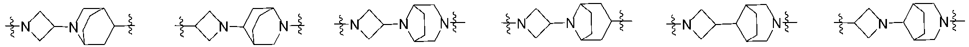 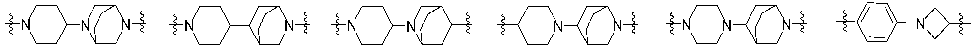 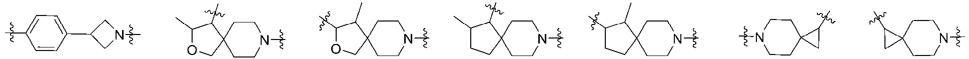 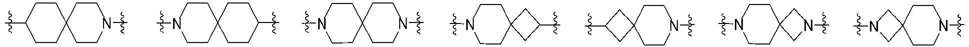 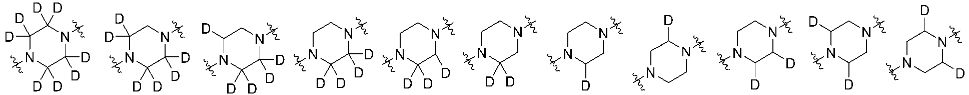 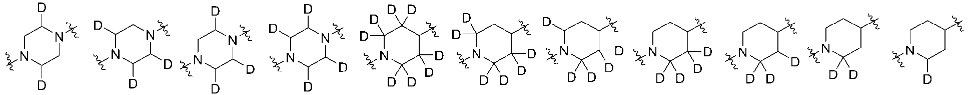 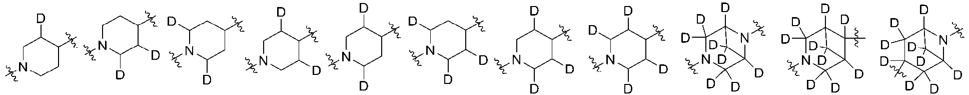 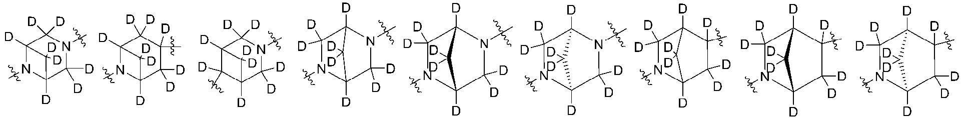 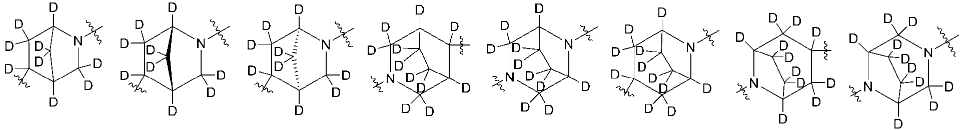 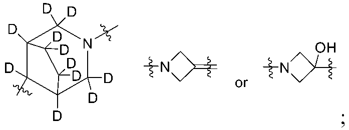 wherein the groups are each independently optionally substituted with one or more (e.g., 1-5, such as 1, 2, 3, 4, or 5) substituents selected from the group consisting of deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), R^{c12}-R^{c11}, and any combination thereof, wherein R^{c11} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), and R^{c12} represents halogen (e.g., fluorine, chlorine, bromine, or iodine), R^{c13}R^{c14}N-, hydroxy, carboxyl, ethynyl, or vinyl, wherein R^{c13} and R^{c14} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy.

In some embodiments, R_{c} of the compound of Formula (I) represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -NH-CH₂-*, -N(CH₃)-CH₂-*, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 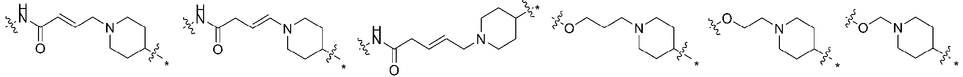 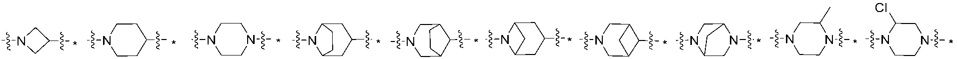 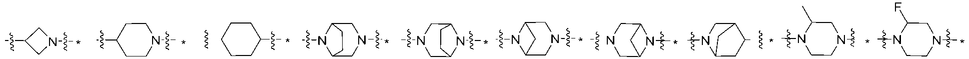 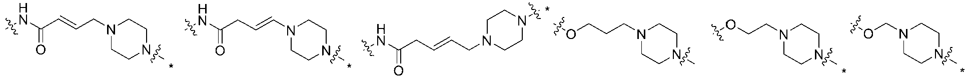 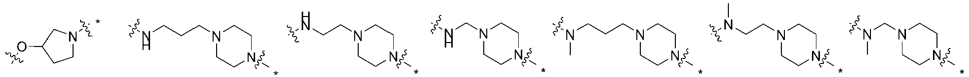 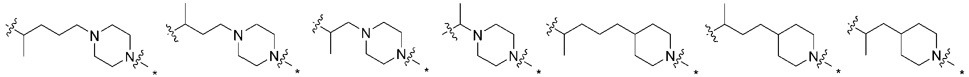 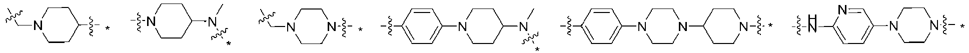 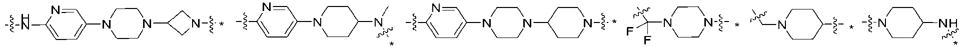 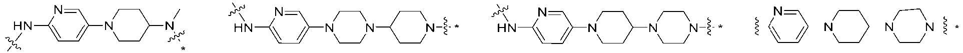 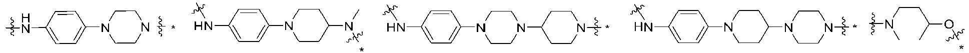 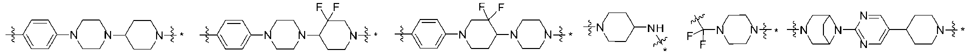 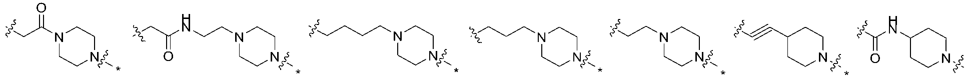 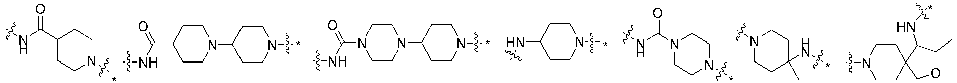 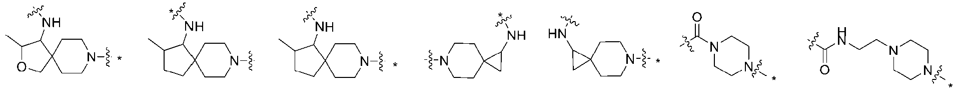 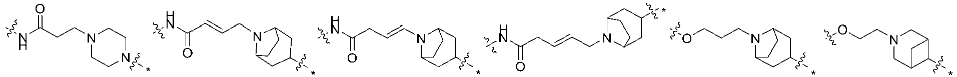 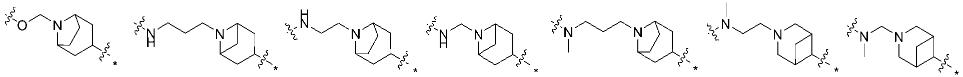 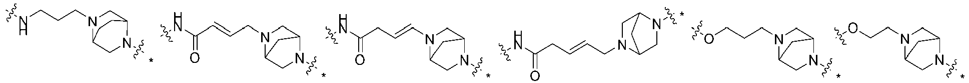 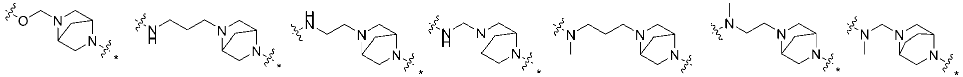 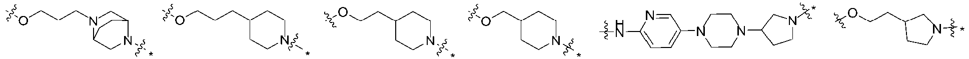 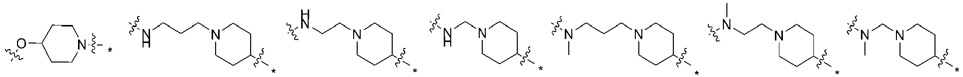 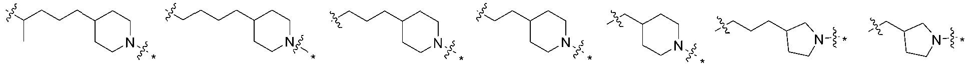 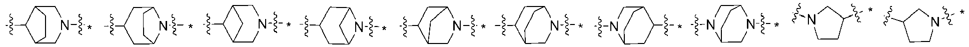 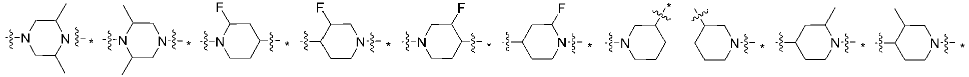 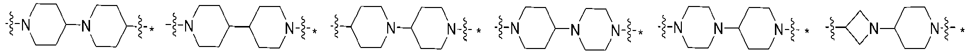 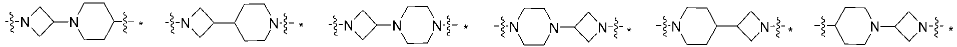 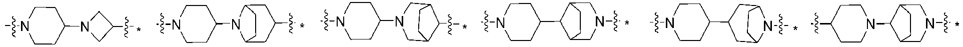 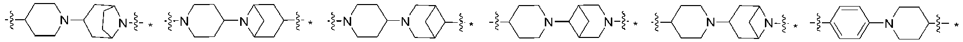 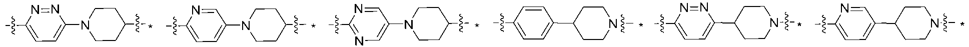 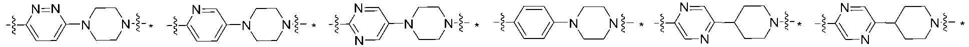 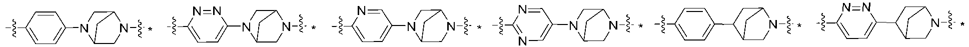 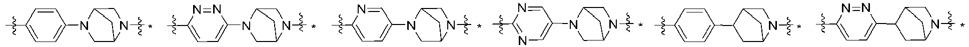 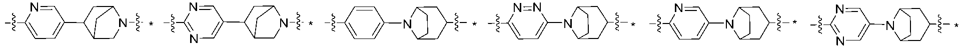 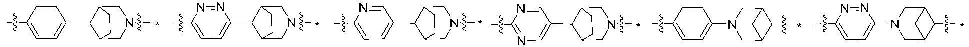 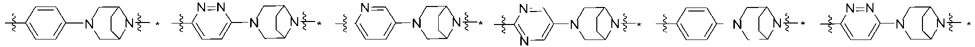 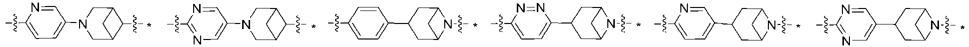 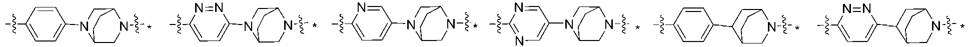 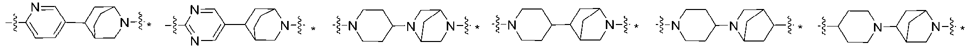 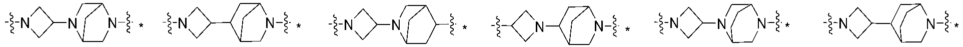 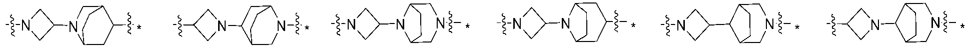 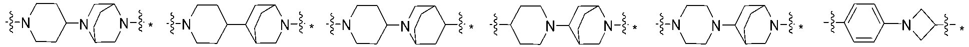 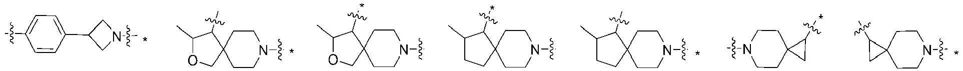 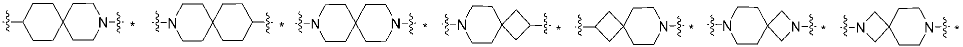 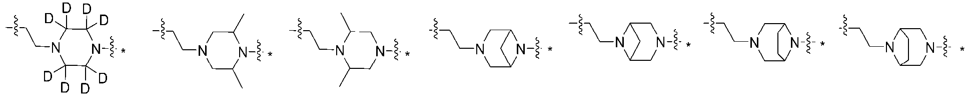 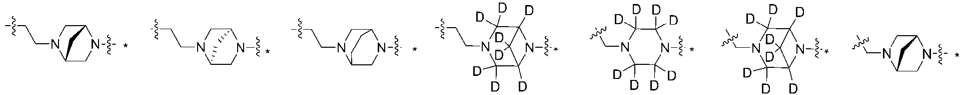 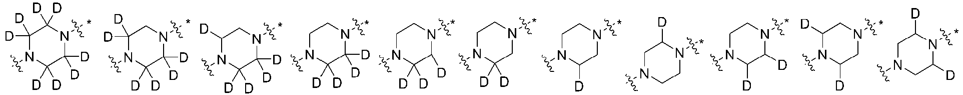 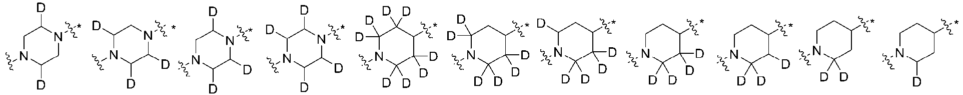 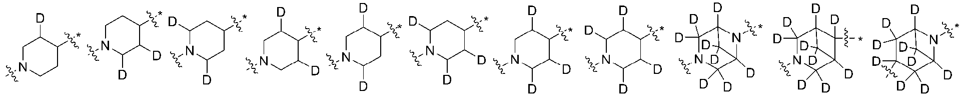 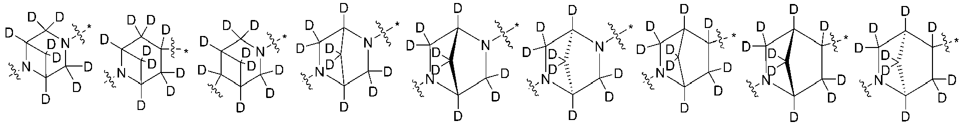 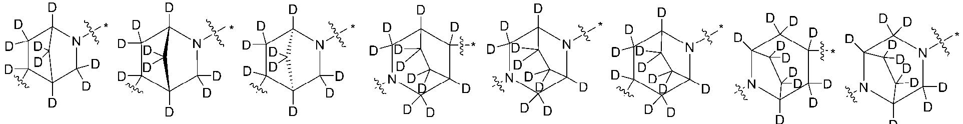 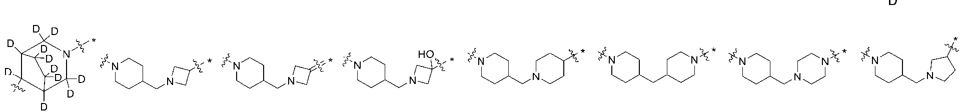 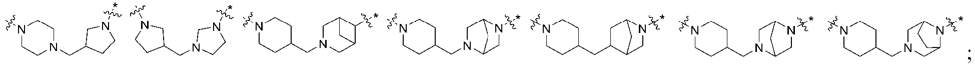 wherein the groups are each independently optionally substituted with one or more (e.g., 1-5, such as 1, 2, 3, 4, or 5) substituents selected from the group consisting of deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxy, mercapto, cyano, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), R^{c12}-R^{c11}-, and any combination thereof, wherein R^{c11} represents optionally substituted C₁₋₆ alkylene (e.g., optionally substituted C₁₋₄ alkylene, such as optionally substituted methylene, ethylene, or propylene), or optionally substituted C₂₋₆ alkenylene (e.g., optionally substituted C₂₋₄ alkenylene, such as optionally substituted vinylene, propenylene, butenylene), and R^{c12} represents halogen (e.g., fluorine, chlorine, bromine, or iodine), R^{c13}R^{c14}N-, hydroxy, carboxyl, ethynyl, or vinyl, wherein R^{c13} and R^{c14} are identical or different and each independently represents hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and
symbol * indicates the point of attachment to LIN.

In some embodiments, examples of PBM in the compound of Formula (I) include, but are not limited to, a structure of the following formula:

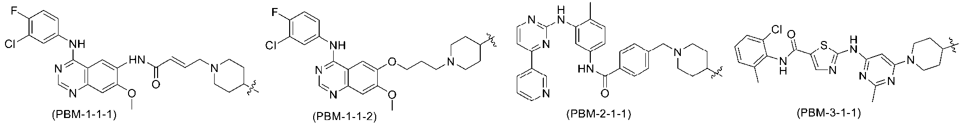

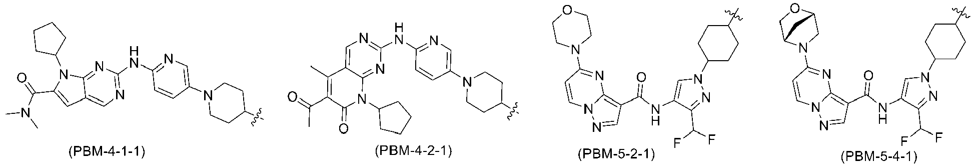

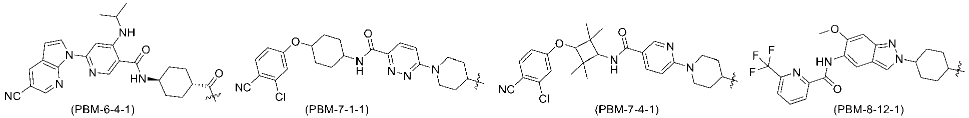

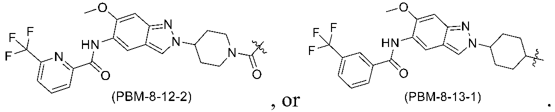

In some embodiments, the ULM of the compound of Formula (I) represents a ligand moiety capable of binding to an E3 ubiquitin ligase, and represents the following structure of the Formula (ULM): 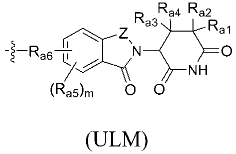
wherein Z represents C(O) or CH₂;
Rₐ₁, Rₐ₂, Rₐ₃, and Rₐ₄ are identical or different and each independently represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
m represents an integer of 0, 1, 2, or 3;
Rₐ₆ represents: 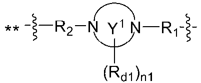
   wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20; and
   R₁ represents N(R_{w}), where R_{w} represents hydrogen or C₁₋₃ alkyl (e.g., methyl, ethyl, or propyl), and R₂ represents a bond, or R₂ represents CH₂, C(O), **-NHC(O)-, or **-N(CH₃)C(O)-, or R₂ represents a structure of the following formula: 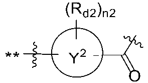 wherein ring Y² represents cycloalkylene or heterocyclylene containing at least one nitrogen atom, and (R_{d2})ₙ₂ indicates that ring Y² is optionally substituted with n2 R_{d2} groups, wherein each R_{d2} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n2 represents an integer of 0-20;
      or
   R₁ represents a bond, and R₂ represents N(R_{w}), where R_{w} represents hydrogen or C₁₋₃ alkyl, or R₂ represents **C(O)N(R_{w}), where R_{w} represents hydrogen or C₁₋₃ alkyl, or R₂ represents **-CH=N-; or
Rₐ₆ represents: 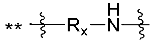 where Rₓ represents NH or **-CH=N-; or
Rₐ₆ represents: 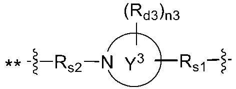
   wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20; and
   wherein Rₛ₁ represents 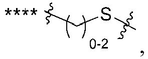 symbol **** indicates the point of attachment to ring Y³; and Rₛ₂ represents N(R_{w}) or **C(O)N(R_{w}), where R_{w} represents hydrogen or C₁₋₃ alkyl; and
   symbol ** in each of the above formulae indicates the point of attachment to LIN.

In some embodiments, Z in the structure of Formula (ULM) represents C(O).

In some embodiments, Z in the structure of Formula (ULM) represents CH₂.

In some embodiments, Rₐ₁, Rₐ₂, Rₐ₃, and Rₐ₄ in the structure of Formula (ULM) represent hydrogen.

In some embodiments, m in the structure of Formula (ULM) represents an integer of 0.

In some embodiments, m in the structure of Formula (ULM) represents an integer of 1, 2, or 3.

In some embodiments, each Rₐ₅ in the structure of Formula (ULM) is the same or different and each independently represents halogen (e.g., fluorine, chlorine, bromine, or iodine).

In some embodiments, Rₐ₆ in the structure of Formula (ULM) represents: 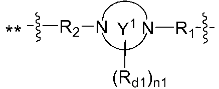 wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20; and
(i) R₁ represents N(R_{w}), where R_{w} represents hydrogen or C₁₋₃ alkyl, and R₂ represents a bond, or R₂ represents CH₂, C(O), **-NHC(O)-, or **-N(CH₃)C(O)-, or R₂ represents a structure of the following formula: 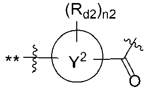 wherein ring Y² represents cycloalkylene or heterocyclylene containing at least one nitrogen atom, and (R_{d2})ₙ₂ indicates that ring Y² is optionally substituted with n2 R_{d2} groups, wherein each R_{d2} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂-etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n2 represents an integer of 0-20;
   or
(ii) R₁ represents a bond, and R₂ represents N(R_{w}), where R_{w} represents hydrogen or C₁₋₃ alkyl, or R₂ represents **C(O)N(R_{w}), where R_{w} represents hydrogen or C₁₋₃ alkyl, or R₂ represents **-CH=N-; and
symbol ** in each of the above formulae indicates the point of attachment to LIN.

In some embodiments, ring Y¹ in Rₐ₆ represents 4- to 20-membered heterocyclylene containing at least two nitrogen atoms. Examples of the 4- to 20-membered heterocyclylene containing at least two nitrogen atoms include, but are not limited to, 4- to 20-membered heterocyclylene containing 2 to 4 nitrogen atoms. Representative examples include: diazetidinylene, imidazolidylene, pyrazolidylene, piperazinylene, 1,2,3,4-tetrahydropyrazinylene, hexahydropyrimidinylene, hexahydropyridazinylene, diazacycloheptanylene, diazacyclooctylene, diazabicyclo[3.1.1]heptanylene, diazabicyclo [2.2.1]heptanylene, diazabicyclo[3.2.1]octanylene, diazabicyclo[2.2.2]octanylene, diazaspiro[2.3]hexylene, diazaspiro[3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydropyrrolo[3,4-c]pyrrolylene, or decahydronaphthyridinylene, or 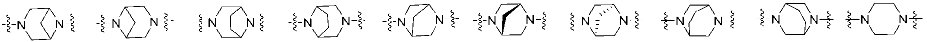 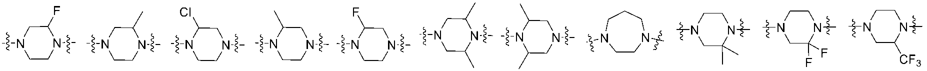 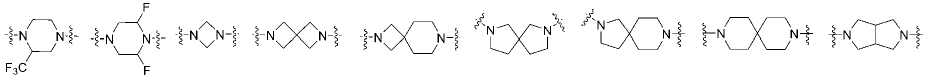 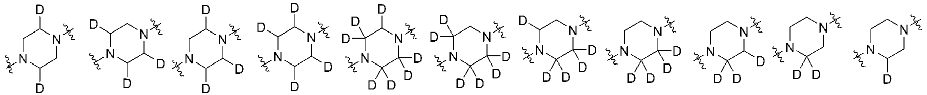 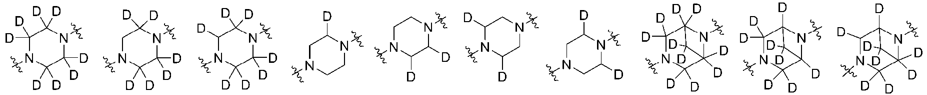 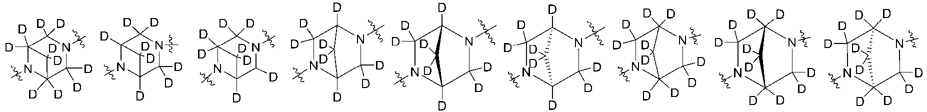 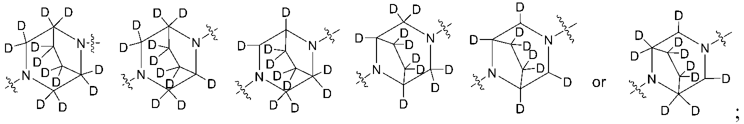
wherein ring Y¹ is optionally substituted with n1 R_{d1} groups, and each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n1 represents an integer of 0-20.

(R_{d1})ₙ₁ as described herein indicates that ring Y¹ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, R₁ in Rₐ₆ represents N(R_{w}), wherein R_{w} represents hydrogen or C₁₋₃ alkyl, and R₂ represents a bond.

In some embodiments, R₁ in Rₐ₆ represents N(R_{w}), where R_{w} represents hydrogen or C₁₋₃ alkyl, and R₂ represents CH₂, C(O), **-NHC(O)-, or **-N(CH₃)C(O)-, and symbol ** indicates the point of attachment to LIN.

In some embodiments, R₁ in Rₐ₆ represents N(R_{w}), where R_{w} represents hydrogen or C₁₋₃ alkyl, and R₂ represents a structure of the following formula: 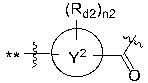
wherein ring Y² represents C₃₋₂₀ cycloalkylene or 4- to 20-membered heterocyclylene containing at least one nitrogen atom, and (R_{d2})ₙ₂ indicates that ring Y² is optionally substituted with n2 R_{d2} groups, wherein each R_{d2} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n2 represents an integer of 0-20; and
symbol ** indicates the point of attachment to LIN.

In some embodiments, ring Y² in Rₐ₆ represents C₃₋₂₀ cycloalkylene (e.g., C₃₋₁₅ cycloalkylene, or C₃₋₁₁ cycloalkylene), or 4- to 20-membered heterocyclylene containing 1 to 4 nitrogen atoms (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene). In some embodiments, examples of C₃₋₂₀ cycloalkylene (e.g., C₃₋₁₅ cycloalkylene) include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, C₅₋₁₅ bridged cycloalkylene (e.g., adamantanylene, noradamantanylene, norbomylene). Ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and n2 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, ring Y² in Rₐ₆ represents 4- to 20-membered heterocyclylene containing 1 to 4 nitrogen atoms (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene). 4- to 20-membered heterocyclylene containing 1 to 4 nitrogen atoms is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing 1 to 4 nitrogen atoms and optionally containing a heteroatom(s) selected from nitrogen, oxygen, and sulfur. Examples of the 4- to 20-membered heterocyclylene containing 1 to 4 nitrogen atoms include, but are not limited to, e.g., azetidinylene, diazetidinylene, pyrrolidinylene, imidazolidylene, pyrazolidylene, piperidinylene, tetrahydropyridylene, piperazinylene, 1,2,3,4-tetrahydropyrazinylene, hexahydropyrimidinylene, hexahydropyridazinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, azaspiro[2.3]hexylene, azaspiro[3.3]heptanylene, azaspiro[2.4]heptanylene, azaspiro[3.4]octanylene, azaspiro[3.5]nonanylene, azaspiro[4.4]nonanylene, azaspiro[4.5]decanylene, azaspiro[5.5]undecanylene, diazaspiro[2.3]hexylene, diazaspiro[3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydrocyclopenta[c]pyrrolylene, octahydropyrrolo[3,4-c]pyrrolylene, or decahydronaphthyridinylene, or 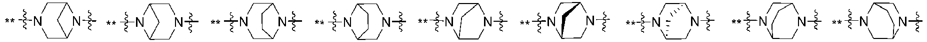 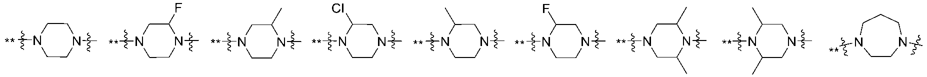 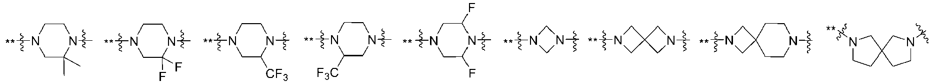 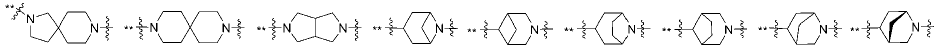 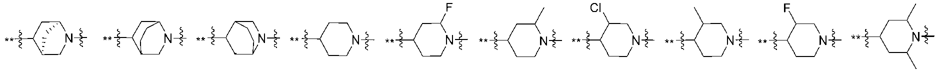 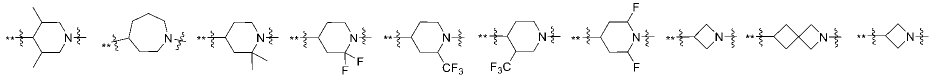 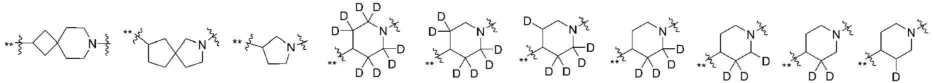 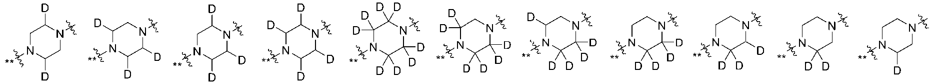 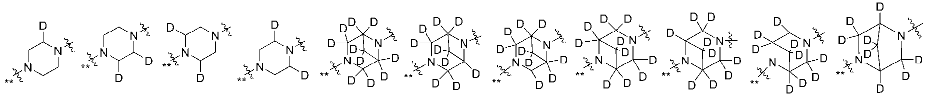 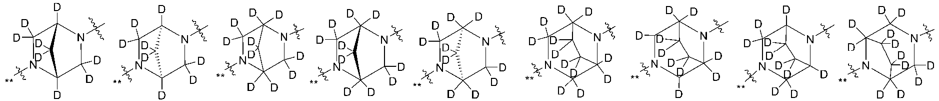 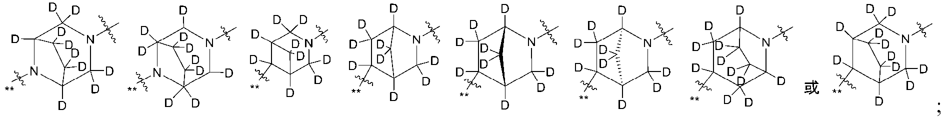
symbol ** indicates the point of attachment to LIN; and
ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n2 represents an integer of 0-20, e.g., an integer of 0, 1, 2, 3, 4, 5, 6, 7, 8, 9, 10, 11, 12, 13, 14, 15, or 20.

In some embodiments, Rₐ₆ in the structure of Formula (ULM) represents: 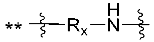 wherein Rₓ represents NH or **-CH=N-, and symbol ** indicates the point of attachment to LIN.

In some embodiments, Rₐ₆ in the structure of Formula (ULM) represents: 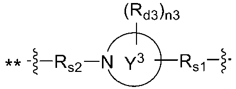
wherein ring Y³ represents heterocyclylene containing one nitrogen atom (e.g., 4- to 20-membered heterocyclylene containing one nitrogen atom), and (R_{d3})ₙ₃ indicates that ring Y³ is optionally substituted with n3 R_{d3} groups, wherein each R_{d3} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20 (e.g., an integer of 0, 1, 2, 3, 4, 5, 6, 7, 8, 9, 10, 11, 12, 13, 14, 15, or 20); and
wherein Rₛ₁ represents 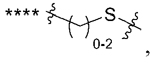 where symbol **** indicates the point of attachment to ring Y³;
Rₛ₂ represents N(R_{w}) or **C(O)N(R_{w}), where R_{w} represents hydrogen or C₁₋₃ alkyl (e.g., methyl, ethyl, or propyl); and
symbol ** indicates the point of attachment to LIN.

In some embodiments, ring Y³ in Rₐ₆ represents 4- to 20-membered heterocyclylene containing one nitrogen atom (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene). 4- to 20-membered heterocyclylene containing one nitrogen atom is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered heterocyclylene containing one nitrogen atom include, but are not limited to, e.g., azetidinylene, pyrrolidinylene, piperidinylene, tetrahydropyridylene, azepanylene, azacyclooctylene, azabicyclo[3.1.1]heptanylene, azabicyclo[2.2.1]heptanylene, azabicyclo[3.2.1]octanylene, azabicyclo[2.2.2]octanylene, azaspiro[2.3]hexylene, azaspiro[3.3]heptanylene, azaspiro[2.4]heptanylene, azaspiro[3.4]octanylene, azaspiro[3.5]nonanylene, azaspiro[4.4]nonanylene, azaspiro[4.5]decanylene, azaspiro[5.5]undecanylene, octahydrocyclopenta[c]pyrrolylene, or octahydropyrrolo[3,4-c]pyrrolylene, or 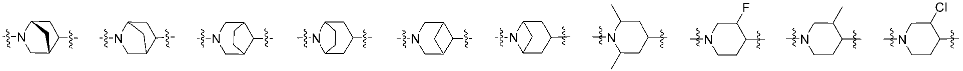 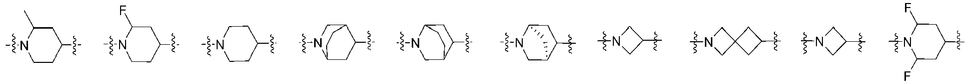 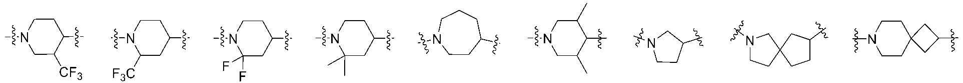 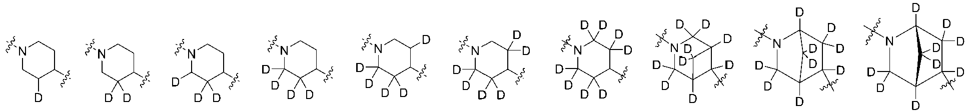 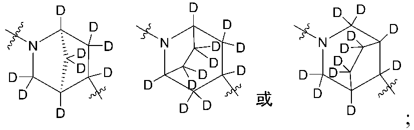
the ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n3 represents an integer of 0-20.

In some embodiments, Rₛ₁ in Rₐ₆ represents S.

In some embodiments, Rₛ₁ in Rₐ₆ represents ****-CH₂-S- or ****-CH₂CH₂-S-, wherein symbol **** indicates the point of attachment to ring Y³.

In some embodiments, Rₐ₆ in the compound of Formula (I) represents:
-NH-NH-, or **-CH=N-NH-; 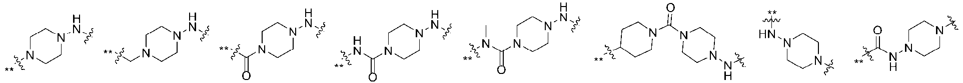 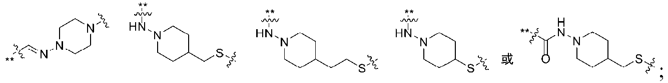 symbol ** indicates the point of attachment to LIN.

In some embodiments, ULM of the compound of Formula (I) represents a structure of Formula (ULM-1-1), Formula (ULM-1-2), Formula (ULM-1-3) , or Formula (ULM-1-4): 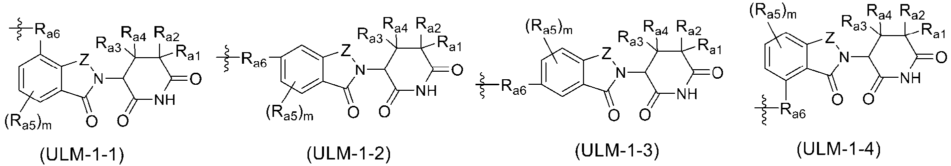
wherein Z represents C(O) or CH₂;
Rₐ₁, Rₐ₂, Rₐ₃, and Rₐ₄ are identical or different and each independently represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
m represents an integer of 0, 1, 2, or 3;
Rₐ₆ represents: 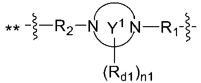
   wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20 (e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units); and
   R₁ represents N(R_{w}), where R_{w} represents hydrogen or C₁₋₃ alkyl, and R₂ represents a bond, or R₂ represents CH₂, C(O), **-NHC(O)-, or **-N(CH₃)C(O)-, or R₂ represents a structure of the following formula: 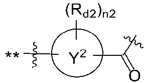 wherein ring Y² represents cycloalkylene or heterocyclylene containing at least one nitrogen atom, and (R_{d2})ₙ₂ indicates that ring Y² is optionally substituted with n2 R_{d2} groups, wherein each R_{d2} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n2 represents an integer of 0-20 (e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units);
      or
   R₁ represents a bond, and R₂ represents N(R_{w}), where R_{w} represents hydrogen or C₁₋₃ alkyl, or R₂ represents **C(O)N(R_{w}), where R_{w} represents hydrogen or C₁₋₃ alkyl, or R₂ represents **-CH=N-; or
R ₐ₆ represents: 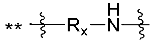 wherein Rₓ represents NH or **-CH=N-; or
R ₐ₆ represents: 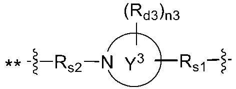
   wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20 (e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units); and
   wherein Rₛ₁ represents 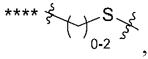 symbol **** indicates the point of attachment to ring Y³; and Rₛ₂ represents N(R_{w}) or **C(O)N(R_{w}), where R_{w} represents hydrogen or C₁₋₃ alkyl; and
   symbol ** in each of the above formulae indicates the point of attachment to LIN.

In some embodiments, ULM of the compound of Formula (I) represents a structure of the following formula: 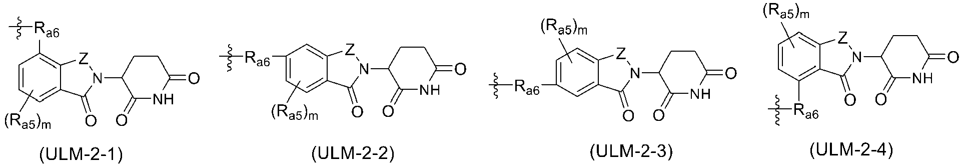 wherein Z, (Rₐ₅)ₘ, and Rₐ₆ are as defined herein.

In some embodiments, LIN of the compound of Formula (I) represents: 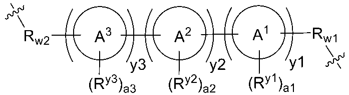
wherein R_{w2} is linked to PBM, and R_{w1} is linked to Rₐ₆;
R_{w2} represents a bond, C(O), C(O)NH, or NHC(O);
each ring A¹ is identical or different and each independently represents heterocyclylene (e.g., 4- to 20-membered heterocyclylene), cycloalkylene (e.g., C₃₋₂₀ cycloalkylene), arylene (e.g., C₅₋₂₀ arylene), or heteroarylene (e.g., 5- to 20-membered heteroarylene), y1 represents an integer of 0, 1, 2, or 3, and (R^{y1})ₐ₁ indicates that each ring A¹ is independently optionally substituted with a1 R^{y1} substituents, wherein each R^{y1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a1 represents an integer of 0-10;
each ring A² is identical or different and each independently represents heterocyclylene (e.g., 4- to 20-membered heterocyclylene), cycloalkylene (e.g., C₃₋₂₀ cycloalkylene), arylene (e.g., C₅₋₂₀ arylene), or heteroarylene (e.g., 5- to 20-membered heteroarylene), y2 represents an integer of 0, 1, 2, or 3, and (R^{y2})ₐ₂ indicates that each ring A² is independently optionally substituted with a2 R^{y2} substituents, wherein each R^{y2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a2 represents an integer of 0-10;
each ring A³ is identical or different and each independently represents heterocyclylene (e.g., 4- to 20-membered heterocyclylene), cycloalkylene (e.g., C₃₋₂₀ cycloalkylene), arylene (e.g., C₅₋₂₀ arylene), or heteroarylene (e.g., 5- to 20-membered heteroarylene), y3 represents an integer of 0, 1, 2, or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a3 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof.

In some embodiments, R_{w2} in LIN of the compound of Formula (I) represents a bond.

In some embodiments, R_{w2} in LIN of the compound of Formula (I) represents C(O).

In some embodiments, R_{w2} in LIN of the compound of Formula (I) represents C(O)NH or NHC(O).

In some embodiments, each ring A¹ in LIN of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene), cycloalkylene (e.g., C₃₋₂₀ cycloalkylene), arylene (e.g., C₅₋₂₀ arylene, such as C₆₋₂₀ arylene, C₅₋₁₅ arylene, or C₆₋₁₅ arylene), or heteroarylene (e.g., 5- to 20-membered heteroarylene, such as 5- to 15-membered, 5- to 10-membered, 5- to 9-membered, 5- to 8-membered, 5- to 7-membered, or 5- to 6-membered heteroarylene). In some embodiments, 4- to 20-membered heterocyclylene is 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic, or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4- to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A¹ is independently optionally substituted with a1 R^{y1} groups, wherein each R^{y1} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl, or C₂₋₆ alkenyl. a1 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, y1 in LIN of the compound of Formula (I) represents an integer of 0, 1, 2, or 3.

In some embodiments, each ring A¹ in LIN of the compound of Formula (I) is identical or different and each independently represents the following optionally substituted groups: 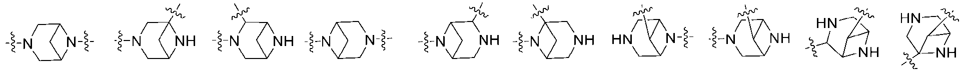 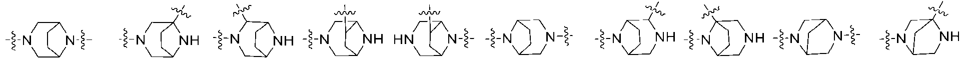 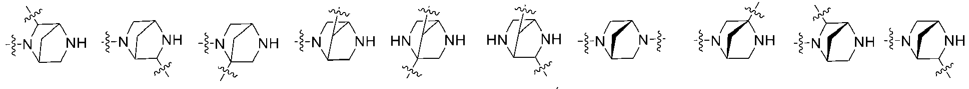 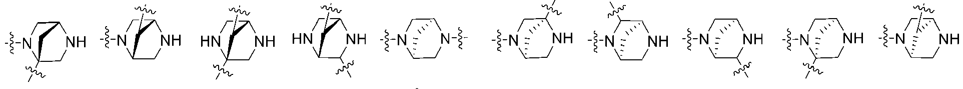 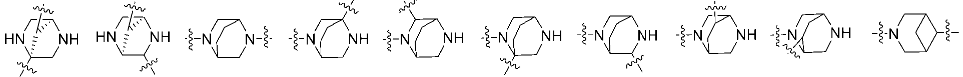 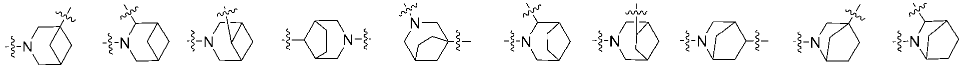 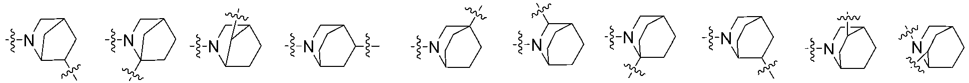 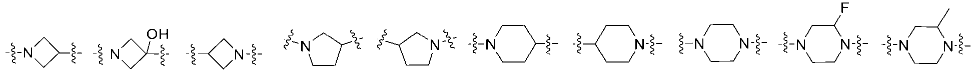 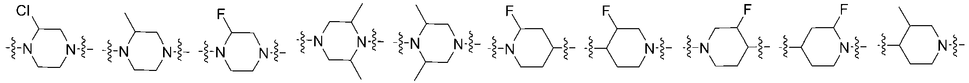 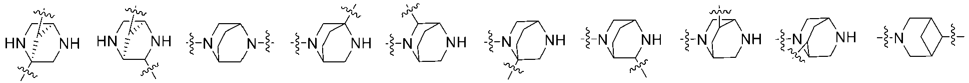 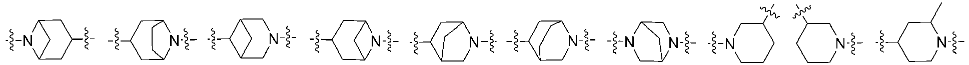 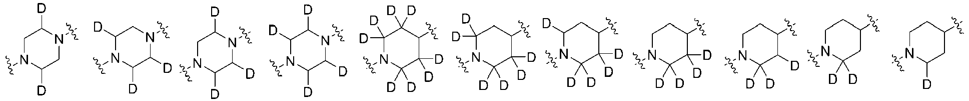 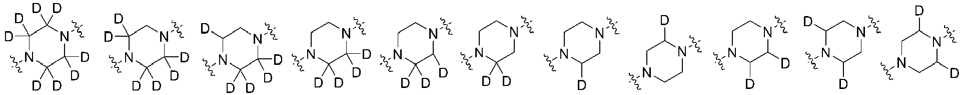 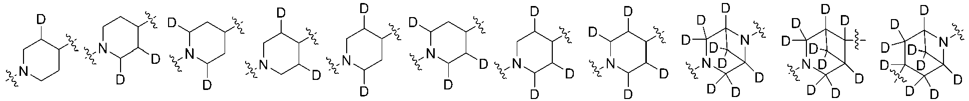 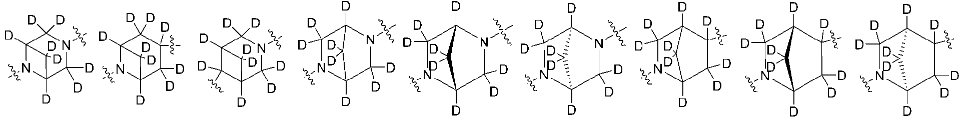 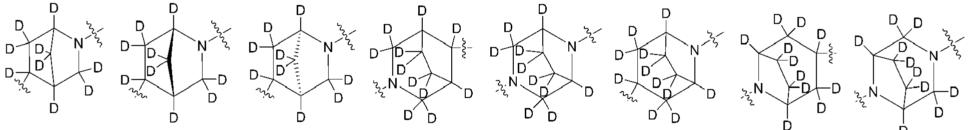 or 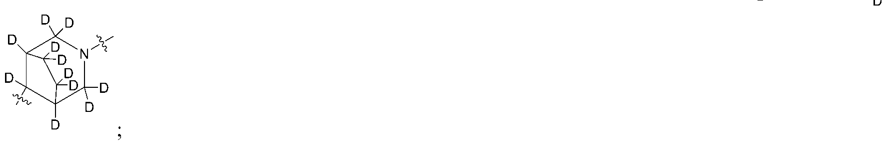 each ring A¹ is independently optionally substituted with a1 R^{y1} substituents, and each R^{y1} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), or C₂₋₆ alkenyl (e.g., C₂₋₄ alkenyl, such as vinyl, propenyl, or butenyl). a1 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, each ring A² in LIN of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene), cycloalkylene (e.g., C₃₋₂₀ cycloalkylene), arylene (e.g., C₅₋₂₀ arylene, such as C₆₋₂₀ arylene, C₅₋₁₅ arylene, or C₆₋₁₅ arylene), or heteroarylene (e.g., 5- to 20-membered heteroarylene, such as 5- to 15-membered, 5- to 10-membered, 5- to 9-membered, 5- to 8-membered, 5- to 7-membered, or 5- to 6-membered heteroarylene). In some embodiments, 4- to 20-membered heterocyclylene is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of 4- to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of 5- to 20-membered arylene include, but are not limited to, e.g., phenylene or naphthylene. In some embodiments, 5- to 20-membered heteroarylene is a 5- to 20-membered (e.g., 5- to 15-membered, 5- to 12-membered, 5- to 9-membered, 5- to 8-membered, 5-to 7-membered, or 5- to 6-membered) heteroarylene containing one nitrogen atom and optionally containing a heteroatom(s) selected from nitrogen, oxygen, and sulfur. In some embodiments, examples of 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Each ring A² is independently optionally substituted with a2 R^{y2} groups, wherein each R^{y2} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl, or C₂₋₆ alkenyl. a2 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, y2 of the compound of Formula (I) represents an integer of 0, 1, 2, or 3.

In some embodiments, each ring A² in LIN of the compound of Formula (I) is identical or different and each independently represents the following optionally substituted groups: 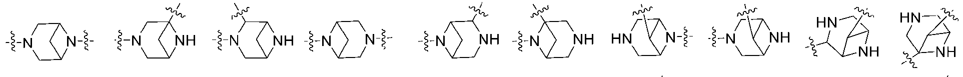 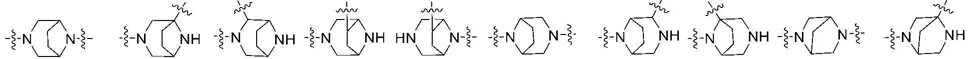 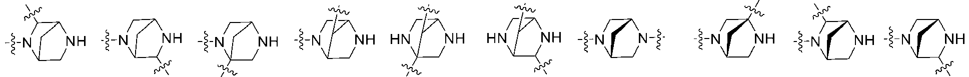 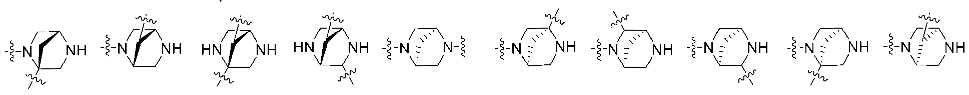 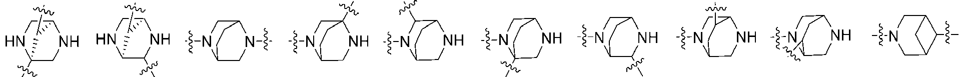 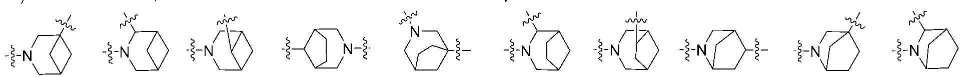 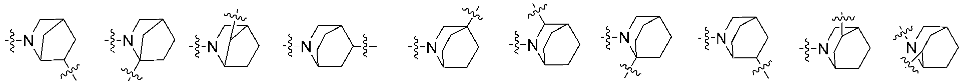 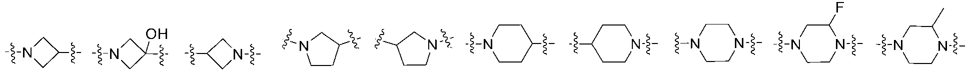 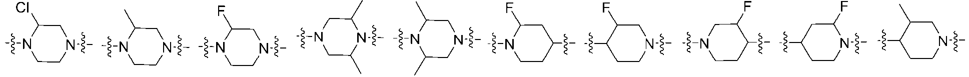 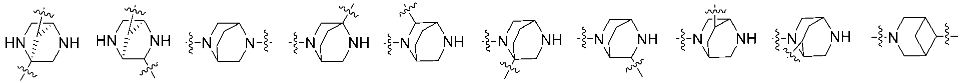 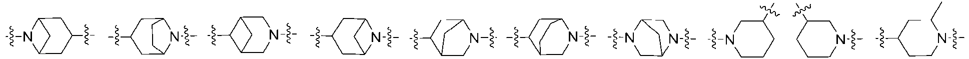 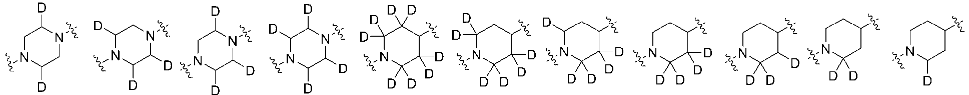 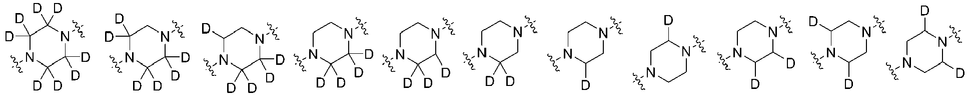 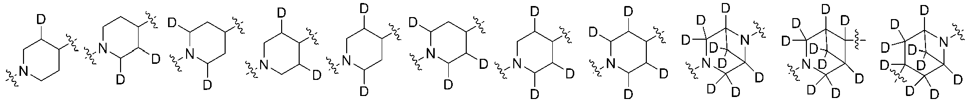 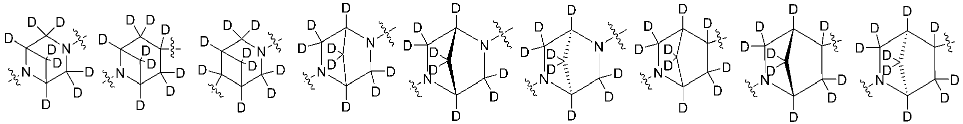 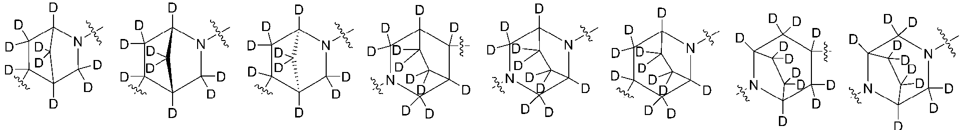 or 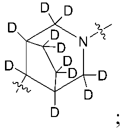 each ring A² is independently optionally substituted with a2 R^{y2} groups, wherein each R^{y2} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), or C₂₋₆ alkenyl (e.g., C₂₋₄ alkenyl, such as vinyl, propenyl, or butenyl). a2 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, each ring A³ in LIN of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered heterocyclylene), cycloalkylene (e.g., C₃₋₂₀ cycloalkylene), arylene (e.g., C₅₋₂₀ arylene, such as C₆₋₂₀ arylene, C₅₋₁₅ arylene, or C₆₋₁₅ arylene), or heteroarylene (e.g., 5- to 20-membered heteroarylene, such as 5- to 15-membered, 5- to 10-membered, 5- to 9-membered, 5- to 8-membered, 5- to 7-membered, or 5- to 6-membered heteroarylene). In some embodiments, 4- to 20-membered heterocyclylene is 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of 4- to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of 5- to 20-membered arylene include, but are not limited to, e.g., phenylene or naphthylene. In some embodiments, 5- to 20-membered heteroarylene is 5- to 20-membered (e.g., 5- to 15-membered, 5- to 12-membered, 5- to 9-membered, 5- to 8-membered, 5-to 7-membered, or 5- to 6-membered) heteroarylene containing one nitrogen atom and optionally containing a heteroatom(s) selected from nitrogen, oxygen, and sulfur. In some embodiments, examples of 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A³ is independently optionally substituted with a3 R^{y3} groups, wherein each R^{y3} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl, or C₂₋₆ alkenyl. a3 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, y3 of the compound of Formula (I) represents an integer of 0, 1, 2, or 3.

In some embodiments, each ring A³ of the compound of Formula (I) is identical or different and each independently represents the following optionally substituted groups: 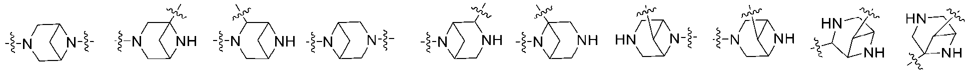 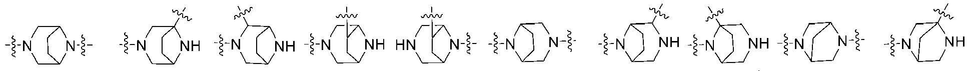 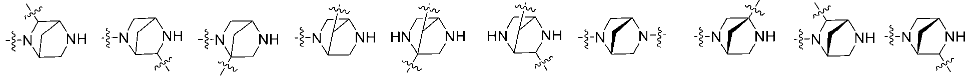 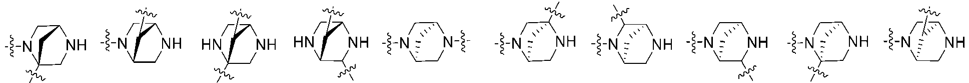 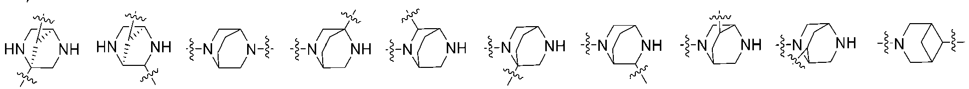 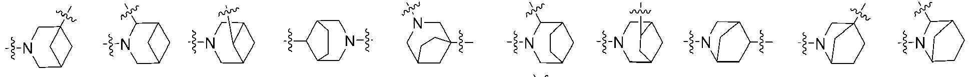 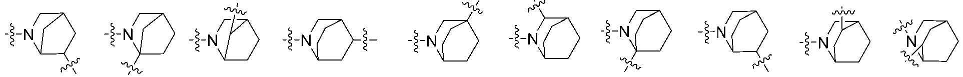 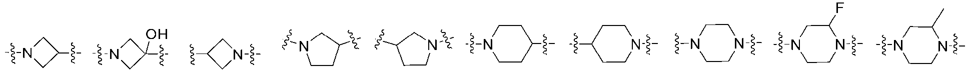 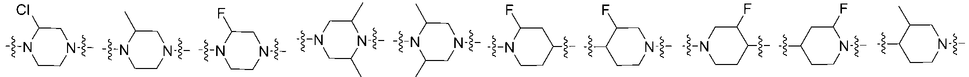 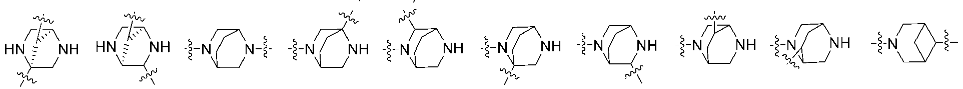 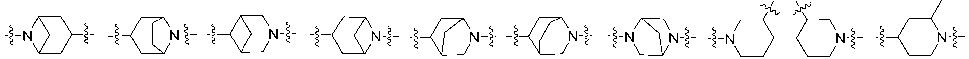 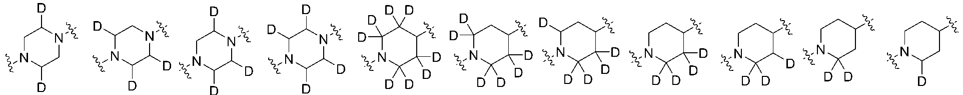 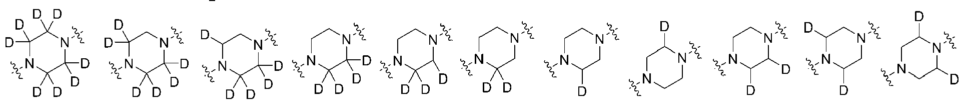 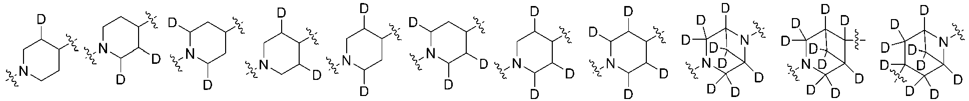 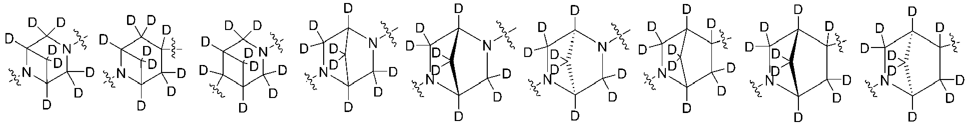 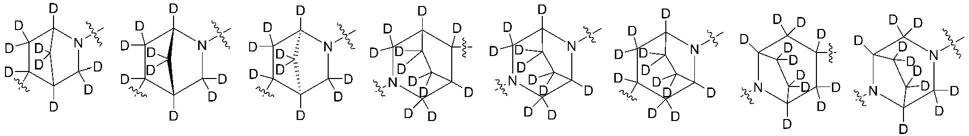 or 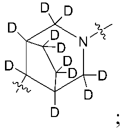 each ring A³ is independently optionally substituted with a3 R^{y3} substituents, and each R^{y3} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), or C₂₋₆ alkenyl (e.g., C₂₋₄ alkenyl, such as vinyl, propenyl, or butenyl). a3 represents an integer of 0-10, e.g., an integer of 0, 1, 2, 3, 4, 5, 6, 7, 8, 9, or 10. The number of substituents is not theoretically limited in any way, or is automatically limited by the size of the building units.

In some embodiments, non-limiting examples of the moiety 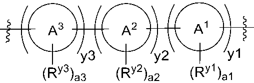 in each of the general formulae of the LIN of the compound of Formula (I) include, but are not limited to, the following groups: 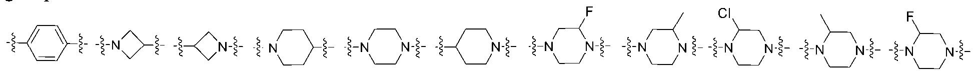 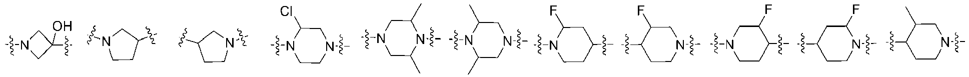 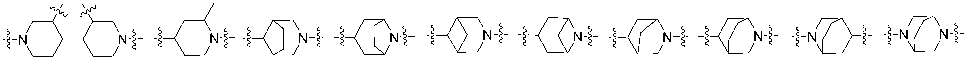 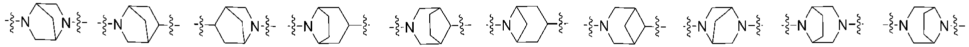 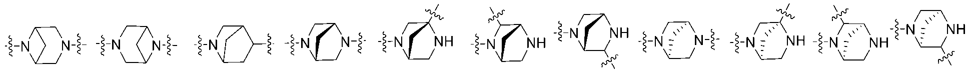 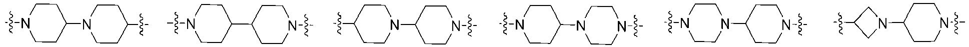 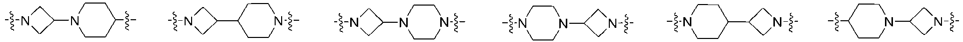 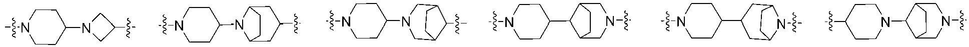 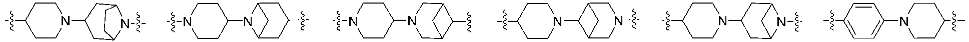 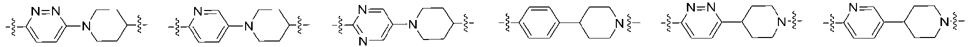 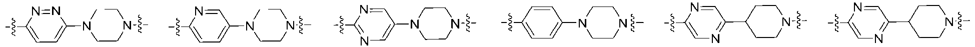 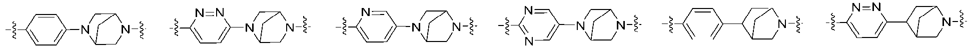 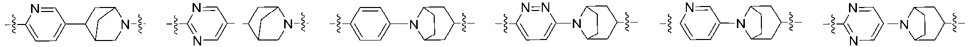 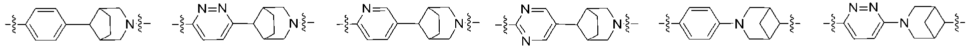 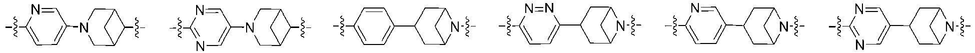 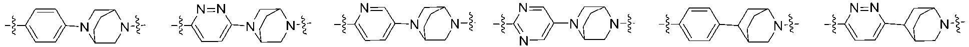 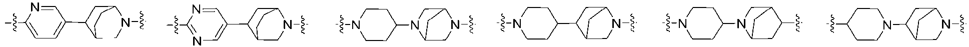 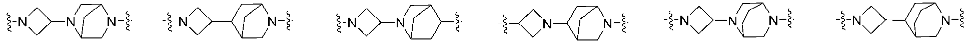 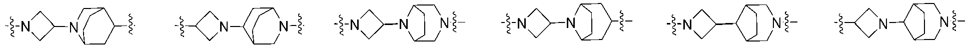 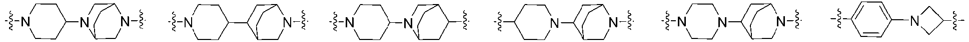 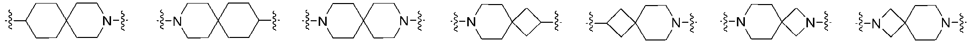 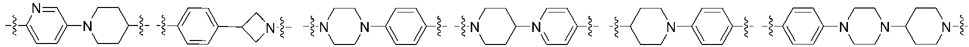 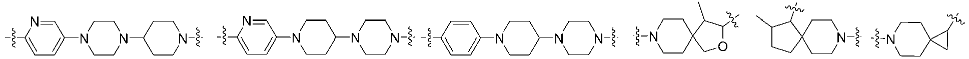 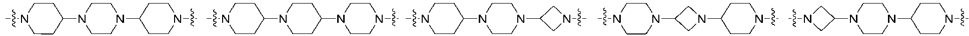 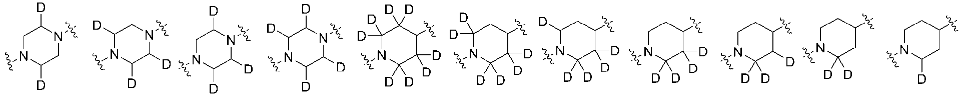 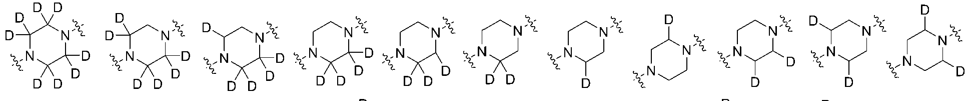 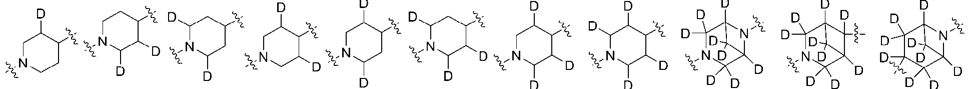 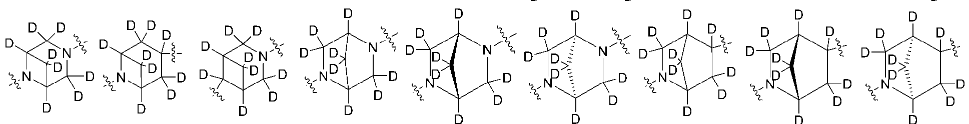 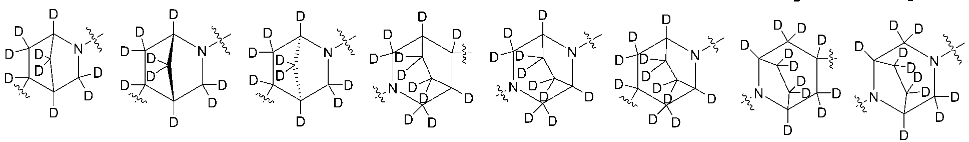 or 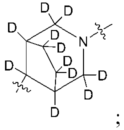 wherein the groups are optionally substituted with a substituent selected from the group consisting of deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy, halogen, amino, hydroxy, mercapto, cyano, carboxyl, C₂₋₆ alkynyl, and C₂₋₆ alkenyl. In the present disclosure, unless otherwise specified, any one end of the general formulas provided above may be connected to R_{w1} of the compound of Formula (I), while the other end is connected to R_{w2}.

In some embodiments, R_{w1} in the general formulae of the LIN of the compound of Formula (I) represents a bond.

In some embodiments, R_{w1} in the general formulae of the LIN of the compound of Formula (I) represents an optionally substituted linear or branched C₁₋₂₀ alkylene, wherein any one or more (e.g., 1-15, 1-10, 1-9, 1-8, 1-7, 1-6, 1-5, 1-4, 1-3, or 1-2, or 1) methylene groups in the main carbon chain skeleton of the linear or branched C₁₋₂₀ alkylene are optionally replaced by one or more (e.g., 1-15, 1-10, 1-9, 1-8, 1-7, 1-6, 1-5, 1-4, 1-3, or 1-2, or 1) groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxyl, amino, mercapto, nitro, halogen, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₃₋₆ cycloalkyl, halogenated C₃₋₆ cycloalkyl, deuterated C₃₋₆ cycloalkyl, 4- to 10-membered heterocyclyl, and any combination thereof; and wherein one or more hydrogens of said linear or branched C₁₋₂₀ alkylene are optionally replaced by a substituent selected from the group consisting of deuterium, hydroxyl, amino, mercapto, nitro, halogen, cyano, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₃₋₆ cycloalkyl, halogenated C₃₋₆ cycloalkyl, deuterated C₃₋₆ cycloalkyl, 4- to 10-membered heterocyclyl, and any combination thereof.

In some embodiments, R_{w1} in the general formulae of the LIN of the compound of Formula (I) represents a structure of the following formulae:
-C₁₋₁₅ alkylene-;
-(C(Rₐ₈)(Rₐ₉))ₙ₄-(Rₐ(C(Rₐ₁₀)(Rₐ₁₁))ₙ₅)ₘ₃-;
-(C(Rₐ₈)(Rₐ₉))ₙ₄-(Rₐ(C(Rₐ₁₀)(Rₐ₁₁))ₙ₅)ₘ₃-(Rₐ(C(Rₐ₁₂)(Rₐ₁₃))ₙ₆)ₘ₄-;
-(C(Rₐ₈)(Rₐ₉))ₙ₄-(Rₐ(C(Rₐ₁₀)(Rₐ₁₁))ₙ₅)ₘ₃-(Rₐ(C(Rₐ₁₂)(Rₐ₁₃))ₙ₆)ₘ₄-(Rₐ(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅Rₐ)ₘ₃-;
-(C(Rₐ₈)(R₄₉))ₙ₉-((C(Rₐ₁₀)(Rₐ₁₁))ₙ₅Rₐ₁₁)ₘ₃-((C(Rₐ₁₂)(Rₐ₁₃))ₙ₆Rₐ)ₘ₄-;
-(C(Rₐ₈)(R₄₉))ₙ₄-((C(Rₐ₁₀)(Rₐ₁₁))ₙ₅Rₐ)ₘ₃-((C(Rₐ₁₂)(Rₐ₁₃))ₙ₆Rₐ)ₘ₄-((C(Rₐ₁₄)(Rₐ₁₅))ₐ₇Rₐ)ₘ₅-;
~(C(Rₐ₈)(Rₐ₉))ₙ₄-(R_{b}(C(Rₐ₁₀)(Rₐ₁₁))ₙ₅)ₘ₃-;
-(C(Rₐ₈)(Rₐ₉))ₙ₄-(R_{b}(C(Rₐ₁₀)(Rₐ₁₁))ₙ₅)ₘ₃-(R_{b}(C(Rₐ₁₂)(Rₐ₁₃))ₙ₆)ₘ₄-;
-(C(Rₐ₈)(Rₐ₉))ₙ₄-(R_{b}(C(Rₐ₁₀)(Rₐ₁₁)ₙ₅)ₘ₃-(R_{b}(C(Rₐ₁₂)(Rₐ₁₃)ₙ₆)ₘ₄-(R_{b}(C(Rₐ₁₄)(Rₐ₁₅))ₙ₇)ₘ₅;
-(C(Rₐ₈)(Rₐ₉))ₙ₄-((C(Rₐ₁₀)(Rₐ₁₁))ₙ₅R_{b})ₘ₃-;
-(C(Rₐs)(Rₐ₉))ₙ₄-((C(Rₐio)(Rₐ₁₁))ₙ₅Re)ₘ₃-((C(Rₐi₂)(Rₐ₁₃))ₙ₆Re)ₘ₄-;
-(C(Rₐ₈)(Rₐ₉))ₙ₄-((C(Rₐ₁₀)(Rₐ₁₁))ₙ₅R_{b})ₘ₃-((C(Rₐ₁₂)(Rₐ₁₃))ₙ₆R_{b})ₘ₄-((C(Rₐ₁₄)(Rₐ₁₅))ₙ₇R_{b})ₘ₅-;
-(C(Rₐ₈)(Rₐ₉))ₙ₄-(Rₐ-R_{b}-(C(Rₐ₁₀)(Rₐ₁₁))ₙ₅)ₘ₃-;
-(C(Rₐ₈)(Rₐ₉))ₙ₄-(Rₐ(C(Rₐ₁₀(Rₐ₁₁))ₙ₅)ₘ₃-(R_{b}(C(Rₐ₁₂)(Rₐ₁₃))ₙ₆)ₘ₄-;
-(C(Rₐ₈)(Rₐ₉))ₙ₄-(R_{b}-Rₐ-(C(Rₐ₁₀)(Rₐ₁₁))ₙ₅)ₘ₃-;
-(C(Rₐ₈)(Rₐ₉))ₙ₄-(R_{b}(C(Rₐ₁₀)(Rₐ₁₁))ₙ₅)ₘ₃-(Rₐ(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁))ₙ₅-Rₐ-R_{b}))ₘ₃-;
-(C(Rₐ₈)(Rₐ₉))ₙ₄-((C(Rₐ)(Rₐ₁₁))ₙ₅Rₐ)ₘ₃-((C(Rₐ₁₂)(Rₐ₁₃))ₙ₆Re)ₘ₄-;
-(C(Rₐ₈)(R₄₉))ₙ₄-((C(Rₐ₁₀)(Rₐ₁₁))ₙ₅-R_{b}-Rₐ)ₘ₃-; or
-(C(Rₐ₈)(Rₐ₉))ₙ₄-((C(Rₐ₁₀)(Rₐ₁₁))ₙ₅R_{b})ₘ₃-((C(Rₐ₁₂)(Rₐ₁₃))ₙ₆Rₐ)ₘ₄-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxyl, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n4, n5, n6, n7, m3, m4, and m5 each independently represent an integer of 1, 2, 3, 4, 5, 6, 7, 8, 9, or 10; and
any one or more hydrogens in the main carbon chain skeleton of said C₁₋₁₅ alkylene are optionally replaced by a substituent selected from the group consisting of: deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof. In the present disclosure, unless otherwise specified, any one end of the general formulas provided above may be connected to Rₐ₆ of the compound of Formula (I), while the other end is connected to ring A¹. The number of carbon atoms of each of the above general formulas, namely the sum of n4+ n5*m3, the sum of n4+ n5*m3+ n6*m4, the sum of n4+ n5*m3+ n6*m4+ n7*m, each independently is an integer less than or equal to 20.

In some embodiments, R_{w1} in the general formulae of the LIN of the compound of Formula (I) represents -C₁₋₁₅ alkylene-, wherein any one or more hydrogens in the main carbon chain skeleton of said C₁₋₁₅ alkylene are optionally replaced by a substituent selected from the group consisting of: deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, R_{w1} in the general formulae of the LIN of the compound of Formula (I) represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, - (CH₂)₁₂-, -(CH₂)₁₃-, -(CH₂)₁₄-, or -(CH₂)₁₅-;
wherein any one or more hydrogens of the groups are optionally replaced by a substituent selected from the group consisting of: deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, R_{w1} in the general formulae of the LIN of the compound of Formula (I) represents a structure of the following formula:
-(C(Rₐ₈)(Rₐ₉))ₙ₄-(O(C(Rₐ₁₀)(Rₐ₁₁))ₙ₅)ₘ₃-;
-(C(Rₐ₈)(Rₐ₉))ₙ₄-(O(C(Rₐ₁₀)(Rₐ₁₁))ₙ₅)ₘ₃-(O(C(Rₐ₁₂)(Rₐ₁₃))ₙ₆)ₘ₄-;
-(C(Rₐ₈)(Rₐ₉))ₙ₄-(O(C(Rₐ₁₀)(Rₐ₁₁))ₙ₅)ₘ₃-(O(C(Rₐ₁₂)(Rₐ₁₃))ₙ₆)ₘ₄- (O(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅O)ₘ₃-;
-(C(Rₐ₈)(Rₐ₉))ₙ₄-((C(Rₐ₁₀)(Rₐ₁₁))ₙ₅O)ₘ₃-((C(Rₐ₁₂)(Rₐ₁₃))ₙ₆O)ₘ₄-;
-(C(Rₐ₈)(Rₐ₉))ₙ₄-((C(Rₐ₁₀)(Rₐ₁₁))ₙ₅O)ₘ₃-((C(Rₐ₁₂)(Rₐ₁₃))ₙ₆O)ₘ₄- ((C(Rₐ₁₄)(Rₐ₁₅))ₙ₇O)ₘ₅-;
-(C(Rₐ₈)(Rₐ₉))ₙ₄-(N(Rₐ₇)(C(Rₐ₁₀)(Rₐ₁₁))ₙ₅)ₘ₃-;
-(C(Rₐ₈)(Rₐ₉))ₙ₄-(N(Rₐ₇)(C(Rₐ₁₀)(Rₐ₁₁))ₙ₅)ₘ₃-(N(Rₐ₇)(C(Rₐ₁₂)(Rₐ₁₃))ₙ₆)ₘ₄-;
-(C(Rₐ₈)(Rₐ₉))ₙ₄-(N(Rₐ₇)(C(Rₐ₁₀)(Rₐ₁₁))ₙ₅)ₘ₃-(N(Rₐ₇)(C(Rₐ₁₂)(Rₐ₁₃))ₙ₆)ₘ₄-; (N(Rₐ₇)(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅N(Rₐ₇))ₘ₃-;
-(C(Rₐ₈)(Rₐ₉))ₙ₄-((C(Rₐ₁₀)(Rₐ₁₁))ₙ₅N(Rₐ₇))ₘ₃-((C(Rₐ₁₂)(Rₐ₁₃))ₙ₆N(Rₐ₇))ₘ₄-;
-(C(Rₐ₈)(Rₐ₉))ₙ₄-((C(Rₐ₁₀)(Rₐ₁₁))ₙ₅N(Rₐ₇))ₘ₃-((C(Rₐ₁₂)(Rₐ₁₃))ₙ₆N(Rₐ₇))ₘ₄-((C(Rₐ₁₄)(Rₐ₁₅))ₙ₇N(Rₐ₇))ₘ₅-;
-(C(Rₐ₈)(Rₐ₉))ₙ₄-(C(O)N(Rₐ₇)-(C(Rₐ₁₀)(Rₐ₁₁))ₙ₅)ₘ₃-;
-(C(Rₐ₈)(Ra₉))ₙ₄-(C(O)N(Rₐ₇)-(C(Rₐ₁₀)(Rₐ₁₁))ₙ₅)ₘ₃-(C(O)N(Rₐ₇)-(C(Rₐ₁₂)(Rₐ₁₃))ₙ₆)ₘ₄-;
-(C(Rₐ₈)(Ra₉))ₙ₄-(C(O)N(Rₐ₇)-(C(Rₐ₁₀)(Rₐ₁₁))ₙ₅)ₘ₃-(C(O)N(Rₐ₇)-(C(Rₐ₁₂)(Rₐ₁₃))ₙ₆)ₘ₄-(C(O)N(Rₐ₇)-(C(Rₐ₁₄)(Rₐ₁₅)ₙ₇)ₘ₅-;
-(C(Rₐ₈)(Rₐ₉))ₙ₄-((C(Rₐ₁₀)(Rₐ₁₁))ₙ₅-C(O)N(Rₐ₇))ₘ₃-;
-(C(Rₐ₈)(Rₐ₉))ₙ₄-((C(Rₐ₁₀)(Rₐ₁₁))ₙ₅-C(O)N(Rₐ₇))ₘ₃-((C(Rₐ₁₂)(Rₐ₁₃))ₙ₆-C(O)N(Rₐ₇))ₘ₄-;
-(C(Rₐ₈)(Rₐ₉))ₙ₄-((C(Rₐ₁₀)(Rₐ₁₁))ₙ₅-C(O)N(Rₐ₇))ₘ₃-((C(Rₐ₁₂)(Rₐ₁₃))ₙ₆-C(O)N(Rₐ₇))ₘ₄-((C(Rₐ₁₄)(Rₐ₁₅))ₙ₇-C(O)N(Rₐ₇))ₘ₅-;
-(C(Rₐ₈)(Rₐ₉))ₙ₄-(C(O)N(Rₐ₇)-(C(Rₐ₁₀)(Rₐ₁₁))ₙ₅)ₘ₃-(O-(C(Rₐ₁₂)(Rₐ₁₃))ₙ₆)ₘ₄-;
-(C(Rₐ₈)(Rₐ₉))ₙ₄-C(O)N(Rₐ₇)-(C(Rₐ₁₀)(Rₐ₁₁))ₙ₅-(O(C(Rₐ₁₂)(Rₐ₁₃))ₙ₆)ₘ₃-;
-(C(Rₐ₈)(Rₐ₉))ₙ₄-((C(Rₐ₁₀)(Rₐ₁₁))ₙ₅-C(O)N(Rₐ₇))ₘ₃-( (C(Rₐ₁₂)(Rₐ₁₃))ₙ₆O)ₘ₄-;
-(C(Rₐ₈)(Rₐ₉))ₙ₄-(C(Rₐ₁₀)(Rₐ₁₁))ₙ₅-C(O)N(Rₐ₇)-((C(Rₐ₁₂)(Rₐ₁₃))ₙ₆O)ₘ₃-;
-(C(Rₐ₈)(Rₐ₉))ₙ₄-(N(Rₐ₇)C(O)-(C(Rₐ₁₀)(Rₐ₁₁))ₙ₅)ₘ₃-;
-(C(Rₐ₈)(Rₐ₉))ₙ₄-(N(Rₐ₇)C(O)-(C(Rₐ₁₀)(Rₐ₁₁))ₙ₅)ₘ₃-(O-(C(Rₐ₁₂)(Rₐ₁₃)))ₙ₆)ₘ₄-;
-(C(Rₐ₈)(Rₐ₉))ₙ₄-(N(Rₐ₇)C(O)-(C(Rₐ₁₀)(Rₐ₁₁))ₙ₅)ₘ₃-(O-(C(Rₐ₁₂)(Rₐ₁₃))ₙ₆)ₘ₄-(O-(C(Rₐ₁₄)(Rₐ₁₅))ₙ₇)ₘ₅-;
-(C(Rₐ₈)(Rₐ₉))ₙ₄-N(Rₐ₇)C(O)-(C(Rₐ₁₀)(Rₐ₁₁)ₙ₅-(O(C(Rₐ₁₂)(Rₐ₁₃)ₙ₆)ₘ₃-;
-(C(Rₐ₈)(Rₐ₉))ₙ₄-N(Rₐ₇)C(O)N(Rₐ₇)-(C(Rₐ₁₀)(Rₐ₁₁))ₙ₅-;
-(C(Rₐ₈)(Rₐ₉))ₙ₄-((C(Rₐ₁₀)(Rₐ₁₁))ₙ₅-N(Rₐ₇)C(O))ₘ₃-;
-(C(Rₐ₈)(Rₐ₉))ₙ₄-((C(Rₐ₁₀)(Rₐ₁₁))ₙ₅-N(Rₐ₇)C(O))ₘ₃-((C(Rₐ₁₂)(Rₐ₁₃)))ₙ₆-O)ₘ₄-;
-(C(Rₐ₈)(Rₐ₉))ₙ₄-((C(Rₐ₁₀)(Rₐ₁₁))ₙ₅-N(Rₐ₇)C(O))ₘ₃-((C(Rₐ₁₂)(Rₐ₁₃)))ₙ₆-O)ₘ₄-((C(Rₐ₁₄)(Rₐ₁₅))ₙ₇O)ₘ₅-;
-(C(Rₐ₈)(Rₐ₉))ₙ₄- (C(Rₐ₁₀)(Rₐ₁₁))ₙ₅-N(Rₐ₇)C(O)-((C(Rₐ₁₂)(Rₐ₁₃))ₙ₆O)ₘ₃-;
-(C(Rₐ₈)(Rₐ₉))ₙ₄-( arylene-(C(Rₐ₁₀)(Rₐ₁₁))ₙ₅)ₘ₃-;
-(C(Rₐ₈)(Rₐ₉))ₙ₄-(arylene-(C(Rₐ₁₀)(Rₐ₁₁))ₙ₅)ₘ₃- arylene-(C(Rₐ₁₂)(Rₐ₁₃))ₙ₆-;
-(C(Rₐ₈)(Rₐ₉))ₙ₄-((C(Rₐ₁₀)(Rₐ₁₁))ₙ₅- arylene)ₘ₃-;
-C(Rₐ₈)(Rₐ₉))ₙ₄-((C(Rₐ₁₀)(Rₐ₁₁))ₙ₅- arylene)ₘ₃-(C(Rₐ₁₂)(Rₐ₁₃))ₙ₆- arylene-;
-(C(Rₐ₈)(Rₐ₉))ₙ₄-(heterocyclylene-(C(Rₐ₁₀)(Rₐ₁₁))ₙ₅)ₘ₃-;
-(C(Rₐ₈)(Rₐ₉))ₙ₄-(heterocyclylene-(C(Rₐ₁₀)(Rₐ₁₁)ₙ₅)ₘ₃-(heterocyclylene-(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁)ₙ₅-heterocyclylene)ₘ₃-;
-(C(Rₐ₈)(Rₐ₉))ₙ₄-((C(Rₐ₁₀)(Rₐ₁₁))ₙ₅-heterocyclylene)ₘ₃-((C(Rₐ₁₂)(Rₐ₁₃))ₙ₆-heterocyclylene)ₘ₄-;
-(C(Rₐ₈)(Rₐ₉))ₙ₄-(heteroarylene-(C(Rₐ₁₀)(Rₐ₁₁))ₙ₅)ₘ₃-;
-(C(Rₐ₈)(Rₐ₉))ₙ₄-(heteroarylene-(C(Rₐ₁₀)(Rₐ₁₁))ₙ₅)ₘ₃-(heteroarylene-(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁))ₙ₅-heteroarylene)ₘ₃-;
-(C(Rₐ₈)(Rₐ₉))ₙ₄-((C(Rₐ₁₀)(Rₐ₁₁))ₙ₅-heteroarylene)ₘ₃-((C(Rₐ₁₂)(Rₐ₁₃))ₙ₆-heteroarylene)ₘ₄-;
-(C(Rₐ₈)(Rₐ₉))ₙ₄-(cycloalkylene-(C(Rₐ₁₀)(Rₐ₁₁))ₙ₅)ₘ₃-;
-(C(Rₐ₈)(Rₐ₉))ₙ₄-(cycloalkylene-(C(Rₐ₁₀)(Rₐ₁₁))ₙ₅)ₘ₃-(cycloalkylene-(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁))ₙ₅-cycloalkylene)ₘ₃-; or
-(C(Rₐ₈)(Rₐ₉))ₙ₄-((C(Rₐ₁₀)(Rₐ₁₁))ₙ₅-cycloalkylene)ₘ₃-((C(Rₐ₁₂)(Rₐ₁₃))ₙ₆-cycloalkylene)ₘ₄-;
wherein each Rₐ₇ independently represents H or C₁₋₃ alkyl;
the cycloalkylene (e.g., C₃₋₂₀ cycloalkylene), arylene (e.g., C₅₋₂₀ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, or any combination thereof; and
n4, n5, n6, n7, m3, m4, and m5 each independently represent an integer of 1, 2, 3, 4, 5, 6, 7, 8, 9, or 10. The total number of carbon atoms of alkylene in each of the above general formulas, namely the sum of n4+ n5*m3, the sum of n4+ n5*m3+ n6*m4, the sum of n4+ n5*m3+ n6*m4+ n7*m5, each independently is an integer less than or equal to 20.

In some embodiments, in the general formula LIN of the compound of Formula (I), examples of cycloalkylene in the general structural formula of R_{w1} include, but are not limited to, C₃₋₂₀ cycloalkylene, C₃₋₁₅ cycloalkylene, and C₃₋₁₁ cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. The cycloalkylene is optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, in the general formula LIN of the compound of Formula (I), examples of arylene in the general structural formula of R_{w1} include, but are not limited to, C₅₋₂₀ arylene, C₆₋₂₀ arylene, C₅₋₁₅ arylene, and C₆₋₁₅ arylene, e.g., phenylene or naphthylene. The arylene is optionally substituted with a substituent selected from the group consisting of deuterium, hydroxyl, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, in the general formula LIN of the compound of Formula (I), examples of heterocyclylene in the general structural formula of R_{w1} include, but are not limited to, 4- to 20-membered , 4-to 15-membered , 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, e.g., azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azepanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and 5- to 20-membered azaspirocycloalkylene. The heterocyclylene is optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, in the general formula LIN of the compound of Formula (I), examples of heteroarylene in the general structural formula of R_{w1} include, but are not limited to, 5- to 20-membered heteroarylene, 5- to 15-membered, 5- to 10-membered, 5- to 9-membered, 5- to 8-membered, 5- to 7-membered, and 5- to 6-membered heteroarylene, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. The heteroarylene is optionally substituted with a substituent selected from the group consisting of deuterium, hydroxyl, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, examples of R_{w1} in the general formulae of the LIN of the compound of Formula (I) include, but are not limited to, the following groups:
-O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, -(CH₂)₅-O-, -(CH₂)₆-O-, -(CH₂)₇-O-, - (CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, - (CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, - (CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, - (CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, - (CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -CH(CH₃)-O-(CH₂)₉-, -CH(CH₃)-O-(CH₂)₁₀-, - (O(CH₂)₂)₂-, -(O(CH₂)₂)₃-, -(O(CH₂)₂)₄-, -(O(CH₂)₂)₅-, -(O(CH₂)₂)₆-, -(O(CH₂)₂)₇-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, - (CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, - (CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)1-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -N(Rₐ₇)-(CH₂)₇-, -N(Rₐ₇)-(CH₂)₈-, -N(Rₐ₇)-(CH₂)₉-, -N(Rₐ₇)-(CH₂)₁₀-, -(CH₂)₂-N(Rₐ₇)-(CH₂)₁-, - (CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, - (CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, - (CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, -C(O)NH(CH₂)₂-, -C(O)NH(CH₂)₃-, - C(O)NH(CH₂)₄-, -C(O)NH(CH₂)₅-, -CH₂C(O)NHCH₂-, -(CH₂)₂C(O)NH(CH₂)₂-, -(CH₂)₂C(O)NH(CH₂)₃-, - (CH₂)₂C(O)NH(CH₂)₄-, -(CH₂)₂C(O)NH(CH₂)₅-, -(CH₂)₃C(O)NH(CH₂)₃-, -(CH₂)₃C(O)NH(CH₂)₄-, - (CH₂)₄C(O)NH(CH₂)₄-, -(CH₂)₅C(O)NH(CH₂)₅-, -(CH₂)₆C(O)NH(CH₂)₇-, -(CH₂)₆C(O)NH(CH₂)₆-, - (CH₂)₇C(O)NH(CH₂)₇-, -(CH₂)₂C(O)NH(CH₂)₂-O-(CH₂)₂-, -NHC(O)CH₂-, -NHC(O)(CH₂)₂-, - NHC(O)(CH₂)₃-, -NHC(O)(CH₂)₄-, -NHC(O)(CH₂)₅-, -CH₂NHC(O)CH₂-, -(CH₂)₂NHC(O)(CH₂)₂-, - (CH₂)₂NHC(O)(CH₂)₃-, -(CH₂)₂NHC(O)(CH₂)₄-, -(CH₂)₂NHC(O)(CH₂)₅-, -(CH₂)₃NHC(O)(CH₂)₃-, - (CH₂)₃NHC(O)(CH₂)₄-, -(CH₂)₄NHC(O)(CH₂)₄-, -(CH₂)₅NHC(O)(CH₂)₅-, -(CH₂)₆NHC(O)(CH₂)₇-, - (CH₂)₆NHC(O)(CH₂)₆-, -(CH₂)₇NHC(O)(CH₂)₇-, -(CH₂)₄NHC(O)(CH₂)₈-, -(CH₂)₂NHC(O)(CH₂)₂-O-(CH₂)₂-, - (CH₂)₄NHC(O)CH₂-, -phenylene-CH₂-, -phenylene-(CH₂)₂-, -phenylene-(CH₂)₃-, -phenylene-(CH₂)₄-, - phenylene-(CH₂)₅-, -phenylene-(CH₂)₆-, -phenylene-(CH₂)₇-, -phenylene-(CH₂)₈-, -CH₂-phenylene-CH₂-, - CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, - (CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, - (CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, -(CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, - (CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, - (CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, - (CH₂)₈-phenylene-(CH₂)₆-, -(CH₂)₈-phenylene-(CH₂)₇-, -N(Rₐ₇)-CH₂-phenylene-CH₂-, -N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, -N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, - CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, - (CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, -(CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, - (CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, - (CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -piperidinylene-CH₂-, -piperidinylene-(CH₂)₂-, -piperidinylene-(CH₂)₃-, -piperidinylene-(CH₂)₄-, -piperidinylene-(CH₂)₅-, -piperidinylene-(CH₂)₆-, -piperidinylene-(CH₂)₇-, -piperidinylene-(CH₂)₈-, - CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)ₐ-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, - (CH₂)₃-piperidinylene-(CH₂)ₐ-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)ₐ-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, -(CH₂)₈-piperidinylene-(CH₂)₇-, - N(Rₐ₇)-CH₂-piperidinylene-CH₂-, -N(Rₐ₇)-CH₂-piperidinylene-(CH₂)₂-, -N(Rₐ₇)-CH₂-piperidinylene-(CH₂)₃-, - N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, -N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇)-CH₂-piperidinylene-(CH₂)₆-, - CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, -piperazinylene-CH₂-, -piperazinylene-(CH₂)₂-, - piperazinylene-(CH₂)₃-, -piperazinylene-(CH₂)₄-, -piperazinylene-(CH₂)₅-, -piperazinylene-(CH₂)₆-, - piperazinylene-(CH₂)₇-, -piperazinylene-(CH₂)₈-, -CH₂-piperazinylene-CH₂-, -CH₂-piperazinylene-(CH₂)₂-, - CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, - (CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene, and piperidinylene are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof. In the present disclosure, unless otherwise specified, any one end of the above groups may be connected to Rₐ₆ of the compound of Formula (I), while the other end is connected to ring A¹. For example, in the group -O-CH₂-, the O may be connected to Rₐ₆ of the compound of Formula (I), and the CH₂ may be connected to A¹ of the compound of Formula (I), and vice versa.

In some embodiments, when R_{w2} and R_{w1} in the general formulae of the LIN of the compound of Formula (I) both represent a bond, and y1, y2, and y3 all represent an integer of 0, then LIN represents a bond (i.e., LIN is absent), and Rₐ₆ of the compound of Formula (I) is directly connected to the R_{c} group of the PBM.

In some embodiments, examples of LIN of the compound of Formula (I) include, but are not limited to, the following groups:
C(O), C(O)NH, NHC(O), -CH₂-, -(CH₂)₂-, -(CH₂)₃-, -(CH₂)₄-, -(CH₂)₅-, -(CH₂)₆-, -(CH₂)₇-, -(CH₂)₈-, - (CH₂)₉-, -(CH₂)₁₀-, -(CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, - (CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, - (CH₂)₁-O-(CH₂)₇-, -(CH₂)₁-O-(CH₂)₈-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₃-O-(CH₂)₁-, - (CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, - (CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, - (CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, - CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -(O(CH₂)₂)₂-, -(O(CH₂)₂)₃-, -(O(CH₂)₂)₄-, -(O(CH₂)₂)₅-, - (O(CH₂)₂)₆-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, - CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, - (CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, - CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, - (CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, (CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, - (CH₂)₁-N(Rₐ₇)-(CH₂)₆-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, - N(Rₐ₇)-(CH₂)₆-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH2)₅-, -(CH₂)₂-N(Rₐ₇)-(CH₂)₆-, -(CH₂)₃-N(Rₐ₇)-(CH₂)₁-, -(CH₂)₃-N(Rₐ₇)-(CH₂)₂-, - (CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)6-N(Rₐ₇)-(CH₂)₁-, -(CH₂)₆-N(Rₐ₇)-(CH₂)₂-, -(CH₂)₆-N(Rₐ₇)-(CH₂)₃-, - (CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, - C(O)NHCH₂-, -C(O)NH(CH₂)₂-, -C(O)NH(CH₂)₃-, -C(O)NH(CH₂)₄-, -C(O)NH(CH₂)₅-, -CH₂C(O)NHCH₂-, - (CH₂)₂C(O)NH(CH₂)₂-, -(CH₂)₂C(O)NH(CH₂)₃-, -(CH₂)₂C(O)NH(CH₂)₄-, -(CH₂)₂C(O)NH(CH₂)₅-, - (CH₂)₃C(O)NH(CH₂)₃-, -(CH₂)₃C(O)NH(CH₂)₄-, -(CH₂)₄C(O)NH(CH₂)₄-, -(CH₂)₅C(O)NH(CH₂)₅-, - (CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, - NHC(O)(CH₂)₅-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₂NHC(O)(CH₂)₂-O-(CH₂)₂-, -(CH₂)₄NHC(O)CH₂-, - CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, - (CH₂)₄-phenylene-(CH₂)₅-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)ₐ-, -(CH₂)₂-piperidinylene-(CH₂)₅-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)ₐ-, -(CH₂)₃-piperidinylene-(CH₂)₅-, - CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -(CH₂)₂-piperazinylene-(CH₂)₁-, -(CH₂)₂-piperazinylene-(CH₂)₂-, - (CH₂)₂-piperazinylene-(CH₂)₃-, -(CH₂)₂-piperazinylene-(CH₂)₄-, -(CH₂)₂-piperazinylene-(CH₂)₅-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)ₐ-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -( CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, or -(CH₂)₈-piperazinylene-(CH₂)₅-;
wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl. In the present disclosure, unless otherwise specified, any one end of the above groups may be connected to Rₐ₆ of the compound of Formula (I), while the other end is connected to R_{c} of PBM.

In some embodiments, examples of LIN of the compound of Formula (I) include, but are not limited to, the following groups: 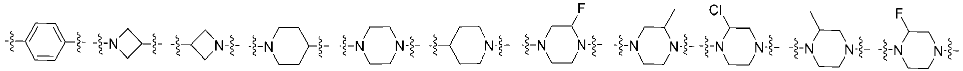 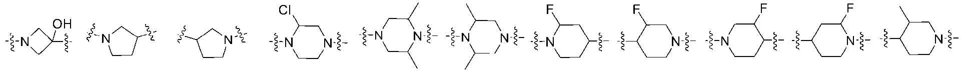 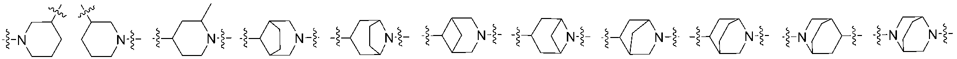 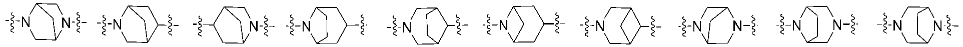 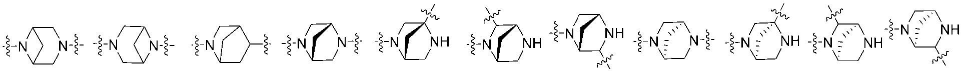 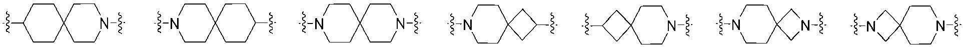 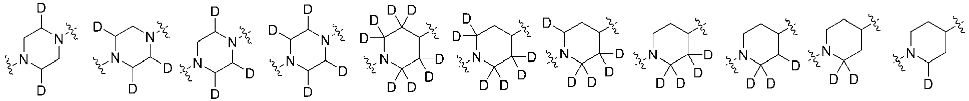 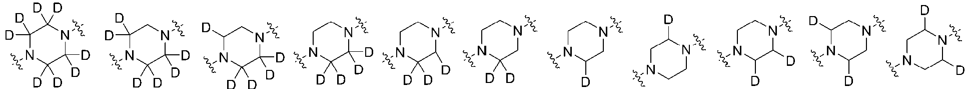 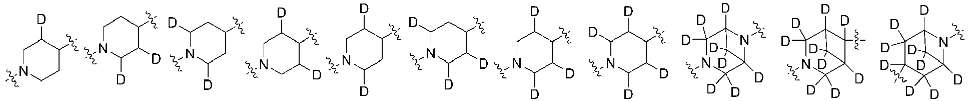 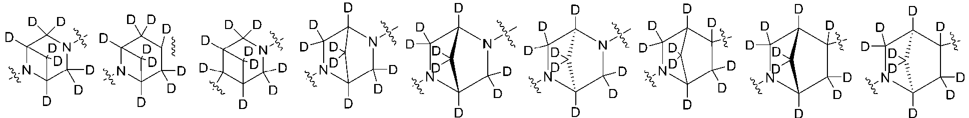 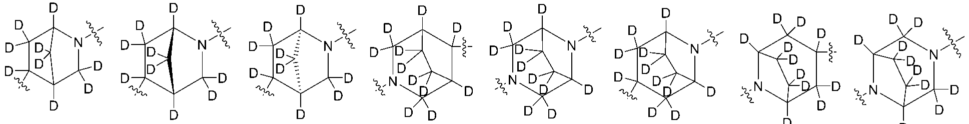 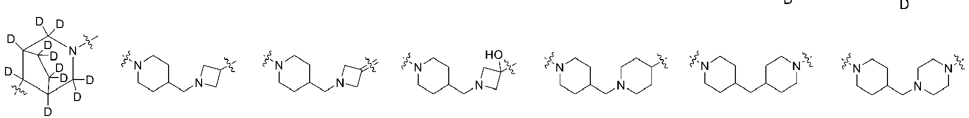 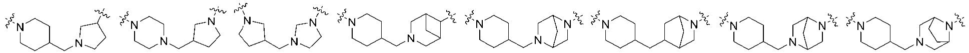 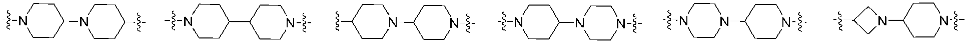 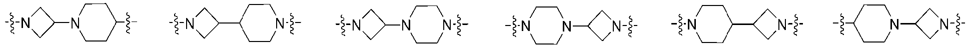 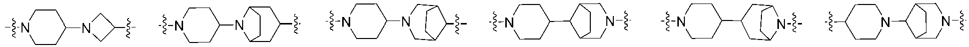 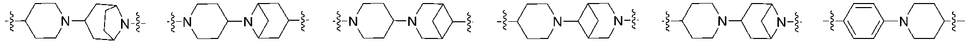 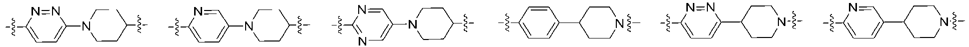 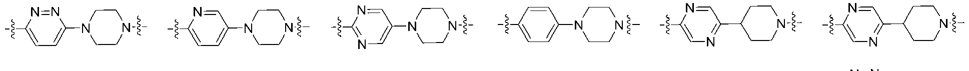 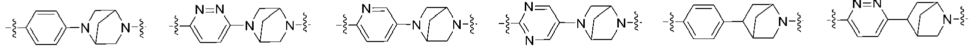 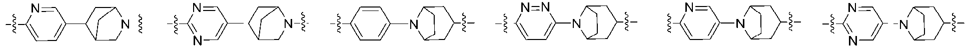 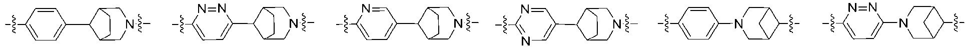 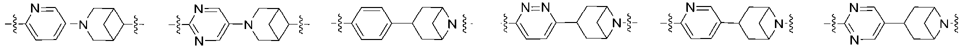 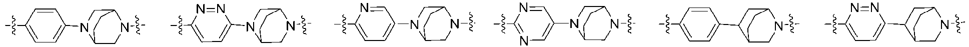 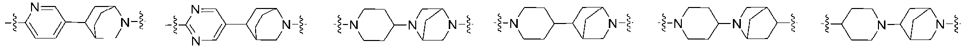 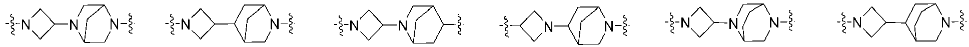 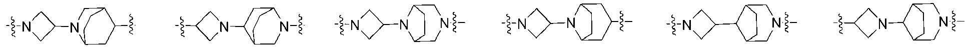 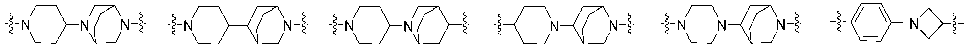 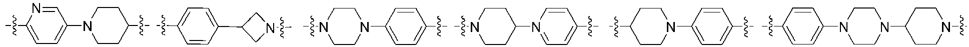 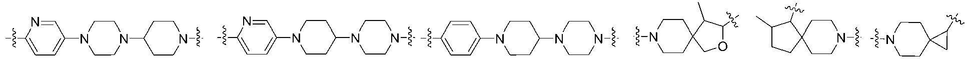 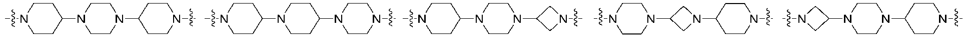 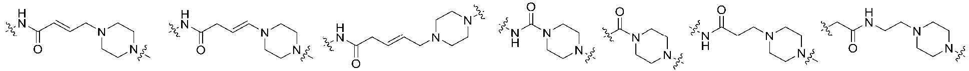 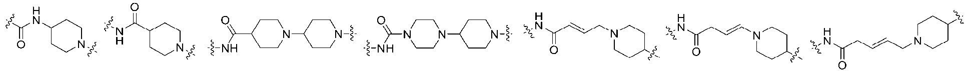 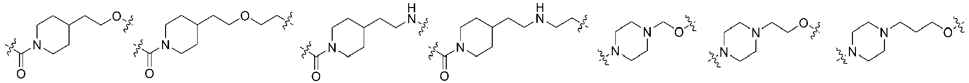 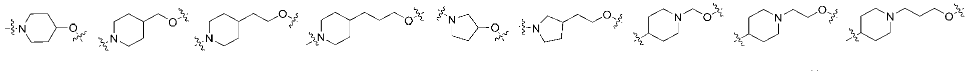 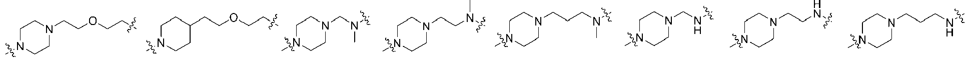 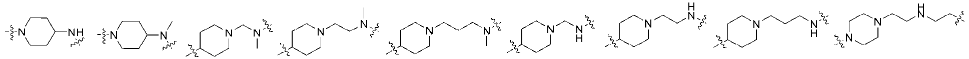 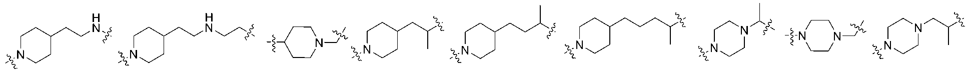 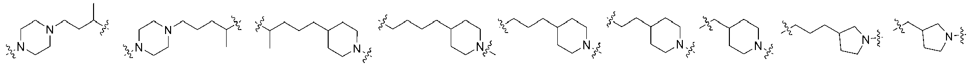 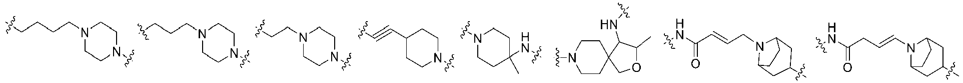 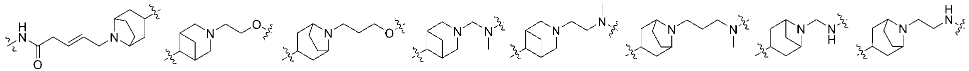 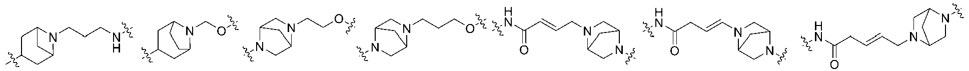 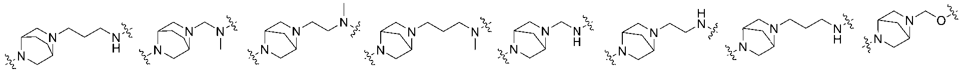 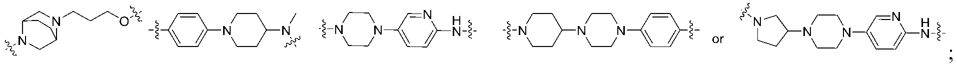 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl. In the present disclosure, unless otherwise specified, any one end of the above groups may be connected to Rₐ₆ of the compound of Formula (I), while the other end is connected to R_{c} of PBM.

In some embodiments, the compound of Formula (I) is also represented by Formula (I-1), Formula (I-2), Formula (I-3), Formula (I-4), Formula (I-5), Formula (I-6), Formula (I-7), or Formula (I-8): 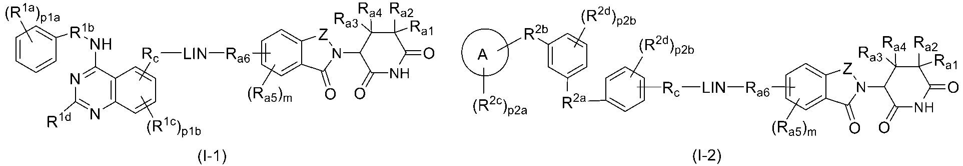 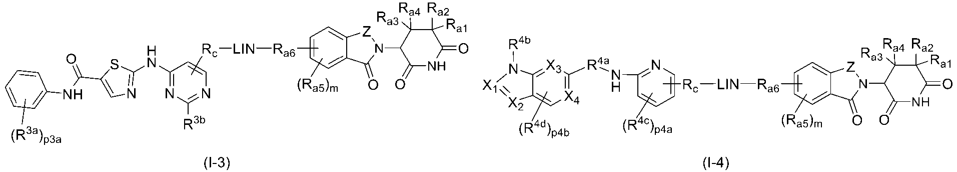 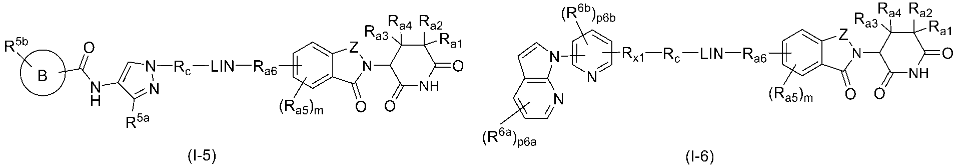 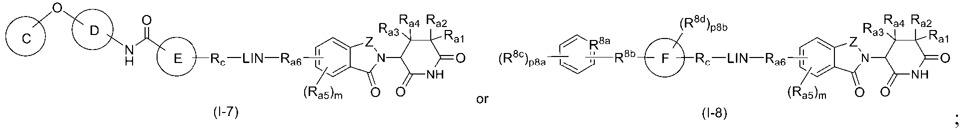 wherein Z, Rₐ₁, Rₐ₂, Rₐ₃, Rₐ₄, (Rₐ₅)ₘ, Rₐ₆, LIN, R_{c}, (R^{1a})ₚ₁ₐ, R^{1b}, (R^{1c})_{p1b}, R^{1d}, ring A, R^{2a}, R^{2b}, (R^{2c})ₚ₂ₐ, (R^{2d})_{p2b}, (R^{3a})ₚ₃ₐ, R^{3b}, X₁, X₂, X₃, X₄, R^{4a}, R^{4b}, (R^{4c})ₚ₄ₐ, (R^{4d})_{p4b}, ring B, R^{5a}, R^{5b}, (R6a) (R^{6b})_{p6b}, Rₓ₁, ring C, ring D, ring E, ring F, R^{8a}, R^{8b}, (R^{8c})ₚ₈ₐ, and (R^{8d})_{p8b} are as defined in the compounds of formula (I) of the present disclosure and the respective sub-embodiments thereof.

In some embodiments, the compounds of Formula (I) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided.

**Table 1. The compounds of the present disclosure**

| Compound No. | Structure of compound | Compound No. | Structure of compound |
|---|---|---|---|
| GT-0976 4 | 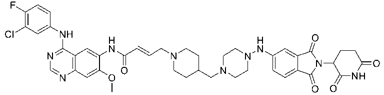 | GT-0695 1 | 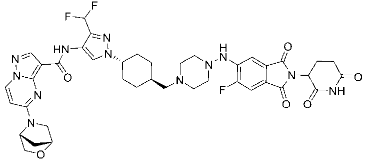 |
| | 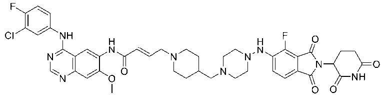 | GT-0960 3 | 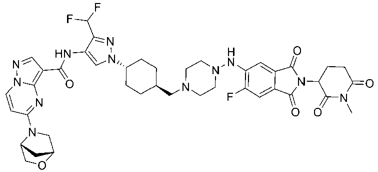 |
| GT-0992 0 | 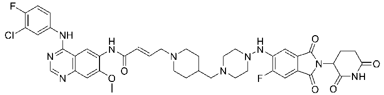 | GT-0954 8 | 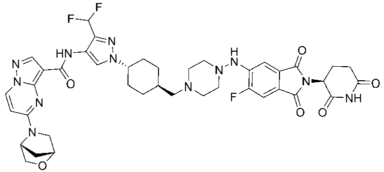 |
| | 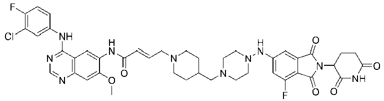 | GT-0954 9 | 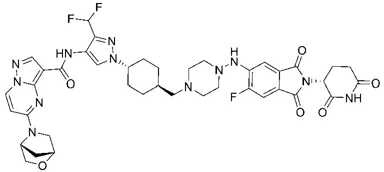 |
| GT-0992 1 | 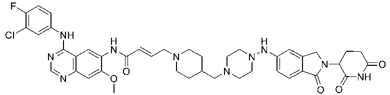 | GT-1002 2 | 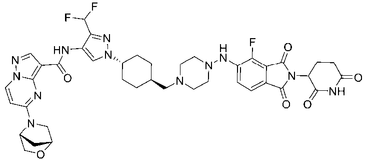 |
| GT-0992 2 | 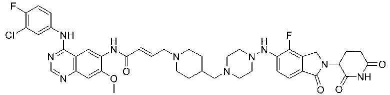 | GT-1004 6 | 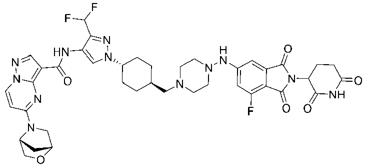 |
| | 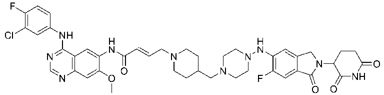 | GT-0818 0 | 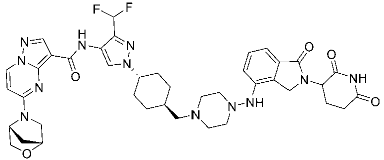 |
| | 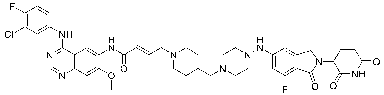 | GT-1004 7 | 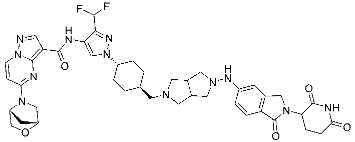 |
| | 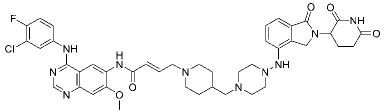 | GT-1004 8 | 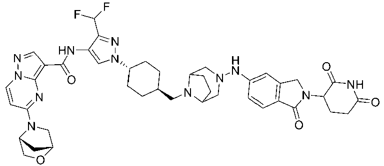 |
| GT-0992 3 | 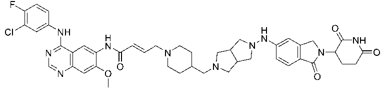 | GT-1004 9 | 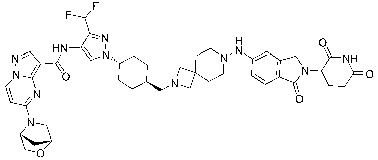 |
| GT-0992 4 | 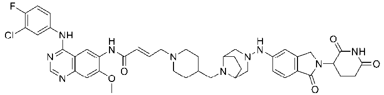 | GT-1005 0 | 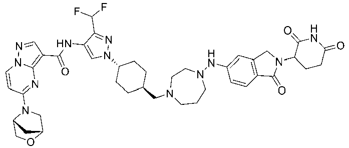 |
| GT-0992 5 | 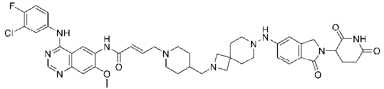 | GT-1005 1 | 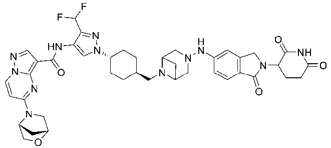 |
| GT-0992 6 | 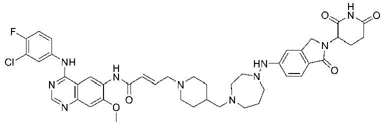 | GT-1005 2 | 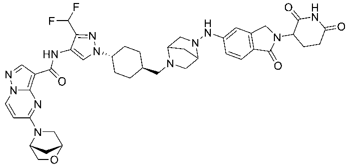 |
| | 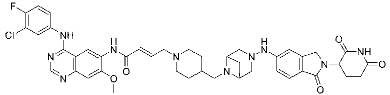 | GT-0976 5 | 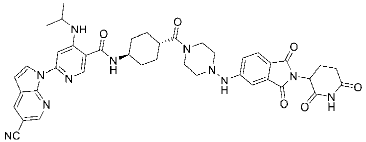 |
| | 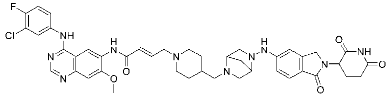 | GT-1002 4 | 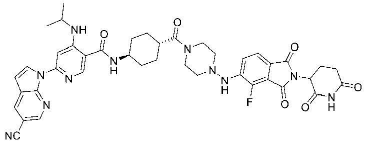 |
| GT-0962 8 | 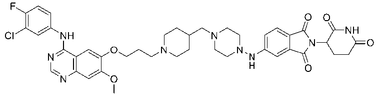 | GT-0976 6 | 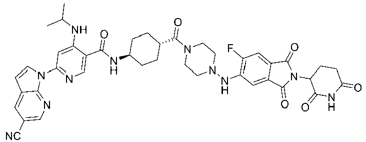 |
| GT-1002 5 | 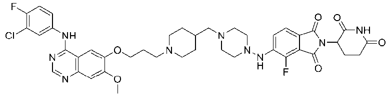 | GT-1005 4 | 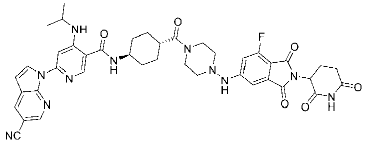 |
| GT-0963 0 | 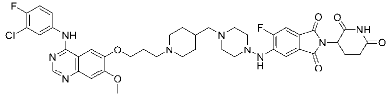 | GT-0976 7 | 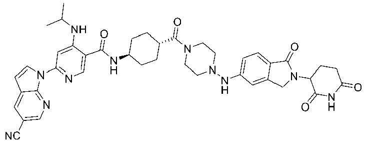 |
| GT-1015 3 | 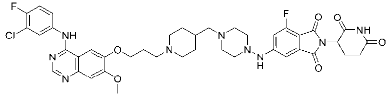 | GT-0976 8 | 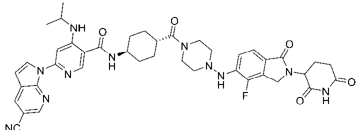 |
| | 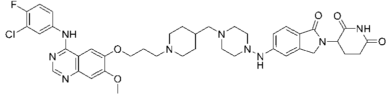 | GT-0976 9 | 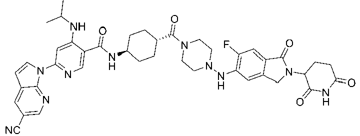 |
| GT-0963 4 | 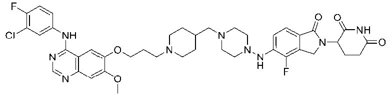 | GT-0977 0 | 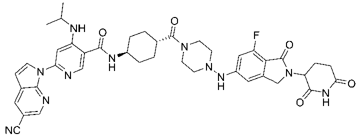 |
| GT-1015 4 | 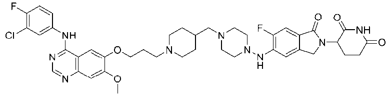 | GT-0977 1 | 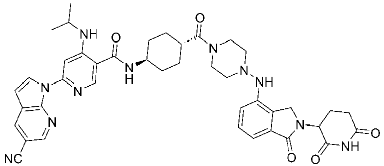 |
| GT-0963 1 | 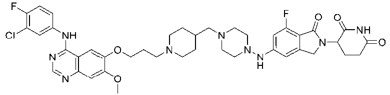 | GT-0977 2 | 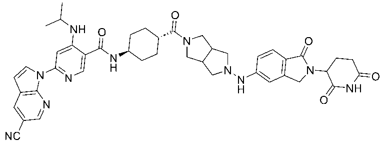 |
| GT-0968 1 | 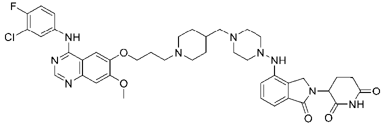 | GT-0977 3 | 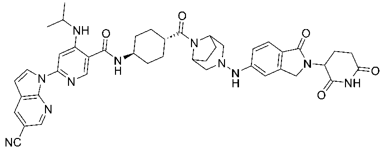 |
| GT-0968 2 | 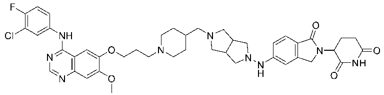 | GT-0977 4 | 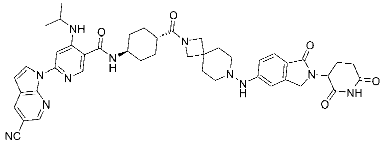 |
| GT-0963 2 | 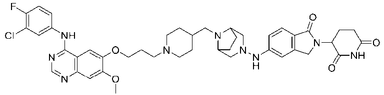 | GT-0977 5 | 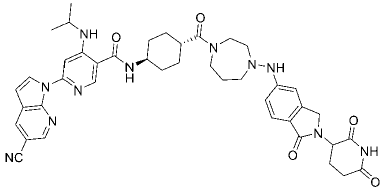 |
| GT-0963 3 | 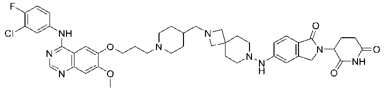 | GT-0977 6 | 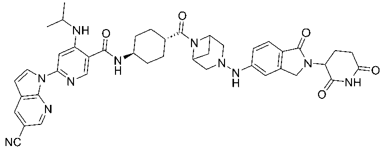 |
| GT-0968 3 | 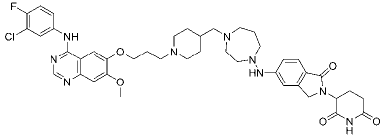 | GT-0977 7 | 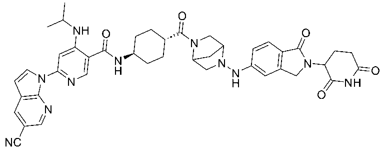 |
| | 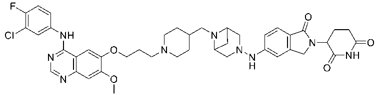 | GT-0534 8 | 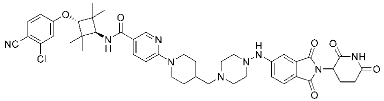 |
| GT-0968 4 | 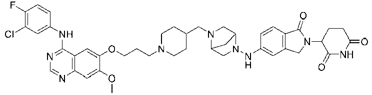 | GT-1002 3 | 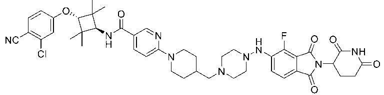 |
| | 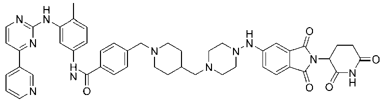 | GT-0694 9 | 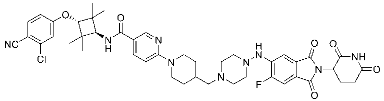 |
| | 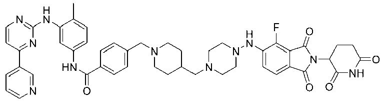 | GT-1005 3 | 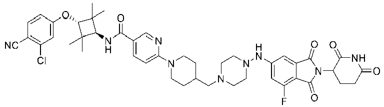 |
| | 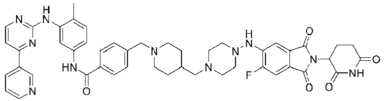 | GT-0709 1 | 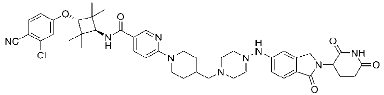 |
| | 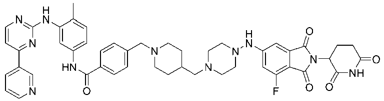 | GT-0710 6 | 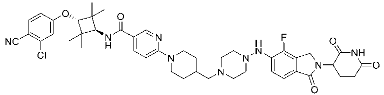 |
| | 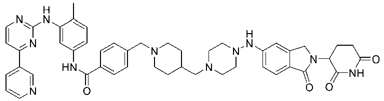 | GT-0710 7 | 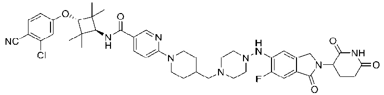 |
| | 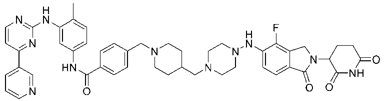 | GT-0710 8 | 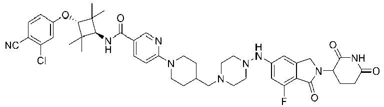 |
| | 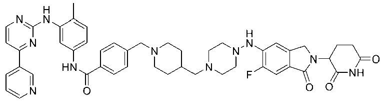 | GT-0818 9 | 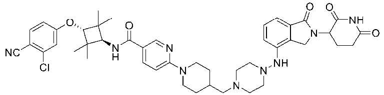 |
| | 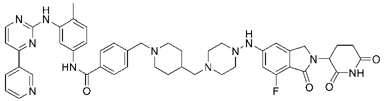 | GT-0992 7 | 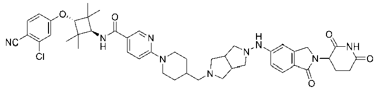 |
| | 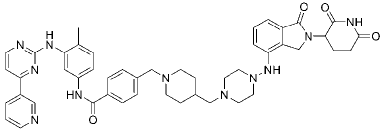 | GT-0992 8 | 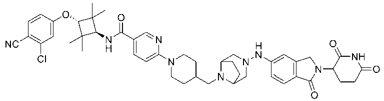 |
| | 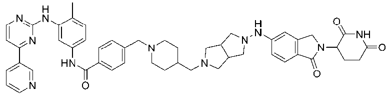 | GT-0992 9 | 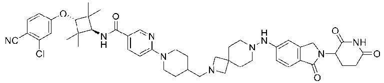 |
| | 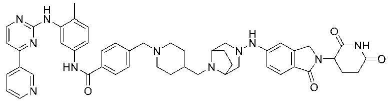 | GT-0993 0 | 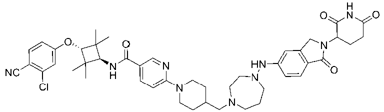 |
| | 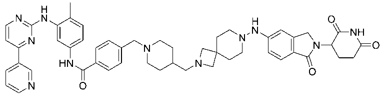 | GT-0993 1 | 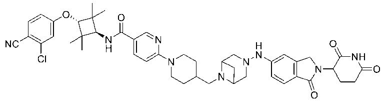 |
| | 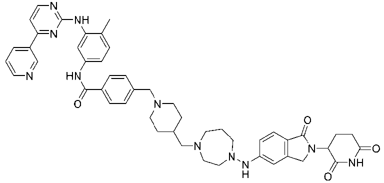 | GT-0993 2 | 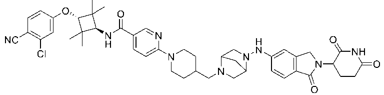 |
| | 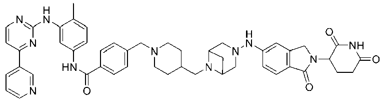 | GT-0523 4 | 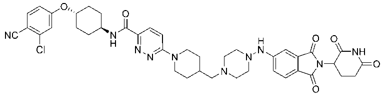 |
| | 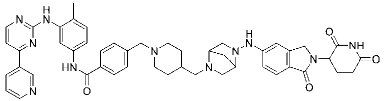 | GT-1002 1 | 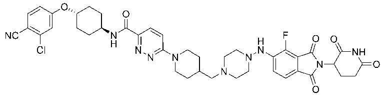 |
| | 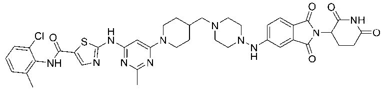 | GT-0695 0 | 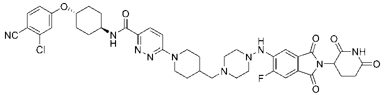 |
| | 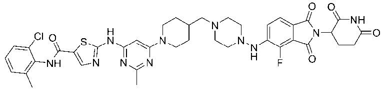 | GT-1003 9 | 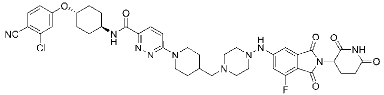 |
| | 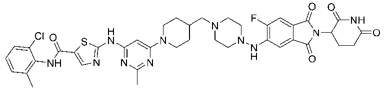 | GT-0709 0 | 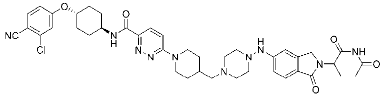 |
| | 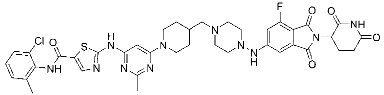 | GT-0719 5 | 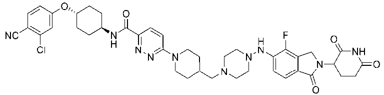 |
| | 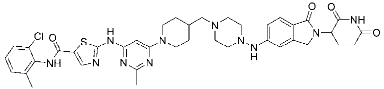 | GT-0719 6 | 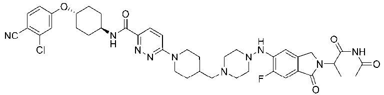 |
| | 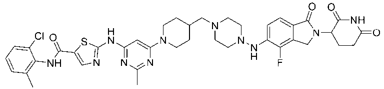 | GT-0710 0 | 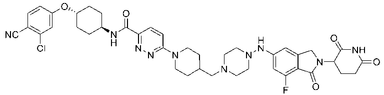 |
| | 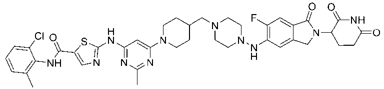 | GT-0817 9 | 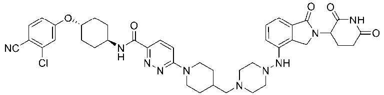 |
| | 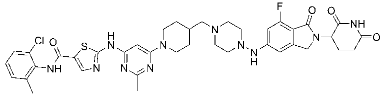 | GT-1004 0 | 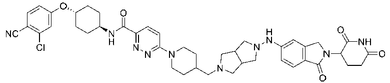 |
| | 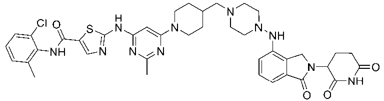 | GT-1004 1 | 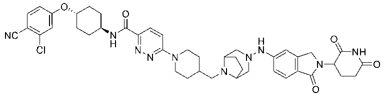 |
| | 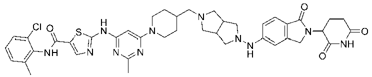 | GT-1004 2 | 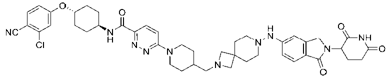 |
| | 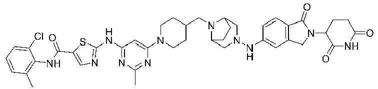 | GT-1004 3 | 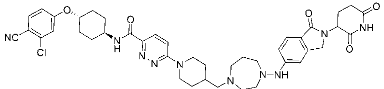 |
| | 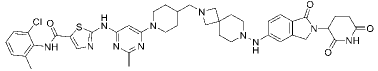 | GT-1004 4 | 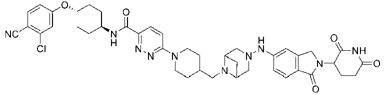 |
| | 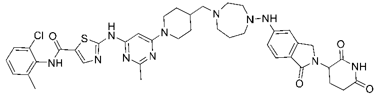 | GT-1004 5 | 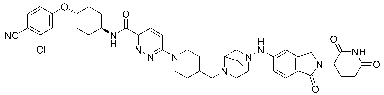 |
| | 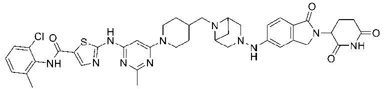 | GT-0710 9 | 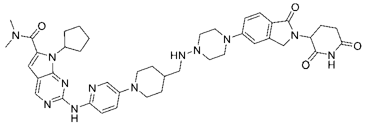 |
| | 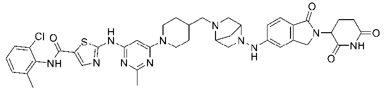 | GT-0724 1 | 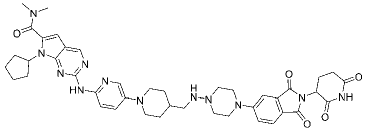 |
| GT-0520 7 | 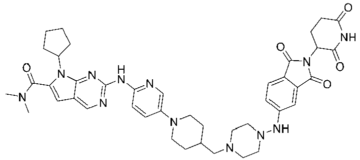 | GT-0709 9 | 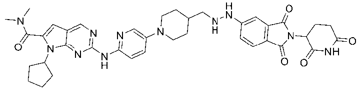 |
| GT-1002 0 | 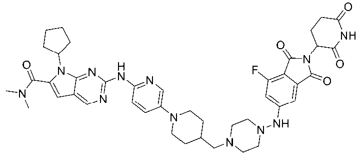 | GT-0710 1 | 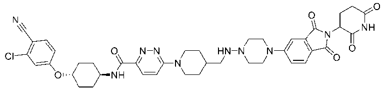 |
| GT-0694 8 | 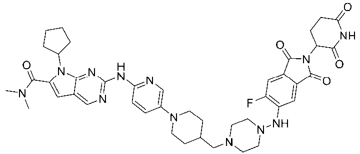 | GT-0711 0 | 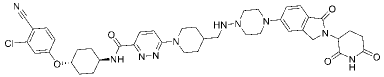 |
| GT-1003 2 | 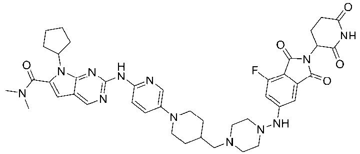 | GT-0709 8 | 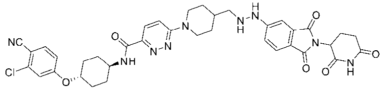 |
| GT-0708 9 | 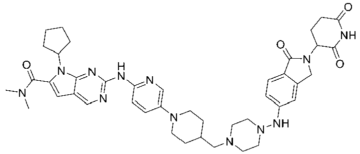 | GT-0711 2 | 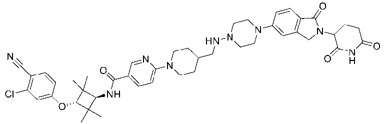 |
| GT-0709 5 | 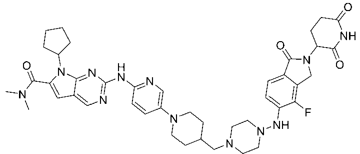 | GT-0717 9 | 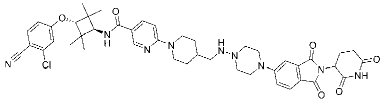 |
| GT-0709 6 | 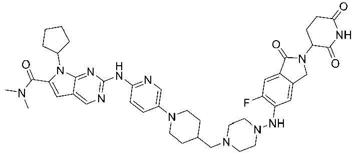 | GT-0718 0 | 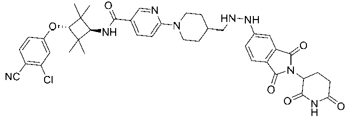 |
| GT-0709 7 | 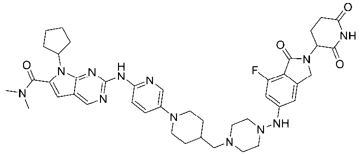 | GT-0711 1 | 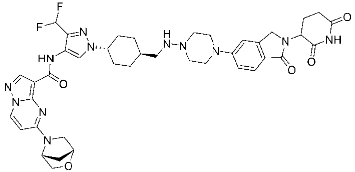 |
| GT-0818 8 | 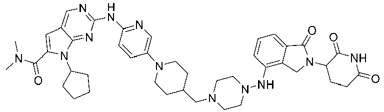 | GT-0739 4 | 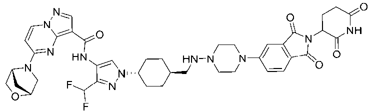 |
| GT-1003 3 | 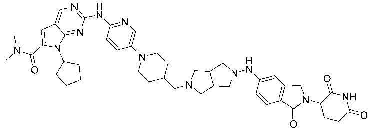 | GT-0739 5 | 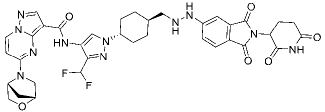 |
| GT-1003 4 | 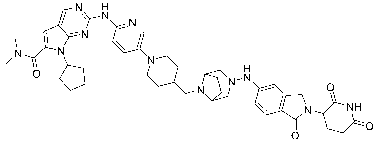 | GT-0719 4 | 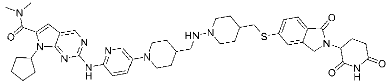 |
| GT-1003 5 | 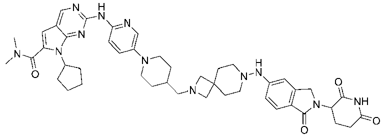 | GT-0710 2 | 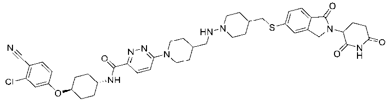 |
| GT-1003 6 | 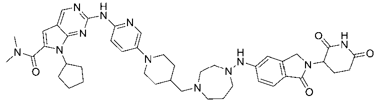 | GT-0739 6 | 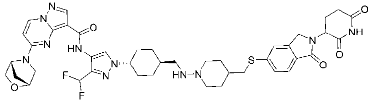 |
| GT-1003 7 | 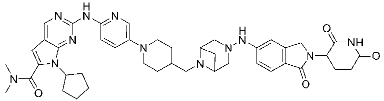 | GT-0718 1 | 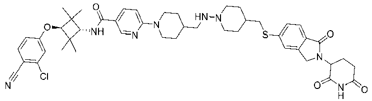 |
| GT-1003 8 | 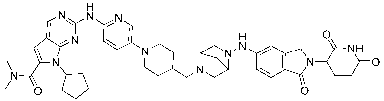 | GT-0694 3 | 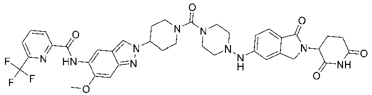 |
| GT-0694 1 | 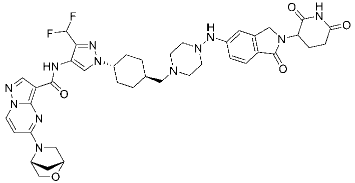 | GT-0694 6 | 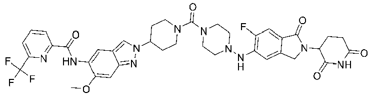 |
| GT-0710 3 | 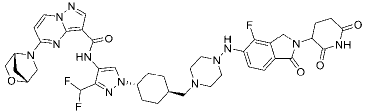 | GT-0694 5 | 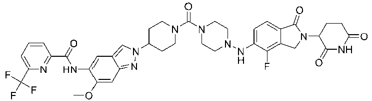 |
| GT-0710 4 | 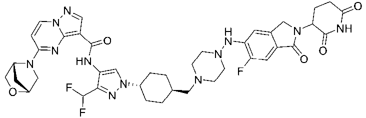 | GT-0694 7 | 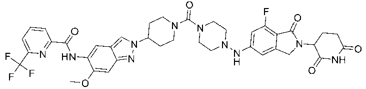 |
| GT-0710 5 | 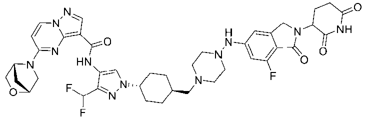 | GT-0712 6 | 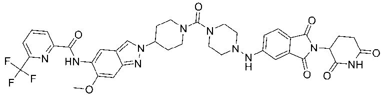 |
| GT-0530 0 | 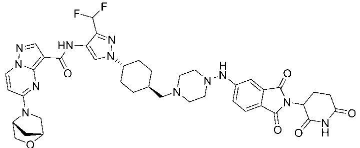 | GT-0694 4 | 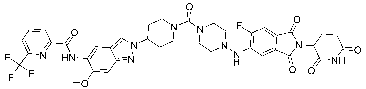 |

### Compound of Formula (II)

The present disclosure provides a compound of Formula (II): 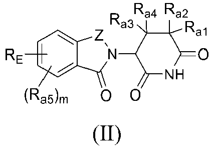 or salts (including pharmaceutically acceptable salts), stereoisomers (including enantiomers), solvates, isotopically enriched analogs, prodrugs, or polymorphs thereof,
wherein Z represents C(O) or CH₂;
Rₐ₁, Rₐ₂, Rₐ₃, and Rₐ₄ are identical or different and each independently represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
m represents an integer of 0, 1, 2, or 3;
R_{E} represents: 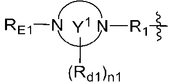
   wherein ring Y¹ represents heterocyclylene containing at least two nitrogen atoms, and (R_{d1})ₙ₁ indicates that ring Y¹ is optionally substituted with n1 R_{d1} groups, wherein each P_{d1} independently represents deuterium, halogen, hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂-etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20; and
   R₁ represents N(R_{w}), where R_{w} represents hydrogen or C₁₋₃ alkyl, and R_{E1} represents hydrogen; or
   R₁ represents a bond, and R_{E1} represents NH₂; or
R_{E} represents: 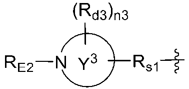
   wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20; and
   wherein Rₛ₁ represents 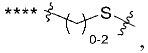 symbol **** indicates the point of attachment to ring Y³; and R_{E2} represents NH₂;
provided that the following compounds are excluded:
   3-(1-oxo-5-(piperazin-1-ylamino)isoindolin-2-yl)piperidine-2,6-dione.

The compounds of Formula (II) of the present disclosure, or their salts (including pharmaceutically acceptable salts), stereoisomers (including enantiomers), solvates, isotope-enriched analogs, prodrugs, or polymorphs, possess CRBN-binding activity and contribute to the recruitment of substrate proteins. They can act as molecular glues binding to the CRBN E3 ubiquitin ligase and can also be applied to bifunctional small-molecule protein degraders. The compounds of Formula (II) of the present disclosure exhibit antitumor activity and/or favorable pharmacokinetic properties.

In some embodiments, Z of the compound of Formula (II) represents C(O).

In some embodiments, Z of the compound of Formula (II) represents CH₂.

In some embodiments, Rₐ₁, Rₐ₂, Rₐ₃, and Rₐ₄ of the compound of Formula (II) are identical or different and each independently represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy.

In some embodiments, Rₐ₁, Rₐ₂, Rₐ₃, and Rₐ₄ of the compound of Formula (II) represent hydrogen.

In some embodiments, m of the compound of Formula (II) represents an integer of 0.

In some embodiments, m of the compound of Formula (II) represents an integer of 1, 2, or 3.

In some embodiments, each Rₐ₅ in the compound of Formula (II) is identical or different and each independently represents halogen (e.g., fluorine, chlorine, bromine, or iodine).

In some embodiments, R_{E} of the compound of Formula (II) represents: 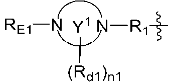 wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20; and
(i) R₁ represents N(R_{w}), where R_{w} represents hydrogen or C₁₋₃ alkyl, and R_{E1} represents hydrogen; or
(ii) R₁ represents a bond, and R_{E1} represents NH₂.

In some embodiments, ring Y¹ in R_{E} of the compound of Formula (II) represents 4- to 20-membered heterocyclylene containing at least two nitrogen atoms. Examples of the 4- to 20-membered heterocyclylene containing at least two nitrogen atoms include, but are not limited to, 4- to 20-membered heterocyclylene containing 2 to 4 nitrogen atoms. Representative examples include: diazetidinylene, imidazolidylene, pyrazolidylene, piperazinylene, 1,2,3,4-tetrahydropyrazinylene, hexahydropyrimidinylene, hexahydropyridazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, diazaspiro[2.3]hexylene, diazaspiro[3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydropyrrolo[3,4-c]pyrrolylene, and decahydronaphthyridinylene, or 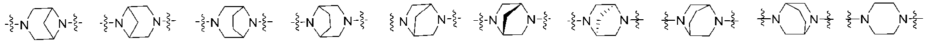 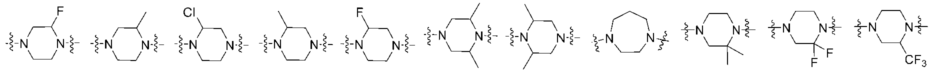 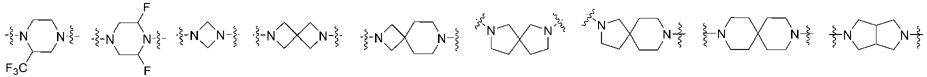 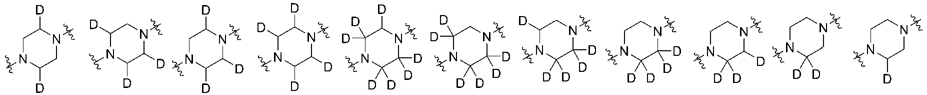 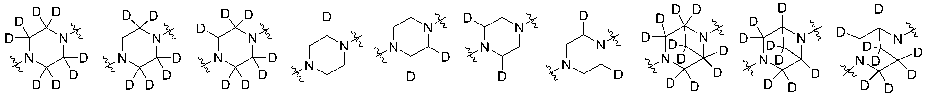 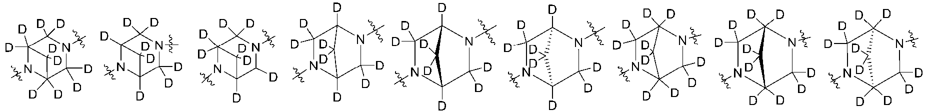 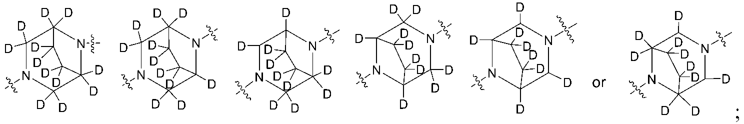
wherein ring Y¹ is optionally substituted with n1 R_{d1} groups, and each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n1 represents an integer of 0-20.

In some embodiments, ring Y¹ in R_{E} of the compound of Formula (II) is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20, e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units.

In some embodiments, R₁ in R_{E} of the compound of Formula (II) represents N(R_{w}), where R_{w} represents hydrogen or C₁₋₃ alkyl (e.g., methyl, ethyl, or propyl), and R_{E1} represents hydrogen.

In some embodiments, when R₁ in R_{E} of the compound of Formula (II) represents N(R_{w}), where R_{w} represents hydrogen or C₁₋₃ alkyl (e.g., methyl, ethyl, or propyl), R_{E1} may represent an amino protecting group, such as tert-butoxycarbonyl (boc) and the like.

In some embodiments, R₁ in R_{E} of the compound of Formula (II) represents a bond, and R_{E1} represents NH₂. In some embodiments, NH₂ represented by R_{E1} may further have a protecting group, such as tert-butoxycarbonyl (boc) and the like.

In some embodiments, R_{E} of the compound of Formula (II) represents: 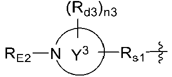
wherein ring Y³ represents heterocyclylene containing one nitrogen atom (e.g., 4- to 20-membered heterocyclylene containing one nitrogen atom), and (R_{d3})ₙ₃ indicates that ring Y³ is optionally substituted with n3 R_{d3} groups, wherein each R_{d3} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20; and
wherein Rₛ₁ represents 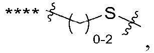 symbol **** indicates the point of attachment to ring Y³; and
R_{E2} represents NH₂.

In some embodiments, ring Y³ in R_{E} of the compound of Formula (II) represents 4- to 20-membered heterocyclylene containing one nitrogen atom (e.g., 4- to 15-membered, 4- to 12-membered, 4- to 11-membered, 4- to 10-membered, 4- to 9-membered, 4- to 8-membered, 4- to 7-membered, 4- to 6-membered, 5-to 15-membered, or 5- to 9-membered heterocyclylene). 4- to 20-membered heterocyclylene containing one nitrogen atom is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered heterocyclylene containing one nitrogen atom include, but are not limited to, e.g., azetidinylene, pyrrolidinylene, piperidinylene, tetrahydropyridylene, azepanylene, azacyclooctylene, azabicyclo[3.1.1]heptanylene, azabicyclo[2.2.1]heptanylene, azabicyclo[3.2.1]octanylene, azabicyclo[2.2.2]octanylene, azaspiro[2.3]hexylene, azaspiro[3.3]heptanylene, azaspiro[2.4]heptanylene, azaspiro[3.4]octanylene, azaspiro[3.5]nonanylene, azaspiro[4.4]nonanylene, azaspiro[4.5]decanylene, azaspiro[5.5]undecanylene, octahydrocyclopenta[c]pyrrolylene, or octahydropyrrolo[3,4-c]pyrrolylene, or 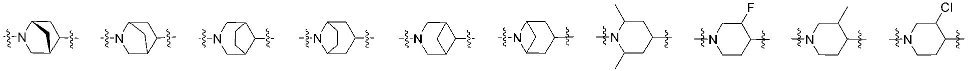 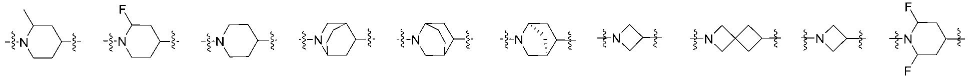 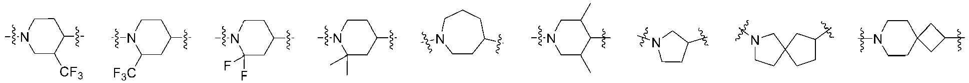 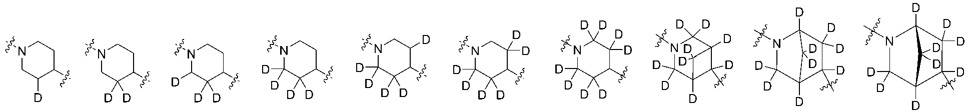 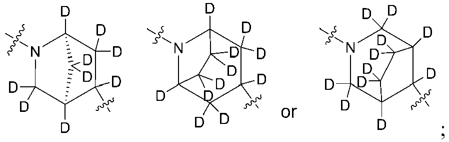
the ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n3 represents an integer of 0-20.

In some embodiments, in R_{E} of the compound of Formula (II), Rₛ₁ represents S, and R_{E2} represents NH₂.

In some embodiments, in R_{E} of the compound of Formula (II), Rₛ₁ represents ****-CH₂-S- or ****-CH₂CH₂-S-, wherein symbol **** indicates the point of attachment to ring Y³, and R_{E2} represents NH₂.

In some embodiments, in the compound of Formula (II), when Z represents CH₂, m represents an integer of 0, R₁ represents N(R_{w}), where R_{w} represents hydrogen, and ring Y¹ represents piperazinyl, ring Y¹ is substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 1-20.

In some embodiments, the compound of Formula (II) is also represented by Formula (II-1), Formula (II-2), Formula (II-3) , or Formula (II-4): 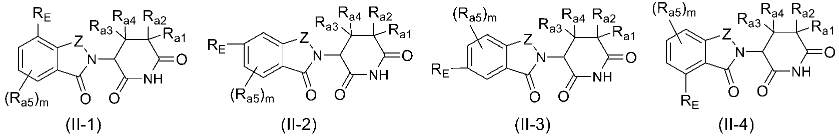
wherein Z represents C(O) or CH₂;
Rₐ₁, Rₐ₂, Rₐ₃, and Rₐ₄ are identical or different and each independently represents hydrogen, deuterium, halogen (e.g., fluorine, chlorine, bromine, or iodine), C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or deuterated C₁₋₆ alkoxy;
m represents an integer of 0, 1, 2, or 3;
R_{E} represents: 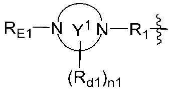
wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0-20 (e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units); and
R₁ represents N(R_{w}), where R_{w} represents hydrogen or C₁₋₃ alkyl, and R_{E1} represents hydrogen; or
R₁ represents a bond, and R_{E1} represents NH₂; or
R_{E} represents: 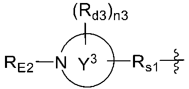
wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n3 represents an integer of 0-20 (e.g., an integer of 0, 1, 2, 3, 4, 5, 6, 7, 8, 9, 10, 11, 12, 13, 14, 15, or 20. The number of substituents is not theoretically limited in any way, or is automatically limited by the size of the building units); and
wherein Rₛ₁ represents 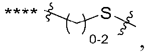 symbol **** indicates the point of attachment to ring Y³; and
R_{E2} represents NH₂;
provided that the following compounds are excluded:
3-(1-oxo-5-(piperazin-1-ylamino)isoindolin-2-yl)piperidine-2,6-dione.

In some embodiments, the compounds of Formula (II) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 2 below are provided.

**Table 2. The compounds of the present disclosure**

| Compound No. | Structure of compound | Compound No. | Structure of compound |
|---|---|---|---|
| GT-05579 | 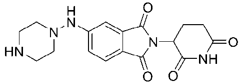 | GT-07051 | 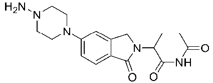 |
| GT-09916 | 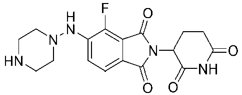 | GT-07008 | 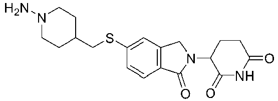 |
| GT-04310 | 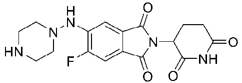 | GT-07965 | 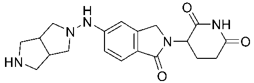 |
| GT-09995 | 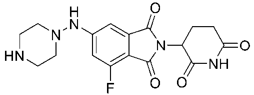 | GT-07966 | 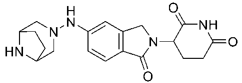 |
| GT-06985 | 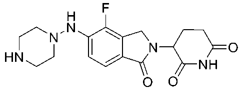 | GT-08006 | 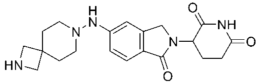 |
| GT-06988 | 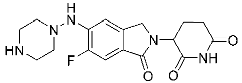 | GT-08007 | 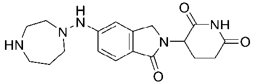 |
| GT-06989 | 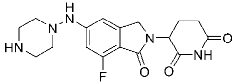 | GT-08008 | 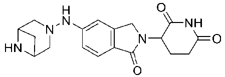 |
| GT-07368 | 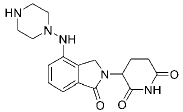 | GT-08009 | 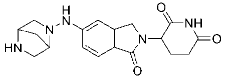 |
| GT-07007 | 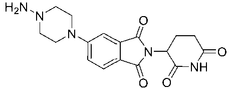 | GT-08195 | 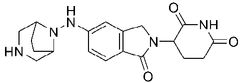 |

### II. Other Forms of Compounds (including salts, enantiomers, stereoisomers, solvates, isotopically enriched analogs, prodrugs, or polymorphs of compounds)

The compounds of the present disclosure have the structures of any one of Formula (I), Formula (I-1), Formula (I-2), Formula (I-3), Formula (I-4), Formula (I-5), Formula (I-6), Formula (I-7), Formula (I-8), Formula (II), Formula (II-1), Formula (II-2), Formula (II-3) , or Formula (II-4). Unless otherwise specified, all references to the compounds of the present disclosure also include compounds of any one of Formula (I), Formula (I-1), Formula (I-2), Formula (I-3), Formula (I-4), Formula (I-5), Formula (I-6), Formula (I-7), Formula (I-8), Formula (II), Formula (II-1), Formula (II-2), Formula (II-3) , or Formula (II-4) and specific compounds within the scope of these general formulae.

It should be recognized that compounds of the present disclosure (including compounds of Formula (I), Formula (I-1), Formula (I-2), Formula (I-3), Formula (I-4), Formula (I-5), Formula (I-6), Formula (I-7), Formula (I-8), Formula (II), Formula (II-1), Formula (II-2), Formula (II-3), or Formula (II-4)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorphs forms or salts of the compounds of the present disclosure (including compounds of Formula (I), Formula (I-1), Formula (I-2), Formula (I-3), Formula (I-4), Formula (I-5), Formula (I-6), Formula (I-7), Formula (I-8), Formula (II), Formula (II-1), Formula (II-2), Formula (II-3) , or Formula (II-4)) are also provided. The salts may be pharmaceutically acceptable salts, including but not limited to hydrohalides (including hydrochlorides, hydrobromides), sulfates, maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate, etc. The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or ²H).

### III. Pharmaceutical Compositions/Formulations

In some embodiments, the present disclosure provides a pharmaceutical composition comprising as active ingredient the compound of the present disclosure (the compound of Formula (I), or the compound of Formula (II)) or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil, and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of the present disclosure (the compounds of Formula (I) or Formula (II)) or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulations and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of the present disclosure (the compounds of Formula (I) or Formula (II)) into conventional, dispersible, chewable, orally disintegrating or rapidly dissolving formulations, or sustained-release capsules or controlled-release capsules as needed.

The compounds of the present disclosure (the compounds of Formula (I) or Formula (II)), as an active ingredient, are contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25 mg per kilogram body weight of the subject per day.

The compounds of the present disclosure (the compounds of Formula (I) or Formula (II)) or pharmaceutically acceptable salts thereof may be conveniently administered in any suitable unit dosage form. Suitable unit dosage form specifications include, but are not limited to, less than 1 mg, 1 mg to 3000 mg, 5 mg to 1000 mg, for example, 5 to 500 mg, 25 to 250 mg of active ingredient per unit dosage form.

### IV. Kits/Packaged Products

The compounds of the present disclosure (the compound of Formula (I) or Formula (II)) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases. In some embodiments, the diseases or disorders include, but are not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, inflammatory bowel disease (including Crohn's disease and ulcerative colitis); keratoconjunctivitis sicca; auto inflammatory diseases, including gout etc.; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for treating and/or preventing a disease or disorder in a subject, comprising administering to the subject a therapeutically effective amount of the compound of Formula (I) or a pharmaceutically acceptable salt thereof, or the pharmaceutical composition of the present disclosure comprising as an active ingredient the compounds of Formula (I) of the present disclosure or a pharmaceutically acceptable salt thereof, wherein the diseases or disorders include, but are not limited to, tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases. In some embodiments, the diseases or disorders include, but are not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, inflammatory bowel disease (including Crohn's disease and ulcerative colitis); keratoconjunctivitis sicca; auto inflammatory diseases, including gout etc.; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

In the method for preventing and/or treating a disease or disorder in a subject, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The compound of Formula (II) of the present disclosure, or a pharmaceutically acceptable salt, solvate, isotopically enriched analog, polymorph, prodrug, stereoisomer (including enantiomer), or mixture of stereoisomers thereof, is a novel CRBN E3 ubiquitin ligase ligand, which can act as a molecular glue binding to the CRBN E3 ligase.

The compound of Formula (II) of the present disclosure, or a pharmaceutically acceptable salt, solvate, isotopically enriched analog, polymorph, prodrug, stereoisomer (including enantiomer), or mixture of stereoisomers thereof, can be used for the preparation of a Cereblon protein (CRBN) binder.

3-(1-oxo-5-(piperazin-1-ylamino)isoindolin-2-yl)piperidine-2,6-dione (GT-04304), or a salt, enantiomer, stereoisomer, solvate, isotopically enriched analog, prodrug, or polymorph thereof can bind to CRBN E3 ligase, and thus can also be used for the preparation of a Cereblon protein (CRBN) binder.

The compound of Formula (II) of the present disclosure, or a pharmaceutically acceptable salt, solvate, isotopically enriched analog, polymorph, prodrug, stereoisomer (including enantiomer), or mixture of stereoisomers thereof, can be used for the preparation of the compound of Formula (I) of the present disclosure. The compound of Formula (I) of the present disclosure can be used for treating and/or preventing a disease or disorder selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

The compound of Formula (II) of the present disclosure or a pharmaceutically acceptable salt, solvate, isotopically enriched analog, polymorph, prodrug, stereoisomer (including enantiomer), or mixture of stereoisomers thereof can be used as a medicament. Especially the compound of Formula (II) of the present disclosure or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder associated with a cereblon protein.

The disease or disorder associated with cereblon protein includes: tumors, cancers, and autoimmune diseases. In some embodiments, the disease or disorder associated with cereblon protein includes, but is not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt' s lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); and autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, and inflammatory bowel disease (including Crohn's disease and ulcerative colitis).

The present disclosure provides a method for preventing and/or treating a disease or disorder associated with cereblon protein in a subject, comprising administering to the subject a therapeutically effective amount of the compounds of Formula (II) of the present disclosure or a pharmaceutically acceptable salt thereof, or the pharmaceutical composition of the present disclosure comprising as an active ingredient the compounds of Formula (II) of the present disclosure or a pharmaceutically acceptable salt thereof. In some embodiments, the disease or disorder associated with cereblon protein includes: tumors, cancers, and autoimmune diseases. In some embodiments, the disease or disorder associated with cereblon protein includes, but is not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt' s lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); and autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, and inflammatory bowel disease (including Crohn's disease and ulcerative colitis).

In the method for preventing and/or treating diseases or disorders associated with cereblon protein, the compound of Formula (II) of the present disclosure or the pharmaceutical composition comprising as an active ingredient the compound of Formula (II) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of the present disclosure (the compounds of Formula (I) or Formula (II)), or a pharmaceutically acceptable salt thereof, or the pharmaceutical composition comprising, as an active ingredient, the compound of the present disclosure (the compounds of Formula (I) or Formula (II)) or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example, mammal, including but not limited to primate (such as human being), cow, sheep, goat, horse, dog, cat, rabbit, guinea pig, rat, mice, etc.

### VI. Preparation Methods

The present disclosure also provides a method for preparing the compound of Formula (I), comprising using a compound of Formula (M1) and a compound of Formula (M2) as starting materials to obtain the compound of Formula (I): 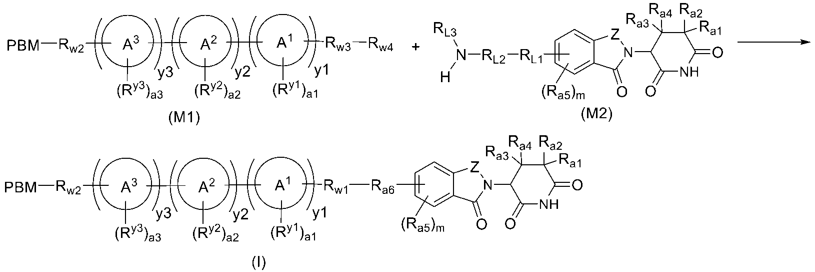
wherein PBM, Rₐ₁, Rₐ₂, Rₐ₃, Rₐ₄, (Rₐ₅)ₘ, Z, R_{w2}, ring A¹, ring A², ring A³, y1, y2, y3, (R^{y1}))ₐ₁, (R^{y2})ₐ₂, and (R^{y3})ₐ₃ are as defined in the compounds of formula (I) of the present disclosure and the respective sub-embodiments thereof;
R_{w3} represents a bond or an optionally substituted linear or branched C₁₋₁₉ alkylene, wherein any one or more methylene groups in the main carbon chain skeleton of the linear or branched C₁₋₁₉ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₁₉ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and wherein one or more hydrogens of said linear or branched C₁₋₁₉ alkylene are optionally replaced by a substituent selected from the group consisting of deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
R_{w4} represents CHO, COOH, or 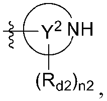 wherein ring Y² and (R_{d2})ₙ₂ are as defined in the compounds of formula (I) of the present disclosure and the respective sub-embodiments thereof;
wherein (1) when R_{w4} represents CHO or COOH,
   (a1) R_{L1} represents N(R_{w}), and R_{L2} and R_{L3}, together with the nitrogen atom to which they are attached, form 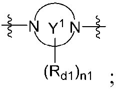 or
   (a2) R_{L1} and R_{L2} together form 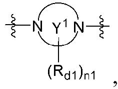 and R_{L3} represents H or C₁₋₃ alkyl; or
   (a3) R_{L1} represents NH, R_{L2} represents a bond, and R_{L3} represents H; or
   (a4) R_{L1} represents 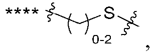 wherein the symbol **** indicates the point of attachment to R_{L2}, R_{L2} represents 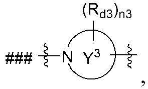 wherein the symbol ### indicates the point of attachment to N, and ring Y³ and (R_{d3})ₙ₃ are as defined in the compounds of formula (I) of the present disclosure and the respective sub-embodiments thereof, and R_{L3} represents H or C₁₋₃ alkyl; or
wherein (2) when R_{w4} represents 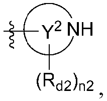 R_{L1} represents N(R_{w}), and R_{L2} and R_{L3}, together with the nitrogen atom to which they are attached, form 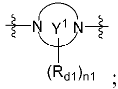 and
wherein each of the above R_{w}, ring Y¹, and (R_{d1})ₙ₁ is as defined in the compounds of formula (I) of the present disclosure and the respective sub-embodiments thereof.

In some embodiments of the method for preparing the compound of Formula (I) in the present disclosure, when R_{w4} represents CHO, the compound of Formula (M1) and the compound of Formula (M2) undergo a reductive amination reaction to prepare the compound of Formula (I), wherein 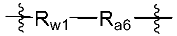 in the compound of Formula (I) correspondingly represents a structure represented by the following general formula:

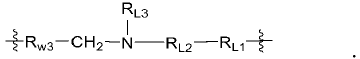

In some embodiments of the method for preparing the compound of Formula (I) in the present disclosure, the reductive amination reaction can be carried out in the presence of sodium triacetoxyborohydride and an organic solvent (e.g., 1,2-dichloroethane, N,N-dimethylformamide, or dichloromethane) at a temperature ranging from room temperature to 80 °C (e.g., 40 °C to 60 °C, 40 °C to 50 °C, or 50 °C to 60 °C). Alternatively, the reductive amination reaction can be carried out in the presence of sodium borohydride and 1,4-dioxane at a temperature ranging from room temperature to 80 °C (e.g., 40 °C to 60 °C, 40 °C to 50 °C, or *50* °C to 60 °C). In some embodiments, the molar ratio of the compound of Formula (M1) to the compound of Formula (M2) can be, for example, from 1.0 to 2:1, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) in the present disclosure, when R_{w4} represents COOH, the compound of Formula (M1) and the compound of Formula (M2) undergo an amide condensation reaction to prepare the compound of Formula (I), wherein 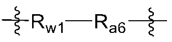 in the compound of Formula (I) correspondingly represents a structure represented by the following general formula:

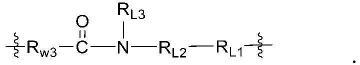

In some embodiments of the method for preparing the compound of Formula (I) in the present disclosure, the amide condensation reaction can be carried out in the presence of HATU/DIEA/DMF, or HOAt/EDCI/TEA/DCM at room temperature. In some embodiments, the molar ratio of the compound of Formula (M1) to the compound of Formula (M2) can be, for example, from 1.0 to 2:1, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) in the present disclosure, when R_{w4} represents 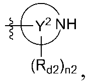 the compound of Formula (M1) and the compound of Formula (M2) undergo a urea formation reaction to prepare the compound of Formula (I), wherein 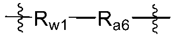 in the compound of Formula (I) correspondingly represents a structure represented by the following general formula:

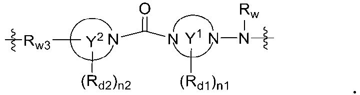

In some embodiments of the method for preparing the compound of Formula (I) in the present disclosure, the urea formation reaction can be carried out by adding triphosgene to a mixture of the compound of Formula (M1) and N,N-diisopropylethylamine at room temperature, followed by the addition of the compound of Formula (M2). In some embodiments, the molar ratio of the compound of Formula (M1) to the compound of Formula (M2) can be, for example, from 1.0 to 2:1, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) in the present disclosure, when the groups contain an active hydrogen or a reactive group that is not intended to participate in the scheme reaction, it can be protected by techniques and methods well known to those skilled in the art, such as using protecting groups for the active hydrogen or reactive group. For example, when the reactive group not intended to participate in the reaction scheme is piperidinyl or piperazinyl, conventional protecting groups such as Boc and the like can be used to protect the hydrogen on the nitrogen. For example, when the reactive group not intended to participate in the reaction scheme is -CHO, conventional protecting groups can be used to protect the aldehyde group, such as forming an acetal. The deprotection of the protecting groups can be achieved by techniques and methods well known to those skilled in the art. For example, the removal of the Boc protecting group can be achieved under acidic conditions, such as hydrochloric acid or trifluoroacetic acid. As another example, the removal of the acetal protecting group can be achieved under acidic conditions, such as sulfuric acid, hydrochloric acid, or trifluoroacetic acid.

The present disclosure also provides a method for preparing the compound of Formula (II), comprising using a compound of Formula (M3) and a compound of Formula (M4) as starting materials to obtain the compound of Formula (II): 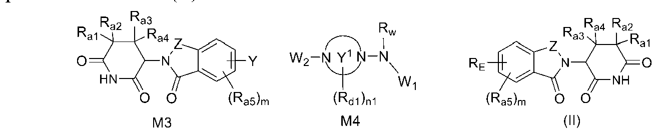
wherein Rₐ₁, Rₐ₂, Rₐ₃, Rₐ₄, (Rₐ₅)ₘ, Z, ring Y¹, R_{w}, and (R_{d1})ₙ₁ are as defined in the compounds of formula (II) of the present disclosure and the respective sub-embodiments thereof;
Y represents F or Br;
W₁ represents H, and W₂ represents an amino protecting group; or
W₁ represents an amino protecting group, and W₂ represents H; and
R_{E} represents: 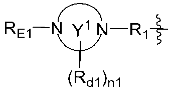
   wherein ring Y¹ represents heterocyclylene containing at least two nitrogen atoms, and (R_{d1})ₙ₁ indicates that ring Y¹ is optionally substituted with n1 R_{d1} groups, wherein each P_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
   R₁ represents N(R_{w}), where R_{w} represents hydrogen or C₁₋₃ alkyl, and R_{E1} represents hydrogen; or
   R₁ represents a bond, and R_{E1} represents NH₂.

In some embodiments of the method for preparing the compound of Formula (II) in the present disclosure, when Y represents F, the compound of Formula (M3) and the compound of Formula (M4) undergo a palladium-catalyzed coupling reaction to prepare the compound of Formula (II). In some embodiments of the method for preparing the compound of Formula (II) in the present disclosure, the palladium-catalyzed coupling reaction can be carried out in the presence of cesium carbonate, a palladium catalyst, and N,N-dimethylformamide at a temperature of, for example, 50 °C to 100 °C (e.g., 80 °C).

In some embodiments of the method for preparing the compound of Formula (II) in the present disclosure, when Y represents Br, the compound of Formula (M3) and the compound of Formula (M4) undergo a substitution reaction to prepare the compound of Formula (II). In some embodiments of the method for preparing the compound of Formula (II) in the present disclosure, the substitution reaction can be carried out in the presence of N,N-diisopropylethylamine and dimethyl sulfoxide at a temperature of, for example, 80 °C to 150 °C (e.g., 130 °C).

In some embodiments of the method for preparing the compound of Formula (II) in the present disclosure, the amino protecting group can be, for example, Boc and the like. The deprotection of the protecting groups can be achieved by techniques and methods well known to those skilled in the art. For example, the removal of the Boc protecting group can be achieved under acidic conditions, such as hydrochloric acid or trifluoroacetic acid.

### VII. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R₂ represents a bond" means that R₂ is a bond linker. In other words, when R₂ represents a bond, ring Y¹ in Rₐ₆ is directly bonded to LIN. For example, the wording "LIN represents a bond" means that LIN is a bond linker. In other words, when LIN represents a bond, the PBM in the compound of Formula (I) is directly linked to the Rₐ₆ in the structure of Formula (ULM).

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

As used herein, the term "replaced with" in the expression "any one or more methylene groups in the main carbon chain skeleton of a linear or branched C₁₋₂₀ alkylene are optionally be replaced by one or more groups selected from: Rₐ, R_{b}, and any combination thereof" used alone or in combination, has a definition known in the art, namely, it may refer to one or more groups Rₐ, R_{b}, or any combination of groups Rₐ and R_{b} optionally replacing any one or more methylene groups in the main carbon chain skeleton of a C₁₋₂₀ alkylene. Herein, examples of the above-mentioned expression may include, but are not limited to, that any one or more (e.g., 1-20 or 1-15, 1-10, 1-9, 1-8, 1-7, 1-6, 1-5, 1-4, 1-3, or 1-2, or 1) methylene groups in the main carbon chain skeleton of C₁₋₂₀ alkylene optionally are replaced by one or more (e.g., 1-20 or 1-15, 1-10, 1-9, 1-8, 1-7, 1-6, 1-5, 1-4, 1-3, or 1-2, or 1) groups Rₐ as defined herein, and/or one or more (e.g., 1-20 or 1-15, 1-10, 1-9, 1-8, 1-7, 1-6, 1-5, 1-4, 1-3, or 1-2, or 1) groups R_{b}, and/or one or more (e.g., 1-20 or 1-15, 1-10, 1-9, 1-8, 1-7, 1-6, 1-5, 1-4, 1-3, or 1-2, or 1) of any combination of groups Rₐ and R_{b}, and the resulting main chain skeleton group conforms to the covalent bond theory. When two or more methylene groups in the main carbon chain skeleton of the linear or branched C₁₋₂₀ alkylene are replaced by two or more groups Rₐ, the respective groups Rₐ are not directly connected to each other. When any one or more methylene groups in the main carbon chain skeleton of the linear or branched C₁₋₂₀ alkylene are replaced by one or more groups Rₐ, and/or one or more groups R_{b}, and/or one or more of any combination of groups Rₐ and R_{b}, the resulting main chain skeleton group may contain one or more (e.g., 1-20 or 1-15, 1-10, 1-9, 1-8, 1-7, 1-6, 1-5, 1-4, 1-3, or 1-2, or 1) of the following fragments, such as but not limited to: "-(CH₂)₀₋₅-Rₐ-(CH₂)₀₋₅-" fragment, "-Rₐ-(CH₂)₁₋₅-Rₐ-" fragment, "-(CH₂)₀₋₅-(R_{b})₀₋₅-(CH₂)₀₋₅-" fragment, "-(R_{b})₀₋₅-(CH₂)₀₋₅-(R_{b})₀₋₅-" fragment, "-(CH₂)₀₋₅-Rₐ-(R_{b})₀₋₅-(CH₂)₀₋₅-" fragment, "-Rₐ-(CH₂)₀₋₅-(R_{b})₀₋₅-(CH₂)₀₋₅-" fragment, "-(CH₂)₀₋₅-Rₐ-(CH₂)₀₋₅-(R_{b})₀₋₅-" fragment etc., wherein each Rₐ is the same or different, each R_{b} is the same or different, and as defined herein.

Herein, a bond interrupted by a wavy line shows the point of attachment of the depicted group to the rest of the molecule. For example, the group of Formula (ULM) depicted below 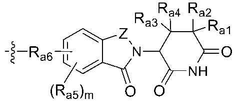 shows the point of attachment of Rₐ₆ in said group to LIN of the compound of Formula (I). Herein, when a depicted group has two bonds interrupted by a wavy line, for example, when ring A² represents 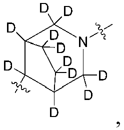 unless otherwise specified, it indicates that either end (e.g., nitrogen atom) of said group may be connected to ring A¹, while the other end is connected to ring A³ in LIN.

As used herein, the expression "one or more hydrogens of the C_{x-y} alkylene are replaced by...", used alone or in combination, means that any one or more hydrogens of the linear or branched C_{x-y} alkylene is replaced by a substituent(s) as defined herein. In other words, the C_{x-y} alkylene optionally has one or more substituents as defined herein. Herein, the term "one or more" may refer to part or all hydrogen atoms of each referenced alkylene group, including but not limited to 1-40 hydrogens, e.g., 1-30, such as 1-25, 1-20, 1-15, 1-10, 1-5, 1-4, 1-3, 1-2 or 1 hydrogen atoms. The number of hydrogens to be replaced is in principle not limited in any way, or is automatically limited by the size of the building unit.

As used herein, the term "deuterated" used alone or in combination, indicates that one or more hydrogen atoms in the referenced group are replaced by deuterium atoms.

As used herein, the term "oxo" or "oxo group" used alone or in combination, refers to = O.

As used herein, the term "C(O)" or "C(=O)" used alone or in combination, refers to a carbonyl group.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, C₁₋₄ alkyl, and C₁₋₃ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, C₁₋₃ alkyl, C₁₋₃ alkoxy, trifluoromethyl, and 4- to 15-membered heterocyclyl.

As used herein, the term "halogenated alkyl", used alone or in combination, refers to a linear or branched alkyl group substituted with one or more halogens, wherein one or more hydrogen atom(s) of the alkyl group are replaced with one or more halogens. The term "halogenated Cₓ₋C_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present disclosure refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated C₁₋₃ alkyl" or "halogenated C₁₋C₃ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated Cₓ₋C_{y} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present disclosure refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. Examples of the C₁-C₂₀ alkylene in the present disclosure may include, but are not limited to C₁-C₂₀ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene, C₁-C₉ alkylene, C₁-C₈ alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, and pentadecylene. In the present disclosure, the "alkylene" is optionally substituted with one or more (e.g., 1-10, 1-6, 1-5, 1-4, or 1-3) substituents optionally selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, and cyano.

As used herein, the term "alkoxy", used alone or in combination, refers to a linear or branched alkoxy group having structural formula of alkyl-O-. Optionally, the alkyl portion of the alkoxy group may contain 1-10 (e.g., 1-6, 1-4, or 1-3) carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "halogenated alkoxy", used alone or in combination, refers to an alkoxy group substituted with one or more halogen atoms. Optionally, the alkyl portion of the alkoxy group may contain 1-10 (e.g., 1-6, 1-4, or 1-3) carbon atoms. Examples of "halogenated alkoxy" include, but are not limited to, halogenated C₁₋₆ alkoxy or halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-.

As used herein, the term "alkylamino", used alone or in combination, refers to a linear or branched alkylamino group having structural formula of alkyl-NH-. Optionally, the alkyl portion of the alkylamino group may contain 1-10 (e.g., 1-6, 1-4, or 1-3) carbon atoms. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "C₁-C₃ alkyl-NH-" or "C₁₋₃ alkyl-NH-" refers to a linear or branched alkyl-NH- group containing from 1 to 3 carbon atoms. Representative examples of C₁₋₃ alkyl-NH-include, but are not limited to, methyl-NH-, ethyl-NH-, n-propyl-NH-, and isopropyl-NH-.

As used herein, the term "amino-substituted alkylene", used alone or in combination, refers to a linear or branched alkylene group substituted with amino having structural formula of NH₂-alkylene. Optionally, the alkylene portion of the amino-substituted alkylene group may contain 1-10 (e.g., 1-6, 1-4, or 1-3) carbon atoms. The term "amino-substituted C₁₋₃ alkylene" or "amino-C₁₋₃ alkyl-" refers to a linear or branched alkylene group containing from 1 to 3 carbon atoms which is substituted with amino. Representative examples of amino-substituted C₁₋₃ alkylene include, but are not limited to, NH₂-CH₂-, NH₂-CH₂CH₂-, and NH₂-CH₂CH₂CH₂-.

As used herein, the term "alkyl-NHC(O)-", used alone or in combination, refers to a linear or branched alkyl-NHC(O)- group having structural formula of alkyl-NHC(O)-. Optionally, the alkyl portion of the alkyl-NHC(O)- group may contain 1-10 (e.g., 1-6, 1-4, or 1-3) carbon atoms. The term "C₁₋₃ alkyl-NHC(O)-" or "C₁-C₃ alkyl-NHC(O)-" refers to a linear or branched alkyl-NHC(O)- group containing from 1 to 3 carbon atoms. Representative examples of C₁₋₃ alkyl-NHC(O)- include, but are not limited to, CH₃-NHC(O)-, CH₃CH₂-NHC(O)-, and CH₃CH₂CH₂-NHC(O)-.

As used herein, the term "alkyl-C(O)NH-", used alone or in combination, refers to a linear or branched alkyl-C(O)NH- group having structural formula of alkyl-C(O)NH-. Optionally, the alkyl portion of the alkyl-C(O)NH- group may contain 1-10 (e.g., 1-6, 1-4, or 1-3) carbon atoms. The term "C₁₋₃ alkyl-C(O)NH-" or "C₁-C₃ alkyl-C(O)NH-" refers to a linear or branched alkyl-C(O)NH- group containing from 1 to 3 carbon atoms. Representative examples of C₁₋₃ alkyl-C(O)NH- include, but are not limited to, CH₃-C(O)NH-, CH₃CH₂-C(O)NH-, and CH₃CH₂CH₂-C(O)NH-.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, 6- to 9-membered, 6- to 20-membered, or 6- to 10-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic, tetracyclic, or polycyclic heteroaryl groups, which contain one aromatic ring having one or more (e.g., 1-4, 1-3, 1-2, or 1) heteroatoms independently selected from O, S and N, and the remaining rings which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, and 6,7-dihydrothieno[3,2-d]pyrimidinyl. Examples of tricyclic, tetracyclic, or polycyclic heteroaryl groups include, but are not limited to, acridinyl, benzindolyl, carbazolyl, dibenzofuranyl, xanthyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, 6- to 9-membered, 6- to 20-membered, or 6- to 10-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic, tetracyclic or polycyclic heteroarylene groups, which contain one aromatic ring having one or more (e.g., 1-4, 1-3, 1-2, or 1) heteroatoms independently selected from O, S and N, and the remaining ring(s) which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, chromanylene, and 6,7-dihydrothieno[3,2-d]pyrimidinylene. Examples of tricyclic, tetracyclic or polycyclic heteroarylene groups include, but are not limited to, acridinylene, benzindolylene, carbazolylene, dibenzofuranylene, xanthylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano.

As used herein, the term "aryl", used alone or in combination, refers to a monovalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, tri-, or poly-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, and cyano.

As used herein, the term "arylene", used alone or in combination, refers to a divalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenylene (e.g., 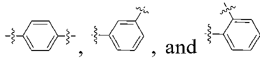 ), naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, tri-, or poly-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, and cyano.

As used herein, the term "cycloalkyl", used alone or in combination, refers to a saturated or partially unsaturated (i.e., containing one or more double bonds, but not having a fully conjugated π-electron system) monocyclic or bicyclic or tricyclic or polycyclic cyclic hydrocarbon radical, which in some embodiments contains from 3 to 30 carbon atoms (i.e., C₃₋₃₀ cycloalkyl), or from 3 to 25 carbon atoms (i.e., C₃₋₂₅ cycloalkyl), or from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl), or from 4 to 20 carbon atoms ( i.e., C₄₋₂₀ cycloalkyl), or from 4 to 15 carbon atoms ( i.e., C₄₋₁₅ cycloalkyl), or from 4 to 12 carbon atoms (i.e., C₄₋₁₂ cycloalkyl), or from 4 to 10 carbon atoms (i.e., C₄₋₁₀ cycloalkyl). The term "cycloalkyl" includes monocyclic, bicyclic, tricyclic, and polycyclic cyclic hydrocarbon radical having from 3 to 30 carbon atoms. Examples of the term "cycloalkyl" include, but are not limited to, monocyclic cycloalkyl, bridged cycloalkyl (e.g., C₅₋₃₀ bridged cycloalkyl or C₅₋₂₀ bridged cycloalkyl or C₅₋₁₅ bridged cycloalkyl or C₇₋₁₅ bridged cycloalkyl), fused cycloalkyl (e.g., C₅₋₃₀ fused cycloalkyl, C₅₋₂₀ fused cycloalkyl, C₅₋₁₅ fused cycloalkyl, C₆₋₃₀ fused cycloalkyl, C₆₋₂₀ fused cycloalkyl, C₆₋₁₅ fused cycloalkyl, C₇₋₃₀ fused cycloalkyl, C₇₋₂₀ fused cycloalkyl, C₇₋₁₅ fused cycloalkyl, and C₈₋₁₅ fused cycloalkyl), and spiro-cycloalkyl (e.g., C₅₋₃₀ spiro-cycloalkyl, C₅₋₂₀ spiro-cycloalkyl, C₅₋₁₅ spiro-cycloalkyl, C₆₋₃₀ spiro-cycloalkyl, C₆₋₂₀ spiro-cycloalkyl, C₆₋₁₅ spiro-cycloalkyl, C₇₋₃₀ spiro-cycloalkyl, C₇₋₂₀ spiro-cycloalkyl, C₇₋₁₅ spiro-cycloalkyl, and C₈₋₁₅ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, C₅₋₂₀ spiro-cycloalkyl, C₅₋₁₅ spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano. Examples of the term "C₃₋₆ cycloalkyl" include, but are not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₅₋₃₀ spiro-cycloalkyl", used alone or in combination, refers to a spiro-cycloalkyl group containing from 5 to 30 carbon atoms (e.g., but not limited to, 5-20, 5-15, 7-20, 7-15, 5-11, 5-10, and 7-9 carbon atoms). The term "C₅₋₃₀ spirocycloalkyl" includes "C₅₋₂₀ spirocycloalkyl", "C₅₋₁₅ spirocycloalkyl", "C₇₋₁₅ spirocycloalkyl", and "C₇₋₂₀ spirocycloalkyl", and representative examples thereof include, but are not limited to, spiro[3.3]heptanyl, spiro[2.5]octanyl, spiro[3.5]nonanyl, spiro[4.4]nonanyl, spiro[4.5]decanyl, and spiro[5.5]undecanyl. The "C₅₋₃₀ spiro-cycloalkyl" is optionally further substituted with one or more substituents selected from the group consisting of C₁₋₃ alkyl, halogenated C₁₋₃ alkyl, C₁₋₃ alkoxy, deuterated C₁₋₃ alkoxy, C₁₋₃ alkylamino, amino, oxo, halogen, hydroxy, and cyano.

As used herein, the term "C_{x-y} bridged cycloalkyl" (where x and y are integers) used alone or in combination, refers to a bridged cycloalkyl group containing x to y carbon atoms. In the present invention, the term "C₅₋₃₀ bridged cycloalkyl" used alone or in combination, refers to a bridged cycloalkyl group containing from 5 to 30 carbon atoms (e.g., but not limited to, 5-20, 6-20, 5-15, 7-20, 7-15, 5-11, 5-10, and 7-9 carbon atoms). The term "C₅₋₃₀ bridged cycloalkyl" includes "C₅₋₂₀ bridged cycloalkyl", "C₆₋₂₀ bridged cycloalkyl", "C₇₋₂₀ bridged cycloalkyl", "C₅₋₁₅ bridged cycloalkyl", and "C₇₋₁₅ bridged cycloalkyl", and representative examples thereof include, but are not limited to, adamantanyl, noradamantanyl, bornyl, norbomanyl (systematically named bicyclo[2.2.1]heptanyl), 2-oxobicyclo[2.2.1]heptanyl, bicyclo[2.2.1]heptenyl, and cubanyl. Said " C₅₋₃₀ bridged cycloalkyl" may be optionally substituted with 1 to 10 (e.g., 1-6, 1-5, 1-4, or 1-3) substituents selected from: C₁₋₃ alkyl, deuterated C₁₋₃ alkyl, C₁₋₃ alkoxy, halogen, halogenated C₁₋₃ alkyl, C₁₋₃ alkylamino, amino, hydroxy, cyano, and oxo.

As used herein, the term "cycloalkylene", used alone or in combination, refers to a saturated or partially unsaturated (i.e., containing one or more double bonds, but not having a fully conjugated π-electron system) monocyclic, bicyclic, tricyclic, or polycyclic divalent cyclic hydrocarbon radical, which in some embodiments contains from 3 to 30 carbon atoms (i.e., C₃₋₃₀ cycloalkylene), or from 3 to 25 carbon atoms (i.e., C₃₋₂₅ cycloalkylene), from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms (i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene), or from 4 to 20 carbon atoms (i.e., C₄₋₂₀ cycloalkylene), or from 4 to 15 carbon atoms ( i.e., C₄₋₁₅ cycloalkylene), or from 4 to 12 carbon atoms (i.e., C₄₋₁₂ cycloalkylene), or from 4 to 10 carbon atoms (i.e., C₄₋₁₀ cycloalkylene). The term "cycloalkylene" includes monocyclic, bicyclic, tricyclic, and polycyclic divalent cyclic hydrocarbon radical having from 3 to 30 carbon atoms. Examples of the term "cycloalkylene" include, but are not limited to, monocyclic cycloalkylene, bridged cycloalkylene (e.g., C₅₋₃₀ bridged cycloalkylene, C₅₋₂₀ bridged cycloalkylene, C₅₋₁₅ bridged cycloalkylene or C₇₋₁₅ bridged cycloalkylene), fused cycloalkylene (e.g., C₅₋₃₀ fused cycloalkylene, C₅₋₂₀ fused cycloalkylene, C₅₋₁₅ fused cycloalkylene, C₆₋₃₀ fused cycloalkylene, C₆₋₂₀ fused cycloalkylene, C₆₋₁₅ fused cycloalkylene, C₇₋₃₀ fused cycloalkylene, C₇₋₂₀ fused cycloalkylene, C₇₋₁₅ fused cycloalkylene, and C₈₋₁₅ fused cycloalkylene), and spiro-cycloalkylene (e.g., C₅₋₃₀ spiro-cycloalkylene, C₅₋₂₀ spiro-cycloalkylene, C₅₋₁₅ spiro-cycloalkylene, C₆₋₃₀ spiro-cycloalkylene, C₆₋₂₀ spiro-cycloalkylene, C₆₋₁₅ spiro-cycloalkylene, C₇₋₃₀ spiro-cycloalkylene, C₇₋₂₀ spiro-cycloalkylene, C₇₋₁₅ spiro-cycloalkylene, and C₈₋₁₅ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, C₅₋₂₀ spiro-cycloalkylene (e.g., C₅₋₁₅ spiro-cycloalkylene), adamantanylene, noradamantanylene, and norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano.

As used herein, the term "C_{x-y} spiro-cycloalkylene" (x and y each being an integer), used alone or in combination, refers to a spiro-cycloalkylene group containing from x to y carbon atoms. As used herein, the term "C₅₋₃₀ spiro-cycloalkylene", used alone or in combination, refers to a spiro-cycloalkylene group containing from 5 to 30 carbon atoms (e.g., 5-20, 5-15, 7-20, 7-15, 5-11, 5-10, or 7-9 carbon atoms). The term "C₅₋₃₀ spiro-cycloalkylene" includes "C₅₋₂₀ spiro-cycloalkylene", "C₅₋₁₅ spiro-cycloalkylene", "C₇₋₁₅ spiro-cycloalkylene", and "C₇₋₂₀ spiro-cycloalkylene", and representative examples thereof include, but are not limited to, spiro[3.3]heptylene, spiro[2.5]octylene, spiro[3.5]nonylene, spiro[4.4]nonylene, spiro[4.5]decylene, and spiro[5.5]undecylene. The "C₅₋₃₀ spiro-cycloalkylene" is optionally further substituted with one or more (e.g., 1-10, 1-6, 1-5, 1-4, or 1-3) substituents selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano.

As used herein, the term "C_{x-y} bridged cycloalkylene" (where x and y are integers) used alone or in combination, refers to a bridged cycloalkylene group containing x to y carbon atoms. In the present invention, the term "C₅₋₃₀ bridged cycloalkylene" used alone or in combination, refers to a bridged cycloalkylene group containing 5 to 30 (e.g., 5-20, 6-20, 7-20, 5-15, 7-15, 5-11, 5-10, and 7-9) carbon atoms. The term "C₅₋₃₀ bridged cycloalkylene" includes "C₅₋₂₀ bridged cycloalkylene", "C₆₋₂₀ bridged cycloalkylene", "C₇₋₂₀ bridged cycloalkylene", "C₅₋₁₅ bridged cycloalkylene", and "C₇₋₁₅ bridged cycloalkylene", and representative examples thereof include, but are not limited to, adamantanylene, noradamantanylene, bornylene, bicyclo[2.2.1]heptanylene, 2-oxobicyclo[2.2.1]heptanylene, bicyclo[2.2.1]heptenylene, and cubanylene. Said "C₅₋₃₀ bridged cycloalkylene" may be optionally substituted with one or more (e.g., 1-10, 1-6, 1-5, 1-4, or 1-3) substituents selected from: C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, C₁₋₃ alkyl-NHC(O)-, C₁₋₃ alkyl-C(O)NH-, and cyano.

As used herein, the term "heterocyclyl" or "heterocyclic group", used alone or in combination, refers to a 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5-to 30-membered, 5- to 20-membered, or 5- to 15-membered) saturated or partially unsaturated (i.e., containing one or more double bonds, but not having a fully conjugated π-electron system) monocyclic, bicyclic, tricyclic, or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl group include, but are not limited to, monocyclic heterocyclyl (e.g., 4- to 30-membered monocyclic heterocyclyl, and 4- to 20-membered monocyclic heterocyclyl), bridged heterocyclyl (e.g., 5- to 30-membered bridged heterocyclyl, 5- to 20-membered bridged heterocyclyl, 7- to 20-membered bridged heterocyclyl, and 7- to 15-membered bridged heterocyclyl), fused heterocyclyl (e.g., 5- to 30-membered fused heterocyclyl, and 5- to 20-membered fused heterocyclyl), and spiro-heterocyclyl groups (e.g., 5- to 30-membered spiro-heterocyclyl, and 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, pyrrolidinocyclopropyl, cyclopentylaziridinyl, pyrrolidinocyclobutyl, pyrrolidinopyrrolidinyl (e.g., hexahydropyrrolo[3,4-c]pyrrolyl), pyrrolidinopiperidinyl, pyrrolidinopiperazinyl, pyrrolidinomorpholinyl, piperidinomorpholinyl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "nitrogen-containing heterocyclyl", used alone or in combination, refers to 4-to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5-to 30-membered, 5- to 20-membered, or 5- to 15-membered) saturated or partially unsaturated (i.e., containing one or more double bonds, but not having a fully conjugated π-electron system) monocyclic, bicyclic, tricyclic, or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclyl group include, but are not limited to, nitrogen-containing monocyclic heterocyclyl (e.g., 4- to 30-membered nitrogen-containing monocyclic heterocyclyl, and 4- to 20-membered nitrogen-containing monocyclic heterocyclyl), nitrogen-containing bridged heterocyclyl (e.g., 5- to 30-membered nitrogen-containing bridged heterocyclyl, 5- to 20-membered nitrogen-containing bridged heterocyclyl, 5- to 15-membered nitrogen-containing bridged heterocyclyl, 7- to 20-membered nitrogen-containing bridged heterocyclyl, and 7- to 15-membered nitrogen-containing bridged heterocyclyl), nitrogen-containing fused heterocyclyl (e.g., 5- to 30-membered nitrogen-containing fused heterocyclyl, and 5- to 20-membered nitrogen-containing fused heterocyclyl), and nitrogen-containing spiro-heterocyclyl groups (e.g., 5- to 30-membered nitrogen-containing spiro-heterocyclyl, and 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, pyrrolidinocyclopropyl, cyclopentylaziridinyl, pyrrolidinocyclobutyl, pyrrolidinopyrrolidinyl (e.g., hexahydropyrrolo[3,4-c]pyrrolyl), pyrrolidinopiperidinyl, pyrrolidinopiperazinyl, pyrrolidinomorpholinyl, piperidinomorpholinyl, and azaspirocycloalkyl (e.g., 5- to 20-membered azaspirocycloalkyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "nitrogen-containing bridged heterocyclyl", used alone or in combination, refers to 5- to 30-membered (e.g., 5- to 20-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic, or polycyclic monovalent bridged cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of nitrogen-containing bridged heterocyclyl include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "-O-optionally substituted heterocyclyl", used alone or in combination, refers to a group formed by optionally substituted heterocyclyl connected to oxygen. The definition of "heterocyclyl" may be as defined above. Examples of the term "heterocyclyl" include, but are not limited to, monocyclic heterocyclyl (e.g., 4- to 20-membered monocyclic heterocyclyl), bridged heterocyclyl (e.g., 5- to 20-membered bridged heterocyclyl or 7- to 15-membered bridged heterocyclyl), fused heterocyclyl, and spiro heterocyclyl (e.g., 5- to 20-membered spiro heterocyclyl). Optionally, the heterocyclyl in the term "-O-optionally substituted heterocyclyl" is e.g., a 4- to 20-membered mono-, bi-, tri-, or polycyclic heterocyclyl containing one or more (e.g., 1-4, or 1, 2, 3, or 4) heteroatoms selected from nitrogen, oxygen, and sulfur. Examples of "heterocyclyl" include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). As used herein, the heterocyclyl group is optionally substituted with a substituent selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "heterocyclylene", used alone or in combination, refers to a 4- to 30-membered saturated or partially unsaturated (i.e., containing one or more double bonds, but not having a fully conjugated π-electron system) monocyclic, bicyclic, tricyclic, or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 4- to 30-membered (optionally 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5-to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic, or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 30-membered monocyclic heterocyclylene, and 4- to 20-membered monocyclic heterocyclylene), bridged heterocyclylene (e.g., 5- to 30-membered bridged heterocyclylene, 5- to 20-membered bridged heterocyclylene, 7- to 20-membered bridged heterocyclylene, and 7- to 15-membered bridged heterocyclylene), fused heterocyclylene (e.g., 5- to 30-membered fused heterocyclylene, and 5- to 20-membered fused heterocyclylene) and spiro-heterocyclylene groups (e.g., 5- to 30-membered spiro-heterocyclylene, and 5- to 20-membered spiro-heterocyclylene). Representative examples of monocyclic heterocyclylene include, but are not limited to, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctylene. Examples of the bridged heterocyclylene, fused heterocyclylene, and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, pyrrolidinocyclopropylene, cyclopentylaziridinylene, pyrrolidinocyclobutylene, pyrrolidinopyrrolidinylene (e.g., hexahydropyrrolo[3,4-c]pyrrolylene), pyrrolidinopiperidinylene, pyrrolidinopiperazinylene, pyrrolidinomorpholinylene, piperidinomorpholinylene, and azaspirocycloalkylene (e.g., 5- to 20-membered azaspirocycloalkylene, such as 3-azaspiro[5.5]undecanylene), 5,6,7,8-tetrahydrobenzo[4,5]thieno[2,3-d]pyrimidinylene, 6,7-dihydro-5H-cyclopenta[4,5]thieno[2,3-d]pyrimidinylene, chromanylene, and 6,7-dihydrothieno[3,2-d]pyrimidinylene. The heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "nitrogen-containing heterocyclylene", used alone or in combination, refers to a 4- to 30-membered (e.g., 4- to 30-membered, 4- to 25-membered, 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5-to 20-membered, or 5- to 15-membered) saturated or partially unsaturated (i.e., containing one or more double bonds, but not having a fully conjugated π-electron system) monocyclic, bicyclic, tricyclic, or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene group include, but are not limited to, nitrogen-containing monocyclic heterocyclylene (e.g., 4- to 30-membered nitrogen-containing monocyclic heterocyclylene, and 4- to 20-membered nitrogen-containing monocyclic heterocyclylene), nitrogen-containing bridged heterocyclylene (e.g., 5- to 30-membered nitrogen-containing bridged heterocyclylene, 5- to 20-membered nitrogen-containing bridged heterocyclylene, 7- to 20-membered nitrogen-containing bridged heterocyclylene, and 7- to 15-membered nitrogen-containing bridged heterocyclylene), nitrogen-containing fused heterocyclylene (e.g., 5- to 30-membered nitrogen-containing fused heterocyclylene, and 5- to 20-membered nitrogen-containing fused heterocyclylene), and nitrogen-containing spiro-heterocyclylene (e.g., 5-to 30-membered nitrogen-containing spiro-heterocyclylene, and 5- to 20-membered nitrogen-containing spiro-heterocyclylene). Representative examples of the nitrogen-containing monocyclic heterocyclylene include, but are not limited to, azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of nitrogen-containing bridged heterocyclylene, nitrogen-containing fused heterocyclylene, and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, pyrrolidinocyclopropylene, cyclopentylaziridinylene, pyrrolidinocyclobutylene, pyrrolidinopyrrolidinylene (e.g., hexahydropyrrolo[3,4-c]pyrrolylene), pyrrolidinopiperidinylene, pyrrolidinopiperazinylene, pyrrolidinomorpholinylene, piperidinomorpholinylene, and azaspirocycloalkylene (e.g., 5- to 20-membered azaspirocycloalkylene, such as 3-azaspiro[5.5]undecanylene). The nitrogen-containing heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "nitrogen-containing bridged heterocyclylene", used alone or in combination, refers to 5- to 30-membered (e.g., 5- to 20-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic, or polycyclic bivalent bridged cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing bridged heterocyclylene include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing bridged heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, and optionally deuterated C₁₋₆ alkyl-C(O)NH-.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include C₂₋₈ alkynylene, C₂₋₆ alkynylene, or C₂₋₄ alkynylene, with representative but non-limiting examples including ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include C₂₋₈ alkynyl, C₂₋₆ alkynyl, or C₂₋₄ alkynyl, with representative but non-limiting examples including ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include C₂₋₈ alkenylene, C₂₋₆ alkenylene, or C₂₋₄ alkenylene, with representative but non-limiting examples including vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of C₂₋₆ alkenyl group include C₂₋₈ alkenyl, C₂₋₆ alkenyl, or C₂₋₄ alkenyl, with representative but non-limiting examples including, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

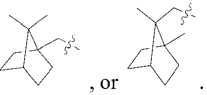

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 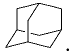 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 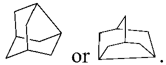 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl, or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural formula:

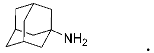

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include Cl⁻, Br, I⁻ and sulfonate (such as p-toluenesulfonate, TsO⁻), and neutral molecular leaving groups include water, ammonia, and alcohol. In the present disclosure, those skilled in the art can select an appropriate leaving group as needed, such as but not limited to -N₃, halogen, methanesulfonyloxy (MsO-), trifluoromethanesulfonyloxy (TfO-) or p-toluenesulfonyloxy (TsO-), etc.

Salts or pharmaceutically acceptable salts, enantiomers, stereoisomers, solvates, polymorphs of the compounds of Formula (I) or Formula (II) of the present disclosure are also encompassed within the scope of the present invention.

In all embodiments of the present disclosure, the salts or pharmaceutically acceptable salts of the compounds of Formula (I) or Formula (II) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, glycolates, trifluoroacetates, terephthalates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil, and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid, and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties, and reactivity. The diastereomeric mixture can be separated by high-resolution analytical operations such as electrophoresis and chromatography, such as HPLC.

As used herein, the term "prodrug" refers to a compound that can be converted in vivo, such as by hydrolysis in the blood, into its biologically active form. When a prodrug is administered to a patient, it releases the parent compound in the body. Prodrugs are generally prepared by modifying functional groups, and such modifications can be removed by conventional operations (e.g., acid/base hydrolysis) or by cleavage in vivo to release the parent drug. Prodrugs include, for example, forms in which the hydroxyl, amino, or mercapto groups of the compounds of the present invention are combined with any group that, when administered to a patient, can be cleaved to re-form the hydroxyl, amino, or mercapto groups. Accordingly, representative examples of prodrugs include, but are not limited to, acetate/amide, formate/amide, tert-butoxycarbonyl (Boc), and benzoate/amide derivatives of the hydroxyl, mercapto, and amino functional groups of the compounds of Formula (I) and Formula (II). In addition, in the case of carboxylic acids (-COOH), esters such as methyl esters, ethyl esters, and the like can be used. These esters may themselves be active and/or may be hydrolyzed under physiological conditions in the human body. Suitable pharmaceutically acceptable, in vivo hydrolyzable ester groups include those that are readily cleaved in the human body to release the parent acid or a salt thereof.

### Description of the Drawings

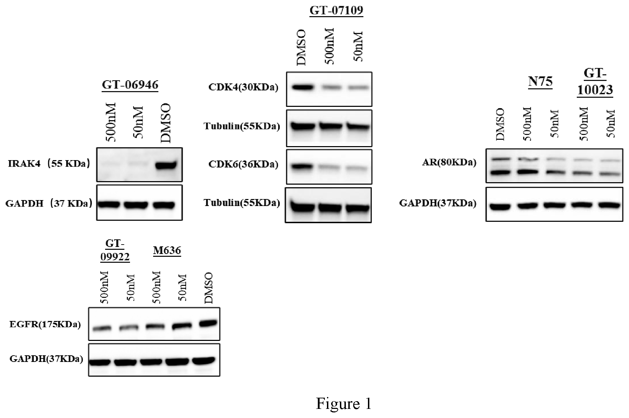

Fig. 1 shows the Western blot assay results of the degradation of target substrate proteins by the compounds of the present disclosure in hPBMC, MDA-MB-231, LNcap, and H1975 cells.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | | | |
|---|---|---|---|
| ACN | acetonitrile | MeCN | acetonitrile |
| AcOH | acetic acid | MeOH | Methanol |
| AcOK | potassium acetate | MS | mass spectrometry |
| AIBN | 2,2'-azobis(2-methylpropionitrile) | MsO- | Methanesulfonyloxy |
| Bn | Benzyl | MsCl | methanesulfonyl chloride |
| Boc | t-Butyloxy carbonyl | NaHMDS | Sodium bis(trimethylsilyl)amide |
| Brettphos Pd G3 | Methanesulfonato(2-dicyclohexylphosphino-3,6-dimethoxy-2',4',6'-tri-i-propyl-1,1'-biphenyl)(2'-amino-1,1'-biphenyl-2-yl)palladium(II) | NBS | N-Bromosuccinimide |
| Brettphos | Dicyclohexyl[3,6-dimethoxy-2',4',6'-tris(1-methylethyl)[1,1'-biphenyl]-2-yl]phosphine | NMI | N-Methylimidazole |
| BINAP | 2,2'-Bis(diphenylphosphino)-1,1'-binaphthalene | ¹H NMR | Proton nuclear magnetic resonance |
| BPO | Dibenzoyl peroxide | HFIP | Hexafluoroisopropanol |
| Cbz | Carbobenzoxy | MeO- | Methoxy |
| CNBr | cyanogen bromide | o/n | Overnight |
| Con. | Concentration | PCC | Pyridinium chlorochromate |
| DCE | 1,2-Dichloroethane | PE | petroleum ether |
| DCM | dichloromethane | Pin₂B₂ | pinacolborane |
| DEA | Diethylamine | PyBOP | Benzotriazol-1-yl-oxy-tris(pyrrolidino)phosphonium hexafluorophosphate |
| DIAD | Diisopropyl azodicarboxylate | Ruphos Pd G3 | Methanesulfonato(2- |
| | | | dicyclohexylphosphino-2',6'-diisopropoxy-1,1'-biphenyl)(2'-amino-1,1'-biphenyl-2-yl)palladium(II) (CAS No.: 1445085-77-7) |
| DIEA, or DIPEA | N, N-diisopropylethylamine | rt | room temperature |
| DMA | N,N-Dimethylacetamide | tBu | tert-butyl |
| DMAP | 4-Dimethylaminopyridine | tBuONa | Sodium tert-butoxide |
| DME | Dimethylether | TBSCl | tert-Butyldimethylchlorosilane |
| DMEDA | N,N'-dimethylethylenediamine | TCFH | N,N,N',N'-Tetramethylchloroformamidinium hexafluorophosphate |
| DMF | N,N-dimethylformamide | TEA | triethylamine |
| DMSO | dimethyl sulfoxide | TFA | trifluoroacetic acid |
| EDCI | 1-(3-dimethylaminopropyl)-3-ethylcarbodiimide hydrochloride | T_{f}O- | Trifluoromethanesulfonate |
| ESI | electrospray ionization | Tf₂O | Triflic anhydride |
| equiv | equivalent | THF | Tetrahydrofuran |
| EtOH | ethanol | THP | tetrahydropyran |
| EtOAc, or EA | Ethyl acetate | TLC | thin layer chromatography |
| HATU | 2-(7-Azabenzotriazol-1-yl)-N,N,N',N'-tetramethyluronium hexafluorophosphate | TMS | tetramethylsilane |
| HPLC | high performance liquid chromatography | TsCl | p-toluenesulfonyl chloride |
| HRMS | high resolution mass spectrometry | TsO- | Tosyloxy |
| IPA | Isopropyl alcohol | TsOH | p-Toluenesulfonic acid |
| LC-MS | liquid chromatography-mass spectrometry | ^{®}T₃P | propylphosphonic anhydride |
| LiHMDS | Lithium hexamethyldisilazide | TTIP | titanium(IV) isopropoxide |
| LRMS | low resolution mass spectrometry | Xantphos | 4,5-bis(diphenylphosphino)-9,9-dimethylxanthene |
| LC | liquid chromatography | Xphos | 2-dicyclohexylphosphino-2',4',6'-triisopropylbiphenyl |
| Me | methyl | | |

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. Supercritical fluid chromatography (SFC) chiral resolution/separation was performed on a SHIMADZU LC-20AP instrument equipped with a Daicel CHIRALPAK^{®}IE column. All reactions were carried out under ambient atmosphere unless otherwise specified. Reaction progress was monitored by TLC or LC-MS.

Solvents and reagents are processed as follows:
the solvents used in the reactions such as DCM, DMF, NMP, anhydrous EtOH, and anhydrous MeOH were commercially available;
preparative HPLC was performed using HPLC-grade CH₃CN and deionized water;
all other reactants, reagents and chemicals were commercially available, unless otherwise specified, or were synthesized using methods known in the art.

Unless otherwise specified, the materials and reagents used in the following examples are commercially available and used directly, or can be synthesized by using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and Formula (II) of the present disclosure according to routine techniques in the art.

### General synthesis method 1 for intermediate compounds:

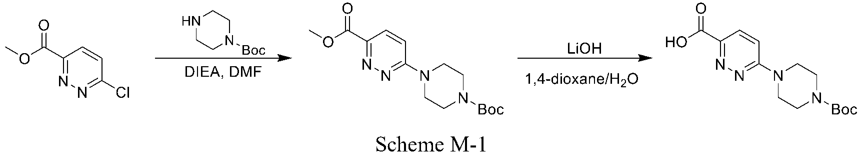

### General synthesis method 2 for intermediate compounds:

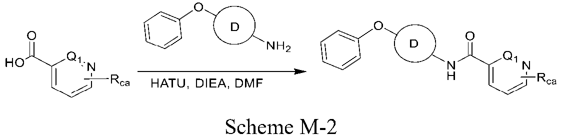

In Scheme M-2, ring D of the substrate amine used and Q₁ of the substrate acid used are as defined in the structure of Formula (PBM-7) and its various embodiments. The R_{ca} of the substrate acid used is a monovalent group corresponding to the divalent group R_{c} in the structure of Formula (PBM-7). For example, R_{ca} represents -CHO, -OH, -NH(R_{d}), -C(O)NH₂, -N(R_{d})-R_{f}-NH(Rₑ), -R_{f}-C(O)NH₂, -R_{f}-C(O)OH, -C(O)OH, - R_{f}-H, -R_{f}-NH(R_{d}), -O-R_{f}-NH(R_{d}), 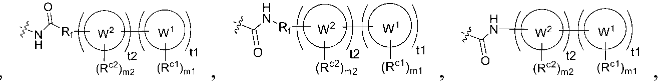 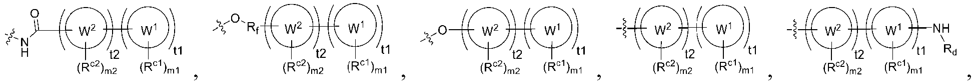 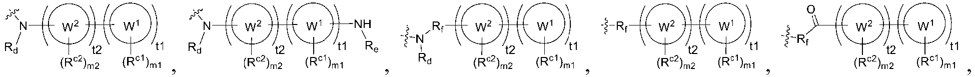 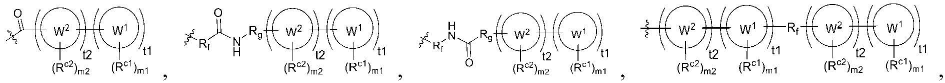 or 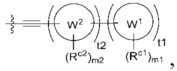 wherein R_{d}, Rₑ, R_{f}, R_{g}, R^{c1}, R^{c2}, ring W¹, ring W², t1, t2, ml, and m2 are as defined in the compound of Formula (I) and its various embodiments in the present disclosure. It should be understood that when the group R_{ca} contains active hydrogen or reactive group that can participate in the reaction of Scheme M-2, the active hydrogen or reactive group can be protected by techniques and methods known to those skilled in the art, such as using protecting groups. For example, when R_{ca} represents a piperidinyl or piperazinyl group, conventional protecting groups such as Boc can be used to protect the hydrogen on the N. For example, when R_{ca} represents -CHO, conventional protecting groups can be used to protect the aldehyde group, such as by forming an acetal. The removal of the protecting group can be achieved by techniques and methods known to those skilled in the art. For instance, the removal of the Boc protecting group can be accomplished under acidic conditions such as hydrochloric acid or trifluoroacetic acid. As another example, the removal of the acetal protecting group can be achieved under acidic conditions, such as sulfuric acid, hydrochloric acid, or trifluoroacetic acid.

In Scheme M-2, to a solution of the substrate acid (1 equiv) and the substrate amine (1 equiv) in DMF were added HATU (1.5 equiv) and DIEA (5 equiv). The mixture was then reacted at room temperature for 30 minutes. After monitoring the reaction via LC-MS and confirming its completion, water was added to the reaction mixture, and the resulting mixture was extracted with ethyl acetate. The organic phase was washed with saturated brine, and concentrated to give a crude product. The crude product was purified by column chromatography (eluent (v/v): DCM/MeOH = 100/1) to afford the target compound.

### General synthesis method 3 for intermediate compounds:

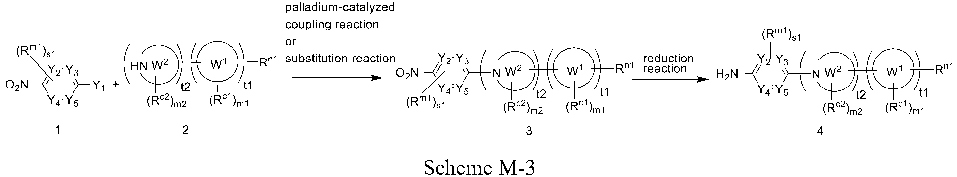

In Scheme M-3, (R^{m1})ₛ₁ of compound 1 indicates that the 6-membered ring to which it is attached is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano, s1 represents an integer of 0, 1, 2, 3 or 4, Y₁ represents halogen (e.g., fluorine, chlorine, or bromine), and Y₂, Y₃, Y₄, and Y₅ each independently represent N or CH, with the provision that Y₂, Y₃, Y₄, and Y₅ are not all N simultaneously. Ring W¹, ring W², t1, t2, (R^{c1})ₘ₁, and (R^{c2})ₘ₂ are as defined in the compound of Formula (I) of the present disclosure. Rⁿ¹ represents Boc, H or -NHRₕ, wherein Rₕ represents hydrogen or C₁₋₆ alkyl (e.g., methyl or ethyl). It should be understood that when ring W¹-Rⁿ¹ contains additional reactive groups (e.g., -NH- or -NH₂) that can participate in the reaction of Scheme M-3, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme M-3 can be appropriately modified and adjusted using techniques and methods well known to those skilled in the art to obtain the desired target compound. In Scheme M-3, the substitution reaction or palladium-catalyzed coupling reaction of step 1 can be conventional techniques and methods known to those skilled in the art. When step 1 is a substitution reaction, Y₁ represents fluorine, and compounds 1 and 2 react under basic conditions (such as K₂CO₃). When step 1 is a palladium-catalyzed coupling reaction, Y₁ represents bromine, and the palladium catalyst and phosphine ligand used can be conventional ones suitable for coupling reactions. There are many types of conventional palladium catalysts for coupling reactions, such as palladium acetate (Pd(OAc)₂), tris(dibenzylideneacetone)dipalladium (Pd₂(dba)₃), diphenylphosphinoferrocene dichloropalladium, tetrakis(triphenylphosphine)palladium, dichlorobis(triphenylphosphine)palladium, palladium on carbon, etc. The phosphine ligand can be a conventional ligand such as Xantphos or BINAP. The reduction reaction of step 2 in Scheme M-3 can be conventional techniques and methods known to those skilled in the art, such as H₂/Pd/C or Fe/NH₄Cl/MeOH.

### General synthesis method 4 for intermediate compounds:

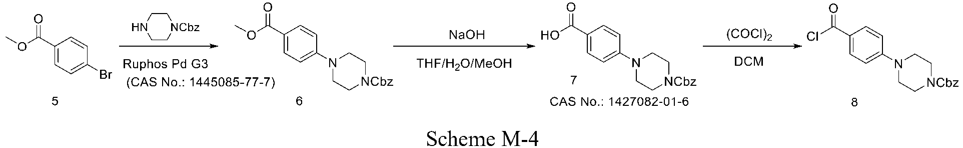

### General synthesis method 5 for intermediate compounds:

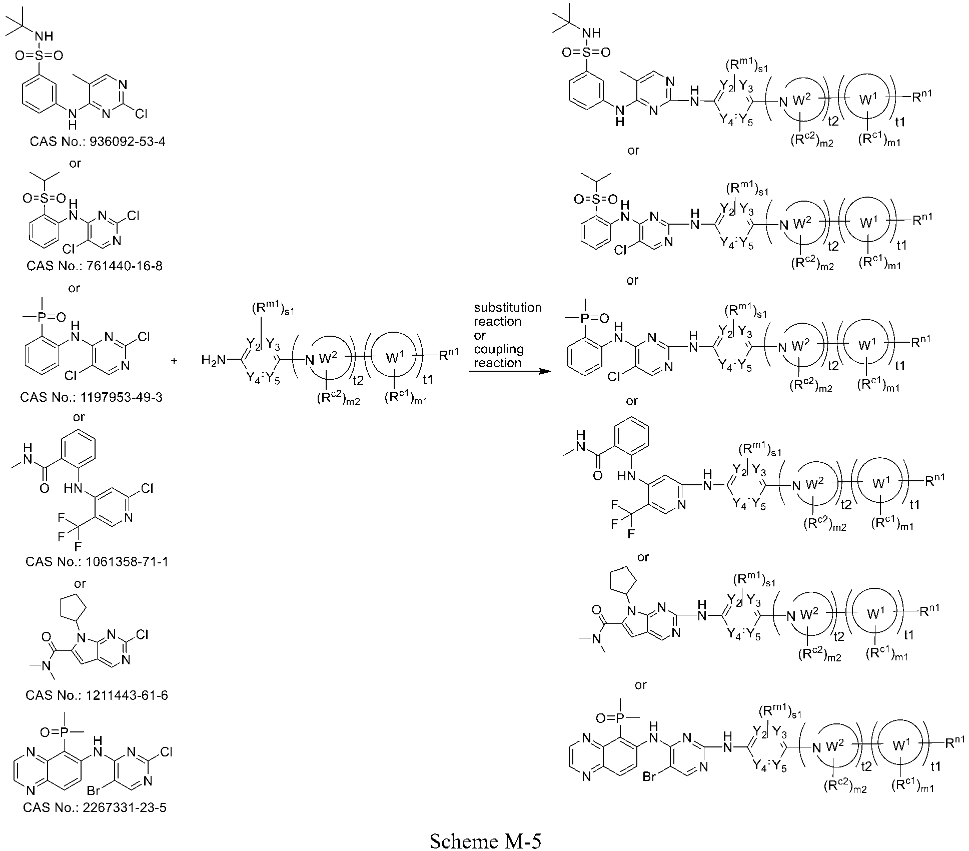

In Scheme M-5, (R^{ml})ₛₗ of the compounds indicates that the 6-membered ring to which it is attached is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano. Y₁ represents halogen (e.g., fluorine, chlorine, or bromine), and Y₂, Y₃, Y₄, and Y₅ each independently represent N or CH, with the provision that Y₂, Y₃, Y₄, and Y₅ are not all N simultaneously. Ring W¹, ring W², t1, t2, (R^{c1})ₘ₁, and (R^{c2})ₘ₂ are as defined in the compound of Formula (I) of the present disclosure. Rⁿ¹ represents 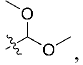 Boc, H, or -NHRₕ, where Rₕ represents hydrogen or C₁₋₆ alkyl (e.g., methyl or ethyl). It should be understood that when ring W¹-Rⁿ¹ contains additional reactive groups (such as -NH-, -NH₂, or aldehyde group) that can participate in the reaction of Scheme M-5, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid. As another example, the removal of the acetal protecting group can be achieved under acidic conditions, such as sulfuric acid, hydrochloric acid, or trifluoroacetic acid.

Depending upon the target compound, the reaction substrate, reaction conditions (including reaction dosage, temperature, time, etc.), and work up, etc. in Scheme M-5 can be appropriately modified and adjusted using techniques and methods well known to those skilled in the art to obtain the desired target compound. In Scheme M-5, the substitution reaction or coupling reaction can be conventional techniques and methods known to those skilled in the art. For example, substitution reactions can be carried out under heating conditions in the presence of triethylamine/isopropanol, p-toluenesulfonic acid/n-butanol, DIEA/ethanol, NaHMDS, or Nal/DIEA/DMF; coupling reactions can be performed under heating conditions in the presence of Brettphos Pd G3/Brettphos ligand/Cs₂CO₃.

### General synthesis method 6 for intermediate compounds:

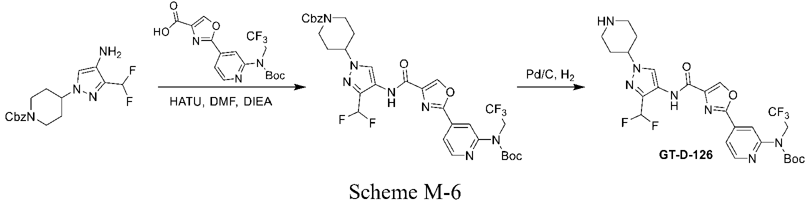

### General synthesis method 7 for intermediate compounds:

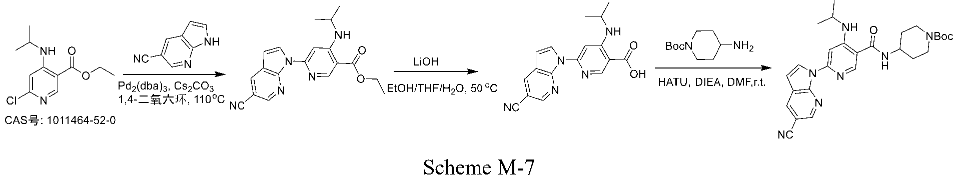

The removal of protecting groups can be achieved through techniques and methods known to those skilled in the art. For example, the removal of the Boc protecting group can be achieved under acidic conditions such as concentrated sulfuric acid.

### General synthesis method 8 for intermediate compounds:

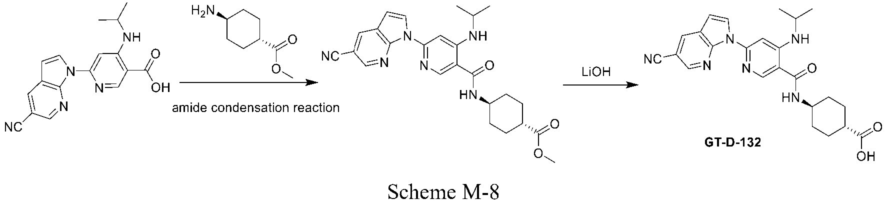

Depending upon the target compound, the reaction substrate, reaction conditions (including reaction dosage, temperature, time, etc.), and work up, etc. in Scheme M-8 can be appropriately modified and adjusted using techniques and methods well known to those skilled in the art to obtain the desired target compound. In Scheme M-8, the amide condensation reaction can be conventional techniques and methods known to those skilled in the art. For example, the amide condensation reaction can be carried out at room temperature in the presence of HATU/DIEA/DMF or HOAt/EDCI/TEA/DCM.

### General synthesis method 9 for intermediate compounds:

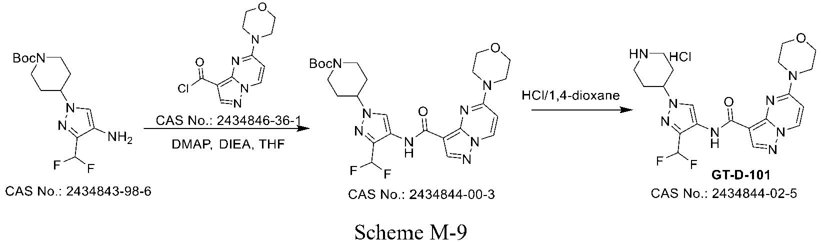

### General synthesis method 10 for intermediate compounds:

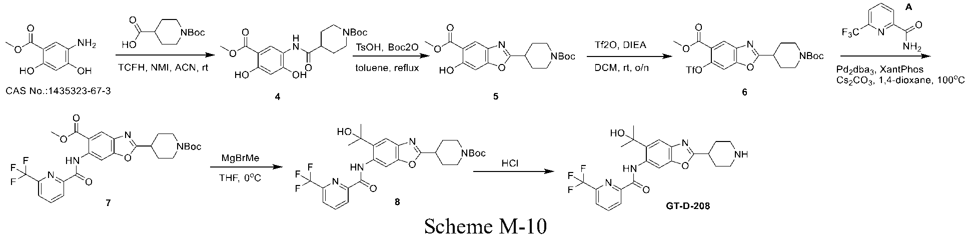

### General synthesis method 11 for intermediate compounds:

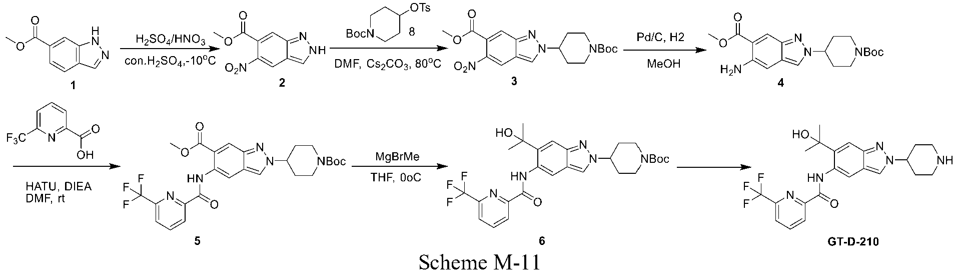

### General synthesis method 12 for intermediate compounds:

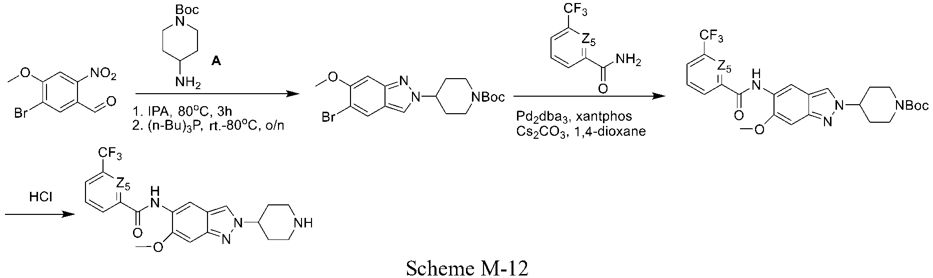

In Scheme M-12, Z₅ represents CH or N.

### General synthesis method 13 for intermediate compounds:

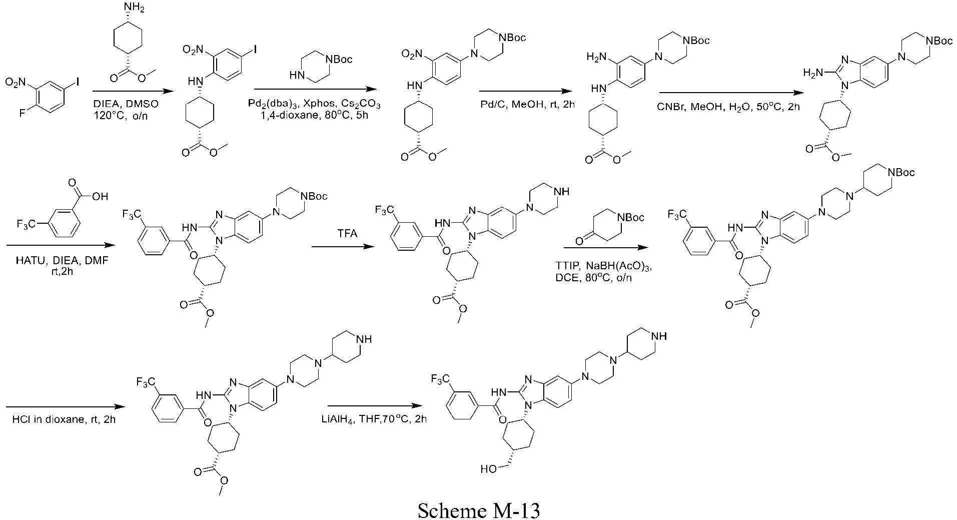

### General synthesis method 1 for compounds of Formula (I):

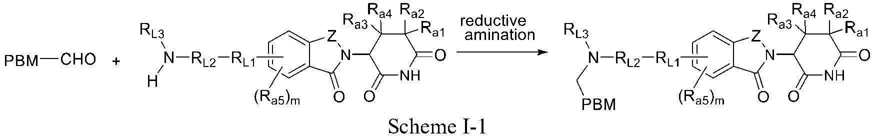

In Scheme I-1, the PBM aldehyde or ketone substrate is a compound whose PBM moiety corresponds to the PBM moiety of the compound of Formula (I) herein. In the PBM substrate aldehyde or ketone, and in the other amine substrate, Rₐ₁, Rₐ₂, Rₐ₃, Rₐ₄, Z, and (Rₐ₅)ₘ are as defined in the compound of Formula (I) herein. In Scheme I-1,
(i) R_{L1} represents NH, and R_{L2} and R_{L3}, together with the nitrogen atom to which they are attached, form 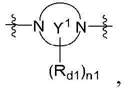 wherein ring Y¹ and (R_{d1})ₙ₁ are as defined in the compound of Formula (I) herein; or
(ii) R_{L1} and R_{L2} together form 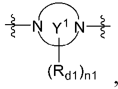 wherein ring Y¹ and (R_{d1})ₙ₁ are as defined in the compound of Formula (I) herein, and R_{L3} represents H; or
(iii) R_{L1} represents NH, R_{L2} represents a bond, and R_{L3} represents H; or
(iv) R_{L1} represents 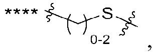 wherein the symbol **** indicates the point of attachment to R_{L2}, R_{L2} represents 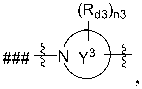 wherein the symbol ### indicates the point of attachment to N, and ring Y³ and (Rₐ₃)ₙ₃ are as defined in the compound of Formula (I) herein, and R_{L3} represents H.

The reductive amination reaction in Scheme I-1 can be carried out by conventional techniques and methods well known to those skilled in the art. For example, the reductive amination reaction can be carried out in the presence of sodium triacetoxyborohydride and 1,2-dichloroethane (or N,N-dimethylformamide) at a temperature ranging from room temperature to 80 °C. Alternatively, the reductive amination reaction can be carried out in the presence of sodium borohydride and 1,4-dioxane at a temperature ranging from room temperature to 80 °C.

For example, the procedure of Scheme I-1 can be as follows: To a solution of the corresponding PBM aldehyde or ketone substrate compound (1.0 equiv) and amine substrate (1.2 equiv) in N,N-dimethylformamide was added sodium acetate (0.9 equiv), and the mixture was stirred at room temperature for 1 h. Then acetic acid (0.9 equiv) and sodium cyanoborohydride (2.8 equiv) were added, and the reaction was carried out at room temperature. After the completion of the reaction was confirmed by LC-MS, the final target compound was obtained by purification via C₁₈ reversed-phase column chromatography.

### General synthesis method 2 for compounds of Formula (I): Urea Formation (Triphosgene)

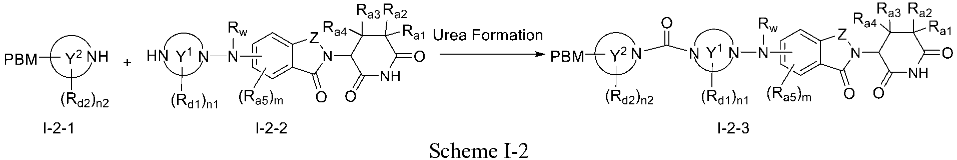

In Scheme I-2, PBM, Z, Rₐ₁, Rₐ₂, Rₐ₃, Rₐ₄, (Rₐ₅)ₘ, R_{w}, ring Y¹, (R_{d1})ₙ₁, ring Y², and (Ra₂)ₙ₂ are as defined in the compound of Formula (I) and its various sub-embodiments of the present disclosure.

The specific procedure of Scheme I-2 can be as follows:

To a solution of compound I-2-1 (40 mg, 0.095 mmol) in dichloromethane (1 mL) was added N,N-diisopropylethylamine (36.84 mg, 0.285 mmol), followed by the addition of triphosgene (0.006 mL, 0.038 mmol) under stirring, and the mixture was stirred at room temperature for 0.5 h. Then a solution of compound I-2-2 (37.35 mg, 0.105 mmol) in N,N-dimethylformamide (1 mL) was added, and the reaction was carried out at room temperature for 1 h. After the completion of the reaction was confirmed by LC-MS, the reaction mixture was filtered, and the filtrate was purified by reversed-phase column chromatography to afford the target product.

### General synthesis method 3 for compounds of Formula (I):

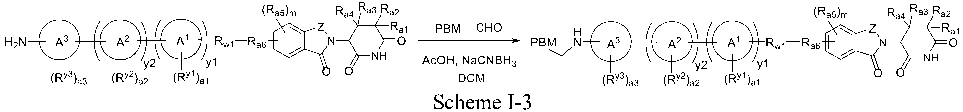

In Scheme I-3, PBM, Rₐ₁, Rₐ₂, Rₐ₃, Rₐ₄, (Rₐ₅)ₘ, Z, Rₐ₆, R_{w1}, ring A¹, ring A², ring A³, y1, y2, (R^{y1})ₐ₁, (R^{y2})ₐ₂, and (R^{y3})ₐ₃ are as defined in the compound of Formula (I) and its various sub-embodiments of the present disclosure.

The reductive amination reaction in Scheme I-3 can be carried out by conventional techniques and methods well known to those skilled in the art. For example, the reductive amination reaction can be carried out in the presence of sodium triacetoxyborohydride and 1,2-dichloroethane (or dichloromethane) at a temperature ranging from room temperature to 80 °C.

For example, to a solution of the corresponding PBM aldehyde or ketone substrate (1.0 equiv) and amine substrate (1.0 equiv) in dichloromethane was added an appropriate amount of acetic acid. The mixture was reacted at room temperature for 30 minutes, then sodium cyanoborohydride (3.0 equiv) was added, and the reaction was continued at room temperature. After the completion of the reaction was confirmed by LC-MS, the final target compound was obtained by purification via C₁₈ reversed-phase column chromatography.

### General synthesis method 4 for compounds of Formula (I):

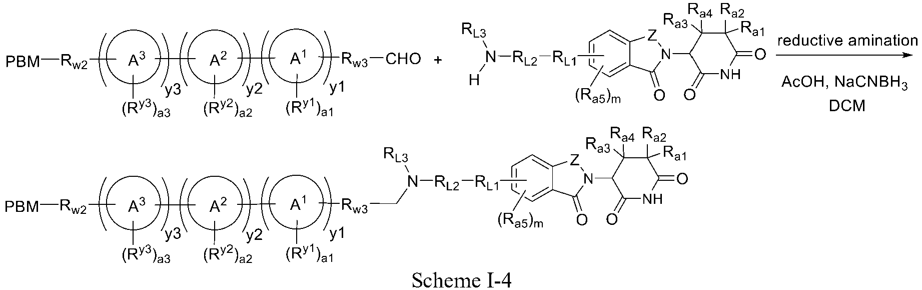

In Scheme I-4, PBM, Rₐ₁, Rₐ₂, Rₐ₃, Rₐ₄, (Rₐ₅)ₘ, Z, R_{w2}, ring A¹, ring A², ring A³, y1, y2, y3, (R^{y1})ₐ₁, (R^{y2})ₐ₂, and (R^{y3})ₐ₃ are as defined in the compound of Formula (I) and its various sub-embodiments of the present disclosure. In Scheme I-4, R_{L1}, R_{L2}, and R_{L3} are as defined in Scheme I-1. -R_{w3}-CH₂- is a non-limiting example of R_{w1} of the compound of Formula (I) herein, wherein R_{w3} represents a bond or an optionally substituted linear or branched C₁₋₁₉ alkylene, wherein any one or more methylene groups in the main carbon chain skeleton of the linear or branched C₁₋₁₉ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₁₉ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and wherein one or more hydrogens of said linear or branched C₁₋₁₉ alkylene are optionally replaced by a substituent selected from the group consisting of deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

The reductive amination reaction in Scheme I-4 can be carried out by conventional techniques and methods well known to those skilled in the art. For example, the reductive amination reaction can be carried out in the presence of sodium triacetoxyborohydride and 1,2-dichloroethane (or dichloromethane) at a temperature ranging from room temperature to 80 °C.

For example, to a solution of the corresponding PBM aldehyde or ketone substrate (1.0 equiv) and amine substrate (1.0 equiv) in dichloromethane was added an appropriate amount of acetic acid. The mixture was reacted at room temperature for 30 minutes, then sodium cyanoborohydride (3.0 equiv) was added, and the reaction was continued at room temperature. After the completion of the reaction was confirmed by LC-MS, the final target compound was obtained by purification via C₁₈ reversed-phase column chromatography.

### General synthesis method 5 for compounds of Formula (I):

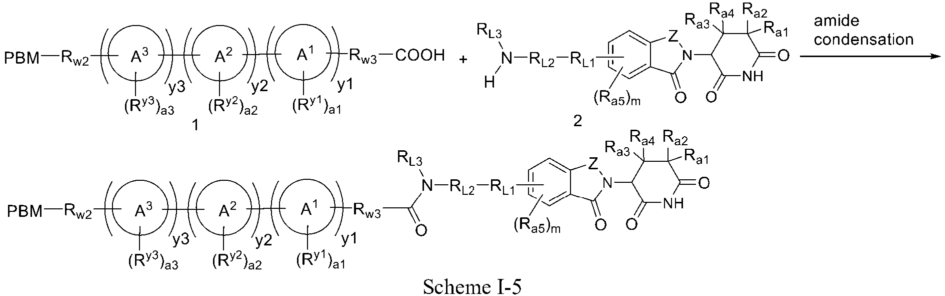

In Scheme I-5, PBM, Rₐ₁, Rₐ₂, Rₐ₃, Rₐ₄, (Rₐ₅)ₘ, Z, R_{w2}, ring A¹, ring A², ring A³, y1, y2, y3, (R^{y1})ₐ₁, (R^{y2})ₐ₂, and (R^{y3})ₐ₃ are as defined in the compound of Formula (I) and its various sub-embodiments herein. R_{L1}, R_{L2}, and R_{L3} in Scheme I-5 are as defined in Scheme I-1. R_{w3} in Scheme I-5 is as defined in Scheme I-4.

The amide condensation reaction in Scheme I-5 can be carried out by conventional techniques and methods well known to those skilled in the art. For example, the amide condensation reaction can be carried out in the presence of HATU/DIEA/DMF, or HOAt/EDCI/TEA/DCM at room temperature.

The specific procedure of Scheme I-5 can be as follows:

To a solution of carboxylic acid substrate 1 (1.1 eq.) and amine substrate 2 (1.0 eq.) in anhydrous DMF (2 mL) were added triethylamine (3.0 eq.) and HATU (1.3 eq.). The reaction mixture was stirred at room temperature for 2 hours. After the completion of the reaction was confirmed by LC-MS, the reaction mixture was filtered, and the filtrate was purified by high performance liquid chromatography (HPLC) to afford the target compound.

### General synthesis method 1 for compounds of Formula (II):

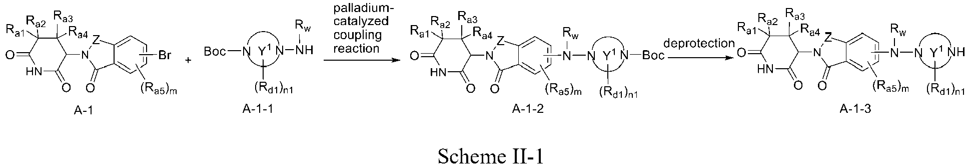

In Scheme II-1, Z, Rₐ₁, Rₐ₂, Rₐ₃, Rₐ₄, (Rₐ₅)ₘ, R_{w}, ring Y¹, and (R_{d1})ₙ₁ are as defined in the compound of Formula (II) and its various sub-embodiments of the present disclosure.

The palladium-catalyzed coupling reaction and deprotection in Scheme II-1 can be carried out by conventional techniques and methods well known to those skilled in the art.

The specific procedure of Scheme II-1 can be as follows:
Step 1: At room temperature, to a 100 mL round-bottom flask were sequentially added compound A-1 (1.0 eq.), compound A-1-1 (1.2 eq.), cesium carbonate (2.0 eq.), palladium catalyst (0.025 eq.), and N,N-dimethylformamide. Under a nitrogen atmosphere, the reaction mixture was heated to 80 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound A-1-2 (gray solid, yield 73%).
Step 2: At room temperature, to a solution of compound A-1-2 in dichloromethane in a 100 mL round-bottom flask was added trifluoroacetic acid. The reaction mixture was stirred for 1 hour, and the completion of the reaction was confirmed by TLC. The reaction mixture was concentrated under reduced pressure, and the resulting residue was lyophilized to afford compound A-1-3.

### General synthesis method 2 for compounds of Formula (II):

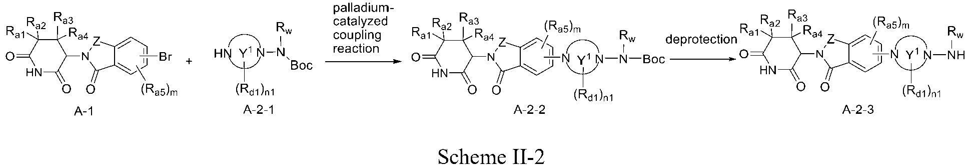

In Scheme II-2, Z, Rₐ₁, Rₐ₂, Rₐ₃, Rₐ₄, (Rₐ₅)ₘ, R_{w}, ring Y¹, and (R_{d1})ₙ₁ are as defined in the compound of Formula (II) and its various sub-embodiments of the present disclosure.

The palladium-catalyzed coupling reaction and deprotection in Scheme II-2 can be carried out by conventional techniques and methods well known to those skilled in the art.

The specific procedure of Scheme II-1 can be as follows:
Step 1: At room temperature, to a 100 mL round-bottom flask were sequentially added compound A-1 (1.0 eq.), compound A-2-1 (1.2 eq.), cesium carbonate (2.0 eq.), palladium catalyst (0.025 eq.), and N,N-dimethylformamide. Under a nitrogen atmosphere, the reaction mixture was heated to 80 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound A-2-2 (gray solid, yield 73%).
Step 2: At room temperature, to a solution of compound A-2-2 in dichloromethane in a 100 mL round-bottom flask was added trifluoroacetic acid. The reaction mixture was stirred for 1 hour, and the completion of the reaction was confirmed by TLC. The reaction mixture was concentrated under reduced pressure, and the resulting residue was lyophilized to afford compound A-2-3.

### General synthesis method 3 for compounds of Formula (II):

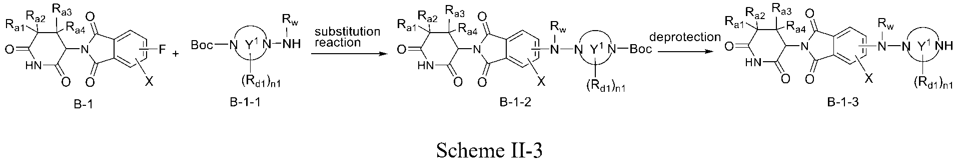

In Scheme II-3, Rₐ₁, Rₐ₂, Rₐ₃, Rₐ₄, R_{w}, ring Y¹, and (R_{d1})ₙ₁ are as defined in the compound of Formula (II) and its various sub-embodiments of the present disclosure. X represents H or F.

The substitution reaction and deprotection in Scheme II-3 can be carried out by conventional techniques and methods well known to those skilled in the art.

The specific procedure of Scheme II-3 can be as follows:
Step 1: At room temperature, to a 100 mL round-bottom flask were sequentially added compound B-1 (1.0 eq.), compound B-1-1 (1.2 eq.), N,N-diisopropylethylamine (5.0 eq.), and dimethyl sulfoxide. Under a nitrogen atmosphere, the reaction mixture was heated to 130 °C and stirred for 16 hours. The completion of the reaction was confirmed by TLC. The reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound B-1-2 (gray solid, yield 62%).
Step 2: At room temperature, to a solution of compound B-1-2 in dichloromethane in a 100 mL round-bottom flask was added trifluoroacetic acid. The reaction mixture was stirred for 1 hour, and the completion of the reaction was confirmed by TLC. The reaction mixture was concentrated under reduced pressure, and the resulting residue was lyophilized to afford compound B-1-3.

### General synthesis method 4 for compounds of Formula (II):

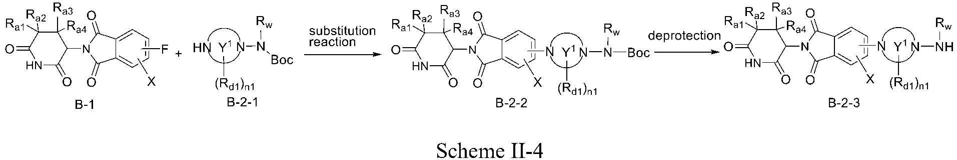

In Scheme II-4, Rₐ₁, Rₐ₂, Rₐ₃, Rₐ₄, R_{w}, ring Y¹, and (R_{d1})ₙ₁ are as defined in the compound of Formula (II) and its various sub-embodiments of the present disclosure. X represents H or F.

The substitution reaction and deprotection in Scheme II-4 can be carried out by conventional techniques and methods well known to those skilled in the art.

The specific procedure of Scheme II-4 can be as follows:
Step 1: At room temperature, to a 100 mL round-bottom flask were sequentially added compound B-1 (1.0 eq.), compound B-2-1 (1.2 eq.), N,N-diisopropylethylamine (5.0 eq.), and dimethyl sulfoxide. Under a nitrogen atmosphere, the reaction mixture was heated to 130 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound B-2-2 (gray solid, yield 62%).
Step 2: At room temperature, to a solution of compound B-2-2 in dichloromethane in a 100 mL round-bottom flask was added trifluoroacetic acid. The reaction mixture was stirred for 1 hour, and the completion of the reaction was confirmed by TLC. The reaction mixture was concentrated under reduced pressure, and the resulting residue was lyophilized to afford compound B-2-3.

### General synthesis method 5 for compounds of Formula (II):

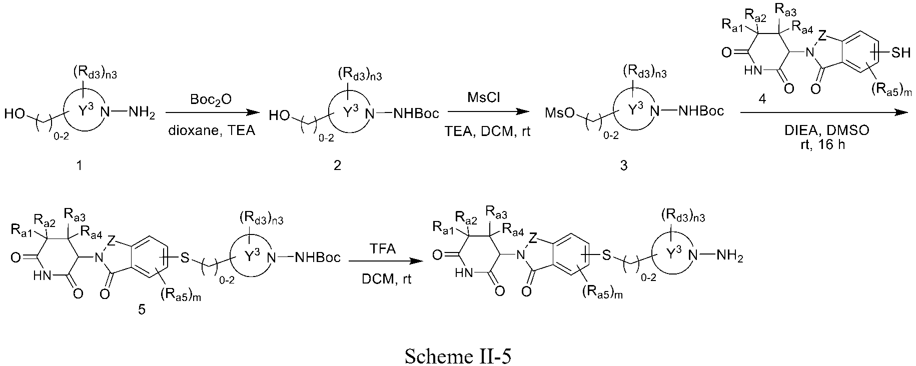

In Scheme II-5, Rₐ₁, Rₐ₂, Rₐ₃, Rₐ₄, Z, (Rₐ₅)ₘ, ring Y³, and (R_{d3})ₙ₃ are as defined in the compound of Formula (I) and its various sub-embodiments of the present disclosure.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

It should be understood that, in each of the above synthetic schemes, when a group contains an active hydrogen or a reactive group that is not intended to participate in the scheme reaction, such active hydrogen or reactive group can be protected by techniques and methods well known to those skilled in the art, for example, using protecting groups. For example, when the reactive group is piperidinyl or piperazinyl, conventional protecting groups such as Boc and the like can be used to protect the hydrogen on the nitrogen. For example, when the reactive group is -CHO, conventional protecting groups can be used to protect the aldehyde group, such as by forming an acetal. The deprotection of the protecting groups can be achieved by techniques and methods well known to those skilled in the art. For example, the removal of the Boc protecting group can be achieved under acidic conditions, such as hydrochloric acid or trifluoroacetic acid. As another example, the removal of the acetal protecting group can be achieved under acidic conditions, such as sulfuric acid, hydrochloric acid, or trifluoroacetic acid.

### Examples

### Intermediate Example 1: Preparation of compound GT-04304

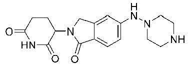

With reference to the method of Scheme II-1, the target compound (GT-04304) was obtained (gray solid, 6.5 g, yield 97 %). ¹H NMR (400 MHz, MeOD) *δ* 7.57 (d, J = 8.4 Hz, 1H), 7.05 (s, 1H), 6.95 (dd, J = 8.4, 1.8 Hz, 1H), 5.08 (dd, J = 13.3, 5.2 Hz, 1H), 4.44 - 4.31 (m, 2H), 3.38 (t, J = 5.1 Hz, 4H), 3.03 (s, 4H), 2.86 (dd, J = 13.3, 5.3 Hz, 1H), 2.81 - 2.72 (m, 1H), 2.45 (dd, J = 13.1, 4.7 Hz, 1H), 2.17 - 2.09 (m, 1H). LCMS (ESI) calcd. for C₁₇H₂₂N₅O₃⁺ [M+H]⁺: 344.17, found, 344.2.

### Example 1 of compound of Formula (II): Preparation of compound GT-06985

With reference to the method of Scheme II-1, the target compound (GT-06985) was obtained (gray solid, 11.7 g, yield 99 %). ¹H NMR (400 MHz, MeOD) *δ* 7.49 (d, J = 8.3 Hz, 1H), 7.45 - 7.36 (m, 1H), 5.09 (dd, J = 13.3, 5.1 Hz, 1H), 4.47 (q, J = 16.7 Hz, 2H), 3.44 - 3.36 (m, 4H), 3.09 (d, J = 4.6 Hz, 4H), 2.95 - 2.85 (m, 1H), 2.81 - 2.75 (m, 1H), 2.48 (qd, J = 13.2, 4.7 Hz, 1H), 2.19 - 2.10 (m, 1H). LCMS (ESI) calcd. for C₁₇H₂₁FN₅O₃⁺ [M+H]⁺: 362.16, found, 362.2.

### Example 2 of compound of Formula (II): Preparation of compound GT-06988

With reference to the method of Scheme II-1, the target compound (GT-06988) was obtained (gray solid, 7.9 g, yield 97 %). ¹H NMR (400 MHz, DMSO-d6) *δ* 10.96 (s, 1H), 8.74 (s, 2H), 7.73 (s, 1H), 7.36 (d, J = 10.7 Hz, 1H), 7.31 (d, J = 7.2 Hz, 1H), 5.05 (dd, J = 13.3, 5.0 Hz, 1H), 4.32 (d, J = 16.8 Hz, 1H), 4.20 (d, J = 16.9 Hz, 1H), 3.25 (s, 4H), 2.98 (s, 4H), 2.92 - 2.82 (m, 1H), 2.59 (d, J = 17.2 Hz, 1H), 2.36 (dd, J = 13.2, 4.4 Hz, 1H), 2.04 - 1.91 (m, 1H). LCMS (ESI) calcd. for C₁₇H₂₁FN₅O₃⁺ [M+H]⁺: 362.16, found, 362.2.

### Example 3 of compound of Formula (II): Preparation of compound GT-06989

With reference to the method of Scheme II-1, the target compound (GT-06989) was obtained (gray solid, 5.4 g, yield 87 %). ¹H NMR (400 MHz, MeOD) *δ* 6.77 (s, 1H), 6.65 (d, J = 12.1 Hz, 1H), 5.03 (dd, J = 13.3, 5.1 Hz, 1H), 4.35 (q, J = 17.0 Hz, 2H), 3.38 (t, J = 4.8 Hz, 4H), 3.02 (s, 4H), 2.85 (dd, J = 13.3, 5.2 Hz, 1H), 2.79 - 2.69 (m, 1H), 2.42 (dd, J = 13.1, 4.7 Hz, 1H), 2.17 - 2.07 (m, 1H). LCMS (ESI) calcd. for C₁₇H₂₁FN₅O₃⁺ [M+H]⁺: 362.16, found, 362.2.

### Example 4 of compound of Formula (II): Preparation of compound GT-07368

With reference to the method of Scheme II-1, the target compound (GT-07368) was obtained (gray solid, 5.4 g, yield 98 %). ¹H NMR (400 MHz, DMSO-d6) δ 10.98 (s, 1H), 8.78 (s, 2H), 7.30 (t, *J* = 7.6 Hz, 1H), 7.17 (s, 1H), 7.08 (dd, *J* = 7.6, 4.4 Hz, 2H), 5.09 (dd, *J =* 13.2, 5.2 Hz, 1H), 4.36 (d, *J =* 17.6 Hz, 1H), 4.24 (d, *J =* 17.6 Hz, 1H), 3.24 (s, 4H), 2.93 (s, 4H), 2.89 (d, *J =* 5.2 Hz, 1H), 2.62 (d, *J =* 17.2 Hz, 1H), 2.40 (qd, *J =* 13.2, 4.4 Hz, 1H), 2.07 - 1.97 (m, 1H). LCMS (ESI) calcd. for C₁₇H₂₂N₅O₃⁺ [M+H]⁺: 344.17, found, 344.1.

### Example 5 of compound of Formula (II): Preparation of compound GT-05579

With reference to the method of Scheme II-1, the target compound (GT-05579) was obtained (gray solid, 4.1 g, yield 97 %). ¹H NMR (400 MHz, DMSO-D6) δ 11.07 (s, 1H), 8.85 (s, 2H), 8.35 (s, 1H), 7.64 (d, *J =* 8.3 Hz, 1H), 7.23 (s, 1H), 7.06 (d, *J =* 7.8 Hz, 1H), 5.05 (dd, *J =* 12.9, 5.3 Hz, 1H), 3.68 (d, *J =* 4.3 Hz, 1H), 3.31 (d, *J =* 18.6 Hz, 4H), 2.88 (td, *J =* 14.6, 5.2 Hz, 4H), 2.56 (dd, *J =* 20.1, 10.7 Hz, 2H), 2.01 (dd, *J =* 9.9, 4.8 Hz, 1H). LCMS (ESI) calcd. for C₁₇H₂₀N₅O₄⁺ [M+H]⁺: 358.15, found, 358.1.

### Example 6 of compound of Formula (II): Preparation of compound GT-04310

With reference to the method of Scheme II-3, the target compound (GT-04310) was obtained (pale yellow solid, 1.5 g, yield 96 %). ¹H NMR (400 MHz, DMSO-D6) δ 11.09 (s, 1H), 8.84 (s, 1H), 8.42 (s, 1H), 7.65 (d, *J =* 10.3 Hz, 1H), 7.57 (d, *J =* 7.0 Hz, 1H), 5.08 (dd, *J =* 12.8, 5.4 Hz, 1H), 3.30 (s, 4H), 2.97 (d, *J =* 33.3 Hz, 4H), 2.93 - 2.83 (m, 1H), 2.56 (dd, *J* = 22.6, 11.2 Hz, 2H), 2.05 - 1.97 (m, 1H). LCMS (ESI) calcd. for C₁₇H₁₉FN₅O₄⁺ [M+H]⁺: 376.14, found, 376.2.

### Example 7 of compound of Formula (II): Preparation of compound GT-07007

With reference to the method of Scheme II-4, the target compound (GT-07007) was obtained (gray solid, 3.6 g, yield 92 %). ¹H NMR (400 MHz, DMSO-d6) δ 11.09 (s, 1H), 9.48 (s, 2H), 7.74 (d, *J =* 8.5 Hz, 1H), 7.41 (d, *J* = 2.0 Hz, 1H), 7.30 (dd, *J* = 8.5, 2.0 Hz, 1H), 5.09 (dd, *J=* 12.9, 5.4 Hz, 1H), 3.58 (s, 4H), 3.02 (s, 4H), 2.92 - 2.83 (m, 1H), 2.57 (dd, *J =* 19.3, 10.3 Hz, 2H), 2.08 - 1.99 (m, 1H). LCMS (ESI) calcd. for C₁₇H₂₀N₅O₄⁺ [M+H]⁺: 358.15, found, 358.2.

### Example 8 of compound of Formula (II): Preparation of compound GT-07051

With reference to the method of Scheme II-4, the target compound (GT-07051) was obtained (gray solid, 1.6 g, yield 97 %). ¹H NMR (400 MHz, DMSO-d6) δ 10.95 (s, 1H), 9.65 (s, 2H), 7.55 (d, *J =* 8.4 Hz, 1H), 7.13 - 7.06 (m, 2H), 5.05 (dd, *J* = 13.3, 5.1 Hz, 1H), 4.34 (d, *J =* 17.0 Hz, 1H), 4.23 (t*, J =* 17.0 Hz, 1H), 3.46 - 3.30 (m, 4H), 3.11 (brs, 4H), 2.94 - 2.84 (m, 1H), 2.58 (d, *J =* 17.2 Hz, 1H), 2.43 - 2.31 (m, 1H), 1.98 - 1.90 (m, 1H). LCMS (ESI) calcd. for C₁₇H₂₂N₅O₃⁺ [M+H]⁺: 344.17, found, 344.2.

### Example 9 of compound of Formula (II): Preparation of compound GT-07008

The target compound GT-07008 was prepared by referring to the method of Scheme II-5.

Step 1: In a 100 mL round-bottom flask, to a solution of (1-aminopiperidin-4-yl)methanol (1.0 eq.) in 1,4-dioxane were sequentially added (Boc)₂O (1.2 eq.) and triethylamine (3.0 eq.). The reaction mixture was stirred at room temperature for 16 hours. The completion of the reaction was confirmed by TLC. The reaction mixture was concentrated under reduced pressure. The resulting concentrate was washed with water, and extracted with ethyl acetate three times. The organic phases were combined, dried over anhydrous sodium sulfate, and concentrated under reduced pressure. The resulting residue was purified by column chromatography (eluent: petroleum ether/ethyl acetate=0-50%) to afford tert-butyl (4-(hydroxymethyl)piperidin-1-yl)carbamate as a white solid (yield 80%).

Step 2: In a 100 mL round-bottom flask, to a solution of tert-butyl (4-(hydroxymethyl)piperidin-1-yl)carbamate (1.0 eq.) in dichloromethane were sequentially added MsCl (1.2 eq.) and triethylamine (3.0 eq.). The reaction mixture was stirred at room temperature for 1 hour. The completion of the reaction was confirmed by TLC. The reaction mixture was washed with water, and extracted with dichloromethane three times. The organic phases were combined, dried over anhydrous sodium sulfate, and concentrated under reduced pressure. The resulting residue was purified by column chromatography (eluent: petroleum ether/ethyl acetate = 0-40%) to afford (1-((tert-butoxycarbonyl)amino)piperidin-4-yl)methyl methanesulfonate as a white solid (yield 85%).

Step 3: In a 100 mL round-bottom flask, to a solution of (1-((tert-butoxycarbonyl)amino)piperidin-4-yl)methyl methanesulfonate (1.3 eq.) and 3-(5-mercapto-1-oxoisoindolin-2-yl)piperidine-2,6-dione (1.0 eq.) in dimethyl sulfoxide was added triethylamine (3.0 eq.). The reaction mixture was stirred at room temperature for 16 hours. The completion of the reaction was confirmed by TLC. The reaction mixture was washed with water, and extracted with ethyl acetate three times. The organic phases were combined, dried over anhydrous sodium sulfate, and concentrated under reduced pressure. The resulting residue was purified by column chromatography (eluent: petroleum ether/ethyl acetate = 0-50%) to afford tert-butyl (4-(((2-(2,6-dioxopiperidin-3-yl)-1-oxoisoindolin-5-yl)thio)methyl)piperidin-1-yl)carbamate as a white solid (yield 50%).

Step 4: At room temperature, to a solution of tert-butyl (4-(((2-(2,6-dioxopiperidin-3-yl)-1-oxoisoindolin-5-yl)thio)methyl)piperidin-1-yl)carbamate in dichloromethane in a 100 mL round-bottom flask was added trifluoroacetic acid. The reaction mixture was stirred for 1 hour, and the completion of the reaction was confirmed by TLC. The reaction mixture was concentrated under reduced pressure, and the resulting residue was lyophilized to afford the target compound GT-07008 (gray solid, 0.6 g, yield 93 %). ¹H NMR (400 MHz, MeOD) δ 11.80 (s, 1H), 10.03 (s, 1H), 8.48 - 8.43 (m, 1H), 8.38 (s, 1H), 8.25 (d, *J =* 7.1 Hz, 1H), 5.91 (dd, *J =* 13.3, 5.1 Hz, 1H), 5.17 (dd, *J* = 50.0, 17.4 Hz, 2H), 4.26 (s, 4H), 3.86 (d, *J* = 6.1 Hz, 2H), 3.77 - 3.68 (m, 1H), 3.42 (d, *J =* 17.0 Hz, 1H), 3.30 - 3.12 (m, 2H), 2.89 - 2.68 (m, 3H), 2.27 - 2.16 (m, 2H). LCMS (ESI) calcd. for C₁₉H₂₅N₄O₃S⁺ [M+H]⁺: 389.16, found, 389.2.

### Example 10 of compound of Formula (II): Preparation of compound GT-07966

With reference to the method of Scheme II-1, the target compound (GT-07966) was obtained (yellow solid, 1.56 g, yield 94 %). ¹H NMR (400 MHz, DMSO-d6) δ 10.93 (s, 1H), 8.99 (s, 2H), 7.56 (s, 1H), 7.46 (d, J = 8.4 Hz, 1H), 6.94(s, 1H), 6.87 (d, J = 8.4 Hz, 1H), 5.03 (dd, J = 13.2, 5.2 Hz, 1H), 4.31 (d, J = 16.8 Hz, 1H), 4.18 (d, J =16.8 Hz, 1H), 4.08 - 4.01 (m, 2H), 2.99 - 2.82 (m, 5H), 2.58 (d,J = 16.8 Hz, 1H), 2.33 (dd, J = 13.2, 4.4 Hz, 1H), 2.15 (d, J = 8.8 Hz, 2H), 1.98 - 1.89 (m, 3H). LCMS (ESI) calcd. for C₁₉H₂₄N₅O₃⁺: 370.19; found, 370.1.

### Example 11 of compound of Formula (II): Preparation of compound GT-08008

With reference to the method of Scheme II-1, the target compound (GT-08008) was obtained (yellow solid, 1.7 g, yield 83 %). ¹H NMR (400 MHz, DMSO-d6) δ 10.93 (s, 1H), 9.49 (s, 1H), 7.98 (s, 1H), 7.47 (d, J = 8.4 Hz, 2H), 7.03(s, 1H), 6.96 (d, J = 8.4 Hz, 1H), 5.03 (dd, J = 13.2, 5.2 Hz, 1H), 4.30 (dd, J = 11.2, 5.6 Hz, 3H), 4.18 (d, J = 16.8 Hz, 1H), 3.46 (d, J = 10.4 Hz, 2H), 3.34 (d, J = 11.2 Hz, 2H), 2.96 - 2.85 (m, 1H), 2.75 - 2.64 (m,1H), 2.58 (d, J = 16.8 Hz, 1H), 2.35 (ddd, J = 26.0, 13.2, 4.4 Hz, 2H), 1.98 - 1.91 (m, 1H). LCMS (ESI) calcd. for C₁₈H₂₂N₅O₃⁺: 356.16; found, 356.1.

### Example 12 of compound of Formula (II): Preparation of compound GT-08009

With reference to the method of Scheme II-1, the target compound (GT-08009) was obtained (yellow solid, 1.2 g, yield 75 %). ¹H NMR (400 MHz, DMSO-d6) δ 10.93 (s, 1H), 9.03 (s, 1H), 8.77 (s, 1H), 7.72 (s, 1H), 7.45 (d, J = 8.4Hz, 1H), 6.96 (s, 1H), 6.86 (d, J = 8.4 Hz, 1H), 5.03 (dd, J = 13.2, 5.2 Hz, 1H), 4.30 (d, J = 16.4 Hz, 2H),4.17 (d, J = 16.8 Hz, 1H), 3.63 (s, 1H), 3.15 (s, 2H), 3.07 - 2.77 (m, 2H), 2.58 (d, J = 16.8 Hz, 1H), 2.39 -2.14 (m, 2H), 1.95 (dd, J = 9.2, 3.6 Hz, 1H), 1.75 (d, J = 10.8 Hz, 1H), 0.83 (dd, J = 9.6, 6.8 Hz, 1H). LCMS (ESI) calcd. for C₁₈H₂₂N₅O₃⁺: 356.17; found, 356.1.

### Example 13 of compound of Formula (II): Preparation of compound GT-08007

With reference to the method of Scheme II-1, the target compound () was obtained GT-08007 (brown solid, 1.1 g, yield 99 %). ¹H NMR (400 MHz, DMSO-D6) δ 10.94 (s, 1H), 8.91 (s, 2H), 7.73 (s, 1H), 7.45 (d, J = 8.4 Hz, 1H), 6.96 (s, 1H), 6.87 (d, J = 8.4 Hz, 1H), 5.03 (dd, J = 13.2, 5.2 Hz, 1H), 4.31 (d, J = 16.8 Hz, 1H), 4.18 (d, J = 16.8 Hz, 1H), 3.26 (d, J = 4.8 Hz, 4H), 3.12 (s, 2H), 2.91 (ddd, J = 18.4, 13.6, 6.4 Hz, 3H), 2.59 (d, J = 16.8 Hz, 1H), 2.35 (qd, J = 13.2, 4.4 Hz, 1H), 2.04 - 1.82 (m, 3H). LCMS (ESI) calcd. for C₁₃H₂₄N₅O₃⁺ [M+H]⁺: 358.18; found, 358.2.

### Example 14 of compound of Formula (II): Preparation of compound GT-08006

With reference to the method of Scheme II-1, the target compound (GT-08006) was obtained (brown solid, 980 mg, yield 95%). ¹H NMR (400 MHz, DMSO-D6) δ 10.99 (s, 1H), 9.02 (s, 2H), 7.49 (d, *J =* 8.4 Hz, 1H), 7.28 (d, *J =* 16.8 Hz, 1H), 6.94 (s, 1H), 6.88 (d, *J =* 8.4 Hz, 1H), 5.08 (dd, *J =* 13.2, 5.2 Hz, 1H), 4.35 (d, *J* = 16.8 Hz, 1H), 4.21 (d, *J =* 16.8 Hz, 1H), 3.80 (t, *J =* 6.0 Hz, 4H), 3.01 - 2.90 (m, 1H), 2.64 (d, *J =* 16.8 Hz, 5H), 2.39 (ddd, *J* = 26.0, 13.2, 4.4 Hz, 1H), 1.99 (dd, *J* = 17.6, 10.4 Hz, 5H). LCMS (ESI) calcd. for C₂₀H₂₆N₅O₃⁺ [M+H]⁺: 384.20; found, 384.3.

### Example 15 of compound of Formula (II): Preparation of compound GT-07965

With reference to the method of Scheme II-1, the target compound (GT-07965) was obtained (off-white solid, 1.1 g, yield 99 %). ¹H NMR (400 MHz, DMSO-D6) δ 10.94 (s, 1H), 8.93 (s, 1H), 8.76 (s, 1H), 7.44 (d, *J* = 8.4 Hz, 1H), 7.24 (s, 1H), 6.95 (s, 1H), 6.89 (d, *J =* 8.0 Hz, 1H), 5.03 (dd, *J =* 13.2, 4.8 Hz, 1H), 4.29 (d, *J =* 16.8 Hz, 1H), 4.19 (s, 1H), 3.47 (s, 2H), 3.10 - 2.82 (m, 7H), 2.68 (s, 2H), 2.59 (d, *J =* 17.6 Hz, 1H), 2.34 (dt, *J =* 22.4, 11.2 Hz, 1H), 2.02 - 1.90 (m, 1H). LCMS (ESI) calcd. for C₁₉H₂₄N₅O₃⁺ [M+H]⁺: 370.18; found, 370.1.

### Example 16 of compound of Formula (II): Preparation of compound GT-09916

With reference to the method of Scheme II-1, the target compound (GT-09916) was obtained (off-white solid, 503 mg, yield 95 %). ¹H NMR (400 MHz, DMSO-D6) δ 11.10 (s, 1H), 8.75 (s, 2H), 8.41 (s, 1H), 7.58 (d, J = 8.2 Hz, 1H), 7.53 - 7.44 (m, 1H), 5.06 (dd, J = 12.8, 5.4 Hz, 1H), 3.29 (d, J = 20.1 Hz, 4H), 2.96 (d, J = 26.0 Hz, 4H), 2.91 - 2.85 (m, 1H), 2.63 - 2.55 (m, 1H), 2.49 (d, J = 4.9 Hz, 1H), 2.07 - 1.97 (m, 1H). LCMS (ESI) calcd. for C₁₇H₁₉FN₅O₄⁺ [M+H]⁺: 376.14, found, 376.2.

### Example 17 of compound of Formula (II): Preparation of compound GT-09995

With reference to the method of Scheme II-1, the target compound (GT-09995) was obtained (off-white solid, 2110 mg, yield 91 %). ¹H NMR (400 MHz, DMSO-d6) δ 11.09 (s, 1H), 8.87 (s, 2H), 8.75 (s, 1H), 7.04 (s, 1H), 6.85 (s, 1H), 5.05 (dd, J = 12.8, 5.4 Hz, 1H), 3.45 - 3.22 (m, 4H), 3.15 - 2.67 (m, 5H), 2.58 (d, J = 19.9 Hz, 1H), 2.50 - 2.43 (m, 1H), 2.01 (dd, J = 9.1, 3.7 Hz, 1H). LCMS (ESI) calcd. for C₁₇H₁₉FN₅O₄⁺ [M+H]⁺: 376.14, found, 376.2.

### Example 18 of compound of Formula (II): Preparation of compound GT-08195

With reference to the method of Scheme II-1, the target compound (GT-08195) was obtained (gray solid, 480 mg, yield 99%). ¹H NMR (400 MHz, DMSO-d6) δ 10.94 (s, 1H), 9.26 (s, 1H), 8.56 (s, 1H), 7.46 (d, *J* = 8.4 Hz, 2H), 7.09 (s, 1H), 6.99 (d, *J* = 8.4 Hz, 1H), 5.04 (dd, *J =* 13.2, 5.2 Hz, 1H), 4.31 (d, *J =* 16.8 Hz, 1H), 4.18 (d, *J =* 16.8 Hz, 1H), 3.39 (s, 2H), 3.30 (s, 2H), 3.12 (d, *J =* 32.4 Hz, 2H), 2.97 - 2.85 (m, 1H), 2.59 (d, *J =* 17.2 Hz, 1H), 2.42 - 2.28 (m, 1H), 2.20 (s, 2H), 1.94 (t, *J =* 12.8 Hz, 3H). LCMS (ESI) calcd. for C₁₉H₂₄N₅O₃⁺ [M+H]⁺: 370.19, found, 370.2.

### Example 1 of compound of Formula (I): Preparation of compound GT-07089

With reference to the method of Scheme I-1, the target compound (GT-07089) was obtained (white solid, 28 mg, yield 40.95%). ¹H NMR (400 MHz, DMSO-d6) δ 11.62 (s, 1H), 11.09 (s, 1H), 10.95 (s, 1H), 9.02 (s, 1H), 8.21 (dd, J = 9.5, 2.4 Hz, 1H), 8.04 (s, 1H), 7.64 (d, J = 9.5 Hz, 1H), 7.47 (d, J = 8.3 Hz, 1H), 6.97 (s, 1H), 6.91 (d, J = 8.6 Hz, 1H), 6.84 (s, 1H), 5.04 (dd, J = 13.2, 5.0 Hz, 1H), 4.87 - 4.73 (m, 1H), 4.31 (d, J = 16.9 Hz, 2H), 3.69 (s, 3H), 3.55 (s, 2H), 3.25 (d, J = 6.9 Hz, 4H), 3.07 (s, 9H), 2.88 (t, J = 12.5 Hz, 3H), 2.59 (d, J = 16.6 Hz, 1H), 2.35 (dd, J = 19.9, 7.2 Hz, 3H), 2.19 - 1.93 (m, 8H), 1.74 - 1.58 (m, 2H), 1.46 (d, J = 10.5 Hz, 2H). LCMS (ESI) calcd. for C₄₂H₅₃N₁₂O₄⁺ [M+H]⁺: 789.43, found, 789.5.

### Example 2 of compound of Formula (I): Preparation of compound GT-07090

With reference to the method of Scheme I-1, the target compound (GT-07090) was obtained (white solid, 32 mg, yield 47.07%). ¹H NMR (400 MHz, DMSO-d6) δ 10.93 (s, 1H), 10.84 (s, 1H), 8.61 (d, J = 8.1 Hz, 1H), 7.87 (dd, J = 11.3, 9.2 Hz, 2H), 7.51 (d, J = 9.7 Hz, 1H), 7.46 (d, J = 8.4 Hz, 1H), 7.38 (d, J = 2.3 Hz, 1H), 7.13 (dd, J = 8.8, 2.4 Hz, 1H), 6.95 (s, 1H), 6.89 (d, J = 8.3 Hz, 1H), 5.03 (dd, J = 13.2, 5.1 Hz, 1H), 4.52 (dd, J = 23.0, 12.0 Hz, 3H), 4.36 - 4.16 (m, 2H), 3.55 (d, J = 8.5 Hz, 2H), 3.11 (ddd, J = 26.2, 20.2, 8.8 Hz, 10H), 2.95 - 2.83 (m, 1H), 2.58 (d, J = 16.6 Hz, 1H), 2.33 (ddd, J = 40.3, 20.3, 15.8 Hz, 2H), 2.10 (d, J = 9.9 Hz, 2H), 2.04 - 1.84 (m, 5H), 1.70 - 1.47 (m, 4H), 1.35 - 1.23 (m, 2H). LCMS (ESI) calcd. for C₄₁H₄₈ClN₁₀O₅⁺ [M+H]⁺: 795.35, found, 795.3.

### Example 3 of compound of Formula (I): Preparation of compound GT-06941

With reference to the method of Scheme I-1, the target compound (GT-06941) was obtained (white solid, 30 mg, yield 44.80%). ¹H NMR (400 MHz, DMSO-d6) δ 10.94 (s, 1H), 10.42 (s, 1H), 9.51 (d, J = 6.3 Hz, 1H), 8.78 (d, J = 7.7 Hz, 1H), 8.40 (d, J = 3.7 Hz, 1H), 8.27 (t, J = 7.1 Hz, 1H), 7.77 (s, 1H), 7.45 (t, J = 10.2 Hz, 1H), 7.32 - 6.93 (m, 2H), 6.91 - 6.38 (m, 2H), 5.39 - 4.97 (m, 2H), 4.76 (d, J = 18.5 Hz, 1H), 4.37 - 4.15 (m, 3H), 3.87 = 3.71 (m, 2H), 3.66 - 3.53 (m, 4H), 3.32 - 2.98 (m, 8H), 2.90 (s, 1H), 2.64 - 2.54 (m, 1H), 2.35 (dd, J = 13.2, 4.3 Hz, 1H), 2.12 - 1.87 (m, 8H), 1.80 (d, J = 11.7 Hz, 2H), 1.22 (d, J = 12.4 Hz, 2H). LCMS (ESI) calcd. for C₄₀H₄₇F₂N₁₂O₅⁺ [M+H]⁺: 813.38, found, 813.4.

### Example 4 of compound of Formula (I): Preparation of compound GT-07091

With reference to the method of Scheme I-1, the target compound (GT-07091) was obtained (white solid, 35 mg, yield 52.67%). ¹H NMR (400 MHz, DMSO-d6) δ 10.94 (s, 1H), 10.67 (s, 1H), 8.58 (d, J = 2.0 Hz, 1H), 8.18 (d, J = 8.3 Hz, 1H), 7.95 (d, J = 8.5 Hz, 1H), 7.90 (d, J = 8.8 Hz, 1H), 7.47 (d, J = 8.4 Hz, 1H), 7.21 (d, J = 2.4 Hz, 2H), 7.01 (dd, J = 8.8, 2.4 Hz, 1H), 6.95 (s, 1H), 6.89 (d, J = 8.3 Hz, 1H), 5.03 (dd, J = 13.2, 5.1 Hz, 1H), 4.45 (d, J = 13.1 Hz, 2H), 4.37 - 4.15 (m, 3H), 4.07 (d, J = 9.1 Hz, 1H), 3.53 - 3.43 (m, 3H), 3.24 - 3.00 (m, 10H), 2.96 - 2.82 (m, 1H), 2.62 - 2.53 (m, 1H), 2.35 (dd, J = 13.0, 4.3 Hz, 1H), 2.23 (s, 1H), 1.96 (dd, J = 16.8, 8.9 Hz, 3H), 1.43 - 1.25 (m, 2H), 1.23 (s, 6H), 1.13 (s, 6H). LCMS (ESI) calcd. for C₄₄H₅₃ClN₉O₅⁺ [M+H]⁺: 822.39, found, 822.5

### Example 5 of compound of Formula (I): Preparation of compound GT-06948

With reference to the method of Scheme I-1, the target compound (GT-06948) was obtained (white solid, 28 mg, yield 39.36%). ¹H NMR (400 MHz, DMSO-d6) δ 11.60 (s, 1H), 11.09 (s, 1H), 10.98 (s, 1H), 9.01 (s, 1H), 8.55 (s, 1H), 8.19 (dd, *J* = 9.6, 2.4 Hz, 1H), 8.02 (d, *J =* 13.1 Hz, 1H), 7.64 (dd, *J* = 9.8, 4.9 Hz, 2H), 7.56 (d, *J =* 6.9 Hz, 1H), 6.83 (s, 1H), 5.07 (dd, *J =* 12.7, 5.4 Hz, 1H), 4.80 (dd, *J* = 17.4, 8.7 Hz, 1H), 3.59 - 3.48 (m, 5H), 3.33 (d, *J =* 9.1 Hz, 4H), 3.05 (d, *J =* 7.9 Hz, 10H), 2.89 - 2.77 (m, 3H), 2.58 (d, *J =* 18.3 Hz, 1H), 2.30 (s, 2H), 2.14 - 1.92 (m, 8H), 1.64 (d, *J =* 5.8 Hz, 2H), 1.44 (d, *J* = 10.6 Hz, 2H). LCMS (ESI) calcd. for C₄₂H₅₀FN₁₂O₅⁺ [M+H]⁺: 821.40, found, 821.5.

### Example 6 of compound of Formula (I): Preparation of compound GT-06949

With reference to the method of Scheme I-1, the target compound (GT-06949) was obtained (white solid, 29 mg, yield 42.00%). ¹H NMR (400 MHz, DMSO-d6) δ 11.09 (s, 1H), 10.59 (s, 1H), 8.59 (d, J = 1.8 Hz, 1H), 8.52 (s, 1H), 8.35 - 7.86 (m, 3H), 7.69 - 7.54 (m, 2H), 7.20 (t, J = 5.3 Hz, 2H), 7.01 (dd, J = 8.8, 2.3 Hz, 1H), 5.07 (dd, J = 12.7, 5.4 Hz, 1H), 4.46 (d, J = 12.8 Hz, 2H), 4.35 (s, 1H), 4.07 (d, J = 9.2 Hz, 1H), 3.28 (d, J = 8.7 Hz, 4H), 3.16 - 3.00 (m, 6H), 2.85 (ddd, J = 36.7, 29.5, 11.0 Hz, 3H), 2.56 (dd, J = 19.8, 10.2 Hz, 2H), 2.23 (s, 1H), 2.00 (dd, J = 19.0, 9.5 Hz, 3H), 1.39 - 1.25 (m, 2H), 1.23 (s, 6H), 1.13 (s, 6H). LCMS (ESI) calcd. for C₄₄H₅₀ClFN₉O₆⁺ [M+H]⁺: 854.36, found, 854.5.

### Example 7 of compound of Formula (I): Preparation of compound GT-06950

With reference to the method of Scheme I-1, the target compound (GT-06950) was obtained (white solid, 34 mg, yield 48.08%). ¹H NMR (400 MHz, DMSO-d6) δ 11.09 (s, 1H), 10.51 (s, 1H), 8.60 (d, J = 8.2 Hz, 1H), 8.51 (s, 1H), 7.91 - 7.82 (m, 2H), 7.65 (d, J = 10.2 Hz, 1H), 7.57 (d, J = 7.0 Hz, 1H), 7.44 (d, J = 9.7 Hz, 1H), 7.38 (d, J = 2.4 Hz, 1H), 7.13 (dd, J = 8.8, 2.4 Hz, 1H), 5.07 (dd, J = 12.8, 5.4 Hz, 1H), 4.52 (dd, J = 21.2, 11.4 Hz, 3H), 3.85 (dd, J = 11.4, 7.7 Hz, 1H), 3.41 - 3.22 (m, 4H), 3.19 - 3.00 (m, 6H), 2.98 - 2.77 (m, 2H), 2.64 - 2.52 (m, 2H), 2.22 (s, 1H), 2.00 (ddd, J = 45.5, 34.0, 14.0 Hz, 7H), 1.72 - 1.20 (m, 7H), 0.93 (t, J = 7.3 Hz, 1H). LCMS (ESI) calcd. for C₄₁H₄₅ClFN₁₀O₆⁺ [M+H]⁺: 827.32, found, 827.3.

### Example 8 of compound of Formula (I): Preparation of compound GT-06951

With reference to the method of Scheme I-1, the target compound (GT-06951) was obtained (white solid, 27 mg, yield 38.79%). ¹H NMR (400 MHz, DMSO-d6) δ 11.09 (s, 1H), 10.02 (s, 1H), 9.51 (d, J = 6.4 Hz, 1H), 8.78 (d, J = 7.7 Hz, 1H), 8.48 (s, 1H), 8.40 (d, J = 3.7 Hz, 1H), 8.25 (d, J = 5.3 Hz, 1H), 7.65 (d, J = 10.2 Hz, 1H), 7.57 (d, J = 6.9 Hz, 1H), 7.11 (dd, J = 55.2, 52.2 Hz, 1H), 6.66 (dd, J = 165.7, 7.8 Hz, 1H), 5.13 (dd, J = 48.6, 40.4 Hz, 2H), 4.77 (d, J = 19.5 Hz, 1H), 4.24 (s, 1H), 3.87 - 3.72 (m, 2H), 3.67 - 3.43 (m, 4H), 3.33 - 3.28 (m, 3H), 3.20 (t, J = 11.3 Hz, 2H), 3.14 - 3.00 (m, 4H), 2.91 (dd, J = 32.3, 15.2 Hz, 2H), 2.64 - 2.51 (m, 2H), 2.13 - 1.76 (m, 10H), 1.22 (d, J = 11.6 Hz, 2H). LCMS (ESI) calcd. for C₄₀H₄₄F₃N₁₂O₆⁺ [M+H]⁺: 845.35, found, 845.3.

### Example 9 of compound of Formula (I): Preparation of compound GT-07095

With reference to the method of Scheme I-1, the target compound (GT-07095) was obtained (white solid, 23 mg, yield 32.89%). ¹H NMR (400 MHz, DMSO-d6) δ 11.59 (s, 1H), 10.97 (s, 1H), 10.87 (s, 1H), 9.02 (d, J = 2.5 Hz, 1H), 8.19 (d, J = 9.7 Hz, 1H), 7.99 (s, 1H), 7.81 (s, 1H), 7.64 (d, J = 9.5 Hz, 1H), 7.43 (d, J = 8.2 Hz, 1H), 7.38 - 7.27 (m, 1H), 6.84 (s, 1H), 5.05 (dd, J = 13.2, 5.0 Hz, 1H), 4.81 (dd, J = 17.4, 8.7 Hz, 1H), 4.38 (dd, J = 71.9, 16.7 Hz, 2H), 3.71 (s, 2H), 3.51 - 3.42 (m, 3H), 3.29 (dd, J = 18.7, 7.2 Hz, 4H), 3.06 (d, J = 7.7 Hz, 9H), 2.86 (dd, J = 25.0, 14.1 Hz, 3H), 2.60 (d, J = 16.8 Hz, 1H), 2.46 - 2.23 (m, 3H), 2.01 (dd, J = 16.0, 9.6 Hz, 8H), 1.66 (d, J = 5.9 Hz, 2H), 1.44 (d, J = 10.8 Hz, 2H). LCMS (ESI) calcd. for C₄₂H₅₂FN₁₂O₄⁺ [M+H]⁺: 807.42, found, 807.5.

### Example 10 of compound of Formula (I): Preparation of compound GT-07096

With reference to the method of Scheme I-1, the target compound (GT-07096) was obtained (white solid, 26 mg, yield 37.18%). ¹H NMR (400 MHz, DMSO-d6) δ 11.53 (d, J = 20.2 Hz, 1H), 10.96 (s, 1H), 10.80 (s, 1H), 9.00 (d, J = 3.8 Hz, 1H), 8.16 (d, J = 9.5 Hz, 1H), 7.96 (s, 1H), 7.80 (s, 1H), 7.62 (d, J = 9.5 Hz, 1H), 7.36 (d, J = 10.6 Hz, 1H), 7.29 (d, J = 7.2 Hz, 1H), 6.82 (d, J = 2.8 Hz, 1H), 5.05 (dd, J = 13.2, 5.1 Hz, 1H), 4.92 - 4.66 (m, 1H), 4.26 (dd, J = 50.4, 16.9 Hz, 2H), 3.69 (s, 2H), 3.57 (s, 3H), 3.25 (s, 3H), 3.06 (s, 9H), 2.87 (ddd, J = 30.6, 20.9, 8.3 Hz, 3H), 2.59 (d, J = 16.4 Hz, 1H), 2.44 - 2.23 (m, 3H), 2.13 - 1.77 (m, 8H), 1.72 - 1.56 (m, 2H), 1.50 - 1.28 (m, 2H). LCMS (ESI) calcd. for C₄₂H₅₂FN₁₂O₄⁺ [M+H]⁺: 807.42, found, 807.5.

### Example 11 of compound of Formula (I): Preparation of compound GT-07097

With reference to the method of Scheme I-1, the target compound (GT-07097) was obtained (white solid, 33 mg, yield 47.19%). ¹H NMR (400 MHz, DMSO-d6) δ 11.59 (s, 1H), 11.06 (s, 1H), 10.96 (s, 1H), 9.02 (s, 1H), 8.19 (dd, J = 9.7, 2.6 Hz, 2H), 7.99 (s, 1H), 7.63 (d, J = 9.5 Hz, 1H), 6.84 (s, 1H), 6.73 (s, 1H), 6.65 (d, J = 12.0 Hz, 1H), 5.09 - 4.93 (m, 1H), 4.82 (s, 1H), 4.25 (dd, J = 56.5, 17.3 Hz, 2H), 3.72 (s, 5H), 3.24 (s, 4H), 3.07 (s, 9H), 2.85 (d, J = 12.3 Hz, 3H), 2.59 (d, J = 16.1 Hz, 1H), 2.32 (dd, J = 12.4, 8.3 Hz, 3H), 2.02 (dd, J = 22.5, 10.9 Hz, 8H), 1.71 - 1.59 (m, 2H), 1.43 (d, J = 11.3 Hz, 2H). LCMS (ESI) calcd. for C₄₂H₅₂FN₁₂O₄⁺ [M+H]⁺: 807.42, found, 807.5.

### Example 12 of compound of Formula (I): Preparation of compound GT-07195

With reference to the method of Scheme I-1, the target compound (GT-07195) was obtained (white solid, 24 mg, yield 34.78%). ¹H NMR (400 MHz, DMSO-d6) δ 10.96 (s, 1H), 10.50 (s, 1H), 8.60 (d, J = 8.2 Hz, 1H), 7.91 - 7.82 (m, 2H), 7.74 (d, J = 35.5 Hz, 1H), 7.48 - 7.40 (m, 2H), 7.38 (d, J = 2.4 Hz, 1H), 7.32 (t, J = 7.7 Hz, 1H), 7.13 (dd, J = 8.8, 2.4 Hz, 1H), 5.05 (dd, J = 13.3, 5.1 Hz, 1H), 4.52 - 4.23 (m, 4H), 3.95 - 3.84 (m, 1H), 3.55 (s, 2H), 3.31 - 3.02 (m, 10H), 2.96 - 2.84 (m, 1H), 2.59 (d, J = 17.1 Hz, 1H), 2.45 - 2.31 (m, 1H), 2.22 (s, 1H), 2.10 (d, J = 9.2 Hz, 2H), 2.02 - 1.83 (m, 5H), 1.73 - 1.22 (m, 7H). LCMS (ESI) calcd. for C₄₁H₄₇ClFN₁₀O₅⁺ [M+H]⁺: 813.34, found, 813.3.

### Example 13 of compound of Formula (I): Preparation of compound GT-07196

With reference to the method of Scheme I-1, the target compound (GT-07196) was obtained (white solid, 20 mg, yield 28.77%). ¹H NMR (400 MHz, DMSO-D6) δ 10.95 (s, 1H), 10.61 (s, 1H), 8.60 (d, J = 8.2 Hz, 1H), 7.86 (dd, J = 9.2, 3.6 Hz, 2H), 7.79 (s, 1H), 7.47 (d, J = 9.7 Hz, 1H), 7.37 (dd, J = 10.0, 6.5 Hz, 2H), 7.29 (d, J = 7.2 Hz, 1H), 7.13 (dd, J = 8.8, 2.4 Hz, 1H), 5.05 (dd, J = 13.3, 5.1 Hz, 1H), 4.56 - 4.15 (m, 5H), 3.55 (s, 2H), 3.27 - 3.01 (m, 10H), 2.96 - 2.84 (m, 1H), 2.59 (d, J = 16.6 Hz, 1H), 2.36 (dd, J = 13.1, 4.5 Hz, 1H), 2.23 (s, 1H), 2.10 (d, J = 9.7 Hz, 2H), 2.00 - 1.82 (m, 5H), 1.48 (ddt, J = 29.3, 22.0, 12.4 Hz, 7H). LCMS (ESI) calcd. for C₄₁H₄₇ClFN₁₀O₅⁺ [M+H]⁺: 813.34, found, 813.3.

### Example 14 of compound of Formula (I): Preparation of compound GT-07100

With reference to the method of Scheme I-1, the target compound (GT-07100) was obtained (white solid, 35 mg, yield 50.35%). ¹H NMR (400 MHz, DMSO-d6) δ 10.95 (s, 1H), 10.73 (s, 1H), 8.61 (d, J = 8.2 Hz, 1H), 8.07 (d, J = 62.8 Hz, 1H), 7.86 (dd, J = 9.2, 4.3 Hz, 2H), 7.48 (d, J = 9.7 Hz, 1H), 7.38 (d, J = 2.4 Hz, 1H), 7.13 (dd, J = 8.8, 2.4 Hz, 1H), 6.71 (s, 1H), 6.63 (d, J = 12.1 Hz, 1H), 4.99 (dd, J = 13.2, 5.0 Hz, 1H), 4.59 - 4.45 (m, 3H), 4.24 (dd, J = 55.8, 17.3 Hz, 2H), 3.93 - 3.83 (m, 1H), 3.56 (s, 2H), 3.25 - 3.02 (m, 9H), 2.95 - 2.80 (m, 1H), 2.63 - 2.54 (m, 1H), 2.34 (td, J = 12.9, 4.2 Hz, 1H), 2.29 - 2.17 (m, 1H), 2.10 (d, J = 9.9 Hz, 2H), 2.02 - 1.83 (m, 5H), 1.66 - 1.21 (m, 7H). LCMS (ESI) calcd. for C₄₁H₄₇ClFN₁₀O₅⁺ [M+H]⁺: 813.34, found, 813.3.

### Example 15 of compound of Formula (I): Preparation of compound GT-07103

With reference to the method of Scheme I-1, the target compound (GT-07103) was obtained (white solid, 10 mg, yield 14.61%). ¹H NMR (400 MHz, DMSO-d6) δ 10.97 (s, 1H), 10.00 (s, 1H), 9.51 (d, J = 6.5 Hz, 1H), 8.78 (d, J = 7.7 Hz, 1H), 8.40 (d, J = 4.0 Hz, 1H), 8.26 (d, J = 5.5 Hz, 1H), 7.76 (s, 1H), 7.43 (d, J = 8.2 Hz, 1H), 7.32 (t, J = 7.7 Hz, 1H), 7.28 - 6.96 (m, 1H), 6.66 (dd, J = 165.7, 7.8 Hz, 1H), 5.34 - 5.01 (m, 2H), 4.77 (d, J = 20.4 Hz, 1H), 4.35 (dt, J = 25.8, 12.9 Hz, 3H), 3.87 - 3.71 (m, 2H), 3.67 - 3.43 (m, 4H), 3.25 - 2.84 (m, 9H), 2.59 (d, J = 17.2 Hz, 1H), 2.47 - 2.34 (m, 1H), 2.12 - 1.75 (m, 10H), 1.22 (q, J = 11.5 Hz, 2H). LCMS (ESI) calcd. for C₄₀H₄₆F₃N₁₂O₅⁺ [M+H]⁺: 831.37, found, 831.4.

### Example 16 of compound of Formula (I): Preparation of compound GT-07104

With reference to the method of Scheme I-1, the target compound (GT-07104) was obtained (white solid, 16 mg, yield 23.37%). ¹H NMR (400 MHz, DMSO-d6) δ 10.96 (s, 1H), 10.15 (s, 1H), 9.51 (d, J = 6.5 Hz, 1H), 8.78 (d, J = 7.7 Hz, 1H), 8.40 (d, J = 4.0 Hz, 1H), 8.26 (d, J = 5.5 Hz, 1H), 7.77 (s, 1H), 7.37 (d, J = 10.6 Hz, 1H), 7.29 (d, J = 7.2 Hz, 1H), 7.11 (td, J = 53.7, 3.6 Hz, 1H), 6.66 (dd, J = 166.1, 7.8 Hz, 1H), 5.32 - 4.98 (m, 2H), 4.77 (d, J = 19.5 Hz, 1H), 4.26 (dd, J = 49.9, 16.9 Hz, 3H), 3.86 - 3.69 (m, 2H), 3.60 (dd, J = 24.7, 13.4 Hz, 3H), 3.46 (s, 1H), 3.28 - 2.99 (m, 8H), 2.96 - 2.82 (m, 1H), 2.59 (d, J = 16.7 Hz, 1H), 2.43 - 2.30 (m, 1H), 2.00 (td, J = 25.8, 12.1 Hz, 8H), 1.80 (dd, J = 23.5, 11.6 Hz, 2H), 1.22 (dd, J = 24.0, 11.6 Hz, 2H). LCMS (ESI) calcd. for C₄₀H₄₆F₃N₁₂O₅⁺ [M+H]⁺: 831.37, found, 831.4.

### Example 17 of compound of Formula (I): Preparation of compound GT-07105

With reference to the method of Scheme I-1, the target compound (GT-07105) was obtained (white solid, 35 mg, yield 51.13%). ¹H NMR (400 MHz, DMSO-d6) δ 10.95 (s, 1H), 10.29 (s, 1H), 9.51 (d, J = 6.5 Hz, 1H), 8.78 (d, J = 7.7 Hz, 1H), 8.40 (d, J = 4.0 Hz, 1H), 8.27 (t, J = 7.3 Hz, 1H), 8.10 (s, 1H), 7.11 (td, J = 53.8, 3.7 Hz, 1H), 6.90 - 6.39 (m, 3H), 5.18 (d, J = 80.3 Hz, 1H), 4.99 (dd, J = 13.3, 5.0 Hz, 1H), 4.77 (d, J = 18.9 Hz, 1H), 4.36 - 4.14 (m, 3H), 3.85 - 3.71 (m, 2H), 3.68 - 3.41 (m, 5H), 3.11 (t, J = 44.1 Hz, 7H), 2.95 - 2.79 (m, 1H), 2.58 (d, J = 16.3 Hz, 1H), 2.32 (qd, J = 13.1, 4.4 Hz, 1H), 2.00 (ddd, J = 24.8, 18.9, 11.3 Hz, 8H), 1.80 (dd, J = 23.2, 11.5 Hz, 2H), 1.22 (q, J = 11.5 Hz, 2H). LCMS (ESI) calcd. for C₄₀H₄₆F₃N₁₂O₅⁺ [M+H]⁺: 831.37, found, 831.4.

### Example 18 of compound of Formula (I): Preparation of compound GT-07106

With reference to the method of Scheme I-1, the target compound (GT-07106) was obtained (white solid, 36 mg, yield 53.01%). ¹H NMR (400 MHz, DMSO-d6) δ 10.96 (s, 1H), 10.53 (s, 1H), 8.59 (d, J = 2.0 Hz, 1H), 8.24 - 7.88 (m, 3H), 7.78 (s, 1H), 7.42 (d, J = 8.2 Hz, 1H), 7.32 (t, J = 7.7 Hz, 1H), 7.19 (dd, J = 11.3, 5.6 Hz, 2H), 7.01 (dd, J = 8.8, 2.4 Hz, 1H), 5.05 (dd, J = 13.3, 5.0 Hz, 1H), 4.46 (d, J = 16.5 Hz, 3H), 4.35 (s, 1H), 4.28 (d, J = 16.7 Hz, 1H), 4.07 (d, J = 9.1 Hz, 1H), 3.29 - 3.00 (m, 12H), 2.93 - 2.85 (m, 1H), 2.59 (d, J = 16.5 Hz, 1H), 2.40 (dt, J = 13.4, 8.9 Hz, 1H), 2.22 (s, 1H), 2.04 - 1.84 (m, 3H), 1.37 - 1.25 (m, 2H), 1.23 (s, 6H), 1.13 (s, 6H). LCMS (ESI) calcd. for C₄₄H₅₂ClFN₉O₅⁺ [M+H]⁺: 840.38, found, 840.5.

### Example 19 of compound of Formula (I): Preparation of compound GT-07107

With reference to the method of Scheme I-1, the target compound (GT-07107) was obtained (white solid, 36 mg, yield 53.01%). ¹H NMR (400 MHz, DMSO-d6) δ 10.95 (s, 1H), 10.48 (s, 1H), 8.59 (d, J = 2.2 Hz, 1H), 8.17 (d, J = 8.7 Hz, 1H), 7.94 (d, J = 9.1 Hz, 1H), 7.90 (d, J = 8.8 Hz, 1H), 7.78 (s, 1H), 7.36 (d, J = 10.6 Hz, 1H), 7.29 (d, J = 7.2 Hz, 1H), 7.20 (t, J = 5.5 Hz, 2H), 7.01 (dd, J = 8.8, 2.4 Hz, 1H), 5.05 (dd, J = 13.3, 5.1 Hz, 1H), 4.46 (d, J = 13.1 Hz, 2H), 4.35 (s, 1H), 4.32 - 4.17 (m, 2H), 4.07 (d, J = 9.2 Hz, 1H), 3.27 - 2.98 (m, 12H), 2.95 - 2.85 (m, 1H), 2.59 (d, J = 16.8 Hz, 1H), 2.36 (qd, J = 13.1, 4.5 Hz, 1H), 2.22 (s, 1H), 2.05 - 1.87 (m, 3H), 1.37 - 1.25 (m, 2H), 1.23 (s, 6H), 1.13 (s, 6H). LCMS (ESI) calcd. for C₄₄H₅₂ClFN₉O₅⁺ [M+H]⁺: 840.38, found, 840.5.

### Example 20 of compound of Formula (I): Preparation of compound GT-07108

With reference to the method of Scheme I-1, the target compound (GT-07108) was obtained (white solid, 31 mg, yield 45.65%). ¹H NMR (400 MHz, DMSO-d6) δ 10.95 (s, 1H), 10.74 (s, 1H), 8.57 (d, J = 1.9 Hz, 1H), 8.09 (dd, J = 86.9, 8.8 Hz, 3H), 7.90 (d, J = 8.7 Hz, 1H), 7.22 (t, J = 8.3 Hz, 2H), 7.01 (dd, J = 8.8, 2.4 Hz, 1H), 6.71 (s, 1H), 6.63 (d, J = 12.1 Hz, 1H), 4.99 (dd, J = 13.2, 5.0 Hz, 1H), 4.46 (d, J = 13.1 Hz, 2H), 4.37 - 4.14 (m, 4H), 4.07 (d, J = 9.2 Hz, 1H), 3.25 - 3.01 (m, 10H), 2.95 - 2.73 (m, 2H), 2.62 - 2.53 (m, 1H), 2.34 (td, J = 13.0, 4.4 Hz, 1H), 2.23 (s, 1H), 1.96 (dd, J = 22.1, 8.8 Hz, 3H), 1.38 - 1.26 (m, 2H), 1.23 (s, 6H), 1.13 (s, 6H). LCMS (ESI) calcd. for C₄₄H₅₂ClFN₉O₅⁺ [M+H]⁺: 840.38, found, 840.5.

### Example 21 of compound of Formula (I): Preparation of compound GT-05207

With reference to the method of Scheme I-1, the target compound (GT-05207) was obtained (white solid, 41 mg, yield 45.2%). ¹H NMR (400 MHz, DMSO-D6) δ 11.50 (s, 1H), 11.09 (s, 1H), 10.75 (s, 1H), 9.00 (s, 1H), 8.53 (s, 1H), 8.15 (dd, *J* = 9.6, 2.7 Hz, 1H), 7.92 (s, 1H), 7.65 (d, *J =* 8.3 Hz, 1H), 7.58 (d, *J =* 9.6 Hz, 1H), 7.26 (s, 1H), 7.17 - 7.00 (m, 1H), 6.83 (s, 1H), 5.06 (dd, *J* = 12.8, 5.4 Hz, 1H), 4.86 - 4.71 (m, 1H), 3.71 (d, *J* = 12.0 Hz, 2H), 3.60 - 3.54 (m, 3H), 3.13 - 3.09 (m, 3H), 3.06 (s, 8H), 2.93 - 2.87 (m, 1H), 2.80 (t, *J* = 11.2 Hz, 2H), 2.59 (d, *J =* 17.7 Hz, 3H), 2.33 - 2.26 (m, 2H), 2.08 - 1.94 (m, 9H), 1.68 - 1.59 (m, 2H), 1.44 - 1.34 (m, 2H). LCMS (ESI) calcd. for C₄₂H₅₁N₁₂O₅ ⁺ [M+H]⁺: 803.41, found, 803.5.

### Example 22 of compound of Formula (I): Preparation of compound GT-05234

With reference to the method of Scheme I-1, the target compound (GT-05234) was obtained (yellow solid, 24 mg, yield 26.5%). ¹H NMR (400 MHz, DMSO-d6) δ 11.08 (s, 1H), 10.34 (s, 1H), 8.60 (d, *J =* 8.3 Hz, 1H), 8.48 (s, 1H), 7.85 (t, 2H), 7.65 (d, *J* = 8.3 Hz, 1H), 7.43 - 7.34 (m, 2H), 7.26 (s, 1H), 7.14 (dd, *J* = 8.8, 2.3 Hz, 1H), 7.10 - 6.98 (m, 1H), 5.06 (dd, *J =* 13.0, 5.4 Hz, 1H), 4.59 - 4.47 (m, 3H), 3.86 (d, *J =* 7.8 Hz, 1H), 3.59 - 3.56 (m, 1H), 3.29 (s, 2H), 3.18 - 3.05 (m, 8H), 2.95 - 2.85 (m, 1H), 2.59 (d, *J =* 17.3 Hz, 1H), 2.22 (s, 1H), 2.11 (d, *J =* 10.0 Hz, 2H), 1.98 - 1.88 (m, 4H), 1.67 - 1.60 (m, 2H), 1.56 - 1.50 (m, 2H), 1.34 - 1.24 (m, 3H), 0.94 (t, *J =* 7.3 Hz, 2H). LCMS (ESI) calcd. for C₄₁H₄₆ClN₁₀O₆ ⁺ [M+H]⁺: 809.33, found, 809.3.

### Example 23 of compound of Formula (I): Preparation of compound GT-05300

With reference to the method of Scheme I-1, the target compound (GT-05300) was obtained (yellow solid, 22 mg, yield 19.7%). ¹H NMR (400 MHz, DMSO-D6) δ 11.09 (s, 1H), 10.08 (s, 1H), 9.51 (d, *J =* 6.5 Hz, 1H), 8.79 (d, *J* = 7.6 Hz, 1H), 8.41 (d, *J* = 4.0 Hz, 1H), 8.26 (d, *J* = 5.6 Hz, 1H), 7.65 (d, *J* = 8.3 Hz, 1H), 7.26 (s, 1H), 7.14 - 7.05 (m, 1H), 5.10 - 5.03 (m, 1H), 4.77 (d, *J =* 19.8 Hz, 1H), 4.30 - 4.17 (m, 1H), 3.84 - 3.80 (m, 1H), 3.67 - 3.55 (m, 3H), 3.46 (s, 2H), 3.28 (s, 3H), 3.18 - 2.98 (m, 6H), 2.94 - 2.81 (m, 1H), 2.62 - 2.54 (m, 1H), 2.11 - 2.05 (m, 2H), 2.04 - 1.86 (m, 6H), 1.85 - 1.71 (m, 2H), 1.22 (dd, *J* = 23.8, 11.9 Hz, 2H). LCMS (ESI) C₄₀H₄₅F₂N₁₂O₆⁺ [M+H]⁺: calcd. for 827.35, found, 827.4.

### Example 24 of compound of Formula (I): Preparation of compound GT-05348

With reference to the method of Scheme I-1, the target compound (GT-05348) was obtained (yellow solid, 38 mg, yield 43%). ¹H NMR (400 MHz, DMSO-D6) δ 11.07 (s, 1H), 10.31 (s, 1H), 8.59 (s, 1H), 8.48 (s, 1H), 8.08 (d, *J* = 8.7 Hz, 1H), 7.91 (d, *J* = 8.7 Hz, 1H), 7.78 (d, 1H), 7.65 (d, *J* = 8.4 Hz, 1H), 7.26 (s, 1H), 7.21 (d, *J =* 2.2 Hz, 1H), 7.12 - 6.97 (m, 3H), 5.06 (dd, *J =* 13.0, 5.2 Hz, 1H), 4.44 (d, *J* = 12.5 Hz, 2H), 4.33 (s, 1H), 4.07 (d, *J =* 9.1 Hz, 1H), 3.59 (s, 2H), 3.33 - 3.28 (m, 2H), 3.09 (d, *J =* 27.5 Hz, 8H), 2.87 (t, *J =* 12.4 Hz, 1H), 2.70 - 2.55 (m, 2H), 2.19 (s, 1H), 2.06 - 1.98 (m, 1H), 1.94 (d, *J* = 11.9 Hz, 2H), 1.33 - 1.27 (m, 1H), 1.23 (s, 7H), 1.13 (s, 6H). LCMS (ESI) calcd. for C₄₄H₅₁ClN₉O₆⁺ [M+H]⁺: 836.36, found, 836.3.

### Example 25 of compound of Formula (I): Preparation of compound GT-07241

With reference to the method of Scheme I-1, the target compound (GT-07241) was obtained (white solid, 18 mg, yield 25.87%). ¹H NMR (400 MHz, DMSO-d6) δ 11.54 (s, 1H), 11.09 (s, 2H), 9.00 (d, J = 2.2 Hz, 1H), 8.16 (dd, J = 9.6, 2.5 Hz, 1H), 7.95 (s, 1H), 7.72 (d, J = 8.5 Hz, 1H), 7.62 (d, J = 9.5 Hz, 1H), 7.43 (s, 1H), 7.32 (d, J = 9.0 Hz, 1H), 6.82 (s, 1H), 5.08 (dd, J = 12.9, 5.3 Hz, 1H), 4.86 - 4.71 (m, 1H), 3.70 (d, J = 11.6 Hz, 5H), 3.36 - 3.22 (m, 4H), 3.08 (d, J = 18.7 Hz, 9H), 2.85 (dt, J = 26.6, 12.9 Hz, 3H), 2.57 (dd, J = 18.6, 9.9 Hz, 2H), 2.32 (d, J = 11.3 Hz, 2H), 2.14 - 1.83 (m, 8H), 1.69 - 1.55 (m, 2H), 1.43 (d, J = 9.7 Hz, 2H). LCMS (ESI) calcd. for C₄₂H₅₁N₁₂O₅⁺ [M+H]⁺: 803.41, found, 803.5.

### Example 26 of compound of Formula (I): Preparation of compound GT-07109

With reference to the method of Scheme I-1, the target compound (GT-07109) was obtained (white solid, 10 mg, yield 14.63%). ¹H NMR (400 MHz, DMSO-d6) δ 11.62 (s, 1H), 10.96 (s, 2H), 9.02 (s, 1H), 8.22 (d, J = 9.6 Hz, 1H), 8.07 (s, 1H), 7.65 (d, J = 9.4 Hz, 1H), 7.57 (d, J = 8.4 Hz, 1H), 7.20 - 7.06 (m, 2H), 6.85 (s, 1H), 5.06 (dd, J = 13.3, 5.0 Hz, 1H), 4.85 - 4.79 (m, 1H), 4.21 (s, 2H), 3.70 (d, J = 11.7 Hz, 2H), 3.34 (dd, J = 49.3, 43.5 Hz, 7H), 3.16 - 2.99 (m, 9H), 2.90 (dd, J = 19.7, 11.0 Hz, 3H), 2.64 - 2.54 (m, 1H), 2.36 (dd, J = 19.5, 14.7 Hz, 3H), 2.04 (dd, J = 39.1, 27.8 Hz, 8H), 1.65 (d, J = 5.8 Hz, 2H), 1.49 (d, J = 10.7 Hz, 2H). LCMS (ESI) calcd. for C₄₂H₅₃N₁₂O₄⁺ [M+H]⁺: 789.43, found, 789.5.

### Example 27 of compound of Formula (I): Preparation of compound GT-07099

With reference to the method of Scheme I-1, the target compound (GT-07099) was obtained (white solid, 16 mg, yield 25.16%). ¹H NMR (400 MHz, DMSO-d6) δ 11.55 (s, 1H), 11.06 (d, J = 15.7 Hz, 2H), 9.02 - 8.93 (m, 1H), 8.18 (dd, J = 9.4, 3.0 Hz, 1H), 7.97 (s, 1H), 7.69 (t, J = 7.4 Hz, 1H), 7.59 (d, J = 9.3 Hz, 1H), 7.41 (d, J = 4.4 Hz, 1H), 7.30 - 7.20 (m, 1H), 6.83 (s, 1H), 5.14 - 5.00 (m, 1H), 4.80 (dd, J = 17.4, 8.8 Hz, 1H), 3.76 (s, 3H), 3.06 (s, 6H), 2.87 (ddd, J = 27.9, 21.2, 10.1 Hz, 4H), 2.56 (dd, J = 19.8, 11.0 Hz, 2H), 2.30 (s, 2H), 2.12 - 1.82 (m, 8H), 1.72 - 1.55 (m, 3H), 1.42 (s, 1H). LCMS (ESI) calcd. for C₃₈H₄₄N₁₁O₅⁺ [M+H]⁺: 734.35, found, 734.4.

### Example 28 of compound of Formula (I): Preparation of compound GT-07194

With reference to the method of Scheme I-1, the target compound (GT-07194) was obtained (white solid, 19 mg, yield 26.29%). ¹H NMR (400 MHz, DMSO-d6) δ 11.59 (s, 1H), 10.99 (s, 1H), 9.02 (s, 1H), 8.20 (d, *J* = 7.9 Hz, 1H), 8.06 (s, 1H), 7.64 (d, *J* = 8.1 Hz, 2H), 7.59 (s, 1H), 7.45 (d, *J* = 7.9 Hz, 1H), 6.82 (d, *J* = 15.0 Hz, 1H), 5.10 (dd, *J* = 13.3, 5.1 Hz, 1H), 4.83 (dd, *J* = 17.3, 8.7 Hz, 1H), 4.43 (d, *J* = 17.5 Hz, 2H), 3.68 (d, *J =* 12.0 Hz, 2H), 3.54 (s, 2H), 3.06 (s, 9H), 2.98 (d, *J =* 13.5 Hz, 2H), 2.93 - 2.80 (m, 4H), 2.60 (d, *J =* 16.3 Hz, 1H), 2.43 - 2.26 (m, 3H), 2.09 - 1.88 (m, 10H), 1.70 (dd, *J* = 36.2, 30.6 Hz, 6H), 1.42 (d, *J=* 10.2 Hz, 2H). LCMS (ESI) calcd. for C₄₄H₅₆N₁₁O₄S⁺ [M+H]⁺: 834.42, found, 834.5.

### Example 29 of compound of Formula (I): Preparation of compound GT-07101

With reference to the method of Scheme I-1, the target compound (GT-07101) was obtained (white solid, 15 mg, yield 21.68%). ¹H NMR (400 MHz, DMSO-d6) δ 11.08 (s, 1H), 10.79 (s, 1H), 8.60 (d, J = 8.2 Hz, 1H), 7.85 (d, J = 8.9 Hz, 2H), 7.71 (dd, J = 13.2, 5.9 Hz, 1H), 7.51 - 7.40 (m, 2H), 7.38 (d, J = 2.4 Hz, 1H), 7.32 (d, J = 8.6 Hz, 1H), 7.13 (dd, J = 8.8, 2.4 Hz, 1H), 5.08 (dd, J = 12.9, 5.3 Hz, 1H), 4.64 - 4.34 (m, 3H), 3.86 (d, J = 8.1 Hz, 1H), 3.53 - 3.39 (m, 2H), 3.17 (ddd, J = 47.1, 30.4, 8.6 Hz, 8H), 2.95 - 2.82 (m, 1H), 2.57 (dd, J = 18.1, 9.7 Hz, 2H), 2.26 - 1.99 (m, 4H), 1.90 (d, J = 9.8 Hz, 4H), 1.48 (ddt, J = 22.0, 14.6, 9.3 Hz, 8H). LCMS (ESI) calcd. for C₄₁H₄₆ClN₁₀O₆⁺ [M+H]⁺: 809.33, found, 809.3.

### Example 30 of compound of Formula (I): Preparation of compound GT-07110

With reference to the method of Scheme I-1, the target compound (GT-07110) was obtained (white solid, 22 mg, yield 32.36%). ¹H NMR (400 MHz, DMSO-d6) δ 10.95 (s, 2H), 8.61 (d, J = 8.2 Hz, 1H), 7.91 (d, J = 9.6 Hz, 1H), 7.85 (d, J = 8.8 Hz, 1H), 7.57 (t, J = 9.2 Hz, 2H), 7.38 (d, J = 2.4 Hz, 1H), 7.19 - 7.07 (m, 3H), 5.05 (dd, J = 13.2, 5.1 Hz, 1H), 4.50 (dd, J = 19.7, 9.1 Hz, 3H), 4.37 (s, 2H), 3.90 - 3.81 (m, 1H), 3.37 (d, J = 69.5 Hz, 8H), 3.10 (dd, J = 27.8, 9.1 Hz, 4H), 2.97 - 2.83 (m, 1H), 2.58 (d, J = 16.6 Hz, 1H), 2.44 - 2.29 (m, 1H), 2.13 (t, J = 20.3 Hz, 3H), 2.02 - 1.83 (m, 5H), 1.67 - 1.44 (m, 4H), 1.32 (dd, J = 21.5, 10.7 Hz, 2H). LCMS (ESI) calcd. for C₄₁H₄₈ClN₁₀O₅⁺ [M+H]⁺: 795.35, found,795.4.

### Example 31 of compound of Formula (I): Preparation of compound GT-07098

With reference to the method of Scheme I-1, the target compound (GT-07098) was obtained (white solid, 25 mg, yield 39.51%). ¹H NMR (400 MHz, DMSO-d6) δ 10.08 (d, J = 6.0 Hz, 1H), 9.93 (s, 1H), 7.60 (dd, J = 8.0, 5.5 Hz, 1H), 6.91 - 6.83 (m, 2H), 6.70 (dd, J = 24.0, 8.2 Hz, 1H), 6.52 (t, J = 9.1 Hz, 1H), 6.44 - 6.34 (m, 2H), 6.31 - 6.18 (m, 1H), 6.13 (dd, J = 8.8, 2.3 Hz, 1H), 4.13 - 3.99 (m, 1H), 3.62 - 3.45 (m, 3H), 2.23 (t, J = 11.5 Hz, 1H), 2.08 (t, J = 11.7 Hz, 1H), 1.90 (dd, J = 29.7, 17.8 Hz, 2H), 1.71 (d, J = 4.1 Hz, 1H), 1.56 (dd, J = 15.6, 12.7 Hz, 2H), 1.15 - 0.86 (m, 8H), 0.71 - 0.44 (m, 6H), 0.29 (d, J = 9.2 Hz, 1H). LCMS (ESI) calcd. for C₃₇H₃₉ClN₉O₆⁺ [M+H]⁺: 740.27, found, 740.3.

### Example 32 of compound of Formula (I): Preparation of compound GT-07102

With reference to the method of Scheme I-1, the target compound (GT-07102) was obtained (white solid, 10 mg, yield 4 13.92%). ¹H NMR (400 MHz, DMSO-d6) δ 10.98 (s, 1H), 8.67 - 8.44 (m, 1H), 7.85 (d, J = 8.9 Hz, 2H), 7.63 (d, J = 8.0 Hz, 1H), 7.57 (s, 1H), 7.49 - 7.40 (m, 2H), 7.38 (d, J = 2.3 Hz, 1H), 7.13 (dd, J = 8.8, 2.4 Hz, 1H), 5.09 (dd, J = 13.2, 4.9 Hz, 1H), 4.66 - 4.26 (m, 5H), 3.86 (s, 1H), 3.12 (ddd, J = 25.1, 19.4, 9.4 Hz, 8H), 2.96 - 2.85 (m, 2H), 2.62 (s, 1H), 2.44 - 2.36 (m, 1H), 2.10 - 1.81 (m, 9H), 1.57 (ddd, J = 32.5, 23.2, 10.2 Hz, 9H), 1.30 (dd, J = 14.7, 7.3 Hz, 2H). LCMS (ESI) calcd. for C₄₃H₅₁ClN₉O₅S⁺ [M+H]⁺: 840.34, found, 840.3.

### Example 33 of compound of Formula (I): Preparation of compound GT-07394

With reference to the method of Scheme I-1, the target compound (GT-07394) was obtained (white solid, 22 mg, yield 32.29%). ¹H NMR (500 MHz, DMSO-D6) δ 11.09 (s, 1H), 10.67 (s, 1H), 9.50 (d, J = 7.3 Hz, 1H), 8.78 (d, J = 7.7 Hz, 1H), 8.42 (d, J = 5.4 Hz, 1H), 8.25 (d, J = 6.9 Hz, 1H), 7.77 - 7.68 (m, 1H), 7.42 (d, J = 10.4 Hz, 1H), 7.30 (dd, J = 18.6, 5.4 Hz, 1H), 7.11 (td, J = 53.8, 4.4 Hz, 1H), 6.66 (dd, J = 207.5, 7.8 Hz, 1H), 5.29 - 5.02 (m, 2H), 4.77 (d, J = 21.7 Hz, 1H), 4.22 (dd, J = 11.5, 8.7 Hz, 1H), 3.79 (t, J = 21.1 Hz, 2H), 3.67 - 3.49 (m, 7H), 3.19 (d, J = 23.8 Hz, 3H), 3.06 (s, 2H), 2.94 - 2.84 (m, 1H), 2.57 (dt, J = 13.3, 5.9 Hz, 2H), 2.14 - 1.72 (m, 10H), 1.44 - 1.15 (m, 2H). LCMS (ESI) calcd. for C₄₀H₄₅F₂N₁₂O₆⁺ [M+H]⁺: 827.36, found, 827.3.

### Example 34 of compound of Formula (I): Preparation of compound GT-07111

With reference to the method of Scheme I-1, the target compound (GT-07111) was obtained (white solid, 20 mg, yield 29.86%). ¹H NMR (400 MHz, DMSO-d6) δ 10.96 (s, 1H), 10.73 (s, 1H), 9.51 (d, J = 5.8 Hz, 1H), 8.80 (t, J = 8.0 Hz, 1H), 8.42 (d, J = 4.3 Hz, 1H), 8.27 (t, J = 7.4 Hz, 1H), 7.56 (t, J = 9.5 Hz, 1H), 7.26 - 6.96 (m, 3H), 6.93 - 6.42 (m, 1H), 5.32 - 5.02 (m, 2H), 4.77 (d, J = 17.3 Hz, 1H), 4.36 (d, J = 17.2 Hz, 4H), 3.73 (dt, J = 42.2, 10.3 Hz, 4H), 3.45 (d, J = 9.9 Hz, 4H), 3.27 (s, 4H), 3.05 (d, J = 5.7 Hz, 2H), 2.98 - 2.85 (m, 1H), 2.59 (d, J = 16.7 Hz, 1H), 2.38 (dt, J = 13.6, 9.1 Hz, 1H), 2.15 - 1.70 (m, 11H), 1.23 (dd, J = 23.4, 11.8 Hz, 2H). LCMS (ESI) calcd. for C₄₀H₄₇F₂N₁₂O₅⁺ [M+H]⁺: 813.38, found,813.3.

### Example 35 of compound of Formula (I): Preparation of compound GT-07395

With reference to the method of Scheme I-1, the target compound (GT-07395) was obtained (white solid, 45 mg, yield 72.08%). ¹H NMR (500 MHz, DMSO-D6) δ 11.17 - 11.01 (m, 1H), 9.51 (t, J = 6.8 Hz, 1H), 8.78 (d, J = 7.7 Hz, 1H), 8.45 - 8.38 (m, 1H), 8.26 (dt, J = 9.3, 4.7 Hz, 1H), 7.80 - 7.65 (m, 1H), 7.48 - 7.27 (m, 2H), 7.26 - 6.99 (m, 2H), 6.90 - 6.44 (m, 1H), 5.33 - 5.05 (m, 2H), 4.77 (d, J = 20.9 Hz, 1H), 4.33 - 4.21 (m, 1H), 3.85 - 3.81 (m, 2H), 3.45 (d, J = 10.0 Hz, 1H), 2.98 - 2.81 (m, 2H), 2.64 - 2.53 (m, 2H), 1.93 (dddd, J = 47.0, 34.3, 33.1, 16.6 Hz, 12H), 1.36 (ddd, J = 35.7, 23.2, 11.2 Hz, 2H). LCMS (ESI) calcd. for C₃₆H₃₈F₂N₁₁O₆⁺ [M+H]⁺: 758.30, found,758.3

### Example 36 of compound of Formula (I): Preparation of compound GT-07396

With reference to the method of Scheme I-1, the target compound (GT-07396) was obtained (white solid, 16 mg, yield 22.63%). ¹H NMR (500 MHz, DMSO-D6) δ 10.99 (s, 1H), 9.50 (d, J = 7.4 Hz, 1H), 8.78 (d, J = 7.7 Hz, 1H), 8.39 (t, J = 6.6 Hz, 1H), 8.26 (t, J = 9.2 Hz, 1H), 7.67 - 7.61 (m, 1H), 7.57 (d, J = 6.7 Hz, 1H), 7.49 - 7.41 (m, 1H), 7.11 (td, J = 53.7, 4.3 Hz, 1H), 6.66 (dd, J = 207.5, 7.8 Hz, 1H), 5.31 - 5.02 (m, 2H), 4.76 (d, J = 22.5 Hz, 1H), 4.52 - 4.29 (m, 2H), 4.25 - 4.12 (m, 1H), 3.79 (t, J = 21.3 Hz, 2H), 3.65 - 3.58 (m, 2H), 3.44 - 3.27 (m, 6H), 3.09 - 3.02 (m, 2H), 2.95 - 2.87 (m, 2H), 2.60 (d, J = 16.9 Hz, 1H), 2.43 - 2.34 (m, 1H), 2.00 (ddd, J = 37.8, 23.9, 9.5 Hz, 10H), 1.81 - 1.69 (m, 3H), 1.48 - 1.37 (m, 1H), 1.22 - 0.99 (m, 2H). LCMS (ESI) calcd. for C₄₂H₅₀F₂N₁₁O₅S⁺ [M+H]⁺: 858.37, found,858.3

### Example 37 of compound of Formula (I): Preparation of compound GT-07179

With reference to the method of Scheme I-1, the target compound (GT-07179) was obtained (white solid, 32 mg, yield 47.35%). ¹H NMR (400 MHz, DMSO-d6) δ 11.09 (s, 1H), 10.91 (s, 1H), 8.56 (d, J = 1.9 Hz, 1H), 8.21 (d, J = 8.9 Hz, 1H), 8.00 (d, J = 8.8 Hz, 1H), 7.90 (d, J = 8.7 Hz, 1H), 7.71 (t, J = 8.9 Hz, 1H), 7.42 (d, J = 7.2 Hz, 1H), 7.32 (d, J = 8.7 Hz, 1H), 7.26 (d, J = 9.1 Hz, 1H), 7.21 (d, J = 2.4 Hz, 1H), 7.01 (dd, J = 8.8, 2.4 Hz, 1H), 5.08 (dd, J = 12.9, 5.3 Hz, 1H), 4.43 (t, J = 14.8 Hz, 2H), 4.35 (s, 1H), 4.08 (s, 1H), 3.59 (s, 4H), 3.31 - 3.06 (m, 8H), 2.90 (dd, J = 22.3, 8.4 Hz, 1H), 2.64 - 2.53 (m, 2H), 2.22 (s, 1H), 2.08 - 1.84 (m, 3H), 1.43 (dd, J = 76.8, 10.8 Hz, 2H), 1.23 (s, 6H), 1.12 (s, 6H). LCMS (ESI) calcd. for C₄₄H₅₁ClN₉O₆⁺ [M+H]⁺: 836.37, found, 836.4.

### Example 38 of compound of Formula (I): Preparation of compound GT-07112

With reference to the method of Scheme I-1, the target compound (GT-07112) was obtained (white solid, 25 mg, yield 37.59%). ¹H NMR (400 MHz, DMSO-D6) δ 10.95 (s, 1H), 10.82 (s, 1H), 8.57 (d, J = 2.0 Hz, 1H), 8.18 (d, J = 8.4 Hz, 1H), 7.94 (s, 1H), 7.90 (d, J = 8.8 Hz, 1H), 7.56 (d, J = 8.4 Hz, 1H), 7.21 (d, J = 2.4 Hz, 2H), 7.12 (d, J = 12.2 Hz, 2H), 7.01 (dd, J = 8.8, 2.4 Hz, 1H), 5.05 (dd, J = 13.3, 5.1 Hz, 1H), 4.44 (d, J = 13.3 Hz, 2H), 4.29 (dt, J = 49.8, 13.9 Hz, 4H), 4.07 (d, J = 9.1 Hz, 1H), 3.43 (s, 3H), 3.26 - 3.03 (m, 8H), 2.96 - 2.85 (m, 1H), 2.59 (d, J = 16.8 Hz, 1H), 2.45 - 2.33 (m, 1H), 2.19 (s, 1H), 2.02 - 1.89 (m, 3H), 1.41 - 1.26 (m, 2H), 1.23 (s, 6H), 1.12 (s, 6H). LCMS (ESI) calcd. for C₄₄H₅₃ClN₉O₅⁺ [M+H]⁺: 822.39, found, 822.5.

### Example 39 of compound of Formula (I): Preparation of compound GT-07180

With reference to the method of Scheme I-1, the target compound (GT-07180) was obtained (white solid, 33 mg, yield 53.23%). ¹H NMR (400 MHz, DMSO-d6) δ 11.08 (d, J = 5.3 Hz, 1H), 10.99 (s, 1H), 8.59 - 8.43 (m, 1H), 8.25 (d, J = 9.1 Hz, 1H), 8.04 (s, 1H), 7.90 (d, J = 8.8 Hz, 1H), 7.70 (dd, J = 18.7, 8.3 Hz, 1H), 7.44 - 7.19 (m, 4H), 7.01 (dd, J = 8.8, 2.4 Hz, 1H), 5.12 - 5.01 (m, 1H), 4.44 (d, J = 11.0 Hz, 2H), 4.35 (s, 1H), 4.07 (d, J = 9.1 Hz, 1H), 3.26 (dt, J = 55.9, 11.8 Hz, 3H), 3.02 - 2.85 (m, 2H), 2.75 - 2.52 (m, 2H), 2.22 - 1.82 (m, 4H), 1.60 (d, J = 11.3 Hz, 1H), 1.42 - 1.27 (m, 1H), 1.23 (s, 6H), 1.12 (s, 6H). LCMS (ESI) calcd. for C₄₀H₄₄ClN₈O₆⁺ [M+H]⁺: 767.31, found, 767.3.

### Example 40 of compound of Formula (I): Preparation of compound GT-07181

With reference to the method of Scheme I-1, the target compound (GT-07181) was obtained (white solid, 25 mg, yield 35.66%). ¹H NMR (400 MHz, DMSO-d6) δ 10.98 (s, 1H), 8.56 (t, J = 3.7 Hz, 1H), 8.22 (d, J = 9.1 Hz, 1H), 8.03 (d, J = 8.3 Hz, 1H), 7.90 (d, J = 8.7 Hz, 1H), 7.63 (d, J = 8.0 Hz, 1H), 7.57 (s, 1H), 7.44 (d, J = 8.0 Hz, 1H), 7.25 (d, J = 9.0 Hz, 1H), 7.20 (d, J = 2.4 Hz, 1H), 7.01 (dd, J = 8.8, 2.4 Hz, 1H), 5.09 (dd, J = 13.2, 5.1 Hz, 1H), 4.51 - 4.25 (m, 6H), 4.07 (d, J = 9.1 Hz, 1H), 3.22 - 2.75 (m, 10H), 2.60 (d, J = 16.6 Hz, 1H), 2.38 (ddd, J = 26.4, 13.3, 4.5 Hz, 1H), 2.07 - 1.82 (m, 6H), 1.79 - 1.47 (m, 3H), 1.25 (d, J = 18.7 Hz, 8H), 1.12 (s, 6H). LCMS (ESI) calcd. for C₄₆H₅₆ClN₈O₅S⁺ [M+H]⁺: 867.38, found, 867.3.

### Example 41 of compound of Formula (I): Preparation of compound GT-07126

With reference to the method of Scheme I-2, the target compound (GT-07126) was obtained (white solid, 31 mg, yield 40.49%). ¹H NMR (500 MHz, DMSO-D6) δ 11.08 (s, 1H), 10.51 (s, 1H), 8.69 (s, 1H), 8.46 (d, J = 7.7 Hz, 1H), 8.41 (d, J = 7.8 Hz, 1H), 8.39 (s, 1H), 8.26 - 8.19 (m, 1H), 8.14 (s, 1H), 7.63 (d, J = 8.3 Hz, 1H), 7.20 (d, J = 17.0 Hz, 1H), 7.16 (s, 1H), 7.05 (s, 1H), 5.05 (dd, J = 12.7, 5.4 Hz, 1H), 4.73 - 4.55 (m, 1H), 3.98 (s, 3H), 3.75 (d, J = 13.1 Hz, 2H), 3.62 (s, 6H), 3.00 (t, J = 11.4 Hz, 3H), 2.93 - 2.83 (m, 2H), 2.60 - 2.52 (m, 2H), 2.13 - 1.97 (m, 5H). LCMS (ESI) calcd. for C₃₈H₃₈F₃N₁₀O₇⁺ [M+H]⁺: 803.29, found, 803.3.

### Example 42 of compound of Formula (I): Preparation of compound GT-06944

With reference to the method of Scheme I-2, the target compound (GT-06944) was obtained (white solid, 17 mg, yield 21.72%). ¹H NMR (500 MHz, DMSO-D6) δ 11.10 (s, 1H), 10.50 (s, 1H), 8.69 (s, 1H), 8.46 (d, J = 7.7 Hz, 1H), 8.39 (q, J = 7.4 Hz, 2H), 8.22 (dd, J = 7.8, 0.8 Hz, 1H), 8.18 (s, 1H), 7.63 (d, J = 10.3 Hz, 1H), 7.53 (d, J = 7.0 Hz, 1H), 7.17 (d, J = 7.4 Hz, 1H), 5.07 (dd, J = 12.9, 5.4 Hz, 1H), 4.63 (ddd, J = 15.4, 10.9, 4.2 Hz, 1H), 3.98 (s, 3H), 3.70 (t, J = 32.1 Hz, 4H), 3.19 - 2.63 (m, 9H), 2.62 - 2.55 (m, 1H), 2.52 (d, J = 9.1 Hz, 1H), 2.14 - 1.95 (m, 5H). LCMS (ESI) calcd. for C₃₃H₃₇F₄N₁₀O₇⁺ [M+H]⁺: 821.28, found, 821.3.

### Example 43 of compound of Formula (I): Preparation of compound GT-06943

With reference to the method of Scheme I-2, the target compound (GT-06943) was obtained (white solid, 23 mg, yield 24.46%). ¹H NMR (500 MHz, DMSO-D6) δ 10.95 (s, 1H), 10.51 (s, 1H), 8.69 (s, 1H), 8.46 (d, J = 7.8 Hz, 1H), 8.43 - 8.37 (m, 2H), 8.22 (d, J = 7.7 Hz, 1H), 7.45 (d, J = 8.4 Hz, 1H), 7.16 (d, J = 5.1 Hz, 1H), 6.94 (s, 1H), 6.85 (d, J = 8.3 Hz, 1H), 5.03 (dd, J = 13.3, 5.1 Hz, 1H), 4.70 - 4.53 (m, 1H), 4.24 (dd, J = 69.3, 17.0 Hz, 2H), 3.98 (s, 3H), 3.75 (d, J = 12.9 Hz, 2H), 3.29 (s, 5H), 3.00 (t, J = 11.7 Hz, 2H), 2.94 - 2.84 (m, 2H), 2.75 (d, J = 21.1 Hz, 2H), 2.58 (d, J = 16.4 Hz, 1H), 2.34 (dt, J = 13.2, 10.7 Hz, 1H), 2.05 (dt, *J* = 11.2, 9.8 Hz, 4H), 1.99 - 1.89 (m, 1H). LCMS (ESI) calcd. for C₃₈H₄₀F₃N₁₀O₆⁺ [M+H]⁺: 789.31, found, 789.4.

### Example 44 of compound of Formula (I): Preparation of compound GT-06945

With reference to the method of Scheme I-2, the target compound (GT-06945) was obtained (white solid, 15 mg, yield 19.50%). ¹H NMR (500 MHz, DMSO-D6) δ 10.98 (s, 1H), 10.51 (s, 1H), 8.70 (s, 1H), 8.46 (d, J = 7.7 Hz, 1H), 8.41 (dd, J = 9.6, 5.8 Hz, 2H), 8.22 (d, J = 7.7 Hz, 1H), 7.35 (d, J = 10.7 Hz, 1H), 7.31 (d, J = 7.2 Hz, 1H), 7.17 (s, 1H), 5.05 (dd, J = 13.3, 5.1 Hz, 1H), 4.64 (s, 1H), 4.27 (dd, J = 67.9, 17.0 Hz, 2H), 3.99 (s, 3H), 3.75 (d, J = 12.9 Hz, 2H), 3.49 - 3.17 (m, 3H), 2.91 (ddd, J = 49.7, 33.7, 13.6 Hz, 8H), 2.61 (s, 1H), 2.35 (qd, J = 13.2, 4.5 Hz, 1H), 2.06 (dt, J = 38.2, 22.3 Hz, 5H). LCMS (ESI) calcd. for C₃₈H₃₉F₄N₁₀O₆⁺ [M+H]⁺: 807.30, found, 807.3.

### Example 45 of compound of Formula (I): Preparation of compound GT-06946

With reference to the method of Scheme I-2, the target compound (GT-06946) was obtained (white solid, 12 mg, yield 15.60%). ¹H NMR (500 MHz, DMSO-D6) δ 10.98 (s, 1H), 10.52 (s, 1H), 8.70 (s, 1H), 8.47 (d, J = 7.8 Hz, 1H), 8.44 - 8.39 (m, 2H), 8.23 (d, J = 7.7 Hz, 1H), 7.42 (d, J = 8.2 Hz, 1H), 7.33 (t, J = 7.7 Hz, 1H), 7.17 (s, 1H), 5.06 (dd, J = 13.3, 5.1 Hz, 1H), 4.71 - 4.60 (m, 1H), 4.37 (dd, J = 90.0, 16.8 Hz, 2H), 3.99 (s, 3H), 3.75 (d, J = 12.9 Hz, 2H), 3.43 - 3.09 (m, 5H), 3.01 (t, J = 11.6 Hz, 2H), 2.96 - 2.87 (m, 1H), 2.79 (d, J = 8.8 Hz, 3H), 2.59 (d, J = 16.9 Hz, 1H), 2.40 (qd, J = 13.1, 4.5 Hz, 1H), 2.16 - 1.91 (m, 5H). LCMS (ESI) calcd. for C₃₈H₃₉F₄N₁₀O₆⁺ [M+H]⁺: 807.30, found, 807.3.

### Example 46 of compound of Formula (I): Preparation of compound GT-06947

With reference to the method of Scheme I-2, the target compound (GT-06947) was obtained (white solid, 27 mg, yield 35.09%). ¹H NMR (500 MHz, DMSO-D6) δ 10.96 (s, 1H), 10.51 (s, 1H), 8.69 (s, 1H), 8.46 (d, J = 7.7 Hz, 1H), 8.43 - 8.37 (m, 2H), 8.22 (dd, J = 7.7, 0.8 Hz, 1H), 7.72 (s, 1H), 7.16 (s, 1H), 6.70 (s, 1H), 6.57 (d, J = 10.6 Hz, 1H), 4.99 (dd, J = 13.3, 5.1 Hz, 1H), 4.62 (dt, J = 15.3, 5.5 Hz, 1H), 4.36 - 4.11 (m, 2H), 3.99 (d, J = 15.6 Hz, 3H), 3.74 (d, J = 12.9 Hz, 2H), 3.46 (s, 3H), 3.38 - 3.22 (m, 2H), 3.17 - 2.79 (m, 6H), 2.57 (d, J = 16.8 Hz, 1H), 2.31 (qd, J = 13.4, 4.7 Hz, 1H), 2.15 - 1.91 (m, 5H). LCMS (ESI) calcd. for C₃₈H₃₉F₄N₁₀O₆⁺ [M+H]⁺: 807.30, found, 807.3.

### Example 47 of compound of Formula (I): Preparation of compound GT-09603

With reference to the method of Scheme I-1, the target compound (GT-09603) was obtained (white solid, 50 mg, yield 94.22 %). ¹H NMR (400 MHz, DMSO-D6) δ 9.51 (d, J = 6.5 Hz, 2H), 8.79 (d, J = 7.7 Hz, 1H), 8.49 - 8.37 (m, 2H), 8.26 (d, J = 5.6 Hz, 1H), 7.67 (d, J = 10.1 Hz, 1H), 7.58 (d, J = 6.8 Hz, 1H), 7.12 (dd, J = 55.8, 52.3 Hz, 1H), 6.67 (dd, J = 164.5, 8.0 Hz, 1H), 5.31 - 5.04 (m, 2H), 4.77 (d, J = 22.3 Hz, 1H), 4.24 (s, 1H), 3.82 (d, J = 10.7 Hz, 2H), 3.67 - 3.55 (m, 4H), 3.45 (d, J = 10.7 Hz, 2H), 3.14 (s, 4H), 3.06 (s, 2H), 3.01 (s, 3H), 2.93 (d, J = 12.7 Hz, 1H), 2.76 (d, J = 16.3 Hz, 1H), 2.57 (s, 1H), 2.01 (dd, J = 34.7, 14.1 Hz, 8H), 1.81 (d, J = 11.6 Hz, 2H). LCMS (ESI) calcd. for C₄₁H₄₆F₃N₁₂O₆⁺ [M+H]⁺: 859.36, found, 859.4.

### Example 48 of compound of Formula (I): Preparation of compounds GT-09548 and GT-09549

The enantiomeric mixture product GT-06951 obtained from Example 8 of compound of Formula (I) was resolved and separated by supercritical fluid chromatography (SFC) to afford target compound GT-09548 and target compound GT-09549, respectively. The SFC preparation method and conditions are as follows.

The SFC preparation method is as follows:

| | |
|---|---|
| System: | K-Prep LAB100G |
| Column name: | DAICELCHIRALCEL^{®}IE |
| Column dimensions: | 250*40 mm 10 µm |
| Mobile phase A: | ethanol |
| Mobile phase B: | acetonitrile |
| Mobile phase A: Mobile phase B | 75:25 |
| Wavelength: | 254 nm |
| Flow rate: | 60ml/min |
| Column temperature: | room temperature |
| Injection volume: | 3mL |
| Cycle time: | 11min |
| Solvents: | acetonitrile (redistilled grade) |
| | ethanol (redistilled grade) |
| Sample solution preparation: | The sample was dissolved in approximately 40 mL of ethanol. |

The obtained target compound GT-09548 (white solid, 32 mg, yield 40%): ¹H NMR (400 MHz, DMSO-d6) δ 11.08 (s, 1H), 9.50 (d, J = 6.1 Hz, 1H), 8.79 (d, J = 7.8 Hz, 1H), 8.39 (d, J = 4.0 Hz, 1H), 8.26 (d, J = 5.6 Hz, 1H), 8.11 (s, 1H), 7.69 - 7.52 (m, 1H), 7.46 (d, J = 7.0 Hz, 1H), 7.11 (t, J = 53.7 Hz, 1H), 6.91 - 6.44 (m, 1H), 5.30 - 5.04 (m, 2H), 4.77 (d, J = 17.9 Hz, 1H), 4.21 (d, J = 13.0 Hz, 1H), 3.87 - 3.58 (m, 4H), 3.45 (d, J = 9.5 Hz, 1H), 2.86 (d, J = 17.6 Hz, 5H), 2.58 (d, J = 18.4 Hz, 2H), 2.18 (d, J = 7.0 Hz, 2H), 2.11 - 1.88 (m, 9H), 1.75 (d, J = 10.3 Hz, 2H), 1.59 (s, 1H), 1.30 (s, 1H), 1.14 - 1.00 (m, 2H). LCMS (ESI) calcd. for [M+H]⁺: 845.35, found, 845.3.

The obtained target compound GT-09549 (white solid, 36 mg, yield 42%): ¹H NMR (400 MHz, DMSO-d6) δ 11.08 (s, 1H), 9.50 (d, J = 6.1 Hz, 1H), 8.78 (d, J = 7.7 Hz, 1H), 8.39 (d, J = 4.2 Hz, 1H), 8.26 (d, J = 5.6 Hz, 1H), 8.11 (s, 1H), 7.60 (t, J = 10.7 Hz, 1H), 7.46 (d, J = 7.0 Hz, 1H), 7.26 - 6.91 (m, 1H), 6.89 - 6.38 (m, 1H), 5.30 - 4.99 (m, 2H), 4.77 (d, J = 17.9 Hz, 1H), 4.21 (d, J = 13.8 Hz, 1H), 3.89 - 3.46 (m, 4H), 3.02 - 2.75 (m, 5H), 2.63 - 2.52 (m, 2H), 2.23 - 1.89 (m, 11H), 1.74 (dd, J = 14.8, 8.5 Hz, 2H), 1.62 (s, 2H), 1.36 (d, J = 12.6 Hz, 1H), 1.06 (d, J = 12.4 Hz, 2H). LCMS (ESI) calcd. for C₄₀H₄₄F₃N₁₂O₆⁺ [M+H]⁺: 845.35, found, 845.3.

### Example 49 of compound of Formula (I): Preparation of compound GT-10020

With reference to the method of Scheme I-1, the target compound (GT-10020) was obtained (white solid, 26 mg, yield 48.73%). ¹H NMR (400 MHz, DMSO-D6) δ 11.47 (s, 1H), 11.10 (s, 1H), 10.54 (s, 1H), 9.00 (s, 1H), 8.49 (s, 1H), 8.14 (d, J = 7.3 Hz, 1H), 7.92 (s, 1H), 7.60 (dd, J = 8.8, 4.3 Hz, 2H), 7.54 - 7.45 (m, 1H), 6.83 (s, 1H), 5.07 (dd, J = 12.7, 5.5 Hz, 1H), 4.88 - 4.76 (m, 1H), 3.71 (d, J = 12.3 Hz, 2H), 3.58 (s, 2H), 3.26 (s, 4H), 3.06 (s, 10H), 2.84 (dd, J = 24.2, 12.5 Hz, 3H), 2.61 (s, 2H), 2.33 (d, J = 1.8 Hz, 2H), 1.99 (d, J = 13.1 Hz, 8H), 1.66 (d, J = 5.9 Hz, 2H), 1.40 (d, J = 11.3 Hz, 2H). LCMS (ESI) calcd. for C₄₂H₅₀FN₁₂O₅⁺ [M+H]⁺: 821.40, found, 821.4.

### Example 50 of compound of Formula (I): Preparation of compound GT-10032

With reference to the method of Scheme I-1, the target compound (GT-10032) was obtained (white solid, 41 mg, yield 76.84%). ¹H NMR (400 MHz, DMSO-D6) δ 11.54 (s, 1H), 11.09 (s, 1H), 10.86 (s, 1H), 9.01 (s, 1H), 8.97 (s, 1H), 8.16 (dd, J = 9.7, 2.5 Hz, 1H), 7.93 (s, 1H), 7.59 (d, J = 9.5 Hz, 1H), 7.11 (s, 1H), 6.83 (s, 1H), 5.05 (dd, J = 12.8, 5.4 Hz, 1H), 4.82 (p, J = 8.6 Hz, 1H), 3.72 (s, 2H), 3.59 (s, 2H), 3.29 - 3.22 (m, 4H), 3.09 (d, J = 19.4 Hz, 10H), 2.91 - 2.77 (m, 3H), 2.61 (s, 2H), 2.32 (d, J = 10.2 Hz, 2H), 2.01 (dd, J = 15.0, 10.3 Hz, 8H), 1.72 - 1.61 (m, 2H), 1.41 (d, J = 10.6 Hz, 2H). LCMS (ESI) calcd. for C₄₂H₅₀FN₁₂O₅⁺ [M+H]⁺: 821.40, found, 821.4.

### Example 51 of compound of Formula (I): Preparation of compound GT-08188

With reference to the method of Scheme I-1, the target compound (GT-08188) was obtained (white solid, 44 mg, yield 64.70%). ¹H NMR (400 MHz, DMSO-d6) δ 11.61 (s, 1H), 11.14 (s, 1H), 11.01 (s, 1H), 9.02 (s, 1H), 8.20 (dd, J = 9.6, 2.6 Hz, 1H), 8.02 (s, 1H), 7.64 (d, J = 9.6 Hz, 1H), 7.31 (t, J = 7.7 Hz, 1H), 7.12 (d, J = 7.7 Hz, 1H), 7.07 (d, J = 7.3 Hz, 1H), 6.84 (s, 1H), 5.11 (dd, J = 13.2, 5.0 Hz, 1H), 4.82 (p, J = 8.6 Hz, 1H), 4.33 (d, J = 35.0 Hz, 2H), 3.70 (d, J = 12.0 Hz, 2H), 3.54 (s, 2H), 3.28 (d, J = 24.8 Hz, 4H), 3.06 (s, 10H), 2.96 - 2.80 (m, 3H), 2.63 (d, J = 16.7 Hz, 1H), 2.42 - 2.24 (m, 3H), 2.14 - 1.93 (m, 8H), 1.71 - 1.61 (m, 2H), 1.45 (d, J = 10.9 Hz, 2H). LCMS (ESI) calcd. for C₄₂H₅₂N₁₂O₄⁺ [M+H]⁺: 788.42, found, 788.4.

### Example 52 of compound of Formula (I): Preparation of compound GT-10033

With reference to the method of Scheme I-1, the target compound (GT-10033) was obtained (white solid, 37 mg, yield 69.85%). ¹H NMR (400 MHz, DMSO-D6) δ 11.56 (s, 1H), 10.94 (s, 1H), 10.74 (s, 1H), 8.91 (d, J = 80.0 Hz, 1H), 8.24 - 8.07 (m, 1H), 7.95 (s, 1H), 7.59 (d, J = 9.5 Hz, 1H), 7.52 - 7.42 (m, 1H), 7.02 - 6.90 (m, 2H), 6.84 (s, 1H), 5.04 (dd, J = 13.2, 5.0 Hz, 1H), 4.82 (p, J = 8.6 Hz, 1H), 4.25 (d, J = 34.1 Hz, 2H), 3.89 (s, 2H), 3.72 (d, J = 11.8 Hz, 2H), 3.21 (s, 2H), 3.06 (s, 8H), 2.98 (d, J = 9.1 Hz, 3H), 2.82 (t, J = 11.0 Hz, 4H), 2.62 (t, J = 21.2 Hz, 3H), 2.32 (d, J = 4.7 Hz, 3H), 2.09 - 1.93 (m, 8H), 1.66 (d, J = 5.8 Hz, 2H), 1.40 (d, J = 11.7 Hz, 2H). LCMS (ESI) calcd. for C₄₄H₅₅N₁₂O₄⁺ [M+H]⁺: 815.45, found, 815.4.

### Example 53 of compound of Formula (I): Preparation of compound GT-10034

With reference to the method of Scheme I-1, the target compound (GT-10034) was obtained (white solid, 32 mg, yield 60.41%). ¹H NMR (400 MHz, DMSO-D6) δ 11.57 (s, 1H), 10.93 (d, J = 12.2 Hz, 2H), 9.01 (s, 1H), 8.18 (dd, J = 9.6, 2.8 Hz, 1H), 7.98 (s, 1H), 7.64 (d, J = 9.5 Hz, 1H), 7.47 (d, J = 8.4 Hz, 1H), 7.00 (s, 1H), 6.93 (d, J = 8.0 Hz, 1H), 6.83 (s, 1H), 5.04 (dd, J = 13.2, 5.1 Hz, 1H), 4.88 - 4.73 (m, 1H), 4.38 - 4.18 (m, 2H), 4.08 (s, 2H), 3.72 (d, J = 12.3 Hz, 3H), 3.46 (s, 2H), 3.34 (d, J = 12.1 Hz, 1H), 3.06 (s, 6H), 2.99 - 2.77 (m, 7H), 2.59 (d, J = 16.8 Hz, 1H), 2.34 (dd, J = 12.8, 4.6 Hz, 3H), 2.22 (s, 3H), 1.99 (dd, J = 17.7, 10.5 Hz, 7H), 1.72 - 1.61 (m, 2H), 1.46 (d, J = 10.5 Hz, 2H). LCMS (ESI) calcd. for C₄₄H₅₅N₁₂O₄⁺ [M+H]⁺: 815.45, found, 815.4.

### Example 54 of compound of Formula (I): Preparation of compound GT-10035

With reference to the method of Scheme I-1, the target compound (GT-10035) was obtained (white solid, 32 mg, yield 59.39%). ¹H NMR (400 MHz, DMSO-D6) δ 11.55 (s, 1H), 11.30 (s, 1H), 10.94 (s, 1H), 9.01 (s, 1H), 8.16 (dd, J = 9.7, 2.7 Hz, 1H), 7.94 (s, 1H), 7.59 (d, J = 9.6 Hz, 1H), 7.45 (d, J = 8.3 Hz, 1H), 6.99 - 6.85 (m, 2H), 6.83 (s, 1H), 5.03 (d, J = 8.2 Hz, 1H), 4.82 (s, 1H), 4.36 - 4.16 (m, 2H), 4.03 (s, 2H), 3.86 (s, 2H), 3.70 (s, 6H), 3.17 (s, 2H), 3.06 (s, 6H), 2.95 - 2.86 (m, 1H), 2.77 (t, J = 11.6 Hz, 2H), 2.59 (d, J = 16.2 Hz, 2H), 2.35 - 2.26 (m, 3H), 1.96 (t, J = 21.6 Hz, 11H), 1.72 - 1.61 (m, 2H), 1.39 (s, 2H). LCMS (ESI) calcd. for C₄₅H₅₇N₁₂O₄⁺ [M+H]⁺: 829.46, found, 829.4.

### Example 55 of compound of Formula (I): Preparation of compound GT-10036

With reference to the method of Scheme I-1, the target compound (GT-10036) was obtained (white solid, 32 mg, yield 61.31%). ¹H NMR (400 MHz, DMSO-D6) δ 11.61 (s, 1H), 11.02 (s, 1H), 10.94 (s, 1H), 9.02 (s, 1H), 8.20 (dd, J = 9.6, 2.5 Hz, 1H), 8.03 (s, 1H), 7.63 (d, J = 9.5 Hz, 1H), 7.46 (d, J = 8.4 Hz, 1H), 6.98 (s, 1H), 6.91 (d, J = 8.4 Hz, 1H), 6.84 (s, 1H), 5.03 (dd, J = 13.2, 5.1 Hz, 1H), 4.82 (p, J = 8.7 Hz, 1H), 4.31 (d, J = 16.6 Hz, 2H), 3.71 (d, J = 11.8 Hz, 2H), 3.58 (d, J = 9.5 Hz, 3H), 3.39 - 3.28 (m, 2H), 3.17 - 3.01 (m, 10H), 2.93 - 2.80 (m, 3H), 2.59 (d, J = 17.1 Hz, 1H), 2.38 - 2.27 (m, 3H), 2.24 - 1.84 (m, 11H), 1.74 - 1.59 (m, 2H), 1.43 (d, J = 10.6 Hz, 2H). LCMS (ESI) calcd. for C₄₃H₅₅N₁₂O₄⁺ [M+H]⁺: 803.45, found, 803.4.

### Example 56 of compound of Formula (I): Preparation of compound GT-10037

With reference to the method of Scheme I-1, the target compound (GT-10037) was obtained (white solid, 22 mg, yield 42.26%). ¹H NMR (400 MHz, DMSO-D6) δ 11.49 (s, 1H), 11.05 (s, 1H), 10.94 (s, 1H), 9.00 (s, 1H), 8.14 (d, J = 9.4 Hz, 2H), 7.93 (s, 1H), 7.60 (d, J = 9.4 Hz, 1H), 7.52 - 7.44 (m, 1H), 7.07 - 6.85 (m, 2H), 6.82 (s, 1H), 5.04 (dd, J = 12.9, 5.1 Hz, 1H), 4.82 (t, J = 8.6 Hz, 1H), 4.47 - 4.16 (m, 4H), 3.71 (d, J = 11.3 Hz, 2H), 3.62 (s, 4H), 3.09 (d, J = 26.9 Hz, 8H), 2.94 (dd, J = 15.9, 10.8 Hz, 2H), 2.77 (t, J = 10.8 Hz, 2H), 2.59 (d, J = 17.7 Hz, 1H), 2.38 - 2.28 (m, 3H), 2.10 - 1.89 (m, 9H), 1.65 (d, J = 5.8 Hz, 2H), 1.43 (d, J = 11.7 Hz, 2H). LCMS (ESI) calcd. for C₄₃H₅₃N₁₂O₄⁺ [M+H]⁺: 801.43, found, 801.4.

### Example 57 of compound of Formula (I): Preparation of compound GT-10038

With reference to the method of Scheme I-1, the target compound (GT-10038) was obtained (white solid, 28 mg, yield 53.78%). ¹H NMR (400 MHz, DMSO-D6) δ 11.60 (s, 1H), 10.94 (s, 1H), 9.02 (s, 1H), 8.20 (d, J = 8.6 Hz, 1H), 8.01 (s, 1H), 7.63 (d, J = 8.3 Hz, 1H), 7.46 (dd, J = 8.1, 6.6 Hz, 1H), 7.05 - 6.86 (m, 2H), 6.84 (s, 1H), 5.03 (dd, J = 13.1, 4.9 Hz, 1H), 4.87 - 4.76 (m, 1H), 4.42 - 4.19 (m, 3H), 3.91 (s, 2H), 3.64 - 3.54 (m, 4H), 3.28 - 3.14 (m, 2H), 3.06 (s, 7H), 2.98 - 2.74 (m, 4H), 2.59 (d, J = 16.5 Hz, 1H), 2.33 (dd, J = 12.3, 8.3 Hz, 4H), 2.03 (dd, J = 35.3, 23.9 Hz, 8H), 1.65 (d, J = 5.6 Hz, 2H), 1.44 (d, J = 10.7 Hz, 2H). LCMS (ESI) calcd. for C₄₃H₅₃N₁₂O₄⁺ [M+H]⁺: 801.43, found, 801.4.

### Example 58 of compound of Formula (I): Preparation of compound GT-10021

With reference to the method of Scheme I-1, the target compound (GT-10021) was obtained (white solid, 28 mg, yield 52.79%). ¹H NMR (400 MHz, DMSO-D6) δ 11.10 (s, 1H), 10.25 (s, 1H), 8.59 (d, J = 8.2 Hz, 1H), 8.47 (s, 1H), 7.85 (dd, J = 9.2, 4.3 Hz, 2H), 7.60 (d, J = 8.2 Hz, 1H), 7.53 - 7.46 (m, 1H), 7.42 (d, J = 9.7 Hz, 1H), 7.39 (d, J = 2.4 Hz, 1H), 7.14 (dd, J = 8.8, 2.4 Hz, 1H), 5.07 (dd, J = 12.8, 5.5 Hz, 1H), 4.53 (dd, J = 20.5, 11.2 Hz, 3H), 3.98 - 3.83 (m, 1H), 3.58 (s, 2H), 3.30 - 3.18 (m, 4H), 3.09 (dd, J = 26.1, 14.6 Hz, 6H), 2.94 - 2.83 (m, 1H), 2.59 (d, J = 20.2 Hz, 2H), 2.22 (s, 1H), 2.11 (d, J = 10.4 Hz, 2H), 2.06 - 1.98 (m, 1H), 1.92 (t, J = 12.5 Hz, 4H), 1.70 - 1.49 (m, 4H), 1.35 - 1.23 (m, 2H). LCMS (ESI) calcd. for C₄₁H₄₅ClFN₁₀O₆⁺ [M+H]⁺: 827.32, found, 827.3.

### Example 59 of compound of Formula (I): Preparation of compound GT-10039

With reference to the method of Scheme I-1, the target compound (GT-10039) was obtained (white solid, 28 mg, yield 52.79%). ¹H NMR (400 MHz, DMSO-D6) δ 11.09 (s, 1H), 10.52 (s, 1H), 8.91 (s, 1H), 8.60 (d, J = 8.3 Hz, 1H), 7.84 (dd, J = 14.8, 6.6 Hz, 2H), 7.40 (dd, J = 10.5, 6.1 Hz, 2H), 7.14 (dd, J = 8.8, 2.4 Hz, 1H), 6.84 (s, 1H), 5.05 (dd, J = 12.7, 5.4 Hz, 1H), 4.53 (dd, J = 20.5, 11.1 Hz, 3H), 3.86 (d, J = 7.7 Hz, 1H), 3.57 (s, 2H), 3.19 - 3.01 (m, 8H), 2.94 - 2.81 (m, 1H), 2.60 (s, 2H), 2.28 - 2.06 (m, 3H), 1.95 (dd, J = 30.9, 16.4 Hz, 4H), 1.58 (td, J = 27.1, 12.5 Hz, 6H), 1.29 (dd, J = 18.0, 10.7 Hz, 3H). LCMS (ESI) calcd. for C₄₁H₄₅ClFN₁₀O₆⁺ [M+H]⁺: 827.32, found, 827.3.

### Example 60 of compound of Formula (I): Preparation of compound GT-08179

With reference to the method of Scheme I-1, the target compound (GT-08179) was obtained (white solid, 16 mg, yield 23.53%). ¹H NMR (400 MHz, DMSO-d6) δ 11.00 (s, 1H), 8.58 (d, J = 8.2 Hz, 1H), 7.86 (d, J = 8.8 Hz, 1H), 7.80 (d, J = 9.6 Hz, 1H), 7.39 (d, J = 2.4 Hz, 1H), 7.36 - 7.26 (m, 2H), 7.14 (dd, J = 8.8, 2.4 Hz, 1H), 7.09 - 6.99 (m, 2H), 6.77 (s, 1H), 5.09 (dd, J = 13.2, 5.1 Hz, 1H), 4.51 (dd, J = 23.9, 8.9 Hz, 3H), 4.30 (dd, J = 38.4, 17.4 Hz, 2H), 3.86 (d, J = 8.4 Hz, 1H), 3.06 - 2.86 (m, 4H), 2.75 (s, 3H), 2.64 (s, 1H), 2.35 (d, J = 18.0 Hz, 2H), 2.23 - 2.01 (m, 6H), 1.86 (dd, J = 32.9, 10.9 Hz, 6H), 1.68 - 1.59 (m, 2H), 1.57 - 1.48 (m, 2H), 1.12 (s, 2H). LCMS (ESI) calcd. for C₄₁H₄₈ClN₁₀O₅⁺ [M+H]⁺: 795.35, found, 795.4.

### Example 61 of compound of Formula (I): Preparation of compound GT-10040

With reference to the method of Scheme I-1, the target compound (GT-10040) was obtained (white solid, 28 mg, yield 53.17%). ¹H NMR (400 MHz, DMSO-D6) δ 10.94 (s, 1H), 10.57 (s, 1H), 8.61 (d, J = 8.2 Hz, 1H), 7.90 - 7.80 (m, 2H), 7.51 - 7.42 (m, 2H), 7.39 (d, J = 2.3 Hz, 1H), 7.14 (dd, J = 8.8, 2.4 Hz, 1H), 6.95 (d, J = 8.6 Hz, 2H), 5.04 (dd, J = 13.1, 5.1 Hz, 1H), 4.61 - 4.46 (m, 3H), 4.24 (dd, J = 50.0, 16.7 Hz, 2H), 3.88 (s, 3H), 3.43 (s, 1H), 3.18 (d, J = 5.2 Hz, 3H), 3.08 (t, J = 11.8 Hz, 3H), 2.98 (d, J = 8.7 Hz, 3H), 2.85 (dd, J = 18.1, 6.2 Hz, 2H), 2.59 (d, J = 17.6 Hz, 2H), 2.33 (s, 1H), 2.11 (d, J = 9.3 Hz, 3H), 2.03 - 1.87 (m, 5H), 1.70 - 1.47 (m, 6H), 1.31 (dd, J = 14.7, 7.4 Hz, 3H). LCMS (ESI) calcd. for C₄₃H₅₀ClN₁₀O₅⁺ [M+H]⁺: 821.37, found, 821.3.

### Example 62 of compound of Formula (I): Preparation of compound GT-10041

With reference to the method of Scheme I-1, the target compound (GT-10041) was obtained (white solid, 30 mg, yield 56.97%). ¹H NMR (400 MHz, DMSO-D6) δ 10.94 (s, 1H), 10.48 (s, 1H), 8.60 (d, J = 8.2 Hz, 1H), 7.86 (dd, J = 9.2, 3.6 Hz, 2H), 7.60 (s, 1H), 7.51 - 7.36 (m, 3H), 7.14 (dd, J = 8.8, 2.4 Hz, 1H), 6.99 (s, 1H), 6.92 (d, J = 8.1 Hz, 1H), 5.04 (dd, J = 13.3, 5.1 Hz, 1H), 4.60 - 4.47 (m, 3H), 4.25 (dd, J = 53.5, 17.0 Hz, 2H), 4.07 (s, 2H), 3.92 - 3.81 (m, 1H), 3.37 (d, J = 11.5 Hz, 2H), 3.20 - 3.13 (m, 2H), 3.07 (t, J = 11.6 Hz, 2H), 2.94 (d, J = 14.8 Hz, 4H), 2.59 (d, J = 16.3 Hz, 1H), 2.21 (s, 4H), 2.11 (d, J = 9.4 Hz, 2H), 1.94 (dd, J = 20.2, 12.8 Hz, 4H), 1.67 - 1.49 (m, 5H), 1.31 (dd, J = 14.5, 7.4 Hz, 3H). LCMS (ESI) calcd. for C₄₃H₅₀ClN₁₀O₅⁺ [M+H]⁺: 821.37, found, 821.3.

### Example 63 of compound of Formula (I): Preparation of compound GT-10042

With reference to the method of Scheme I-1, the target compound (GT-10042) was obtained (white solid, 30 mg, yield 56.01%). ¹H NMR (400 MHz, DMSO-D6) δ 11.18 (s, 1H), 10.94 (s, 1H), 8.61 (d, J = 8.2 Hz, 1H), 7.87 (dd, J = 9.2, 4.5 Hz, 2H), 7.52 - 7.44 (m, 2H), 7.39 (d, J = 2.4 Hz, 1H), 7.14 (dd, J = 8.8, 2.4 Hz, 1H), 6.96 (d, J = 30.7 Hz, 2H), 5.03 (dd, J = 13.2, 5.1 Hz, 1H), 4.57 - 4.44 (m, 3H), 4.34 - 4.16 (m, 2H), 4.03 (s, 6H), 3.22 - 3.01 (m, 6H), 2.89 (d, J = 13.1 Hz, 1H), 2.59 (d, J = 17.0 Hz, 1H), 2.36 (dd, J = 13.2, 8.8 Hz, 1H), 2.15 - 1.84 (m, 11H), 1.67 - 1.49 (m, 5H), 1.35 - 1.22 (m, 3H). LCMS (ESI) calcd. for C₄₄H₅₂ClN₁₀O₅⁺ [M+H]⁺: 835.38, found, 835.4.

### Example 64 of compound of Formula (I): Preparation of compound GT-10043

With reference to the method of Scheme I-1, the target compound (GT-10043) was obtained (white solid, 33 mg, yield 63.60%). ¹H NMR (400 MHz, DMSO-D6) δ 10.94 (s, 1H), 10.63 (s, 1H), 8.61 (d, J = 8.2 Hz, 1H), 7.86 (d, J = 8.8 Hz, 2H), 7.46 (dd, J = 9.1, 4.0 Hz, 2H), 7.39 (d, J = 2.4 Hz, 1H), 7.14 (dd, J = 8.8, 2.4 Hz, 1H), 6.96 (s, 1H), 6.89 (d, J = 8.4 Hz, 1H), 5.03 (dd, J = 13.3, 5.1 Hz, 1H), 4.59 - 4.46 (m, 3H), 4.24 (dd, J = 53.0, 16.8 Hz, 2H), 3.86 (d, J = 7.9 Hz, 2H), 3.62 - 3.51 (m, 2H), 3.32 (d, J = 13.3 Hz, 2H), 3.12 (dt, J = 24.2, 10.4 Hz, 7H), 2.92 (d, J = 13.4 Hz, 1H), 2.59 (d, J = 17.1 Hz, 1H), 2.39 - 2.31 (m, 1H), 2.23 - 1.87 (m, 10H), 1.67 - 1.49 (m, 5H), 1.30 (dt, J = 21.8, 10.9 Hz, 3H). LCMS (ESI) calcd. for C₄₂H₅₀ClN₁₀O₅⁺ [M+H]⁺: 809.37, found, 809.4.

### Example 65 of compound of Formula (I): Preparation of compound GT-10044

With reference to the method of Scheme I-1, the target compound (GT-10044) was obtained (white solid, 15 mg, yield 28.98%). ¹H NMR (400 MHz, DMSO-D6) δ 10.94 (s, 1H), 10.79 (s, 1H), 8.60 (d, J = 8.3 Hz, 1H), 8.00 (s, 1H), 7.90 - 7.81 (m, 2H), 7.48 (dt, J = 8.4, 2.9 Hz, 1H), 7.40 (dd, J = 10.9, 6.0 Hz, 2H), 7.14 (dd, J = 8.8, 2.4 Hz, 1H), 7.06 - 6.83 (m, 2H), 5.03 (dd, J = 13.3, 5.0 Hz, 1H), 4.54 - 4.42 (m, 3H), 4.27 (t, J = 25.3 Hz, 2H), 3.92 - 3.80 (m, 1H), 3.60 (s, 2H), 3.20 - 3.13 (m, 3H), 3.04 (t, J = 11.6 Hz, 3H), 2.97 - 2.85 (m, 2H), 2.59 (d, J = 16.7 Hz, 1H), 2.41 - 2.30 (m, 1H), 2.11 (d, J = 8.9 Hz, 3H), 2.00 - 1.86 (m, 5H), 1.68 - 1.52 (m, 6H), 1.33 - 1.28 (m, 3H). LCMS (ESI) calcd. for C₄₂H₄₈ClN₁₀O₅⁺ [M+H]⁺: 807.35, found, 807.4.

### Example 66 of compound of Formula (I): Preparation of compound GT-10045

With reference to the method of Scheme I-1, the target compound (GT-10045) was obtained (white solid, 19 mg, yield 36.71%). ¹H NMR (400 MHz, DMSO-D6) δ 10.94 (s, 2H), 8.61 (d, J = 8.3 Hz, 1H), 7.86 (d, J = 8.7 Hz, 2H), 7.50 - 7.43 (m, 2H), 7.39 (d, J = 2.3 Hz, 1H), 7.14 (dd, J = 8.8, 2.4 Hz, 1H), 6.98 (d, J = 11.9 Hz, 1H), 6.90 (dd, J = 16.9, 8.2 Hz, 1H), 5.03 (dd, J = 13.4, 4.9 Hz, 1H), 4.50 (dd, J = 29.8, 23.7 Hz, 4H), 4.25 (ddd, J = 21.5, 12.1, 4.1 Hz, 3H), 3.93 - 3.84 (m, 1H), 3.25 - 3.14 (m, 3H), 3.07 (t, J = 12.2 Hz, 3H), 2.91 (dd, J = 22.0, 8.8 Hz, 1H), 2.59 (d, J = 16.6 Hz, 1H), 2.40 - 2.31 (m, 1H), 2.23 (s, 2H), 2.11 (d, J = 11.7 Hz, 3H), 1.93 (dd, J = 21.9, 9.5 Hz, 4H), 1.69 - 1.48 (m, 6H), 1.31 (td, J = 14.6, 7.3 Hz, 3H). LCMS (ESI) calcd. for C₄₂H₄₈ClN₁₀O₅⁺ [M+H]⁺: 807.35, found, 807.4.

### Example 67 of compound of Formula (I): Preparation of compound GT-10022

With reference to the method of Scheme I-1, the target compound (GT-10022) was obtained (white solid, 26 mg, yield 49.80%). ¹H NMR (400 MHz, DMSO-D6) δ 11.10 (s, 1H), 9.97 (s, 1H), 9.51 (d, J = 6.7 Hz, 1H), 8.78 (d, J = 7.7 Hz, 1H), 8.46 (s, 1H), 8.40 (d, J = 4.2 Hz, 1H), 8.26 (d, J = 5.4 Hz, 1H), 7.60 (d, J = 8.2 Hz, 1H), 7.48 (t, J = 7.6 Hz, 1H), 7.11 (d, J = 3.8 Hz, 1H), 6.66 (dd, J = 164.3, 7.8 Hz, 1H), 5.34 - 5.02 (m, 2H), 4.77 (d, J = 19.7 Hz, 1H), 4.24 (s, 1H), 3.91 - 3.71 (m, 2H), 3.66 - 3.55 (m, 3H), 3.44 (d, J = 10.0 Hz, 2H), 3.27 - 3.14 (m, 4H), 3.12 - 2.99 (m, 4H), 2.88 (s, 1H), 2.58 (d, J = 20.0 Hz, 1H), 2.01 (ddd, J = 35.0, 24.6, 11.4 Hz, 8H), 1.81 (d, J = 12.4 Hz, 2H), 1.22 (d, J = 11.9 Hz, 2H). LCMS (ESI) calcd. for C₄₀H₄₄F₃N₁₂O₆⁺ [M+H]⁺: 845.35, found, 845.3.

### Example 68 of compound of Formula (I): Preparation of compound GT-10046

With reference to the method of Scheme I-1, the target compound (GT-10046) was obtained (white solid, 23 mg, yield 44.05%). ¹H NMR (400 MHz, DMSO-d6) δ 11.09 (s, 1H), 10.04 (s, 1H), 9.51 (d, J = 6.5 Hz, 1H), 8.87 (s, 1H), 8.79 (d, J = 7.6 Hz, 1H), 8.41 (d, J = 4.1 Hz, 1H), 8.26 (d, J = 5.6 Hz, 1H), 7.32 - 6.97 (m, 2H), 6.67 (dd, J = 165.1, 7.8 Hz, 2H), 5.35 - 4.97 (m, 2H), 4.77 (d, J = 19.9 Hz, 1H), 4.25 (s, 1H), 3.87 - 3.71 (m, 2H), 3.69 - 3.56 (m, 3H), 3.45 (d, J = 10.2 Hz, 2H), 3.31 - 3.20 (m, 2H), 3.08 (d, J = 33.6 Hz, 5H), 2.93 - 2.83 (m, 1H), 2.58 (d, J = 21.1 Hz, 2H), 2.16 - 1.88 (m, 8H), 1.81 (dd, J = 23.5, 12.0 Hz, 2H), 1.22 (q, J = 11.6 Hz, 2H). LCMS (ESI) calcd. for C₄₀H₄₄F₃N₁₂O₆⁺ [M+H]⁺: 845.35, found, 845.3.

### Example 69 of compound of Formula (I): Preparation of compound GT-08180

With reference to the method of Scheme I-1, the target compound (GT-08180) was obtained (white solid, 27 mg, yield 40.32%). ¹H NMR (400 MHz, DMSO-d6) δ 11.01 (s, 1H), 9.47 (t, J = 23.5 Hz, 1H), 8.79 (d, J = 7.5 Hz, 1H), 8.40 (s, 1H), 8.26 (d, J = 5.6 Hz, 1H), 7.35 - 6.94 (m, 4H), 6.91 - 6.46 (m, 2H), 5.32 - 5.06 (m, 2H), 4.77 (d, J = 19.0 Hz, 1H), 4.44 - 4.13 (m, 3H), 3.80 (t, J = 17.1 Hz, 2H), 3.66 - 3.56 (m, 2H), 3.45 (d, J = 10.2 Hz, 4H), 3.31 - 3.26 (m, 2H), 3.21 - 2.92 (m, 4H), 2.66 (dd, J = 33.0, 18.4 Hz, 3H), 2.45 - 2.34 (m, 1H), 2.00 (dd, J = 32.3, 12.2 Hz, 8H), 1.79 (s, 2H), 1.07 (s, 1H). LCMS (ESI) calcd. for C₄₀H₄₇F₂N₁₂O₅⁺ [M+H]⁺: 813.38, found, 813.4.

### Example 70 of compound of Formula (I): Preparation of compound GT-10047

With reference to the method of Scheme I-1, the target compound (GT-10047) was obtained (white solid, 25 mg, yield 48.23%). ¹H NMR (400 MHz, DMSO-d6) δ 10.94 (s, 1H), 10.25 (s, 1H), 9.51 (d, J = 6.2 Hz, 1H), 8.79 (d, J = 7.7 Hz, 1H), 8.42 (d, J = 4.0 Hz, 1H), 8.26 (d, J = 5.5 Hz, 1H), 7.45 (d, J = 8.4 Hz, 1H), 7.04 (dd, J = 66.9, 7.9 Hz, 3H), 6.67 (dd, J = 165.7, 7.8 Hz, 1H), 5.37 - 4.98 (m, 2H), 4.77 (d, J = 18.9 Hz, 1H), 4.37 - 4.11 (m, 3H), 3.91 - 3.76 (m, 3H), 3.64 (d, J = 10.2 Hz, 2H), 3.45 (d, J = 10.2 Hz, 2H), 3.16 (s, 2H), 2.95 (dd, J = 20.4, 11.0 Hz, 4H), 2.88 - 2.75 (m, 2H), 2.59 (d, J = 17.0 Hz, 3H), 2.33 (s, 1H), 1.95 (ddd, J = 62.7, 25.9, 12.3 Hz, 10H), 1.20 (d, J = 13.1 Hz, 2H). LCMS (ESI) calcd. for C₄₂H₄₉F₂N₁₂O₅⁺ [M+H]⁺: 839.39, found, 839.4.

### Example 71 of compound of Formula (I): Preparation of compound GT-10048

With reference to the method of Scheme I-1, the target compound (GT-10048) was obtained (white solid, 20 mg, yield 38.58%). ¹H NMR (400 MHz, DMSO-d6) δ 10.94 (s, 1H), 10.12 (s, 1H), 9.51 (d, J = 6.5 Hz, 1H), 8.79 (d, J = 7.7 Hz, 1H), 8.41 (d, J = 4.0 Hz, 1H), 8.26 (d, J = 5.5 Hz, 1H), 7.58 (s, 1H), 7.47 (d, J = 8.4 Hz, 1H), 7.00 (s, 2H), 6.70 (d, J = 185.8 Hz, 2H), 5.33 - 4.96 (m, 2H), 4.77 (d, J = 19.7 Hz, 1H), 4.26 (d, J = 36.3 Hz, 3H), 4.07 (s, 2H), 3.81 (s, 2H), 3.60 (s, 2H), 3.29 (d, J = 11.7 Hz, 3H), 2.93 (dd, J = 16.5, 8.3 Hz, 4H), 2.64 - 2.54 (m, 1H), 2.40 - 2.31 (m, 1H), 2.22 (s, 4H), 2.13 - 1.91 (m, 8H), 1.81 (dd, J = 23.8, 11.6 Hz, 2H), 1.25 (dd, J = 23.1, 10.8 Hz, 2H). LCMS (ESI) calcd. for C₄₂H₄₉F₂N₁₂O₅⁺ [M+H]⁺: 839.39, found, 839.4.

### Example 72 of compound of Formula (I): Preparation of compound GT-10049

With reference to the method of Scheme I-1, the target compound (GT-10049) was obtained (white solid, 26 mg, yield 49.33%). ¹H NMR (400 MHz, DMSO-d6) δ 10.94 (s, 1H), 10.73 (s, 1H), 9.51 (d, J = 6.1 Hz, 1H), 8.79 (d, J = 7.7 Hz, 1H), 8.41 (d, J = 4.1 Hz, 1H), 8.27 (t, J = 7.4 Hz, 1H), 7.45 (d, J = 8.4 Hz, 1H), 7.18 (dd, J = 53.8, 3.4 Hz, 1H), 6.94 (s, 1H), 6.89 - 6.45 (m, 2H), 5.30 - 5.00 (m, 2H), 4.77 (d, J = 18.5 Hz, 1H), 4.34 - 4.15 (m, 3H), 4.03 (s, 2H), 3.81 (dd, J = 27.9, 17.0 Hz, 4H), 3.64 (d, J = 10.5 Hz, 2H), 3.46 - 3.30 (m, 4H), 3.12 (t, J = 6.0 Hz, 2H), 2.91 (dd, J = 22.2, 8.9 Hz, 1H), 2.59 (d, J = 17.2 Hz, 2H), 2.41 - 2.31 (m, 1H), 2.13 - 1.86 (m, 10H), 1.75 (dd, J = 22.3, 11.5 Hz, 3H), 1.18 (dd, J = 23.9, 11.7 Hz, 2H). LCMS (ESI) calcd. for C₄₃H₅₁F₂N₁₂O₅⁺ [M+H]⁺: 853.41, found, 853.4.

### Example 73 of compound of Formula (I): Preparation of compound GT-10050

With reference to the method of Scheme I-1, the target compound (GT-10050) was obtained (white solid, 24 mg, yield 46.97%). ¹H NMR (400 MHz, DMSO-D6) δ 10.94 (s, 1H), 10.16 (s, 1H), 9.51 (d, J = 6.4 Hz, 1H), 8.79 (d, J = 7.7 Hz, 1H), 8.41 (d, J = 4.1 Hz, 1H), 8.27 (t, J = 7.4 Hz, 1H), 7.77 (s, 1H), 7.46 (d, J = 8.4 Hz, 1H), 7.30 - 6.98 (m, 1H), 6.96 (s, 1H), 6.91 - 6.44 (m, 2H), 5.32 - 4.99 (m, 2H), 4.77 (d, J = 19.0 Hz, 1H), 4.36 - 4.12 (m, 3H), 3.87 - 3.72 (m, 2H), 3.68 - 3.51 (m, 5H), 3.35 - 3.23 (m, 2H), 3.18 - 2.81 (m, 6H), 2.59 (d, J = 17.2 Hz, 1H), 2.41 - 2.29 (m, 1H), 2.13 - 1.74 (m, 12H), 1.21 (dd, J = 23.6, 11.6 Hz, 2H). LCMS (ESI) calcd. for C₄₁H₄₉F₂N₁₂O₅⁺ [M+H]⁺: 827.39, found, 827.4.

### Example 74 of compound of Formula (I): Preparation of compound GT-10051

With reference to the method of Scheme I-1, the target compound (GT-10051) was obtained (white solid, 20 mg, yield 39.24%). ¹H NMR (400 MHz, DMSO-D6) δ 10.94 (s, 1H), 10.51 (s, 1H), 9.51 (d, J = 6.2 Hz, 1H), 8.79 (d, J = 7.7 Hz, 1H), 8.42 (d, J = 4.1 Hz, 1H), 8.26 (d, J = 5.5 Hz, 1H), 8.01 (s, 1H), 7.53 - 7.44 (m, 1H), 7.14 (dd, J = 40.2, 36.8 Hz, 1H), 6.82 (ddd, J = 173.2, 89.2, 8.9 Hz, 3H), 5.32 - 4.99 (m, 2H), 4.77 (d, J = 19.0 Hz, 1H), 4.43 (s, 1H), 4.36 - 4.14 (m, 4H), 3.86 - 3.36 (m, 10H), 3.33 - 3.30 (m, 1H), 3.05 (s, 1H), 2.97 - 2.82 (m, 2H), 2.59 (d, J = 16.6 Hz, 1H), 2.47 - 2.31 (m, 2H), 2.14 - 1.92 (m, 7H), 1.87 - 1.74 (m, 2H), 1.28 - 1.18 (m, 1H). LCMS (ESI) calcd. for C₄₁H₄₇F₂N₁₂O₅⁺ [M+H]⁺: 825.38, found, 825.4.

### Example 75 of compound of Formula (I): Preparation of compound GT-10052

With reference to the method of Scheme I-1, the target compound (GT-10052) was obtained (white solid, 23 mg, yield 45.12%). ¹H NMR (400 MHz, DMSO-D6) δ 10.94 (s, 1H), 10.51 (s, 1H), 9.51 (d, J = 6.2 Hz, 1H), 8.79 (d, J = 7.7 Hz, 1H), 8.42 (d, J = 4.1 Hz, 1H), 8.26 (d, J = 5.5 Hz, 1H), 7.89 (s, 1H), 7.47 (dd, J = 8.4, 2.1 Hz, 1H), 7.28 - 6.96 (m, 2H), 6.94 - 6.45 (m, 2H), 5.32 - 4.93 (m, 2H), 4.77 (d, J = 19.0 Hz, 1H), 4.27 (dt, J = 33.4, 21.2 Hz, 4H), 3.88 - 3.55 (m, 6H), 3.46 (s, 3H), 3.16 (d, J = 8.9 Hz, 2H), 3.10 - 2.84 (m, 2H), 2.59 (d, J = 16.9 Hz, 1H), 2.41 - 2.17 (m, 3H), 2.15 - 1.93 (m, 7H), 1.86 - 1.72 (m, 2H), 1.33 - 1.10 (m, 2H). LCMS (ESI) calcd. for C₄₁H₄₇F₂N₁₂O₅⁺ [M+H]⁺: 825.38, found, 825.4.

### Example 76 of compound of Formula (I): Preparation of compound GT-10023

With reference to the method of Scheme I-1, the target compound (GT-10023) was obtained (white solid, 32 mg, yield 61.80%). ¹H NMR (400 MHz, DMSO-D6) δ 11.10 (s, 1H), 10.05 (s, 1H), 8.61 (d, J = 2.3 Hz, 1H), 8.46 (s, 1H), 8.04 (d, J = 9.0 Hz, 1H), 7.91 (d, J = 8.8 Hz, 1H), 7.72 (d, J = 9.0 Hz, 1H), 7.61 (d, J = 8.2 Hz, 1H), 7.54 - 7.43 (m, 1H), 7.21 (d, J = 2.4 Hz, 1H), 7.01 (dd, J = 8.8, 2.4 Hz, 2H), 5.07 (dd, J = 12.8, 5.5 Hz, 1H), 4.44 (d, J = 13.2 Hz, 2H), 4.33 (s, 1H), 4.06 (d, J = 9.1 Hz, 1H), 3.58 (s, 3H), 3.20 (dd, J = 22.0, 10.8 Hz, 4H), 3.13 - 2.98 (m, 6H), 2.94 - 2.82 (m, 1H), 2.61 (s, 1H), 2.18 (s, 1H), 2.07 - 1.99 (m, 1H), 1.91 (d, J = 11.4 Hz, 2H), 1.23 (s, 8H), 1.13 (s, 6H). LCMS (ESI) calcd. for C₄₄H₅₀ClFN₉O₆⁺ [M+H]⁺: 854.36, found, 854.4.

### Example 77 of compound of Formula (I): Preparation of compound GT-10053

With reference to the method of Scheme I-1, the target compound (GT-10053) was obtained (white solid, 24 mg, yield 46.35%). ¹H NMR (400 MHz, DMSO-D6) δ 11.10 (d, J = 7.7 Hz, 1H), 10.58 (s, 1H), 8.92 (s, 1H), 8.59 (d, J = 2.1 Hz, 1H), 8.12 (d, J = 8.5 Hz, 1H), 7.91 (d, J = 8.7 Hz, 1H), 7.85 (d, J = 7.8 Hz, 1H), 7.21 (d, J = 2.4 Hz, 1H), 7.13 (d, J = 9.1 Hz, 1H), 7.01 (dd, J = 8.8, 2.4 Hz, 1H), 6.84 (s, 1H), 5.05 (dd, J = 12.8, 5.4 Hz, 1H), 4.45 (d, J = 13.0 Hz, 2H), 4.34 (s, 1H), 4.07 (d, J = 9.1 Hz, 1H), 3.63 (s, 3H), 3.23 (s, 4H), 3.15 - 3.01 (m, 6H), 2.95 - 2.82 (m, 1H), 2.61 (s, 1H), 2.21 (s, 1H), 1.99 (dd, J = 17.3, 9.5 Hz, 3H), 1.26 (d, J = 21.3 Hz, 8H), 1.13 (s, 6H). LCMS (ESI) calcd. for C₄₄H₅₀ClFN₉O₆⁺ [M+H]⁺: 854.36, found, 854.4.

### Example 78 of compound of Formula (I): Preparation of compound GT-08189

With reference to the method of Scheme I-1, the target compound (GT-08189) was obtained (white solid, 41 mg, yield 62.12%). ¹H NMR (400 MHz, DMSO-d6) δ 11.01 (s, 1H), 10.71 (s, 1H), 8.58 (d, J = 2.2 Hz, 1H), 8.19 (d, J = 9.2 Hz, 1H), 7.97 (d, J = 7.7 Hz, 1H), 7.91 (d, J = 8.8 Hz, 1H), 7.31 (t, J = 7.7 Hz, 1H), 7.22 (t, J = 4.9 Hz, 2H), 7.09 (dd, J = 15.9, 7.7 Hz, 2H), 7.01 (dd, J = 8.8, 2.4 Hz, 1H), 5.10 (dd, J = 13.3, 5.0 Hz, 1H), 4.54 - 4.16 (m, 6H), 4.08 (d, J = 9.1 Hz, 1H), 3.28 - 3.01 (m, 11H), 2.98 - 2.88 (m, 1H), 2.63 (d, J = 17.0 Hz, 1H), 2.40 (dd, J = 13.1, 4.5 Hz, 1H), 2.24 (s, 1H), 2.02 (dd, J = 17.1, 9.4 Hz, 3H), 1.40 - 1.26 (m, 2H), 1.24 (s, 6H), 1.13 (s, 6H). LCMS (ESI) calcd. for C₄₄H₅₃ClN₉O₅⁺ [M+H]⁺: 822.39, found, 822.4.

### Example 79 of compound of Formula (I): Preparation of compound GT-09927

With reference to the method of Scheme I-1, the target compound (GT-09927) was obtained (white solid, 23 mg, yield 44.73%). ¹H NMR (400 MHz, DMSO-D6) δ 10.94 (s, 1H), 10.48 (s, 1H), 8.59 (d, J = 1.9 Hz, 1H), 8.15 (d, J = 9.4 Hz, 1H), 7.91 (d, J = 8.7 Hz, 2H), 7.46 (t, J = 7.7 Hz, 1H), 7.22 (d, J = 2.4 Hz, 1H), 7.18 (d, J = 9.0 Hz, 1H), 7.01 (dd, J = 8.8, 2.4 Hz, 1H), 6.96 (t, J = 9.2 Hz, 2H), 5.04 (dd, J = 13.3, 5.1 Hz, 1H), 4.46 (d, J = 12.5 Hz, 2H), 4.35 - 4.25 (m, 2H), 4.18 (d, J = 16.7 Hz, 1H), 4.07 (d, J = 9.1 Hz, 1H), 3.90 (s, 2H), 3.38 - 3.35 (m, 1H), 3.22 - 3.06 (m, 5H), 3.00 - 2.80 (m, 6H), 2.61 (s, 2H), 2.57 (s, 1H), 2.40 - 2.31 (m, 1H), 2.15 (s, 1H), 1.97 (dd, J = 18.3, 9.8 Hz, 3H), 1.36 - 1.22 (m, 8H), 1.13 (s, 6H). LCMS (ESI) calcd. for C₄₆H₅₅ClN₉O₅⁺ [M+H]⁺: 848.40, found, 848.4.

### Example 80 of compound of Formula (I): Preparation of compound GT-09928

With reference to the method of Scheme I-1, the target compound (GT-09928) was obtained (white solid, 32 mg, yield 62.23%). ¹H NMR (400 MHz, DMSO-D6) δ 10.94 (s, 1H), 10.36 (s, 1H), 8.60 (d, J = 2.2 Hz, 1H), 8.12 (d, J = 9.1 Hz, 1H), 7.91 (d, J = 8.7 Hz, 1H), 7.85 (d, J = 8.3 Hz, 1H), 7.48 (dd, J = 8.3, 5.0 Hz, 1H), 7.21 (d, J = 2.4 Hz, 1H), 7.13 (d, J = 8.2 Hz, 1H), 7.01 (dd, J = 8.8, 2.5 Hz, 2H), 6.92 (d, J = 8.3 Hz, 1H), 5.04 (dd, J = 13.3, 5.1 Hz, 1H), 4.47 (d, J = 13.3 Hz, 2H), 4.25 (dd, J = 53.0, 16.5 Hz, 3H), 4.06 (t, J = 10.4 Hz, 3H), 3.32 (s, 2H), 3.08 (t, J = 11.7 Hz, 2H), 2.91 (td, J = 17.9, 8.7 Hz, 5H), 2.59 (d, J = 16.8 Hz, 1H), 2.36 (dd, J = 12.9, 8.1 Hz, 1H), 2.22 (s, 5H), 2.05 - 1.90 (m, 3H), 1.37 - 1.27 (m, 2H), 1.23 (s, 6H), 1.13 (s, 6H). LCMS (ESI) calcd. for C₄₆H₅₅ClN₉O₅⁺ [M+H]⁺: 848.40, found, 848.4.

### Example 81 of compound of Formula (I): Preparation of compound GT-09929

With reference to the method of Scheme I-1, the target compound (GT-09929) was obtained (white solid, 35 mg, yield 66.96%). ¹H NMR (400 MHz, DMSO-D6) δ 11.05 (s, 1H), 10.94 (s, 1H), 8.57 (d, J = 2.0 Hz, 1H), 8.19 (d, J = 8.9 Hz, 1H), 7.95 (d, J = 8.4 Hz, 1H), 7.91 (d, J = 8.8 Hz, 1H), 7.45 (d, J = 8.3 Hz, 1H), 7.21 (d, J = 2.4 Hz, 2H), 7.01 (dd, J = 8.8, 2.4 Hz, 1H), 6.95 (s, 1H), 6.87 (d, J = 8.0 Hz, 1H), 5.03 (dd, J = 13.2, 5.0 Hz, 1H), 4.44 (d, J = 12.8 Hz, 2H), 4.38 - 4.16 (m, 3H), 4.12 - 4.00 (m, 3H), 3.85 (s, 4H), 3.38 - 3.25 (m, 2H), 3.16 - 3.06 (m, 4H), 2.96 - 2.83 (m, 2H), 2.59 (d, J = 16.5 Hz, 1H), 2.40 - 2.32 (m, 1H), 2.11 - 1.86 (m, 7H), 1.25 (d, J = 15.8 Hz, 8H), 1.13 (s, 6H). LCMS (ESI) calcd. for C₄₇H₅₇ClN₉O₅⁺ [M+H]⁺: 862.42, found, 862.4.

### Example 82 of compound of Formula (I): Preparation of compound GT-09930

With reference to the method of Scheme I-1, the target compound (GT-09930) was obtained (white solid, 30 mg, yield %). ¹H NMR (400 MHz, DMSO-D6) δ 10.94 (s, 1H), 10.68 (s, 1H), 8.57 (d, J = 2.1 Hz, 1H), 8.21 (d, J = 8.6 Hz, 1H), 7.99 (d, J = 8.7 Hz, 1H), 7.91 (d, J = 8.8 Hz, 1H), 7.45 (t, J = 10.4 Hz, 1H), 7.26 (d, J = 9.5 Hz, 1H), 7.22 (d, J = 2.4 Hz, 1H), 7.01 (dd, J = 8.8, 2.4 Hz, 1H), 6.96 (s, 1H), 6.90 (t, J = 9.4 Hz, 1H), 5.03 (dd, J = 13.2, 5.1 Hz, 1H), 4.46 (d, J = 12.7 Hz, 2H), 4.38 - 4.17 (m, 3H), 4.09 (s, 1H), 3.32 (d, J = 13.9 Hz, 5H), 3.13 (dd, J = 24.4, 9.9 Hz, 6H), 2.99 - 2.84 (m, 2H), 2.59 (d, J = 16.6 Hz, 1H), 2.43 - 2.30 (m, 1H), 2.04 (ddd, J = 34.2, 26.2, 16.7 Hz, 6H), 1.37 - 1.26 (m, 2H), 1.24 (s, 6H), 1.13 (s, 6H). LCMS (ESI) calcd. for C₄₅H₅₅ClN₉O₅⁺ [M+H]⁺: 836.40, found, 836.4.

### Example 83 of compound of Formula (I): Preparation of compound GT-09931

With reference to the method of Scheme I-1, the target compound (GT-09931) was obtained (white solid, 16 mg, yield 31.64 %). ¹H NMR (400 MHz, DMSO-D6) δ 10.94 (s, 1H), 10.51 (s, 1H), 8.64 - 8.59 (m, 1H), 8.03 (d, J = 9.2 Hz, 1H), 7.91 (d, J = 8.7 Hz, 1H), 7.70 (d, J = 9.7 Hz, 1H), 7.49 (dd, J = 8.3, 3.4 Hz, 1H), 7.21 (d, J = 2.4 Hz, 1H), 7.03 - 6.84 (m, 3H), 5.03 (dd, J = 13.4, 4.9 Hz, 1H), 4.45 (d, J = 14.9 Hz, 3H), 4.31 (d, J = 14.1 Hz, 2H), 4.18 (d, J = 16.9 Hz, 1H), 4.06 (d, J = 9.1 Hz, 1H), 3.45 (s, 7H), 3.07 - 2.87 (m, 5H), 2.59 (d, J = 17.2 Hz, 1H), 2.40 - 2.28 (m, 1H), 2.07 (s, 1H), 1.99 - 1.82 (m, 3H), 1.22 (s, 8H), 1.12 (s, 6H). LCMS (ESI) calcd. for C₄₅H₅₃ClN₉O₅⁺ [M+H]⁺: 834.39, found, 834.4.

### Example 84 of compound of Formula (I): Preparation of compound GT-09932

With reference to the method of Scheme I-1, the target compound (GT-09932) was obtained (white solid, 33 mg, yield 65.26%). ¹H NMR (400 MHz, DMSO-D6) δ 10.94 (s, 1H), 10.71 (s, 1H), 9.70 (s, 1H), 8.60 (d, J = 2.1 Hz, 1H), 8.09 (d, J = 8.7 Hz, 1H), 7.91 (d, J = 8.8 Hz, 1H), 7.79 (d, J = 7.7 Hz, 1H), 7.46 (dd, J = 8.3, 6.1 Hz, 1H), 7.21 (d, J = 2.4 Hz, 1H), 7.08 (s, 1H), 7.04 - 6.96 (m, 2H), 6.90 (dd, J = 15.1, 8.4 Hz, 1H), 5.13 - 4.99 (m, 1H), 4.44 (s, 2H), 4.37 - 4.12 (m, 4H), 4.07 (d, J = 9.1 Hz, 1H), 3.59 (s, 6H), 3.17 (s, 2H), 3.05 (d, J = 10.8 Hz, 2H), 2.89 (dd, J = 21.9, 9.5 Hz, 1H), 2.61 (s, 1H), 2.43 - 2.32 (m, 1H), 2.22 (s, 2H), 1.99 (dd, J = 35.9, 10.1 Hz, 3H), 1.23 (s, 8H), 1.13 (s, 6H). LCMS (ESI) calcd. for C₄₅H₅₃ClN₉O₅⁺ [M+H]⁺: 834.39, found, 834.4.

### Example 85 of compound of Formula (I): Preparation of compound GT-09764

With reference to the method of Scheme I-1, the target compound (GT-09764) was obtained (white solid, 10 mg, yield 19.77%). ¹H NMR (400 MHz, DMSO-D6) δ 11.15 (s, 1H), 11.07 (s, 1H), 10.72 (s, 1H), 10.19 (d, J = 11.4 Hz, 1H), 9.13 (s, 1H), 8.85 (s, 1H), 8.50 (s, 1H), 8.00 (dd, J = 6.8, 2.6 Hz, 1H), 7.70 (ddd, J = 8.9, 4.3, 2.6 Hz, 1H), 7.64 (d, J = 8.3 Hz, 1H), 7.53 (t, J = 9.1 Hz, 1H), 7.44 (s, 1H), 7.25 (s, 1H), 7.05 (d, J = 10.1 Hz, 1H), 6.99 (dd, J = 15.1, 7.4 Hz, 1H), 6.80 (d, J = 15.6 Hz, 1H), 5.05 (dd, J = 12.8, 5.4 Hz, 1H), 4.08 (s, 3H), 3.97 (d, J = 14.6 Hz, 2H), 3.57 (s, 6H), 3.10 (d, J = 11.9 Hz, 7H), 3.00 - 2.85 (m, 3H), 2.59 (d, J = 17.6 Hz, 2H), 2.11 (d, J = 11.4 Hz, 2H), 2.04 - 1.95 (m, 1H), 1.65 (d, J = 11.7 Hz, 2H). LCMS (ESI) calcd. for C₄₂H₄₅ClFN₁₀O₆⁺ [M+H]⁺: 839.32, found, 839.3.

### Example 86 of compound of Formula (I): Preparation of compound GT-09920

With reference to the method of Scheme I-1, the target compound (GT-09920) was obtained (white solid, 20 mg, yield 33.19%). ¹H NMR (400 MHz, DMSO-D6) δ 11.24 (s, 1H), 11.07 (s, 2H), 10.49 (s, 1H), 10.14 (s, 1H), 9.12 (s, 1H), 8.83 (s, 1H), 8.48 (s, 1H), 8.01 (dd, J = 6.7, 2.6 Hz, 1H), 7.76 - 7.69 (m, 1H), 7.65 (d, J = 10.2 Hz, 1H), 7.59 - 7.53 (m, 1H), 7.51 (d, J = 9.1 Hz, 1H), 7.44 (s, 1H), 7.06 - 6.95 (m, 1H), 6.80 (d, J = 15.5 Hz, 1H), 5.07 (dd, J = 12.9, 5.4 Hz, 1H), 4.07 (s, 3H), 3.97 (d, J = 15.2 Hz, 2H), 3.57 (s, 6H), 3.10 (d, J = 10.4 Hz, 7H), 3.00 - 2.85 (m, 3H), 2.59 (d, J = 19.3 Hz, 2H), 2.20 - 1.96 (m, 4H), 1.66 (d, J = 13.7 Hz, 1H). LCMS (ESI) calcd. for C₄₂H₄₄ClF₂N₁₀O₆⁺ [M+H]⁺: 857.31, found, 857.3.

### Example 87 of compound of Formula (I): Preparation of compound GT-09921

With reference to the method of Scheme I-1, the target compound (GT-09921) was obtained (white solid, 15 mg, yield 25.86%). ¹H NMR (400 MHz, DMSO-D6) δ 11.34 (d, J = 32.5 Hz, 2H), 10.93 (s, 1H), 10.85 (s, 1H), 10.21 (d, J = 12.8 Hz, 1H), 9.16 (s, 1H), 8.88 (s, 1H), 7.99 (dd, J = 6.8, 2.5 Hz, 1H), 7.77 (s, 1H), 7.69 (ddd, J = 8.9, 4.3, 2.6 Hz, 1H), 7.54 (t, J = 9.0 Hz, 1H), 7.47 (dd, J = 14.4, 6.1 Hz, 2H), 7.02 (dt, J = 14.6, 7.2 Hz, 1H), 6.95 (s, 1H), 6.89 (d, J = 8.4 Hz, 1H), 6.80 (d, J = 15.4 Hz, 1H), 5.03 (dd, J = 13.1, 5.1 Hz, 1H), 4.37 - 4.15 (m, 2H), 4.08 (s, 3H), 3.95 (s, 2H), 3.55 (s, 6H), 3.19 - 3.02 (m, 7H), 2.92 (dd, J = 29.3, 11.7 Hz, 3H), 2.61 (s, 1H), 2.35 (td, J = 13.0, 9.6 Hz, 1H), 2.12 (d, J = 12.2 Hz, 3H), 2.00 - 1.91 (m, 1H), 1.68 (d, J = 12.4 Hz, 1H). LCMS (ESI) C₄₂H₄₇ClFN₁₀O₅⁺ [M+H]⁺: calcd. for 825.34, found, 825.3.

### Example 88 of compound of Formula (I): Preparation of compound GT-09922

With reference to the method of Scheme I-1, the target compound (GT-09922) was obtained (white solid, 15 mg, yield 25.31%). ¹H NMR (400 MHz, DMSO-D6) δ 11.43 (s, 1H), 11.22 (s, 1H), 10.96 (s, 1H), 10.67 (s, 1H), 10.21 (d, J = 12.7 Hz, 1H), 9.15 (s, 1H), 8.86 (s, 1H), 8.00 (dd, J = 6.8, 2.5 Hz, 1H), 7.78 (s, 1H), 7.74 - 7.67 (m, 1H), 7.57 - 7.48 (m, 2H), 7.43 (d, J = 8.2 Hz, 1H), 7.38 - 7.28 (m, 1H), 7.02 (dt, J = 14.1, 7.1 Hz, 1H), 6.80 (d, J = 15.3 Hz, 1H), 5.11 - 4.98 (m, 1H), 4.37 (dd, J = 71.8, 16.8 Hz, 2H), 4.08 (s, 3H), 3.95 (s, 2H), 3.56 (s, 5H), 3.24 (d, J = 15.4 Hz, 6H), 3.06 (s, 3H), 2.92 (dd, J = 26.4, 13.5 Hz, 3H), 2.59 (d, J = 17.5 Hz, 1H), 2.43 - 2.33 (m, 1H), 2.10 (d, J = 11.8 Hz, 2H), 2.01 - 1.91 (m, 1H), 1.68 (d, J = 12.6 Hz, 1H). LCMS (ESI) calcd. for C₄₂H₄₆ClF₂N₁₀O₅⁺ [M+H]⁺: 843.33, found, 843.3.

### Example 89 of compound of Formula (I): Preparation of compound GT-09923

With reference to the method of Scheme I-1, the target compound (GT-09923) was obtained (white solid, 18 mg, yield 30.08%). ¹H NMR (400 MHz, DMSO-D6) δ 11.46 (s, 2H), 10.94 (s, 1H), 10.83 (s, 1H), 10.23 (s, 1H), 9.20 (s, 1H), 8.92 (s, 1H), 7.98 (dd, J = 6.8, 2.5 Hz, 1H), 7.72 - 7.65 (m, 1H), 7.59 - 7.52 (m, 2H), 7.44 (d, J = 8.6 Hz, 1H), 7.02 (s, 1H), 6.95 (s, 1H), 6.81 (d, J = 15.4 Hz, 1H), 5.03 (dd, J = 13.2, 5.0 Hz, 1H), 4.24 (dd, J = 50.7, 16.7 Hz, 2H), 4.09 (s, 3H), 3.92 (d, J = 26.3 Hz, 3H), 3.53 (s, 4H), 3.37 - 3.32 (m, 3H), 3.20 (s, 3H), 2.97 (t, J = 31.0 Hz, 7H), 2.81 (s, 2H), 2.59 (d, J = 17.0 Hz, 2H), 2.33 (s, 1H), 2.14 (d, J = 13.5 Hz, 3H), 1.96 (d, J = 5.1 Hz, 1H), 1.69 (s, 1H). LCMS (ESI) calcd. for C₄₄H₄₉ClFN₁₀O₅⁺ [M+H]⁺: 851.36, found, 851.4.

### Example 90 of compound of Formula (I): Preparation of compound GT-09924

With reference to the method of Scheme I-1, the target compound (GT-09924) was obtained (white solid, 16 mg, yield 26.74%). ¹H NMR (400 MHz, DMSO-D6) δ 11.28 (d, J = 64.4 Hz, 2H), 10.94 (s, 1H), 10.66 (s, 1H), 10.20 (d, J = 12.3 Hz, 1H), 9.15 (s, 1H), 8.86 (s, 1H), 8.00 (dd, J = 6.7, 2.5 Hz, 1H), 7.75 - 7.67 (m, 1H), 7.61 (s, 1H), 7.54 (t, J = 9.1 Hz, 1H), 7.49 - 7.43 (m, 2H), 7.07 - 6.98 (m, 2H), 6.92 (d, J = 8.3 Hz, 1H), 6.80 (d, J = 15.7 Hz, 1H), 5.03 (dd, J = 13.2, 5.0 Hz, 1H), 4.25 (dd, J = 53.5, 17.0 Hz, 2H), 4.08 (s, 5H), 3.96 (s, 2H), 3.45 (s, 5H), 3.31 - 3.19 (m, 5H), 2.95 (d, J = 5.8 Hz, 5H), 2.59 (d, J = 17.5 Hz, 1H), 2.17 (d, J = 33.3 Hz, 5H), 2.00 - 1.91 (m, 1H), 1.71 (d, J = 12.8 Hz, 1H). LCMS (ESI) calcd. for C₄₄H₄₉ClFN₁₀O₅⁺ [M+H]⁺: 851.36, found, 851.4.

### Example 91 of compound of Formula (I): Preparation of compound GT-09925

With reference to the method of Scheme I-1, the target compound (GT-09925) was obtained (white solid, 15 mg, yield 24.66%). ¹H NMR (400 MHz, DMSO-D6) δ 11.40 (d, J = 56.0 Hz, 2H), 10.93 (s, 1H), 10.23 (s, 1H), 9.20 (s, 1H), 8.92 (s, 1H), 7.97 (dd, J = 6.8, 2.4 Hz, 1H), 7.71 - 7.65 (m, 1H), 7.59 - 7.51 (m, 2H), 7.44 (d, J = 8.4 Hz, 1H), 7.02 (dd, J = 14.9, 7.5 Hz, 1H), 6.93 (s, 1H), 6.83 (dd, J = 21.9, 12.1 Hz, 2H), 5.02 (dd, J = 13.1, 5.0 Hz, 1H), 4.38 - 4.15 (m, 2H), 4.09 (s, 3H), 4.00 (s, 2H), 3.94 (s, 2H), 3.86 (s, 2H), 3.59 (s, 4H), 3.16 (s, 4H), 2.92 (d, J = 12.3 Hz, 3H), 2.58 (d, J = 18.0 Hz, 2H), 2.35 (dd, J = 12.8, 8.4 Hz, 1H), 2.18 - 1.82 (m, 7H), 1.63 (d, J = 12.1 Hz, 1H). LCMS (ESI) calcd. for C₄₅H₅₁ClFN₁₀O₅⁺ [M+H]⁺: 865.37, found, 865.4.

### Example 92 of compound of Formula (I): Preparation of compound GT-09926

With reference to the method of Scheme I-1, the target compound (GT-09926) was obtained (white solid, 6 mg, yield 10.17%). ¹H NMR (400 MHz, DMSO-D6) δ 11.29 (s, 2H), 10.93 (s, 1H), 10.75 (s, 1H), 10.21 (d, J = 13.8 Hz, 1H), 9.16 (s, 1H), 8.89 (s, 1H), 7.99 (dd, J = 6.8, 2.3 Hz, 1H), 7.81 (s, 1H), 7.72 - 7.65 (m, 1H), 7.54 (t, J = 9.0 Hz, 1H), 7.50 - 7.40 (m, 2H), 7.07 - 6.91 (m, 2H), 6.88 (d, J = 7.9 Hz, 1H), 6.80 (d, J = 15.3 Hz, 1H), 5.03 (dd, J = 13.3, 5.1 Hz, 1H), 4.35 - 4.15 (m, 2H), 4.08 (s, 3H), 3.94 (s, 2H), 3.50 (s, 6H), 3.11 (s, 7H), 3.01 - 2.86 (m, 4H), 2.61 (s, 1H), 2.34 (d, J = 7.9 Hz, 1H), 2.13 (d, J = 12.7 Hz, 4H), 1.95 (s, 1H), 1.65 (d, J = 11.2 Hz, 1H). LCMS (ESI) calcd. for C₄₃H₄₉ClFN₁₀O₅⁺ [M+H]⁺: 839.36, found, 839.4.

### Example 93 of compound of Formula (I): Preparation of compound GT-09628

With reference to the method of Scheme I-1, the target compound (GT-09628) was obtained (white solid, 32 mg, yield 61.96%). ¹H NMR (400 MHz, DMSO-D6) δ 11.68 (s, 1H), 11.06 (s, 1H), 10.78 (s, 1H), 10.37 (s, 1H), 8.86 (s, 1H), 8.60 (s, 1H), 8.51 (s, 1H), 8.11 (dd, J = 6.8, 2.6 Hz, 1H), 7.84 (ddd, J = 8.9, 4.3, 2.6 Hz, 1H), 7.64 (d, J = 8.3 Hz, 1H), 7.54 (t, J = 9.1 Hz, 1H), 7.39 (s, 1H), 7.25 (s, 1H), 7.07 (s, 1H), 5.05 (dd, J = 12.8, 5.4 Hz, 1H), 4.39 (t, J = 6.0 Hz, 2H), 4.02 (s, 3H), 3.59 (s, 3H), 3.30 - 3.17 (m, 9H), 3.10 (d, J = 19.1 Hz, 4H), 3.01 - 2.84 (m, 3H), 2.59 (d, J = 17.5 Hz, 2H), 2.33 (d, J = 1.9 Hz, 2H), 2.11 (d, J = 13.1 Hz, 2H), 2.06 - 1.96 (m, 1H), 1.63 (d, J = 12.3 Hz, 1H). LCMS (ESI) calcd. for C₄₁H₄₆ClFN₉O₆⁺ [M+H]⁺: 814.32, found, 814.3.

### Example 94 of compound of Formula (I): Preparation of compound GT-10025

With reference to the method of Scheme I-1, the target compound (GT-10025) was obtained (white solid, 27 mg, yield 51.15%). ¹H NMR (400 MHz, DMSO-D6) δ 11.58 (s, 1H), 11.10 (s, 1H), 10.40 (d, J = 42.9 Hz, 2H), 8.85 (s, 1H), 8.56 (s, 1H), 8.49 (d, J = 16.3 Hz, 1H), 8.11 (dd, J = 6.8, 2.6 Hz, 1H), 7.89 - 7.79 (m, 1H), 7.60 (d, J = 8.2 Hz, 1H), 7.54 (t, J = 9.1 Hz, 1H), 7.47 (t, J = 7.6 Hz, 1H), 7.37 (d, J = 5.7 Hz, 1H), 5.06 (dd, J = 12.8, 5.5 Hz, 1H), 4.39 (t, J = 5.9 Hz, 2H), 4.02 (s, 3H), 3.59 (s, 3H), 3.28 - 3.20 (m, 8H), 3.09 (d, J = 6.4 Hz, 4H), 3.00 - 2.84 (m, 3H), 2.59 (d, J = 21.4 Hz, 2H), 2.37 - 2.29 (m, 2H), 2.05 (dd, J = 28.1, 9.4 Hz, 4H), 1.63 (d, J = 12.6 Hz, 1H). LCMS (ESI) calcd. for C₄₁H₄₅ClF₂N₉O₆⁺ [M+H]⁺: 832.31, found, 832.3.

### Example 95 of compound of Formula (I): Preparation of compound GT-09630

With reference to the method of Scheme I-1, the target compound (GT-09630) was obtained (white solid, 21 mg, yield 39.78%). ¹H NMR (400 MHz, DMSO-D6) δ 11.74 (s, 1H), 11.09 (s, 1H), 10.48 (s, 2H), 8.86 (s, 1H), 8.64 (s, 1H), 8.50 (s, 1H), 8.12 (dd, J = 6.8, 2.5 Hz, 1H), 7.85 (dd, J = 8.2, 3.4 Hz, 1H), 7.65 (d, J = 10.3 Hz, 1H), 7.60 - 7.50 (m, 2H), 7.40 (s, 1H), 5.08 (dd, J = 12.7, 5.4 Hz, 1H), 4.41 (d, J = 5.8 Hz, 2H), 4.01 (s, 3H), 3.59 (s, 3H), 3.28 (s, 8H), 3.10 (d, J = 10.6 Hz, 4H), 2.92 (dd, J = 37.9, 12.1 Hz, 3H), 2.59 (d, J = 18.2 Hz, 2H), 2.34 (s, 2H), 2.05 (dd, J = 29.7, 9.7 Hz, 4H), 1.65 (d, J = 13.6 Hz, 1H). LCMS (ESI) calcd. for C₄₁H₄₅ClF₂N₉O₆⁺ [M+H]⁺: 832.31, found, 832.3.

### Example 96 of compound of Formula (I): Preparation of compound GT-10153

With reference to the method of Scheme I-1, the target compound (GT-10153) was obtained (white solid, 19 mg, yield 35.99%). ¹H NMR (400 MHz, DMSO-d6) δ 11.75 (s, 1H), 11.09 (s, 1H), 10.84 (s, 1H), 10.40 (s, 1H), 8.93 (s, 1H), 8.87 (s, 1H), 8.63 (s, 1H), 8.11 (dd, J = 6.8, 2.3 Hz, 1H), 7.88 - 7.80 (m, 1H), 7.55 (t, J = 9.1 Hz, 1H), 7.40 (s, 1H), 6.95 (d, J = 81.9 Hz, 2H), 5.05 (dd, J = 12.7, 5.4 Hz, 1H), 4.40 (d, J = 6.1 Hz, 2H), 4.02 (s, 3H), 3.60 (d, J = 9.7 Hz, 4H), 3.26 (s, 6H), 3.09 (d, J = 24.4 Hz, 4H), 3.00 - 2.83 (m, 3H), 2.60 (s, 2H), 2.33 (s, 2H), 2.11 (d, J = 12.1 Hz, 3H), 2.05 - 1.98 (m, 1H), 1.63 (d, J = 12.5 Hz, 2H). LCMS (ESI) calcd. for C₄₁H₄₅ClF₂N₉O₆⁺ [M+H]⁺: 832.31, found, 832.3.

### Example 97 of compound of Formula (I): Preparation of compound GT-10154

With reference to the method of Scheme I-1, the target compound (GT-10154) was obtained (white solid, 40 mg, yield 77.06%). ¹H NMR (400 MHz, DMSO-d6) δ 11.91 (s, 1H), 10.96 (s, 1H), 10.67 (s, 2H), 8.87 (s, 1H), 8.70 (s, 1H), 8.11 (dd, J = 6.8, 2.4 Hz, 1H), 7.86 (ddd, J = 8.9, 4.3, 2.6 Hz, 1H), 7.79 (s, 1H), 7.53 (dd, J = 9.9, 8.2 Hz, 1H), 7.44 (s, 1H), 7.36 (d, J = 10.6 Hz, 1H), 7.28 (d, J = 7.2 Hz, 1H), 5.05 (dd, J = 13.2, 5.0 Hz, 1H), 4.41 (d, J = 5.8 Hz, 2H), 4.26 (dd, J = 50.1, 16.9 Hz, 2H), 4.01 (s, 3H), 3.58 (s, 3H), 3.24 (s, 7H), 3.07 (s, 3H), 3.01 - 2.86 (m, 3H), 2.59 (d, J = 16.4 Hz, 1H), 2.40 - 2.31 (m, 3H), 2.10 (d, J = 13.6 Hz, 3H), 2.01 - 1.88 (m, 1H), 1.72 - 1.64 (m, 1H). LCMS (ESI) calcd. for C₄₁H₄₇ClF₂N₉O₅⁺ [M+H]⁺: 818.34, found, 818.4.

### Example 98 of compound of Formula (I): Preparation of compound GT-09634

With reference to the method of Scheme I-1, the target compound (GT-09634) was obtained (white solid, 14 mg, yield 40.40%). ¹H NMR (400 MHz, DMSO-D6) δ 11.78 (s, 1H), 10.96 (s, 1H), 10.53 (s, 2H), 8.86 (s, 1H), 8.66 (s, 1H), 8.12 (dd, J = 6.7, 2.5 Hz, 1H), 7.89 - 7.82 (m, 1H), 7.77 (s, 1H), 7.54 (t, J = 9.1 Hz, 1H), 7.43 (d, J = 8.7 Hz, 2H), 7.32 (t, J = 7.6 Hz, 1H), 5.05 (dd, J = 13.2, 5.0 Hz, 1H), 4.55 - 4.27 (m, 4H), 4.01 (s, 3H), 3.58 (s, 4H), 3.22 (s, 7H), 3.06 (s, 4H), 3.00 - 2.85 (m, 3H), 2.59 (d, J = 17.1 Hz, 1H), 2.38 (d, J = 26.4 Hz, 3H), 2.09 (d, J = 13.7 Hz, 3H), 1.95 (dd, J = 18.1, 12.7 Hz, 1H), 1.66 (d, J = 11.8 Hz, 1H). LCMS (ESI) calcd. for C₄₁H₄₇ClF₂N₉O₅⁺ [M+H]⁺: 818.34, found, 818.4.

### Example 99 of compound of Formula (I): Preparation of compound GT-09631

With reference to the method of Scheme I-1, the target compound (GT-09631) was obtained (white solid, 20 mg, yield 38.53%). ¹H NMR (400 MHz, DMSO-D6) δ 11.79 (s, 1H), 10.95 (s, 1H), 10.81 (s, 1H), 10.45 (s, 1H), 8.87 (s, 1H), 8.65 (s, 1H), 8.11 (dd, J = 6.8, 2.5 Hz, 1H), 7.90 - 7.82 (m, 1H), 7.54 (t, J = 9.1 Hz, 1H), 7.41 (s, 1H), 6.71 (s, 1H), 6.63 (d, J = 12.0 Hz, 1H), 4.99 (dd, J = 13.1, 5.0 Hz, 1H), 4.30 (dt, J = 55.8, 11.7 Hz, 4H), 4.01 (s, 3H), 3.60 (d, J = 11.0 Hz, 4H), 3.26 (s, 8H), 3.07 (d, J = 11.5 Hz, 3H), 2.92 (dd, J = 32.2, 12.0 Hz, 3H), 2.58 (d, J = 17.7 Hz, 2H), 2.34 (s, 3H), 2.11 (d, J = 13.2 Hz, 2H), 1.99 - 1.91 (m, 1H), 1.64 (d, J = 12.7 Hz, 1H). LCMS (ESI) calcd. for C₄₁H₄₇ClF₂N₉O₅⁺ [M+H]⁺: 818.34, found, 818.4.

### Example 100 of compound of Formula (I): Preparation of compound GT-09681

With reference to the method of Scheme I-1, the target compound (GT-09681) was obtained (white solid, 14 mg, yield 27.58%). ¹H NMR (400 MHz, DMSO-D6) δ 11.73 (s, 1H), 11.01 (s, 1H), 10.79 (s, 1H), 10.44 (s, 1H), 8.86 (s, 1H), 8.63 (s, 1H), 8.11 (dd, J = 6.8, 2.5 Hz, 1H), 7.84 (ddd, J = 9.0, 4.3, 2.6 Hz, 1H), 7.54 (t, J = 9.1 Hz, 1H), 7.39 (d, J = 4.5 Hz, 1H), 7.31 (t, J = 7.7 Hz, 2H), 7.09 (dd, J = 13.5, 7.6 Hz, 2H), 5.10 (dd, J = 13.2, 5.0 Hz, 1H), 4.34 (dt, J = 51.9, 11.7 Hz, 4H), 4.01 (s, 3H), 3.67 - 3.53 (m, 4H), 3.28 (s, 5H), 3.12 (dd, J = 31.1, 17.0 Hz, 7H), 2.99 - 2.90 (m, 2H), 2.62 (d, J = 17.2 Hz, 1H), 2.42 - 2.30 (m, 3H), 2.22 - 1.99 (m, 4H), 1.65 (d, J = 12.7 Hz, 1H). LCMS (ESI) calcd. for C₄₁H₄₈ClFN₉O₅⁺ [M+H]⁺: 800.35, found, 800.4.

### Example 101 of compound of Formula (I): Preparation of compound GT-09682

With reference to the method of Scheme I-1, the target compound (GT-09682) was obtained (white solid, 22 mg, yield 41.97%). ¹H NMR (400 MHz, DMSO-D6) δ 11.97 (s, 1H), 10.94 (s, 1H), 10.64 (d, J = 77.1 Hz, 1H), 8.90 (s, 1H), 8.70 (s, 1H), 8.10 (dd, J = 6.8, 2.6 Hz, 1H), 7.84 (ddd, J = 8.9, 4.3, 2.6 Hz, 1H), 7.55 (t, J = 9.1 Hz, 1H), 7.49 - 7.39 (m, 2H), 6.95 (s, 1H), 5.03 (dd, J = 13.2, 5.0 Hz, 1H), 4.42 (d, J = 5.8 Hz, 2H), 4.24 (dd, J = 50.6, 16.8 Hz, 2H), 4.01 (d, J = 3.5 Hz, 3H), 3.89 (s, 1H), 3.59 (s, 5H), 3.23 (d, J = 28.0 Hz, 6H), 2.98 (dd, J = 41.1, 15.4 Hz, 6H), 2.81 (s, 1H), 2.59 (d, J = 17.5 Hz, 2H), 2.34 (dd, J = 5.2, 3.5 Hz, 3H), 2.18 - 1.89 (m, 4H), 1.64 (d, J = 12.6 Hz, 1H). LCMS (ESI) calcd. for C₄₃H₅₀ClFN₉O₅⁺ [M+H]⁺: 826.36, found, 826.4.

### Example 102 of compound of Formula (I): Preparation of compound GT-09632Preparation of compound

With reference to the method of Scheme I-1, the target compound (GT-09632) was obtained (white solid, 16 mg, yield 30.52%). ¹H NMR (400 MHz, DMSO-D6) δ 11.76 (s, 1H), 10.94 (s, 1H), 10.54 (d, J = 37.1 Hz, 2H), 8.86 (s, 1H), 8.67 (d, J = 10.9 Hz, 1H), 8.12 (dd, J = 6.8, 2.4 Hz, 1H), 7.91 - 7.81 (m, 1H), 7.61 (s, 1H), 7.54 (t, J = 9.1 Hz, 1H), 7.47 (d, J = 8.4 Hz, 1H), 7.41 (s, 1H), 6.99 (s, 1H), 6.92 (d, J = 8.5 Hz, 1H), 5.03 (dd, J = 13.2, 5.0 Hz, 1H), 4.39 (d, J = 5.6 Hz, 2H), 4.25 (dd, J = 53.3, 17.0 Hz, 2H), 4.04 (d, J = 23.1 Hz, 5H), 3.61 (d, J = 10.9 Hz, 2H), 3.29 - 3.14 (m, 5H), 3.02 - 2.86 (m, 6H), 2.59 (d, J = 17.0 Hz, 1H), 2.34 (d, J = 7.3 Hz, 3H), 2.25 - 2.07 (m, 6H), 2.00 - 1.91 (m, 1H), 1.68 (d, J = 12.1 Hz, 1H). LCMS (ESI) calcd. for C₄₃H₅₀ClFN₉O₅⁺ [M+H]⁺: 826.36, found, 826.4.

### Example 103 of compound of Formula (I): Preparation of compound GT-09633

With reference to the method of Scheme I-1, the target compound (GT-09633) was obtained (white solid, 21 mg, yield 39.39%). ¹H NMR (400 MHz, DMSO-D6) δ 11.98 (s, 1H), 11.23 (s, 1H), 10.93 (s, 1H), 10.49 (s, 1H), 8.90 (s, 1H), 8.70 (s, 1H), 8.10 (dd, J = 6.7, 2.5 Hz, 1H), 7.84 (dd, J = 4.7, 2.7 Hz, 1H), 7.55 (t, J = 9.1 Hz, 1H), 7.44 (d, J = 8.5 Hz, 2H), 6.99 - 6.81 (m, 2H), 5.03 (dd, J = 13.3, 5.1 Hz, 1H), 4.41 (d, J = 6.1 Hz, 2H), 4.33 - 4.15 (m, 2H), 4.02 (s, 5H), 3.85 (s, 2H), 3.20 (d, J = 40.5 Hz, 6H), 3.02 - 2.88 (m, 3H), 2.61 (s, 2H), 2.41 - 2.28 (m, 4H), 1.98 (dd, J = 33.5, 9.7 Hz, 8H), 1.60 (d, J = 12.5 Hz, 2H). LCMS (ESI) calcd. for C₄₄H₅₂ClFN₉O₅⁺ [M+H]⁺: 840.38, found, 840.4.

### Example 104 of compound of Formula (I): Preparation of compound GT-09683

With reference to the method of Scheme I-1, the target compound (GT-09683) was obtained (white solid, 25 mg, yield 48.39%). ¹H NMR (400 MHz, DMSO-D6) δ 11.88 (s, 1H), 10.94 (s, 1H), 10.77 (s, 1H), 10.45 (s, 1H), 8.88 (s, 1H), 8.66 (s, 1H), 8.10 (dd, J = 6.8, 2.6 Hz, 1H), 7.83 (ddd, J = 9.0, 4.3, 2.6 Hz, 1H), 7.55 (t, J = 9.1 Hz, 1H), 7.48 - 7.39 (m, 2H), 6.95 (s, 1H), 6.89 (d, J = 8.1 Hz, 1H), 5.03 (dd, J = 13.3, 5.0 Hz, 1H), 4.40 (t, J = 6.0 Hz, 2H), 4.24 (dd, J = 53.2, 16.4 Hz, 2H), 4.02 (s, 3H), 3.60 (d, J = 10.9 Hz, 5H), 3.28 (d, J = 8.1 Hz, 6H), 3.11 (s, 3H), 3.03 - 2.84 (m, 4H), 2.39 - 2.27 (m, 3H), 2.13 (d, J = 12.9 Hz, 4H), 1.95 (s, 1H), 1.63 (d, J = 12.4 Hz, 1H). LCMS (ESI) calcd. for C₄₂H₅₀ClFN₉O₅⁺ [M+H]⁺: 814.36, found, 814.4.

### Example 105 of compound of Formula (I): Preparation of compound GT-09684

With reference to the method of Scheme I-1, the target compound (GT-09684) was obtained (white solid, 17 mg, yield 32.99%). ¹H NMR (400 MHz, DMSO-D6) δ 11.82 (s, 1H), 10.94 (s, 1H), 10.41 (s, 1H), 8.88 (s, 1H), 8.64 (d, J = 3.3 Hz, 1H), 8.14 - 8.05 (m, 1H), 7.91 - 7.81 (m, 1H), 7.55 (t, J = 9.1 Hz, 1H), 7.46 (dd, J = 8.3, 4.0 Hz, 1H), 7.41 (s, 1H), 7.02 - 6.86 (m, 2H), 5.03 (dd, J = 13.3, 5.1 Hz, 1H), 4.40 (s, 2H), 4.35 - 4.17 (m, 2H), 4.02 (s, 3H), 3.60 (s, 3H), 3.22 (d, J = 44.8 Hz, 8H), 3.01 - 2.86 (m, 3H), 2.59 (d, J = 16.5 Hz, 1H), 2.40 - 2.28 (m, 4H), 2.24 (s, 2H), 2.08 (d, J = 12.5 Hz, 1H), 2.00 - 1.93 (m, 1H), 1.65 (d, J = 10.8 Hz, 2H). LCMS (ESI) calcd. for C₄₂H₄₈ClFN₉O₅⁺ [M+H]⁺: 812.35, found, 812.4.

### Example 106 of compound of Formula (I): Preparation of compound GT-09765

With reference to the method of Scheme I-5, the target compound (GT-09765) was obtained (white solid, 25 mg, yield 40.58%). ¹H NMR (400 MHz, DMSO-D6) δ 11.07 (s, 1H), 8.82 (d, J = 2.0 Hz, 1H), 8.68 (d, J = 2.0 Hz, 1H), 8.58 (s, 2H), 8.51 (d, J = 3.9 Hz, 1H), 8.40 (d, J = 7.4 Hz, 1H), 8.14 (s, 1H), 8.06 (s, 1H), 7.63 (d, J = 8.3 Hz, 1H), 7.21 (s, 1H), 7.07 (s, 1H), 6.90 (d, J = 3.9 Hz, 1H), 5.05 (dd, J = 12.8, 5.3 Hz, 1H), 3.78 (s, 2H), 3.41 (s, 4H), 3.36 - 3.34 (m, 3H), 2.86 (d, J = 11.9 Hz, 2H), 2.64 - 2.53 (m, 3H), 2.06 - 1.96 (m, 1H), 1.93 (s, 2H), 1.76 (d, J = 9.6 Hz, 2H), 1.46 (dd, J = 19.8, 10.2 Hz, 4H), 1.29 (d, J = 6.3 Hz, 6H). LCMS (ESI) C₄₁H₄₄N₁₁O₆⁺ [M+H]⁺: calcd. for 786.35, found, 786.4.

### Example 107 of compound of Formula (I): Preparation of compound GT-10024

With reference to the method of Scheme I-5, the target compound (GT-10024) was obtained (white solid, 28 mg, yield 51.84%). ¹H NMR (400 MHz, DMSO-D6) δ 11.10 (s, 1H), 8.82 (d, J = 2.0 Hz, 1H), 8.68 (d, J = 2.0 Hz, 1H), 8.64 (s, 1H), 8.59 (s, 1H), 8.51 (d, J = 3.9 Hz, 1H), 8.42 (d, J = 7.6 Hz, 1H), 8.17 (s, 1H), 8.04 (s, 1H), 7.58 (d, J = 8.2 Hz, 1H), 7.54 - 7.46 (m, 1H), 6.91 (d, J = 3.9 Hz, 1H), 5.06 (dd, J = 12.7, 5.4 Hz, 1H), 3.78 (dd, J = 12.4, 7.0 Hz, 3H), 3.45 (s, 3H), 2.96 - 2.74 (m, 4H), 2.59 (d, J = 20.3 Hz, 4H), 2.07 - 1.97 (m, 1H), 1.93 (s, 2H), 1.76 (d, J = 9.4 Hz, 2H), 1.54 - 1.41 (m, 4H), 1.30 (d, J = 6.3 Hz, 6H). LCMS (ESI) calcd. for C₄₁H₄₃FN₁₁O₆⁺ [M+H]⁺: 804.34, found, 804.4.

### Example 108 of compound of Formula (I): Preparation of compound GT-09766

With reference to the method of Scheme I-5, the target compound (GT-09766) was obtained (white solid, 27 mg, yield 49.99%). ¹H NMR (400 MHz, DMSO-D6) δ 11.09 (s, 1H), 8.81 (d, J = 2.0 Hz, 1H), 8.67 (d, J = 2.0 Hz, 1H), 8.60 (s, 1H), 8.58 (s, 1H), 8.51 (d, J = 3.9 Hz, 1H), 8.40 (d, J = 7.6 Hz, 1H), 8.17 (s, 1H), 8.05 (s, 1H), 7.63 (d, J = 10.3 Hz, 1H), 7.55 (d, J = 7.0 Hz, 1H), 6.90 (d, J = 3.9 Hz, 1H), 5.07 (dd, J = 12.8, 5.4 Hz, 1H), 3.98 - 3.66 (m, 3H), 3.41 (s, 3H), 3.36 - 3.34 (m, 2H), 2.96 - 2.79 (m, 3H), 2.64 - 2.52 (m, 3H), 2.08 - 1.98 (m, 1H), 1.93 (s, 2H), 1.76 (d, J = 9.7 Hz, 2H), 1.46 (dd, J = 19.4, 10.3 Hz, 4H), 1.29 (d, J = 6.3 Hz, 6H). LCMS (ESI) calcd. for C₄₁H₄₃FN₁₁O₆⁺ [M+H]⁺: 804.34, found, 804.3.

### Example 109 of compound of Formula (I): Preparation of compound GT-10054

With reference to the method of Scheme I-5, the target compound (GT-10054) was obtained (white solid, 18 mg, yield 33.33%). ¹H NMR (400 MHz, DMSO-D6) δ 11.09 (s, 1H), 8.82 (d, J = 2.0 Hz, 1H), 8.69 (d, J = 2.0 Hz, 2H), 8.59 (s, 1H), 8.56 (s, 1H), 8.51 (d, J = 3.9 Hz, 1H), 8.44 (d, J = 7.5 Hz, 1H), 8.03 (s, 1H), 7.02 (s, 1H), 6.91 (d, J = 3.9 Hz, 1H), 6.81 (s, 1H), 5.05 (dd, J = 12.7, 5.4 Hz, 1H), 4.40 (s, 1H), 3.92 (s, 1H), 3.87 - 3.62 (m, 4H), 3.40 - 3.22 (m, 1H), 3.12 - 2.76 (m, 4H), 2.58 (d, J = 20.7 Hz, 3H), 2.06 - 1.97 (m, 1H), 1.93 (s, 2H), 1.75 (s, 2H), 1.58 - 1.40 (m, 4H), 1.30 (d, J = 6.3 Hz, 6H). LCMS (ESI) calcd. for C₄₁H₄₃FN₁₁O₆⁺ [M+H]⁺: 804.34, found, 804.3.

### Example 110 of compound of Formula (I): Preparation of compound GT-09767

With reference to the method of Scheme I-5, the target compound (GT-09767) was obtained (white solid, 26 mg, yield 50.13%). ¹H NMR (400 MHz, DMSO-D6) δ 10.93 (s, 1H), 8.81 (d, J = 2.0 Hz, 1H), 8.68 (d, J = 2.0 Hz, 1H), 8.61 (s, 1H), 8.57 (s, 1H), 8.51 (d, J = 3.9 Hz, 1H), 8.41 (d, J = 7.5 Hz, 1H), 8.04 (s, 1H), 7.45 (d, J = 8.4 Hz, 1H), 6.95 (s, 1H), 6.90 (d, J = 3.9 Hz, 1H), 6.86 (d, J = 8.2 Hz, 1H), 5.02 (dd, J = 13.2, 5.1 Hz, 1H), 4.24 (dd, J = 54.9, 17.0 Hz, 2H), 3.77 (dd, J = 17.9, 11.8 Hz, 3H), 3.43 (s, 3H), 3.35 - 3.31 (m, 2H), 2.95 - 2.82 (m, 2H), 2.73 (s, 1H), 2.58 (d, J = 16.3 Hz, 3H), 2.34 (dd, J = 12.5, 4.2 Hz, 1H), 1.99 - 1.87 (m, 3H), 1.75 (d, J = 9.7 Hz, 2H), 1.45 (q, J = 9.9 Hz, 4H), 1.29 (d, J = 6.3 Hz, 6H). LCMS (ESI) calcd. for C₄₁H₄₆N₁₁O₅⁺ [M+H]⁺: 772.37, found, 772.4.

### Example 111 of compound of Formula (I): Preparation of compound GT-09768

With reference to the method of Scheme I-5, the target compound (GT-09768) was obtained (white solid, 8 mg, yield 15.07%). ¹H NMR (400 MHz, DMSO-D6) δ 10.96 (s, 1H), 8.81 (d, J = 2.0 Hz, 1H), 8.67 (d, J = 2.0 Hz, 1H), 8.57 (s, 2H), 8.51 (d, J = 3.9 Hz, 1H), 8.39 (d, J = 7.5 Hz, 1H), 8.06 (s, 1H), 7.42 (d, J = 8.2 Hz, 2H), 7.34 (t, J = 7.7 Hz, 1H), 6.89 (d, J = 3.9 Hz, 1H), 5.04 (dd, J = 13.2, 5.1 Hz, 1H), 4.36 (dd, J = 71.6, 16.8 Hz, 2H), 3.85 - 3.69 (m, 3H), 3.41 (s, 4H), 3.34 - 3.32 (m, 2H), 2.73 (s, 2H), 2.59 (d, J = 17.3 Hz, 2H), 2.39 (dd, J = 13.5, 4.7 Hz, 1H), 2.01 - 1.90 (m, 3H), 1.74 (s, 2H), 1.46 (dd, J = 19.3, 9.9 Hz, 4H), 1.29 (d, J = 6.3 Hz, 6H). LCMS (ESI) calcd. for C₄₁H₄₅FN₁₁O₅⁺ [M+H]⁺: 790.36, found, 790.3.

### Example 112 of compound of Formula (I): Preparation of compound GT-09769

With reference to the method of Scheme I-5, the target compound (GT-09769) was obtained (white solid, 38 mg, yield 71.60%). ¹H NMR (400 MHz, DMSO-D6) δ 10.95 (s, 1H), 8.81 (d, J = 2.0 Hz, 1H), 8.67 (d, J = 2.0 Hz, 1H), 8.60 (s, 1H), 8.57 (s, 1H), 8.51 (d, J = 3.9 Hz, 1H), 8.40 (d, J = 7.5 Hz, 1H), 8.04 (s, 1H), 7.43 (s, 1H), 7.33 (dd, J = 12.4, 9.0 Hz, 2H), 6.90 (d, J = 3.9 Hz, 1H), 5.03 (dd, J = 13.2, 5.0 Hz, 1H), 4.27 (dd, J = 53.8, 17.1 Hz, 2H), 3.83 - 3.67 (m, 3H), 3.43 (s, 3H), 2.95 - 2.69 (m, 5H), 2.59 (d, J = 17.1 Hz, 2H), 2.39 - 2.30 (m, 1H), 1.94 (dd, J = 21.5, 6.9 Hz, 3H), 1.75 (d, J = 10.4 Hz, 2H), 1.52 - 1.39 (m, 4H), 1.29 (d, J = 6.3 Hz, 6H). LCMS (ESI) calcd. for C₄₁H₄₅FN₁₁O₅⁺ [M+H]⁺: 790.36, found, 790.4.

### Example 113 of compound of Formula (I): Preparation of compound GT-09770

With reference to the method of Scheme I-5, the target compound (GT-09770) was obtained (white solid, 38 mg, yield 71.60%). ¹H NMR (400 MHz, DMSO-D6) δ 10.94 (s, 1H), 8.81 (d, J = 2.0 Hz, 1H), 8.68 (d, J = 2.0 Hz, 2H), 8.58 (s, 1H), 8.50 (d, J = 3.9 Hz, 1H), 8.43 (d, J = 7.6 Hz, 1H), 8.02 (s, 1H), 7.72 (s, 1H), 6.91 (d, J = 3.9 Hz, 1H), 6.71 (s, 1H), 6.59 (d, J = 11.9 Hz, 1H), 4.98 (dd, J = 13.2, 5.1 Hz, 1H), 4.33 - 4.14 (m, 2H), 3.76 (d, J = 28.5 Hz, 3H), 3.46 (s, 3H), 3.38 - 3.35 (m, 2H), 2.91 - 2.80 (m, 2H), 2.58 (d, J = 17.1 Hz, 3H), 2.31 (dd, J = 12.6, 4.1 Hz, 1H), 1.96 - 1.87 (m, 3H), 1.74 (s, 2H), 1.45 (dd, J = 18.4, 9.3 Hz, 4H), 1.29 (d, J = 6.3 Hz, 6H). LCMS (ESI) calcd. for C₄₁H₄₅FN₁₁O₅⁺ [M+H]⁺: 790.36, found, 790.4.

### Example 114 of compound of Formula (I): Preparation of compound GT-09771

With reference to the method of Scheme I-5, the target compound (GT-09771) was obtained (white solid, 35 mg, yield 67.49%). ¹H NMR (400 MHz, DMSO-D6) δ 10.98 (s, 1H), 8.82 (d, J = 2.0 Hz, 1H), 8.69 (d, J = 2.0 Hz, 2H), 8.59 (d, J = 3.1 Hz, 1H), 8.51 (d, J = 3.9 Hz, 1H), 8.43 (d, J = 7.6 Hz, 1H), 8.04 (s, 1H), 7.29 (t, J = 7.7 Hz, 1H), 7.06 (dd, J = 7.7, 3.1 Hz, 2H), 6.91 (d, J = 3.9 Hz, 1H), 5.10 (dd, J = 13.3, 5.1 Hz, 1H), 4.32 (dd, J = 40.7, 17.5 Hz, 2H), 3.86 - 3.57 (m, 5H), 3.41 - 3.31 (m, 3H), 2.98 - 2.87 (m, 1H), 2.77 (d, J = 31.7 Hz, 2H), 2.61 (d, J = 16.8 Hz, 2H), 2.44 - 2.36 (m, 1H), 2.06 - 1.98 (m, 1H), 1.92 (d, J = 7.7 Hz, 2H), 1.76 (d, J = 10.5 Hz, 2H), 1.46 (dd, J = 19.9, 10.1 Hz, 4H), 1.29 (d, J = 6.3 Hz, 6H). LCMS (ESI) calcd. for C₄₁H₄₆N₁₁O₅⁺ [M+H]⁺: 772.37, found, 772.37.

### Example 115 of compound of Formula (I): Preparation of compound GT-09772

With reference to the method of Scheme I-5, the target compound (GT-09772) was obtained (white solid, 36 mg, yield 67.15%). ¹H NMR (400 MHz, DMSO-D6) δ 10.93 (s, 1H), 8.82 (d, J = 1.9 Hz, 1H), 8.70 (t, J = 7.4 Hz, 2H), 8.60 (s, 1H), 8.51 (d, J = 3.9 Hz, 1H), 8.46 (d, J = 7.5 Hz, 1H), 8.01 (s, 1H), 7.44 (d, J = 8.4 Hz, 1H), 6.88 (dd, J = 27.2, 6.3 Hz, 2H), 5.02 (dd, J = 13.3, 4.8 Hz, 1H), 4.24 (ddd, J = 22.2, 16.6, 6.7 Hz, 2H), 3.76 (s, 4H), 3.39 - 3.31 (m, 3H), 3.01 - 2.71 (m, 5H), 2.60 (s, 2H), 2.40 (dd, J = 37.1, 11.7 Hz, 2H), 1.99 - 1.77 (m, 5H), 1.47 (dd, J = 25.6, 12.2 Hz, 4H), 1.30 (d, J = 6.3 Hz, 6H). LCMS (ESI) calcd. for C₄₃H₄₈N₁₁O₅⁺ [M+H]⁺: 798.38, found, 798.4.

### Example 116 of compound of Formula (I): Preparation of compound GT-09773

With reference to the method of Scheme I-5, the target compound (GT-09773) was obtained (white solid, 39 mg, yield 72.75%). ¹H NMR (400 MHz, DMSO-D6) δ 10.94 (s, 1H), 8.82 (d, J = 2.0 Hz, 1H), 8.69 (d, J = 2.0 Hz, 2H), 8.60 (s, 1H), 8.51 (d, J = 3.9 Hz, 1H), 8.46 (d, J = 7.4 Hz, 1H), 8.02 (s, 1H), 7.46 (d, J = 8.3 Hz, 1H), 6.92 (d, J = 3.9 Hz, 2H), 6.83 (d, J = 7.5 Hz, 1H), 5.03 (dd, J = 13.3, 5.1 Hz, 1H), 4.60 - 4.40 (m, 2H), 4.25 (dd, J = 56.1, 16.9 Hz, 2H), 3.77 (d, J = 30.5 Hz, 3H), 3.02 - 2.83 (m, 3H), 2.63 - 2.56 (m, 3H), 2.34 (dd, J = 13.1, 4.6 Hz, 1H), 2.00 (dd, J = 46.7, 10.2 Hz, 6H), 1.78 (dd, J = 35.0, 10.5 Hz, 3H), 1.59 - 1.40 (m, 4H), 1.30 (d, J = 6.3 Hz, 6H). LCMS (ESI) calcd. for C₄₃H₄₈N₁₁O₅⁺ [M+H]⁺: 798.38, found, 798.4.

### Example 117 of compound of Formula (I): Preparation of compound GT-09774

With reference to the method of Scheme I-5, the target compound (GT-09774) was obtained (white solid, 24 mg, yield 44.00%). ¹H NMR (400 MHz, DMSO-D6) δ 10.93 (s, 1H), 8.80 (d, J = 1.9 Hz, 1H), 8.66 (d, J = 2.0 Hz, 1H), 8.55 (s, 2H), 8.49 (d, J = 3.9 Hz, 1H), 8.38 (d, J = 7.7 Hz, 1H), 8.03 (s, 1H), 7.43 (d, J = 8.4 Hz, 1H), 6.90 (d, J = 3.9 Hz, 2H), 6.83 (d, J = 8.4 Hz, 1H), 5.00 (dd, J = 13.2, 5.0 Hz, 1H), 4.23 (dd, J = 55.8, 16.9 Hz, 2H), 3.90 (s, 2H), 3.74 (d, J = 26.5 Hz, 6H), 3.40 - 3.35 (m, 3H), 2.93 - 2.83 (m, 1H), 2.58 (d, J = 16.7 Hz, 2H), 2.39 - 2.29 (m, 1H), 2.18 (s, 1H), 1.99 - 1.87 (m, 3H), 1.78 (d, J = 34.3 Hz, 4H), 1.45 - 1.35 (m, 4H), 1.28 (d, J = 6.3 Hz, 6H). LCMS (ESI) calcd. for C₄₄H₅₀N₁₁O₅⁺ [M+H]⁺: 812.40, found, 812.4.

### Example 118 of compound of Formula (I): Preparation of compound GT-09775

With reference to the method of Scheme I-5, the target compound (GT-09775) was obtained (white solid, 33 mg, yield 62.50%). ¹H NMR (400 MHz, DMSO-D6) δ 10.94 (s, 1H), 8.82 (s, 2H), 8.70 (s, 1H), 8.62 (d, J = 6.8 Hz, 1H), 8.55 - 8.44 (m, 2H), 7.99 (d, J = 2.5 Hz, 1H), 7.45 (d, J = 8.3 Hz, 1H), 6.93 (d, J = 3.8 Hz, 3H), 5.03 (dd, J = 13.3, 5.0 Hz, 1H), 4.24 (d, J = 37.8 Hz, 2H), 3.77 (d, J = 52.8 Hz, 6H), 3.51 - 3.34 (m, 3H), 2.90 (s, 3H), 2.64 - 2.54 (m, 2H), 2.35 (dt, J = 21.8, 11.0 Hz, 1H), 2.07 - 1.84 (m, 5H), 1.78 (s, 1H), 1.50 (dd, J = 18.5, 10.6 Hz, 4H), 1.30 (d, J = 6.2 Hz, 6H). LCMS (ESI) calcd. for C₄₂H₄₈N₁₁O₅⁺ [M+H]⁺: 786.38, found, 786.4.

### Example 119 of compound of Formula (I): Preparation of compound GT-09776

With reference to the method of Scheme I-5, the target compound (GT-09776) was obtained (white solid, 28 mg, yield 53.16%). ¹H NMR (400 MHz, DMSO-D6) δ 10.93 (s, 1H), 8.81 (d, J = 2.0 Hz, 1H), 8.68 (d, J = 2.0 Hz, 1H), 8.61 (s, 1H), 8.57 (s, 1H), 8.51 (d, J = 3.9 Hz, 1H), 8.38 (d, J = 7.3 Hz, 1H), 8.05 (s, 1H), 7.56 (s, 1H), 7.44 (d, J = 8.3 Hz, 1H), 6.90 (d, J = 3.9 Hz, 2H), 6.82 (s, 1H), 5.02 (dd, J = 13.2, 5.0 Hz, 1H), 4.53 (s, 1H), 4.24 (dd, J = 54.2, 17.0 Hz, 3H), 3.79 (dd, J = 12.6, 6.4 Hz, 2H), 3.33 - 3.09 (m, 3H), 3.01 - 2.84 (m, 2H), 2.58 (d, J = 17.3 Hz, 2H), 2.35 (d, J = 15.8 Hz, 2H), 2.19 (s, 1H), 1.95 (d, J = 10.8 Hz, 3H), 1.84 (s, 2H), 1.44 (dd, J = 23.2, 12.4 Hz, 4H), 1.30 (d, J = 6.3 Hz, 6H). LCMS (ESI) calcd. for C₄₂H₄₆N₁₁O₅⁺ [M+H]⁺: 784.37, found, 784.4.

### Example 120 of compound of Formula (I): Preparation of compound GT-09777

With reference to the method of Scheme I-5, the target compound (GT-09777) was obtained (white solid, 30 mg, yield 56.96%). ¹H NMR (400 MHz, DMSO-D6) δ 10.93 (s, 1H), 8.82 (d, J = 1.7 Hz, 1H), 8.68 (d, J = 1.9 Hz, 1H), 8.63 (s, 1H), 8.59 (d, J = 1.3 Hz, 1H), 8.53 - 8.50 (m, 1H), 8.45 - 8.37 (m, 1H), 8.05 (s, 1H), 7.72 (s, 1H), 7.45 (dd, J = 18.1, 10.4 Hz, 1H), 6.99 (s, 1H), 6.89 (dd, J = 13.5, 6.2 Hz, 2H), 5.03 (dd, J = 13.3, 4.9 Hz, 1H), 4.61 (s, 1H), 4.23 (dd, J = 54.2, 17.0 Hz, 2H), 3.79 (d, J = 6.3 Hz, 3H), 3.60 (s, 2H), 3.23 (d, J = 10.1 Hz, 1H), 2.95 - 2.84 (m, 1H), 2.58 (d, J = 17.5 Hz, 2H), 2.34 (dt, J = 21.8, 11.0 Hz, 2H), 1.95 (d, J = 12.3 Hz, 5H), 1.79 (s, 2H), 1.44 (d, J = 22.6 Hz, 4H), 1.30 (d, J = 6.1 Hz, 6H). LCMS (ESI) calcd. for C₄₂H₄₆N₁₁O₅⁺ [M+H]⁺: 784.37, found, 784.4.

### Biological activity assay

### Reagents and Materials

| Reagents and materials | Suppliers or Source |
|---|---|
| Human myeloid monocytic leukemia cell line: MV-4-11 | ATCC (American Type Culture Collection) |
| Human T lymphocytic leukemia cell line: Jurkat | ATCC |
| Human acute lymphoblastic leukemia T lymphocyte: CCRF-CEM | Dalian Meilun Biotech Co., Ltd. |
| Human diffuse large B-cell lymphoma cell line: TMD8 | Kyinno Biotechnology Co., Ltd |
| Human prostate cancer cell line: 22RV1 | ATCC |
| Human breast cancer cell line: MCF-7 | ATCC |
| Human multiple myeloma cell line: MM.1S | ATCC |
| Human breast cancer cell line: MDA-MB-231 | ATCC |
| Human malignant meningioma cell line: JEKO-1 | ATCC |
| Human B lymphoblastoma cell line: RPMI-8226 | ATCC |
| Human acute T lymphoblastic leukemia cell line: Molt-4 | ATCC |
| Human small cell lung cancer cell line: H196 | ATCC |
| Human non-small cell lung cancer cell line: NCI-H1975 | ATCC |
| RPMI-1640 Medium | ATCC |
| IMDM Medium | Gibco company |
| EMEM Medium | ATCC |
| CellTiter-Meiluncell Luminescent Cell Viability Assay Kit | Dalian Meilun Biotech Co., Ltd. |
| Fetal bovine serum (FBS) | Sunrise Science Products company |
| Penicillin-Streptomycin | Beijing TransGen Biotech Co., Ltd. |
| Mycoplasma detection kit | Beijing TransGen Biotech Co., Ltd. |
| STR genotype detection | Shanghai Biowing Applied Biotechnology Co., Ltd. |

### Methods:

### Cell cultures

The tumor cells used in the present disclosure were cultured in the cell culture media listed in Table A at 37 °C in an incubator containing 5% CO₂. All cells were authenticated as the correct cell lines by STR profiling and confirmed to be mycoplasma-negative using a mycoplasma detection kit prior to the experiments.

**Table A. Tumor cells and cell culture medium used in the present disclosure**

| Cell line | Cell culture medium used |
|---|---|
| Jurkat | RPMI 1640 culture medium supplemented with 10% FBS, as well as penicillin at a final concentration of 100 U/mL and streptomycin at a final concentration of 100 µg/mL |
| CCRF-CEM | |
| TMD8 | |
| 22RV1 | |
| RPMI-8226 | |
| Molt-4 | |
| MM.1S | |
| H196 | |
| H1975 | |
| JEKO-1 | |
| MV-4-11 | IMDM culture medium supplemented with 10% FBS, as well as penicillin at a final concentration of 100 U/mL and streptomycin at a final concentration of 100 µg/mL |
| MCF-7 | EMEM culture medium supplemented with 10% FBS, 10 µg/mL insulin, and penicillin at a final concentration of 100 U/mL and streptomycin at a final concentration of 100 µg/mL |

### I. Determination of half inhibitory concentration (IC₅₀) of the compounds against tumor cells:

The IC₅₀ values of the compounds of the present disclosure (including the compounds in Table 1, Table 2, and Examples) were determined using the CellTiter-Meiluncell Luminescent Cell Viability Assay Kit from Dalian Meilun Biotech Co., Ltd. The detailed procedures are as follows: Tumor cells were seeded in 96-well cell culture plates at the densities specified in Table B. On the following day, the test compounds of the present disclosure were subjected to serial dilution. The dilution can be performed according to one of the following two methods:
(1) The first dilution method: the test compounds of the present disclosure (including the compounds in Tables 1 and 2, and Examples) were subjected to 5-fold serial dilutions starting from a highest concentration of 2000 µM, resulting in a total of 9 concentrations from high to low (2000, 400, 80, 16, 3.2, 0.64, 0.128, 0.0256, 0.00512 µM). Subsequently, 0.5 µL of the test compound dilution was transferred into the 96-well plate containing the cells, with each well containing 100 µL of cell culture medium. This resulted in final test compound concentrations of 10, 2, 0.4, 0.08, 0.016, 0.0032, 0.00064, 0.000128, and 0.0000256 µM, respectively, or
(2) As an alternative, a second dilution method can be used: the test compounds of the present disclosure (including the compounds in Tables 1 and 2, and the example compounds) were subjected to a 50-fold gradient dilution starting from the highest concentration of 100 µM, with a total of 2 concentrations (100 µM and 2 µM) being set. In a 96-well plate, the diluted test compounds of the present disclosure and the corresponding detection reagents were added to each well to ensure that the final test concentrations of the test compound in each well reached 500 nM and 10 nM, respectively, so as to evaluate the inhibitory activity of the test compound on tumor cell proliferation at these two concentration levels. The experimental procedure is described below using the diluted test compound at 2 µM as an example (the operating steps for the 100 µM concentration and the gradient concentrations obtained by the first dilution method are similar thereto).

0.5 µL of the above diluted test compounds of the present disclosure (including the compounds in Tables 1 and 2, and the example compounds) were added to 100 µL of seeded cells, resulting in a final compound concentration of 10 nM. After the cells were treated with the compounds of the present disclosure for a certain period of time, a cell viability detection reagent was added to the culture medium according to the manufacturer's instructions of the kit to determine cell viability. The negative control was DMSO, and the reference compounds were the corresponding commercially available inhibitors (including PF06650833 (Zimlovisertib); Enzalutamide (CAS No. 915087-33-1); Ribociclib (CAS No. 1211443-58-1); and Erlotinib (CAS No. 183321-74-6)), all of which were used to treat the cells in the same manner as the compounds of the present disclosure. The growth inhibition of the cells by the compounds of the present disclosure was plotted using Prism GraphPad software, from which the IC₅₀ values of the compounds of the present disclosure were calculated.

**Table B. Seeding protocol and treatment time for tumor cells**

| Cells | Cells seeding protocol | The times for treating cells with the compounds of the present disclosure (hours) |
|---|---|---|
| Jurkat | cells were seeded at a density of 10,000 cells per well in 100 µL of serum-containing RPMI-1640 medium per well | 96 hours |
| RPMI-8226 | | |
| Molt-4 | | |
| TMD8 | | |
| MM.1S | | |
| CCRF-CEM | cells were seeded at a density of 4,000 cells per well in 100 µL of serum-containing RPMI-1640 medium per well | 96 hours |
| H1975 | | |
| 22RV1 | | |
| JEKO-1 | | |
| H196 | cells were seeded at a density of 1,000 cells per | 96 hours |
| | well in 100 µL of serum-containing RPMI-1640 medium per well | |
| MV-4-11 | cells were seeded at a density of 10,000 cells per well in 100 µL of serum-containing IMDM medium per well | 96 hours |
| MCF-7 | cells were seeded at a density of 4,000 cells per well in 100 µL of serum-containing EMEM medium per well | 96 hours |

The results of tumor cell proliferation inhibition are presented in Tables I-1, I-2, I-3, I-4, I-5, I-6, and I-7.

**Table I-1: Inhibitory activity (IC₅₀, nM) of the compounds of the present disclosure, designed based on the CDK4/6 inhibitors, against tumor cell proliferation**

| Comp. No./names | 22RV1 | MCF-7 | MM.1S | MDA-MB-231 | MV-4-11 |
|---|---|---|---|---|---|
| Ribociclib | E | F | F | G | F |
| GT-07089 | C | - | - | - | - |
| GT-07095 | A | C | B | B | - |
| GT-07096 | A+ | - | C | - | - |
| GT-07097 | A++ | - | - | - | - |
| GT-07241 | - | - | C | - | - |
| GT-07194 | - | - | C | - | - |
| GT-06948 | A+ | - | - | - | B |
| GT-05207 | - | A | - | - | - |
| GT-07099 | - | - | B | - | - |

| | | | | | |
|---|---|---|---|---|---|
| Note: in table I-1, the symbols A+++, A++, A+, A, B, C, D, E, F, and G are defined as follows: 0 < A+++ ≤ 10 nM; 10 nM < A++ ≤ 50 nM; 50 nM < A+ ≤ 100 nM; 100 nM < A ≤ 200 nM; 200 nM < B ≤ 400 nM; 400 nM < C ≤ 800 nM; 800 nM < D ≤ 1000 nM; 1000 nM < E ≤ 5000 nM; 5000 nM < F ≤ 10000 nM; 10000 nM < G. The symbol "-" indicates "not tested". | | | | | |

**Table I-2: Inhibitory activity (IC₅₀, nM) of the compounds of the present disclosure, designed based on the CDK4/6 inhibitors, against tumor cell proliferation**

| Comp. No. /names | MCF-7 | | MV-4-11 | | JEKO-1 | | RPMI-8226 | | Molt-4 | |
|---|---|---|---|---|---|---|---|---|---|---|
| | Inhibition Rate (%) at 500 nM | Inhibition Rate (%) at 10 nM | Inhibition Rate (%) at 500 nM | Inhibition Rate (%) at 10 nM | Inhibition Rate (%) at 500 nM | Inhibition Rate (%) at 10 nM | Inhibition Rate (%) at 500 nM | Inhibition Rate (%) at 10 nM | Inhibition Rate (%) at 500 nM | Inhibition Rate (%) at 10 nM |
| Ribocic lib | 1a5 | 1a5 | 1a5 | 1a1 | 1a5 | 1a4 | 1a2 | 1a4 | 1a1 | 1a2 |
| GT-10020 | - | - | - | - | 1a7 | 1a5 | - | - | - | - |
| GT-10032 | 1a7 | 1a6 | 1a8 | 1a5 | 1a9 | 1a7 | 1a7 | 1a4 | - | - |
| GT-10033 | 1a8 | 1a7 | 1a8 | 1a5 | 1a8 | 1a7 | - | - | - | - |
| GT-10034 | 1a8 | 1a8 | 1a11 | 1a10 | 1a8 | 1a8 | 1a9 | 1a7 | - | - |
| GT-10035 | 1a8 | 1a7 | 1a11 | 1a10 | 1a8 | 1a8 | 1a8 | 1a7 | - | - |
| GT-10036 | 1a7 | 1a7 | 1a10 | 1a8 | 1a8 | 1a7 | 1a7 | 1a5 | - | - |
| GT-10037 | 1a7 | 1a6 | 1a10 | 1a8 | 1a8 | 1a5 | - | - | - | - |
| GT-10038 | 1a8 | 1a5 | 1a11 | 1a10 | 1a7 | 1a4 | - | - | 1a9 | 1a8 |

| | | | | | | | | | | |
|---|---|---|---|---|---|---|---|---|---|---|
| Note: in table I-2, the symbols 1a1, 1a2, 1a3, 1a4, 1a5, 1a6, 1a7, 1a8, 1a9, 1a10, and 1a11 are defined as follows: -10% < 1a1 < 0, 0 < 1a2 < 5%, 5% ≤ 1a3 < 10%, 10% ≤ 1a4 < 20%, 20% ≤ 1a5 < 35%, 35% ≤ 1a6 ≤ 40%, 40% < 1a7 < 50%, 50% ≤ 1a8 < 60%, 60% ≤ 1a9 < 70%, 70% ≤ 1a10 < 80%, 80% ≤ 1a11 ≤ 100%. The symbol "-" indicates "not tested". | | | | | | | | | | |

**Table I-3: Inhibitory activity (IC₅₀, nM) of the compounds of the present disclosure, designed based on the AR inhibitors, against tumor cell proliferation**

| Comp. No./names | TMD8 | RPMI-8226 | CCRF-CEM | MCF-7 | Jurkat | MV-4-11 |
|---|---|---|---|---|---|---|
| Enzalutamide | F | G | G | G | G | G |
| GT-05234 | A++ | C | A++ | B | A++ | A+ |
| GT-06950 | B | B | C | - | - | A |
| GT-07108 | B | - | C | C | - | - |
| GT-07107 | A++ | A | A | B | B | - |
| GT-07196 | A++ | B | C | B | C | - |
| GT-07195 | A | B | - | - | - | - |
| GT-07100 | A+ | B | B | - | C | - |
| GT-07090 | A | C | - | - | - | - |
| GT-07179 | B | B | B | B | C | - |
| GT-07112 | B | - | - | - | - | - |
| GT-07101 | B | A | - | C | - | - |
| GT-07102 | B | C | - | C | - | - |
| GT-07110 | B | - | - | - | - | - |
| GT-07181 | C | - | - | - | - | - |
| GT-06949 | A+ | C | A | C | - | - |
| GT-07098 | A | C | - | - | - | - |
| GT-07180 | B | C | - | - | - | - |

| | | | | | | |
|---|---|---|---|---|---|---|
| Note: in table I-3, the symbols A+++, A++, A+, A, B, C, D, E, F, and G are defined as follows: 0 < A+++ ≤ 10 nM; 10 nM < A++ ≤ 50 nM; 50 nM < A+ ≤ 100 nM; 100 nM < A ≤ 200 nM; 200 nM < B ≤ 400 nM; 400 nM < C ≤ 800 nM; 800 nM < D ≤ 1000 nM; 1000 nM < E ≤ 5000 nM; 5000 nM < F ≤ 10000 nM; 10000 nM < G. The symbol "-" indicates "not tested". | | | | | | |

**Table I-4: Inhibitory activity (IC₅₀, nM) of the compounds of the present disclosure, designed based on the AR inhibitors, against tumor cell proliferation**

| Comp. No. /names | MV-4-11 | | TMD8 | | Molt-4 | | RPMI-8226 | | MCF-7 | |
|---|---|---|---|---|---|---|---|---|---|---|
| | Inhibiti on Rate (%) at 500 nM | Inhibiti on Rate (%) at 10 nM | Inhibiti on Rate (%) at 500 nM | Inhibiti on Rate (%) at 10 nM | Inhibiti on Rate (%) at 500 nM | Inhibiti on Rate at 10 nM (%) | Inhibiti on Rate (%) at 500 nM | Inhibiti on Rate (%) at 10 nM | Inhibiti on Rate (%) at 500 nM | Inhibiti on Rate (%) at 10 nM |
| Enzalutam ide | 1b5 | 1b3 | 1b5 | 1b5 | 1b4 | 1b4 | 1b5 | 1b4 | 1b2 | 1b2 |
| GT-10039 | - | - | 1b11 | 1b10 | 1b9 | 1b5 | 1b9 | 1b7 | 1b9 | 1b7 |
| GT-10041 | - | - | - | - | - | - | - | - | 1b7 | 1b6 |
| GT-10042 | 1b11 | 1b11 | 1b7 | 1b5 | - | - | - | - | - | - |
| GT-10043 | 1b11 | 1b8 | - | - | - | - | - | - | - | - |
| GT-10044 | 1b11 | 1b11 | 1b8 | 1b6 | 1b10 | 1b5 | - | - | 1b7 | 1b4 |
| GT-10045 | 1b11 | 1b10 | 1b10 | 1b6 | 1b7 | 1b3 | - | - | - | - |
| GT-10023 | - | - | 1b10 | 1b6 | 1b7 | 1b3 | 1b8 | 1b5 | - | - |
| GT-10021 | 1b11 | 1b8 | 1b11 | 1b9 | 1b10 | 1b5 | 1b10 | 1b7 | 1b7 | 1b4 |
| GT-08179 | 1b10 | 1b5 | 1b7 | 1b4 | - | - | - | - | - | - |
| GT-08189 | 1b9 | 1b1 | 1b8 | 1b5 | - | - | - | - | 1b7 | 1b2 |

| | | | | | | | | | | |
|---|---|---|---|---|---|---|---|---|---|---|
| Note: in table I-4, the symbols 1b1, 1b2, 1b3, 1b4, 1b5, 1b6, 1b7, 1b8, 1b9, 1b10, and 1b11 are defined as follows: -20% < 1b1 < 0, 0 < 1b2 < 5%, 5% ≤ 1b3 < 10%, 10% ≤ 1b4 < 20%, 20% ≤ 1b5 < 30%, 30% ≤ 1b6 ≤ 40%, 40% < 1b7 < 50%, 50% ≤ 1b8 < 60%, 60% ≤ 1b9 < 70%, 70% ≤ 1b10 < 80%, 80% ≤ 1b11 ≤ 100%. The symbol "-" indicates "not tested". | | | | | | | | | | |

**Table I-5: Inhibitory activity (IC₅₀, nM) of the compounds of the present disclosure, designed based on interleukin-1 receptor-associated kinase (IRAK) inhibitors (IRAK1, IRAK2, IRAK-3, and IRAK4), against tumor cell proliferation**

| Comp. No. /names | MV-4-11 | Comp. No. /names | MV-4-11 |
|---|---|---|---|
| PF06650833 | E | GT-06945 | A+++ |
| GT-06943 | A++ | GT-06941 | A+++ |
| GT-06946 | A++ | GT-06944 | A+ |
| GT-06947 | A++ | GT-05300 | B |
| GT-06951 | A+ | | |

| | | | |
|---|---|---|---|
| Note: in table I-5, the symbols A+++, A++, A+, A, B, C, D, E, F, and G are defined as follows: 0 < A+++ ≤ 10 nM; 10 nM < A++ ≤ 50 nM; 50 nM < A+ ≤ 100 nM; 100 nM < A ≤ 200 nM; 200 nM < B ≤ 400 nM; 400 nM < C ≤ 800 nM; 800 nM < D ≤ 1000 nM; 1000 nM < E ≤ 5000 nM; 5000 nM < F ≤ 10000 nM; 10000 nM < G. The symbol "-" indicates "not tested". | | | |

**Table I-6: Inhibitory activity (IC₅₀, nM) of the compounds of the present disclosure, designed based on IRAK inhibitors, against tumor cell proliferation**

| Comp. No. /names | MV-4-11 | | TMD8 | | MM.1S | | H196 | |
|---|---|---|---|---|---|---|---|---|
| | Inhibitio n Rate (%) at 500 nM | Inhibitio n Rate (%) at 10 nM | Inhibitio n Rate (%) at 500 nM | Inhibitio n Rate (%) at 10 nM | Inhibitio n Rate (%) at 500 nM | Inhibitio n Rate (%) at 10 nM | Inhibitio n Rate (%) at 500 nM | Inhibitio n Rate (%) at 10 nM |
| PF06650833 | 1c6 | 1c5 | 1c3 | 1c4 | 1c5 | 1c5 | 1c2 | 1c1 |
| GT-06951 | 1c7 | 1c1 | - | - | - | - | 1c9 | 1c8 |
| GT-09549 | 1c9 | 1c6 | - | - | - | - | 1c9 | 1c8 |
| GT-09548 | 1c9 | 1c6 | - | - | - | - | - | - |
| GT-09603 | 1c7 | 1c3 | - | - | - | - | - | - |
| GT-10024 | 1c7 | 1c7 | - | - | - | - | - | - |
| GT-10022 | 1c8 | 1c6 | - | - | - | - | - | - |
| GT-10046 | 1c9 | 1c4 | - | - | - | - | - | - |
| GT-10047 | 1c11 | 1c8 | - | - | - | - | - | - |
| GT-10048 | 1c11 | 1c11 | 1c7 | 1c7 | 1c7 | 1c7 | 1c6 | 1c7 |
| GT-10049 | 1c11 | 1c11 | - | - | - | - | - | - |
| GT-10050 | 1c11 | 1c9 | - | - | - | - | - | - |
| GT-10051 | 1c11 | 1c11 | - | - | 1c8 | 1c6 | 1c8 | 1c8 |
| GT-10052 | 1c11 | 1c7 | - | - | 1c7 | 1c7 | - | - |
| GT-09767 | 1c8 | 1c7 | 1c8 | 1c8 | - | - | - | - |
| GT-09776 | - | - | 1c9 | 1c6 | 1c7 | 1c6 | - | - |
| GT-09765 | - | - | 1c8 | 1c5 | - | - | - | - |
| GT-09770 | - | - | 1c9 | 1c7 | - | - | - | - |
| GT-09769 | 1c10 | 1c9 | 1c8 | 1c8 | - | - | - | - |
| GT-09773 | 1c10 | 1c8 | 1c9 | 1c8 | - | - | - | - |
| GT-09771 | - | - | 1c8 | 1c5 | 1c8 | 1c7 | - | - |
| GT-09775 | - | - | 1c7 | 1c4 | - | - | - | - |
| GT-09766 | - | - | 1c8 | 1c5 | - | - | - | - |
| GT-07105 | 1c11 | 1c10 | - | - | 1c8 | 1c7 | - | - |
| GT-07103 | 1c9 | 1c4 | - | - | - | - | - | - |
| GT-07104 | 1c11 | 1c11 | 1c7 | 1c5 | 1c7 | 1c5 | - | - |
| GT-07126 | 1c11 | 1c8 | - | - | - | - | - | - |
| GT-07111 | - | - | - | - | 1c7 | 1c4 | - | - |
| GT-07396 | 1c10 | 1c5 | - | - | - | - | - | - |
| GT-08180 | 1c11 | 1c4 | - | - | - | - | - | - |

| | | | | | | | | |
|---|---|---|---|---|---|---|---|---|
| Note: in table I-6, the symbols 1c1, 1c2, 1c3, 1c4, 1c5, 1c6, 1c7, 1c8, 1c9, 1c10, and 1c11 are defined as follows: -10% < 1c1 < 0, 0 < 1c2 < 5%, 5% ≤ 1c3 < 10%, 10% ≤ 1c4 < 20%, 20% ≤ 1c5 < 30%, 30% ≤ 1c6 ≤ 40%, 40% < 1c7 < 50%, 50% ≤ 1c8 < 60%, 60% ≤ 1c9 < 70%, 70% ≤ 1c10 < 80%, 80% ≤ 1c11 ≤ 100%. The symbol "-" indicates "not tested". | | | | | | | | |

**Table I-7: Inhibitory activity (IC₅₀, nM) of the compounds of the present disclosure, designed based on EGFR inhibitors, against tumor cell proliferation**

| Comp. No. /names | MV-4-11 | | MM.1S | | H196 | | H1975 | |
|---|---|---|---|---|---|---|---|---|
| | Inhibition Rate (%) at 500 nM | Inhibition Rate (%) at 10 nM | Inhibition Rate (%) at 500 nM | Inhibition Rate (%) at 10 nM | Inhibition Rate (%) at 500 nM | Inhibition Rate (%) at 10 nM | Inhibition Rate (%) at 500 nM | Inhibition Rate (%) at 10 nM |
| erlotinib | 1d4 | 1d3 | 1d6 | 1d5 | 1d4 | 1d1 | 1d3 | 1d2 |
| GT-09928 | 1d11 | 1d10 | - | - | - | - | - | - |
| GT-09924 | 1d7 | 1d3 | - | - | 1d8 | 1d7 | 1d9 | 1d5 |
| GT-09930 | 1d11 | 1d10 | - | - | - | - | - | - |
| GT-09926 | 1d7 | 1d1 | - | - | - | - | 1d9 | 1d4 |
| GT-09929 | 1d10 | 1d8 | - | - | - | - | - | - |
| GT-09927 | 1d9 | 1d6 | - | - | 1d7 | 1d7 | - | - |
| GT-09921 | 1d7 | 1d5 | 1d7 | 1d6 | 1d9 | 1d9 | 1d9 | 1d5 |
| GT-10025 | 1d7 | 1d4 | - | - | 1d9 | 1d8 | - | - |
| GT-09932 | 1d11 | 1d10 | - | - | - | - | - | - |
| GT-09931 | 1d11 | 1d11 | - | - | - | - | - | - |
| GT-10154 | - | - | - | - | 1d7 | 1d7 | - | - |
| GT-09631 | 1d7 | 1d7 | - | - | - | - | - | - |
| GT-09634 | 1d8 | 1d8 | 1d9 | 1d8 | - | - | - | - |
| GT-09684 | 1d7 | 1d6 | 1d8 | 1d6 | - | - | - | - |
| GT-09632 | 1d7 | 1d5 | - | - | - | - | - | - |
| GT-09681 | - | - | 1d10 | 1d9 | 1d7 | 1d5 | - | - |
| GT-09683 | 1d7 | 1d6 | 1d7 | 1d6 | - | - | - | - |
| GT-09633 | 1d7 | 1d6 | - | - | - | - | - | - |
| GT-09682 | 1d8 | 1d6 | - | - | - | - | - | - |
| GT-09764 | - | - | 1d8 | 1d3 | - | - | 1d9 | 1d5 |
| GT-09922 | - | - | - | - | - | - | 1d8 | 1d4 |

| | | | | | | | | |
|---|---|---|---|---|---|---|---|---|
| Note: in table I-7, the symbols 1d1, 1d2, 1d3, 1d4, 1d5, 1d6, 1d7, 1d8, 1d9, 1d10, and 1d11 are defined as follows: -5% < 1d1 < 0, 0 < 1d2 < 5%, 5% ≤ 1d3 < 10%, 10% ≤ 1d4 < 20%, 20% ≤ 1d5 < 30%, 30% ≤ 1d6 ≤ 40%, 40% < 1d7 < 50%, 50% ≤ 1d8 < 60%, 60% ≤ 1d9 < 70%, 70% ≤ 1d10 < 80%, 80% ≤ 1d11 ≤ 100%. The symbol "-" indicates "not tested". | | | | | | | | |

**Table I-8: Inhibitory activity (IC₅₀, nM) of the compounds of the present disclosure against tumor cell proliferation**

| Comp. No. /names | MV-4-11 | | H196 | |
|---|---|---|---|---|
| | Inhibition Rate (%) at 500 nM | Inhibition Rate (%) at 10 nM | Inhibition Rate (%) at 500 nM | Inhibition Rate (%) at 10 nM |
| pomalidomide | 1e4 | 1e5 | 1e5 | 1e5 |
| GT-08009 | 1e8 | 1e9 | - | - |
| GT-08006 | 1e7 | 1e7 | - | - |
| GT-08195 | 1e8 | 1e8 | - | - |
| GT-09916 | - | - | 1e7 | 1e7 |

| | | | | |
|---|---|---|---|---|
| Note: in table I-8, the symbols 1e1, 1e2, 1e3, 1e4, 1e5, 1e6, 1e7, 1e8, 1e9, 1e10, and 1e11 are defined as follows: -5% < 1e1 < 0, 0 < 1e2 < 5%, 5% ≤ 1e3 < 10%, 10% ≤ 1e4 < 20%, 20% ≤ 1e5 < 30%, 30% ≤ 1e6 ≤ 40%, 40% < 1e7 < 50%, 50% ≤ 1e8 < 60%, 60% ≤ 1e9 < 70%, 70% ≤ 1e10 < 80%, 80% ≤ 1e11 ≤ 100%. The symbol "-" indicates "not tested". | | | | |

The results showed that the compounds of the present disclosure (including the compounds in Tables 1 and 2, and the example compounds) exhibited significant inhibitory effects on proliferation across a variety of tumor cell lines. These tumor cell lines included MV-4-11 cells (human myelomonocytic leukemia cells), Jurkat cells (human T lymphoblastic leukemia cells), CCRF-CEM cells (human acute lymphoblastic leukemia T lymphocytes), TMD8 cells (human diffuse large B-cell lymphoma cells), 22RV1 cells (human prostate cancer cells), MCF-7 cells (human breast cancer cells), MM.1S cells (human multiple myeloma cells), MDA-MB-231 cells (human breast cancer cells), JEKO-1 cells (human mantle cell lymphoma cells), RPMI-8226 cells (human B lymphoblastoma cells), Molt-4 cells (human acute T lymphoblastic leukemia cells), H196 cells (human small cell lung cancer cells), NCI-H1975 cells (human non-small cell lung cancer cells), and others. The inhibitory effects of the compounds of the present disclosure were superior to those of the corresponding positive control drugs.

### II. Determining the Effect of Compounds of the present disclosure on Target Protein Expression by Western Blot

Conventional Western blot assay was performed to evaluate the effects of the compounds of the present disclosure on the expression of target proteins in cells. Cell lines selected for WB detection based on the target of interest were summarized in Table II-1.

**Table II-1**

| Cell Type | Cell Line | Corresponding Target(s) |
|---|---|---|
| hPBMC Cell | hPBMC Cell | IKZF1/2/3, Wee1, CK1α, GSPT1, IRAK1/2/3/4 |
| Adherent Cells | MDA-MB-231 Cell | CDK4/6 |
| | LnCap Cell | AR |
| | H1975 Cell | EGFR |
| | 22RV1 Cell | AR-V7 (Androgen Receptor Splice Variant 7) |

(1) Cell Seeding and Protein Harvesting: The selected cells were processed according to the cell seeding procedure and protein harvesting procedure in Table II-2.

**Table II-2 Cell Seeding and Protein Harvesting Procedures**

| Cell Type | Cell Line | Cell Plating Procedures for Various Cell Types |
|---|---|---|
| hPBMC Cell | hPBMC Cell | Human peripheral blood mononuclear cells (hPBMCs) at a density of 1×10⁶ cells/mL were thawed according to the PBMC Recovery Protocol (Milecell Biotechnologies Inc, Shanghai). The cells were then treated with the compounds of the present disclosure (including the compounds listed in Tables 1 and 2, and the Examples compounds) at concentrations of 50 nM and 500 nM. Controls included a negative control (DMSO) and a positive control (the commercial immunomodulatory drug, e.g., lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay. |
| Adherent Cells | MDA-MB-231 Cell, LnCap Cell, H1975 Cell, 22RV1 Cell | Adherent cells (e.g., MDA-MB-231 cells) were added to 6-well plates at a seeding density of 2.5×10⁵/mL, with a total volume of 2 mL of cell suspension per well, cultured for 12 hours, and then treated with the compounds of the present disclosure (including the compounds listed in Tables 1 and 2, and the Examples compounds) at concentrations of 500 nM and 50 nM. Controls included a negative control (DMSO) and a positive control (corresponding commercial inhibitors), both processed following the identical experimental protocol as the compounds of the present disclosure. After 24 hours of compounds treatment, the culture medium was removed. The cells were washed once with PBS and then lysed with RIPA protein lysate containing protease inhibitors. The resulting lysed proteins were subsequently scraped with a cell scraper, placed on ice for 30 minutes, and centrifuged. The supernatant was aspirated as the extracted total cell protein. The protein concentration of the supernatant was determined using the conventional BCA assay. |

(2) To the collected supernatant, 5X SDS sample loading buffer was added, followed by denaturation at 95°C for 5 minutes. The denatured samples were then separated by SDS-PAGE electrophoresis on a polyacrylamide gel. Proteins were transferred to a nitrocellulose membrane (0.45 µm NC membrane), blocked with blocking buffer at room temperature for 1 hour, and subjected to antibody incubation and development procedures according to the antibody manufacturer's instructions (Cell Signaling Technology).

Half-Maximal Degradation Concentration value (the drug concentration required for degrading proteins by 50%, abbreviated as DC₅₀) reads method: comparing the grayscale values of Western blot bands from drug-treated samples with those from blank DMSO-treated controls, and identifying the drug concentration range at which the grayscale value is half that of the DMSO-treated control bands.

DC₅₀ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

Target protein degradation rate (%) = 1 - target protein remaining (%).

The effects of the compounds of the present disclosure (including the compounds listed in Tables 1 and 2, and the Examples compounds) on substrate proteins expression in cells were shown in Tables IIA1-IIA5 below.

**Table IIA1 Degradation of substrate proteins CDK4 and CDK6 by the Example compounds of the present disclosure designed based on CDK4/6 inhibitors**

| Comp. No. | CDK4 | CDK6 | Comp. No. | CDK4 | CDK6 |
|---|---|---|---|---|---|
| GT-08188 | B | A | GT-10032 | A | A |
| GT-07097 | B | A | GT-10033 | A | B |
| GT-07109 | A | A | GT-10034 | A | B |
| GT-07241 | A | A | GT-10035 | A | B |
| GT-07194 | A | A | GT-10036 | A | B |
| GT-10038 | A | A | GT-10037 | A | A |

**Table IIA2 Degradation of substrate proteins AR and AR-V7 by the Example compounds of the present disclosure designed based on AR inhibitors**

| Comp. No. | AR | AR-V7 |
|---|---|---|
| GT-08189 | A | |
| GT-07196 | | A |
| GT-07195 | | A |
| GT-07179 | | A |
| GT-10023 | | A |

**Table IIA3 Degradation of substrate proteins IRAK2, IRAK3, and IRAK4 by the compounds of the present disclosure designed based on IRAK inhibitors**

| Comp. No. | IRAK4 | IRAK2 | IRAK3 |
|---|---|---|---|
| GT-06943 | A | A | A |
| GT-06946 | A | B | A |
| GT-06947 | A | B | A |
| GT-06945 | A | B | A |
| GT-06941 | A | B | A |
| GT-06944 | A | B | A |
| GT-05300 | A | B | A |
| GT-06951 | A | B | A |
| GT-07105 | A | | |
| GT-07103 | A | | |
| GT-07104 | A | | |
| GT-07126 | A | | |
| GT-07111 | A | | |
| GT-07394 | A | | |
| GT-07396 | A | | |
| GT-09767 | A | | |
| GT-09776 | A | | |
| GT-09777 | A | | |
| GT-09770 | A | | |
| GT-09769 | A | | |
| GT-09768 | A | | |
| GT-09773 | A | | |
| GT-09775 | A | | |
| GT-09774 | A | | |
| GT-09772 | A | | |
| GT-09766 | A | | |
| GT-10046 | A | | |
| GT-10047 | A | | |
| GT-10048 | A | | |
| GT-10049 | A | | |
| GT-10050 | A | | |
| GT-10051 | A | | |
| GT-10054 | A | | |
| GT-09549 | A | | |
| GT-09548 | A | | |

**Table IIA4 Degradation of substrate protein EGFR by the compounds of the present disclosure designed based on EGFR inhibitors**

| Comp. No. | EGFR | Comp. No. | EGFR |
|---|---|---|---|
| Osimertinib | No degradation | GT-10153 | A |
| GT-09922 | A | GT-10154 | A |

**Table IIA5 Degradation of substrate proteins WEE1, IKZF1, IKZF2, IKZF3, CK1α, and GSPT1 by the compounds of Formula (II) of the present disclosure**

| Comp. No. | WEE1 | IKZF 1 | IKZF2 | IKZF3 | GSPT1 | CK1α |
|---|---|---|---|---|---|---|
| Lenalidomide | B | A | B | A | B | B |
| GT-08008 | B | B | B | B | B | A |
| GT-08009 | B | B | B | B | B | A |
| GT-08007 | B | B | B | B | B | A |
| GT-08006 | B | B | B | B | A | A |
| GT-06988 | B | B | B | B | B | A |
| GT-07966 | B | B | B | B | B | A |
| GT-07965 | B | B | B | B | B | A |
| GT-07008 | A | B | B | B | A | A |
| GT-07368 | B | B | B | A | B | B |

| | | | | | | |
|---|---|---|---|---|---|---|
| **Note:** In Tables IIA1 to IIA5, "A" is defined as 0% ≤ substrate protein remaining % ≤ 80%, and "B" is defined as 80%< substrate protein remaining %≤ 100%. | | | | | | |

The degradation effects of the designed and synthesized compounds of Formula (I) and Formula (II) of the present invention on the target substrate proteins are shown in Tables IIA1-IIA5 and Figure 1. The experimental results from Tables IIA1 through IIA5 and Figure 1 demonstrated that the compounds of formula (I) and formula (II) of the present invention can significantly degrade the target substrate proteins. The commercially available inhibitor cannot degrade the target protein (with 100% target protein remaining), whereas the compounds of formula (I) of the present invention achieve significant degradation of the target substrate proteins. Compared to lenalidomide, the compounds of formula (II) of the present invention show a significant improvement in the efficiency of inducing substrate protein degradation and can also selectively induce the degradation of substrate proteins. In summary, the structural diversity of the compounds of the present invention enables the effective degradation of diverse substrate proteins, thereby potentially enabling their use in treating indications associated with these substrate proteins.

### III. Determination of CRBN-binding affinity of compounds

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:
**1.** According to the instructions of the CEREBLON BINDING KITS, serial dilutions were performed for the test compounds of the present disclosure (including the compounds in Table 1, Table 2, and Examples) and for lenalidomide. Specifically, diluent #9 (1X) solution was used as the diluent, and multiple concentration gradients were set. The dilution operation can be performed according to one of the following two methods:
   (1) The first dilution method: The test compounds of the present disclosure (including the compounds in Table 1, Table 2, and Examples) were subjected to 5-fold serial dilutions starting from a highest concentration of 40 µM, resulting in a total of 7 concentrations from high to low (40, 8, 1.6, 0.32, 0.064, 0.0128, 0.00256 µM). Subsequently, the diluted test compounds and corresponding detection reagents were added to each well of a 96-well plate, resulting in final test compound concentrations of 10, 2, 0.4, 0.08, 0.016, 0.0032, and 0.00064 µM, respectively.
   (2) As an alternative, a second dilution method can be used: The test compounds of the present disclosure (including the compounds in Table 1, Table 2, and Examples) were subjected to 4-fold gradient dilutions starting from a highest concentration of 8 µM, resulting in 2 concentrations (8 µM, 2 µM). Into the 96-well plate, the diluted test compounds and corresponding detection reagents were added, resulting in final test compound concentrations of 2 µM and 0.5 µM, respectively. The experimental procedure is described below using the diluted 8 µM test compound and lenalidomide solutions as examples.
**2.** 2.5 µL of the above 8 µM of the tested compounds and lenalidomide solution, as well as the same volume of diluent #9 (1X) solution (solvent control group, Std0) were added to each well of a 96-well plate, respectively. Then, 2.5 µL of human Cereblon WT GST-tagged protein solution was added to each well. Finally, 5 µL of the thoroughly mixed Thalidomide-Re reagent and GST Eu antibody working solution were added to each of the aforementioned wells. The final concentration of the tested compounds and lenalidomide in each well was 2 µM.
**3.** The blank control wells were sequentially added with 2.5 µL of diluent #9 (1X) solution, 2.5 µL of PROTAC binding buffer, and 5 µL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method: ${R}_{compound} = OD 665 {nm}_{compound} / OD 620 {nm}_{compound} - OD 665 {nm}_{control} / OD 620 {nm}_{control}$ ${R}_{Std 0} = OD 665 {nm}_{Std 0} / OD 620 {nm}_{Std 0} - OD 665 {nm}_{control} / OD 620 {nm}_{control}$ $inhibition rate \left(%\right) = \left(1-{R}_{compound}/{R}_{Std 0}\right) * 100$
Note: OD 665 nm _{compound} is the absorbance value of each well of the compounds to be tested of the present disclosure at an emission wavelength of 665 nm.
OD 620 nm _{compound} is the absorbance value of each well of the compounds to be tested of the present disclosure at an emission wavelength of 620 nm.
OD 665 nm _{control} is the absorbance value of the blank control well at an emission wavelength of 665 nm.
OD 620 nm _{control} is the absorbance value of the blank control well at an emission wavelength of 620 nm.
OD 665 nm _{Std0} is the absorbance value of the solvent control well at an emission wavelength of 665 nm.
OD 620 nm _{Std0} is the absorbance value of the solvent control well at an emission wavelength of 620 nm.

The test results were shown in Tables III-1 and III-2 below.

**Table III-1. HTRF inhibitory activity (IC₅₀, µM) of the compounds of the present disclosure (including the compounds in Tables 1, 2, and the Examples compounds)**

| Comp. No. | HTRF IC₅₀ (µM) | Comp. No. | HTRF IC₅₀ (µM) |
|---|---|---|---|
| Lenalidomide | e | GT-07008 | a |
| GT-05579 | a | GT-07007 | c |
| GT-06989 | b | GT-07368 | c |
| GT-06988 | d | GT-07051 | a |

| | | | |
|---|---|---|---|
| Note: in Table III-1, the symbols a, b, c, d, e, and f are defined as follows: 0<a≤0.5µM, 0.5µM<b≤1µM, 1µM<c≤1.5µM, 1.5µM<d<1.9µM, 1.9µM ≤ e ≤10µM, 10µM < f. | | | |

**Table III-2: Screening of CRBN Binding Affinity of the Compounds of the Present Invention (including the compounds in Tables 1 and 2, and Examples Compounds) at Concentrations of 2 µM and 0.5 µM**

| Comp. No. | Inhibiti on Rate (%) at 2 µM | Inhibition Rate (%) at 0.5 µM | Comp. No. | Inhibitio n Rate (%) at 2 µM | Inhibition Rate (%) at 0.5 µM | Comp. No. | Inhibiti on Rate (%) at 2 µM | Inhibition Rate (%) at 0.5 µM |
|---|---|---|---|---|---|---|---|---|
| Lenalidomi de | a4 | a2 | GT-07097 | a7 | a1 | GT-09922 | a7 | a2 |
| GT-08179 | a5 | a3 | GT-07109 | a8 | a3 | GT-09928 | a7 | a7 |
| GT-08189 | a5 | a3 | GT-07194 | a5 | a1 | GT-09924 | a8 | a4 |
| GT-08180 | a8 | a3 | GT-07195 | a5 | a1 | GT-09930 | a8 | a6 |
| GT-08195 | a5 | a3 | GT-07100 | a5 | a2 | GT-09926 | a8 | a6 |
| GT-07105 | a7 | a2 | GT-07090 | a7 | a2 | GT-09929 | a8 | a3 |
| GT-07103 | a8 | a4 | GT-07179 | a5 | a2 | GT-09925 | a8 | a4 |
| GT-07104 | a8 | a1 | GT-07112 | a7 | a4 | GT-09927 | a8 | a3 |
| GT-07111 | a8 | a4 | GT-07102 | a7 | a4 | GT-09923 | a8 | a4 |
| GT-07396 | a8 | a5 | GT-07110 | a6 | a3 | GT-09921 | a8 | a7 |
| GT-07395 | a5 | a3 | GT-07181 | a7 | a3 | GT-09932 | a8 | a8 |
| GT-07966 | a7 | a4 | GT-09631 | a6 | a2 | GT-09931 | a8 | a6 |
| GT-07965 | a5 | a2 | GT-09634 | a8 | a3 | GT-10033 | a8 | a8 |
| GT-09767 | a7 | a6 | GT-09684 | a8 | a2 | GT-10034 | a8 | a5 |
| GT-09776 | a8 | a7 | GT-09632 | a7 | a2 | GT-10035 | a8 | a7 |
| GT-09777 | a8 | a4 | GT-09681 | a6 | a1 | GT-10036 | a8 | a7 |
| GT-09770 | a6 | a3 | GT-09683 | a8 | a1 | GT-10037 | a8 | a7 |
| GT-09769 | a6 | a4 | GT-09633 | a8 | a3 | GT-10038 | a8 | a6 |
| GT-09773 | a7 | a6 | GT-09682 | a8 | a2 | GT-10040 | a8 | a6 |
| GT-09775 | a8 | a5 | GT-07091 | a6 | a3 | GT-10041 | a6 | a4 |
| GT-09774 | a7 | a6 | GT-10047 | a8 | a7 | GT-10042 | a8 | a7 |
| GT-09772 | a8 | a5 | GT-10048 | a8 | a6 | GT-10043 | a8 | a4 |
| GT-06947 | a6 | a4 | GT-10049 | a8 | a3 | GT-10044 | a8 | a6 |
| GT-06941 | a8 | a3 | GT-10050 | a8 | a7 | GT-10045 | a8 | a6 |
| GT-07089 | a8 | a3 | GT-10051 | a8 | a8 | GT-09548 | a6 | a3 |
| GT-07096 | a7 | a1 | GT-10052 | a8 | a7 | | | |

| | | | | | | | | |
|---|---|---|---|---|---|---|---|---|
| Note: in table III-2, the symbols a, a1, a2, a3, a4, a5, a6, a7, and a8 are defined as follows: 0% < a ≤ 10%, 10% < a1 ≤ 20%, 20% < a2 ≤ 30%, 30% < a3 ≤ 40%, 40% < a4 ≤ 53%, 53% < a5 ≤ 60%, 60% < a6 ≤ 70%, 70% < a7 ≤ 80%, 80% < a8 ≤ 100%. | | | | | | | | |

The results in Table III-1 and Table III-2 demonstrate that the compounds of the present invention (including but not limited to the compounds in Tables 1 and 2, and Examples) exhibit lower or comparable IC₅₀ values, or higher inhibition rates compared to lenalidomide, indicating stronger binding affinity to CRBN.

### IV. TNF-α Activity Inhibition Assay

PBMCs cells were cultured at 37°C with 5% CO₂ and seeded in 96-well plates at 1×10⁷ cells/well. The compounds to be tested (including those listed in Tables 1 and 2, and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. IC₅₀ values were calculated using GraphPad Prism 7.0.

TNF-α downregulation is a key mechanism for the anti-tumor action of immunomodulators. The compounds of the present disclosure demonstrated dose-dependent inhibition of TNF-α levels.

### V. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Tables 1 and 2, and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs, or non-rodents (rabbits, dogs, and monkeys). The experimental animals used in this study were mice.

The experimental animals were divided into two groups: a control group (for collecting blank plasma) and an oral administration group (administered at a dose of 10 mg/kg). Blood samples were collected at predetermined time points after administration, such as 0.25 h, 0.5 h, 1 h, 2 h, 4 h, 8 h, and 24 h post-dose. Blank plasma was collected from the control group.

Prior to analysis, plasma samples were processed as follows: To 10 µL of plasma sample were added 10 µL of 50% acetonitrile and 200 µL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

### VI. Effects of the compounds of the present disclosure on the release of inflammatory cytokines, including TNF-α, IL-2, IL-12, IL-10, IL-1α, IL-1β, IL-6, IL-8, and IFN-γ, from PBMCs induced by phytohemagglutinin (PHA) or the eBioscience^{™} Cell Stimulation Cocktail (PMA + Ionomycin)

The present inventors examined the effects of the compound of the present invention on the release of a panel of inflammatory cytokines from peripheral blood mononuclear cells (PBMCs) triggered by phytohemagglutinin (PHA) or the eBioscience^{™} Cell Stimulation Cocktail (containing PMA and Ionomycin). These inflammatory cytokines include TNF-α, IL-2, IL-5, IL-12, IL-10, IL-1α, IL-6, IL-8, IFN-γ, IL-1β, and granulocyte-macrophage colony-stimulating factor (GM-CSF), among others. Previous studies have suggested that these inflammatory cytokines may play important roles in the onset and progression of cancer, autoimmune diseases, infectious diseases, and inflammatory diseases. In autoimmune diseases such as rheumatoid arthritis (RA), psoriatic arthritis (PsA), and ankylosing spondylitis (AS), the expression level of TNF-α is significantly elevated, and it exerts its effects by stimulating synovial cell proliferation and promoting inflammatory responses. IL-1α is associated with cancer, autoimmune diseases, infectious diseases, and other inflammatory conditions, and participates in the inflammatory processes of autoimmune diseases. In diseases such as rheumatoid arthritis and systemic lupus erythematosus, elevated IL-1β levels can reflect the extent and activity of inflammatory responses. IL-2, IL-5, IL-10, IFN-γ, and GM-CSF are also associated with various inflammatory diseases and autoimmune diseases.

The following materials were used in this study:
VI.1 Reagents and Consumable materials

| Name | Supplier or Source |
|---|---|
| Human PBMC cell | Milestone Biotechnologies |
| eBioscience^{™} Cell Stimulation Cocktail | Invitrogen |
| Phytohemagglutinin (PHA) | MCE |
| RPMI 1640 Medium | Gibco |
| Human IFN-y CBA Flex Set E7 100Tst | Becton, Dickinson and Company |
| Human IL-2 CBA Flex Set A4 100Tst | Becton, Dickinson and Company |
| Human TNF-α CBA Flex Set D9 100Tst | Becton, Dickinson and Company |
| Human IL-10 CBA Flex Set B7 100Tst | Becton, Dickinson and Company |
| Human IL-6 CBA Flex Set A7 100Tst | Becton, Dickinson and Company |
| Human IL-8 CBA Flex Set A9 100Tst | Becton, Dickinson and Company |
| Human IL-1β CBA Flex Set B4 100Tst | Becton, Dickinson and Company |
| Human IL-12/IL-23p40 CBA Flex Set E5 100Tst | Becton, Dickinson and Company |
| Human IL-17A CBA Flex Set B5 100Tst | Becton, Dickinson and Company |
| Human IL-1α CBA Flex Set D6 100Tst | Becton, Dickinson and Company |
| Human IL-5 CBA Flex Set A6 100Tst | Becton, Dickinson and Company |
| Human IL-13 CBA Flex Set E6 100Tst | Becton, Dickinson and Company |
| Human IL-4 CBA Flex Set A5 100Tst | Becton, Dickinson and Company |
| Human GM-CSF CBA Flex Set C9 100Tst | Becton, Dickinson and Company |
| Sheath Fluid for Flow Cytometric Analysis (20L) | Becton, Dickinson and Company |
| Human Solbl Ptein CBA Buf Kit 500Tst | Becton, Dickinson and Company |

VI.2 Methods
VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shaken occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of 1×10⁵ cells/well (75 µL/well) and incubated in an incubator for 2 h at 37°C with 5% CO₂ under high humidity conditions.

VI.2.2 Compound Preparation and Addition to Cell Plates
1) The test compounds (including the compounds in Tables 1 and 2, and the Examples compounds) were prepared as 10 mM stock solutions in DMSO.
2) The test compounds were serially diluted 10-fold in DMSO starting from 1 mM (highest concentration) to generate 2 concentration gradients, i.e., 1 mM and 0.1 mM.
3) The test compounds were further diluted in culture medium to five times their final working concentration intended for use.
4) The diluted compounds were added to designated wells according to the plate layout at 25 µL/well, achieving final concentrations starting from 1 µM with 10-fold serial dilution (2 concentration points in total), i.e., 1 µM and 0.1 µM.
5) After 1 h incubation, PHA or PMA+Ionomycin (25 µL/well) was added to the cell wells to reach final concentrations of: 1 µg/mL PHA (TNF-α, IL-10, IL-6, IL-1β, IFN-γ, IL-2, IL-12p40, IL-1α, IL-8), 81nM/mL PMA (IL-4, IL-5, IL-13, GM-CSF, and IL-17A), or 1.34 µM/mL Ionomycin (IL-4, IL-5, IL-13, GM-CSF, and IL-17A).
6) The cell plates were incubated in an incubator for 24 h at 37°C with 5% CO₂ under high humidity. The negative control was DMSO, and the positive control drugs were the corresponding commercial immunomodulatory agents (e.g., lenalidomide) or the corresponding commercial inhibitors (including emavusertib (CAS No.: 1801344-14-8)), all of which were used to treat the cells in the same manner as the compound of the present disclosure.

### Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | H₂O | | | | | | | | | | | |
| B | DMSO | PHA/ PMA+Ionomycin | Cmpd 1-5# (1 µM and 0.1µM, 2 points) | | | | | | | | | |
| C | Cmpd 6-11# (1 µM and 0.1µM, 2 points) | | | | | | | | | | | |
| D | Cmpd 12-17# (1 µM and 0.1µM, 2 points) | | | | | | | | | | | |
| E | Cmpd 18-23# (1 µM and 0.1µM, 2 points) | | | | | | | | | | | |
| F | Cmpd 24-29# (1 µM and 0.1µM, 2 points) | | | | | | | | | | | |
| G | Cmpd 30-35# (1 µM and 0.1µM, 2 points) | | | | | | | | | | | |
| H | Cmpd 36-41# (1 µM and 0.1µM, 2 points) | | | | | | | | | | | |

VI.2.3 Detection
(1) The cell plate was centrifuged and the culture supernatant was aspirated. TNF-α, IL-10, IL-6, IL-1β, IFN-γ, IL-2, IL-12p40, IL-1α, IL-8, IL-4, IL-5, IL-13, GM-CSF, and IL-17A cytokines in the culture supernatant were detected according to the instructions of the flow cytometry CBA (Cytometric Bead Array) assay.
(2) The flow cytometry CBA assay was performed as follows:
   Dilution of standards and samples: Standards and samples were diluted at appropriate ratios to enable accurate quantitative analysis in the assay.
   Bead mixing: Capture beads were mixed with samples or standards and incubated in the dark for 1 hour. Antibody mixing: Detection antibodies were mixed with samples or standards and incubated in the dark for 2 hours. These two steps are the core of the flow cytometry CBA technique, enabling quantitative detection of cytokines through the binding of beads to target molecules.
   Flow cytometric detection: The mixtures were analyzed using a flow cytometer. The flow cytometer excites fluorescent labels on the beads with a laser, collects and analyzes the fluorescence signals, thereby quantitatively determining the cytokine concentrations in the samples.
(3) Data collected by the flow cytometer were further analyzed, typically using software (FACP) for data processing, to generate detailed cytokine concentration reports (for TNF-α, IL-10, IL-6, IL-1β, IFN-γ, IL-2, IL-12p40, IL-1α, IL-8, IL-4, IL-5, IL-13, GM-CSF, and IL-17A). The inhibition rate (%) was calculated as follows: $Inhibition rate \left(%\right) = \left[\left(Ac-As\right)/\left(Ac-Ab\right)\right] \times 100 %$ where:
   As = OA of the sample (cells + PHA / PMA+Ionomycin + test compound)
   Ac = OA of the positive cell control (cells + PHA / PMA+Ionomycin + DMSO)
   Ab = OA of the blank control (cells + medium + DMSO)
(4) IC₅₀ curve fitting and IC₅₀ value calculation were performed using GraphPad Prism 6 software with the following formula/analysis: XY-analysis/Nonlinear regression (curve fit)/Dose response-Inhibition/log (inhibitor) vs. response-Variable slope (four parameters).

The results are presented in Table IV below.

**Table IV: Inhibition rates (%) of the compounds of the present disclosure (including but not limited to the compounds in Tables 1 and 2, and the Example compounds) on inflammatory cytokines at concentrations of 1 µM and 0.1 µM.**

| Comp. No. | **IL-10** | | **IL-1α** | | **IL-1β** | | **IL-12** | | **TNF-α** | | **IL-2** | | **IFN-γ** | | **IL-6** | | **IL-8** | |
|---|---|---|---|---|---|---|---|---|---|---|---|---|---|---|---|---|---|---|
| | 1µ M | 0.1 µM | 1µ M | 0.1 µM | 1µ M | 0.1 µM | 1µ M | 0.1 µM | 1µ M | 0.1 µM | 1µ M | 0.1 µM | 1µ M | 0.1 µM | 1µ M | 0.1 µM | 1µ M | 0.1 µM |
| lenalid omide | IV 4 | IV 5 | IV 10 | IV 10 | IV 12 | IV 12 | IV 13 | IV 12 | IV 4 | IV 6 | IV 0 | IV 1 | - | - | IV 10 | IV 11 | IV 2 | IV 3 |
| GT-08008 | - | - | IV 7 | IV 7 | IV 8 | IV 8 | IV 7 | IV 6 | IV 6 | IV 6 | IV 8 | IV 7 | IV 10 | IV 8 | IV 7 | IV 7 | IV 6 | IV 8 |
| GT-08009 | - | - | IV 6 | IV 10 | IV 7 | IV 11 | IV 7 | IV 10 | IV 6 | IV 8 | IV 7 | IV 8 | IV 8 | IV 13 | IV 8 | IV 10 | IV 7 | IV 8 |
| GT-08006 | - | - | IV 12 | IV 8 | IV 12 | IV 6 | IV 9 | IV 7 | IV 8 | IV 5 | IV 5 | IV 6 | IV 11 | IV 8 | IV 11 | IV 7 | IV 10 | IV 7 |
| GT-04310 | - | - | - | - | - | - | - | - | - | - | IV 7 | IV 6 | IV 9 | IV 12 | IV 5 | IV 7 | - | - |
| GT-05579 | IV 10 | IV 8 | IV 12 | IV 9 | IV 13 | IV 11 | IV 14 | IV 9 | IV 13 | IV 10 | IV 10 | IV 10 | IV 14 | IV 13 | IV 12 | IV 10 | IV 13 | IV 7 |
| GT-06989 | - | - | IV 11 | IV 9 | IV 11 | IV 9 | - | - | - | - | - | - | - | - | IV 10 | IV 9 | IV 10 | IV 8 |
| GT-07966 | - | - | IV 10 | IV 5 | IV 10 | IV 5 | IV 8 | IV 5 | IV 8 | IV 5 | IV 7 | - | IV 9 | - | IV 9 | IV 5 | IV 9 | IV 5 |
| GT-08195 | IV 9 | IV 8 | IV 10 | IV 11 | IV 9 | IV 9 | - | - | - | - | - | - | IV 15 | IV 15 | IV 8 | IV 9 | IV 7 | IV 8 |
| GT-07008 | IV 11 | IV 12 | IV 12 | IV 13 | IV 13 | IV 14 | IV 12 | IV 13 | IV 12 | IV 13 | IV 9 | IV 10 | IV 14 | IV 14 | IV 12 | IV 13 | IV 10 | IV 12 |
| GT-07007 | IV 10 | IV 8 | IV 8 | IV 9 | IV 9 | IV 10 | IV 10 | IV 9 | IV 9 | IV 9 | IV 9 | IV 9 | IV 11 | IV 11 | IV 9 | IV 9 | IV 8 | IV 8 |
| GT-07368 | - | - | IV 11 | IV 9 | IV 12 | IV 8 | IV 12 | IV 8 | IV 8 | IV 5 | IV 5 | IV 5 | IV 11 | IV 8 | IV 11 | IV 9 | IV 8 | IV 6 |
| GT-09916 | IV 8 | IV 9 | IV 8 | IV 9 | IV 8 | IV 8 | IV 6 | IV 5 | IV 8 | IV 7 | IV 10 | IV 10 | IV 11 | IV 12 | IV 9 | IV 8 | IV 8 | IV 6 |
| GT-09995 | IV 15 | IV 9 | IV 15 | IV 15 | IV 15 | IV 12 | IV 15 | IV 10 | IV 15 | IV 9 | IV 12 | IV 11 | IV 14 | IV 12 | IV 15 | IV 12 | IV 15 | IV 14 |
| emavu sertib | IV 4 | IV 3 | IV 13 | IV 3 | IV 14 | IV 3 | IV 14 | IV 3 | IV 8 | IV 2 | IV 2 | IV 2 | - | - | IV 14 | IV 6 | IV 11 | IV 7 |
| GT-07105 | IV 13 | IV 12 | IV 14 | IV 14 | IV 14 | IV 14 | IV 14 | IV 14 | IV 14 | IV 14 | IV 13 | IV 13 | IV 15 | IV 15 | IV 13 | IV 13 | IV 13 | IV 12 |
| GT-07103 | IV 12 | IV 10 | IV 14 | IV 14 | IV 14 | IV 14 | IV 14 | IV 14 | IV 14 | IV 13 | IV 5 | IV 10 | IV 15 | IV 15 | IV 13 | IV 13 | IV 12 | IV 12 |
| GT-07104 | IV 14 | IV 14 | IV 14 | IV 14 | IV 14 | IV 14 | IV 14 | IV 14 | IV 15 | IV 14 | - | - | IV 15 | IV 15 | IV 14 | - | IV 13 | IV 13 |
| GT-07126 | IV 14 | IV 12 | IV 15 | IV 14 | IV 14 | IV 14 | IV 15 | IV 14 | IV 14 | IV 13 | IV 12 | IV 10 | IV 15 | IV 15 | - | IV 13 | IV 14 | IV 12 |
| GT-07111 | IV 13 | IV 13 | IV 14 | IV 14 | IV 15 | IV 14 | IV 15 | IV 14 | IV 14 | IV 14 | IV 7 | IV 10 | IV 15 | IV 15 | - | IV 14 | IV 13 | IV 13 |
| GT-07394 | IV 13 | IV 8 | IV 14 | IV 9 | IV 14 | IV 8 | IV 14 | IV 10 | IV 14 | IV 8 | IV 8 | IV 7 | IV 15 | IV 15 | IV 13 | IV 8 | IV 11 | IV 7 |
| GT-07396 | IV 12 | IV 8 | IV 14 | IV 9 | IV 14 | IV 7 | IV 13 | IV 6 | IV 13 | IV 6 | IV 7 | IV 6 | IV 15 | IV 15 | IV 10 | IV 5 | IV 9 | IV 5 |
| GT-08180 | IV 13 | IV 9 | IV 14 | IV 12 | IV 14 | IV 12 | IV 14 | IV 10 | IV 14 | IV 11 | IV 14 | IV 13 | IV 15 | IV 15 | IV 12 | IV 7 | IV 11 | IV 5 |
| GT-09767 | IV 12 | IV 12 | IV 14 | - | IV 15 | - | IV 14 | IV 8 | IV 13 | IV 6 | IV 13 | IV 12 | IV 14 | IV 12 | - | IV 9 | IV 13 | IV 9 |
| GT-09776 | IV 9 | IV 8 | IV 12 | IV 11 | IV 12 | IV 10 | IV 10 | IV 7 | IV 10 | IV 8 | IV 10 | IV 12 | IV 12 | IV 11 | IV 11 | IV 8 | IV 10 | IV 5 |
| GT-09777 | IV 10 | IV 11 | IV 14 | - | IV 14 | - | IV 14 | IV 8 | IV 13 | IV 8 | IV 11 | IV 12 | IV 14 | IV 11 | IV 14 | IV 6 | IV 13 | IV 5 |
| GT-09765 | IV 13 | IV 8 | IV 14 | IV 14 | IV 14 | IV 14 | IV 15 | IV 13 | IV 13 | IV 12 | IV 10 | IV 9 | IV 15 | IV 15 | - | IV 12 | IV 13 | IV 11 |
| GT-09770 | IV 7 | IV 8 | IV 14 | IV 7 | IV 14 | IV 5 | IV 13 | IV 5 | IV 11 | IV 5 | IV 9 | IV 9 | IV 13 | IV 9 | IV 13 | IV 7 | IV 9 | IV 8 |
| GT-09769 | IV 8 | IV 10 | IV 14 | IV 5 | IV 14 | IV 5 | IV 14 | - | IV 12 | IV 5 | IV 8 | IV 9 | IV 14 | IV 7 | IV 14 | IV 5 | IV 11 | IV 6 |
| GT-09768 | IV 7 | IV 9 | IV 13 | IV 5 | IV 14 | IV 5 | IV 12 | IV 5 | IV 12 | IV 5 | IV 11 | IV 8 | IV 13 | IV 8 | IV 12 | IV 7 | IV 8 | IV 7 |
| GT-09773 | IV 12 | IV 8 | IV 14 | IV 8 | IV 15 | IV 6 | IV 14 | IV 6 | IV 13 | IV 7 | IV 12 | IV 13 | IV 14 | IV 12 | - | IV 5 | IV 14 | - |
| GT-09771 | IV 14 | IV 9 | IV 14 | IV 5 | IV 14 | IV 5 | IV 14 | IV 5 | IV 14 | IV 5 | IV 14 | IV 12 | IV 14 | IV 9 | IV 14 | - | IV 12 | - |
| GT-09775 | IV 10 | IV 8 | IV 14 | IV 7 | IV 14 | IV 6 | IV 14 | IV 7 | IV 11 | IV 6 | IV 5 | IV 13 | IV 13 | IV 11 | IV 14 | IV 5 | IV 11 | - |
| GT-09774 | IV 10 | IV 7 | IV 14 | IV 9 | IV 14 | IV 8 | IV 13 | IV 5 | IV 12 | IV 7 | IV 10 | IV 12 | IV 14 | IV 10 | IV 13 | IV 5 | IV 12 | IV 5 |
| GT-09772 | IV 9 | IV 7 | IV 13 | IV 8 | IV 13 | IV 7 | IV 11 | IV 6 | IV 11 | IV 6 | IV 13 | IV 10 | IV 13 | IV 11 | IV 10 | IV 5 | IV 8 | IV 5 |
| GT-06943 | IV 11 | IV 12 | IV 14 | IV 14 | IV 15 | IV 15 | IV 15 | IV 14 | IV 14 | IV 13 | IV 10 | IV 10 | IV 14 | IV 14 | - | - | IV 14 | IV 13 |
| GT-06946 | IV 12 | IV 13 | IV 14 | IV 14 | IV 15 | IV 15 | IV 15 | IV 15 | IV 14 | IV 14 | IV 9 | IV 10 | IV 14 | IV 14 | - | - | IV 14 | IV 14 |
| GT-06947 | IV 11 | IV 13 | IV 14 | IV 14 | IV 15 | IV 15 | IV 15 | IV 15 | IV 14 | IV 14 | IV 8 | IV 10 | IV 14 | IV 14 | - | - | IV 14 | IV 14 |
| GT-06945 | IV 14 | IV 13 | IV 14 | IV 14 | IV 15 | IV 14 | IV 15 | IV 14 | IV 15 | IV 14 | - | IV 12 | IV 15 | IV 14 | - | IV 14 | IV 14 | IV 13 |
| GT-06941 | IV 14 | IV 12 | IV 14 | IV 14 | IV 15 | IV 14 | IV 15 | IV 14 | IV 15 | IV 14 | - | IV 12 | IV 15 | IV 14 | - | IV 13 | IV 14 | IV 12 |
| GT-06944 | IV 12 | IV 7 | IV 14 | IV 10 | IV 15 | IV 10 | IV 15 | IV 6 | IV 14 | IV 6 | IV 9 | IV 7 | IV 14 | IV 8 | - | IV 9 | IV 14 | IV 8 |
| GT-05300 | IV 12 | IV 7 | IV 14 | IV 12 | IV 14 | IV 13 | IV 14 | IV 13 | IV 14 | IV 11 | IV 7 | IV 8 | IV 14 | IV 13 | - | IV 11 | IV 13 | IV 8 |
| GT-10024 | IV 8 | IV 9 | IV 13 | IV 14 | IV 12 | IV 10 | IV 9 | IV 6 | IV 8 | IV 6 | IV 9 | IV 8 | IV 10 | IV 10 | IV 13 | IV 10 | IV 14 | IV 12 |
| GT-10022 | IV 13 | IV 9 | IV 15 | IV 13 | IV 15 | IV 9 | IV 15 | IV 8 | IV 13 | IV 7 | IV 5 | IV 10 | IV 14 | IV 11 | IV 15 | IV 11 | IV 15 | IV 13 |
| GT-10046 | IV 12 | IV 7 | IV 15 | IV 11 | IV 15 | IV 10 | IV 15 | IV 6 | IV 14 | IV 7 | IV 6 | IV 8 | IV 14 | IV 9 | IV 15 | IV 13 | IV 15 | IV 15 |
| GT-10047 | IV 10 | IV 11 | IV 15 | IV 15 | IV 15 | IV 10 | IV 14 | IV 9 | IV 12 | IV 8 | IV 8 | IV 10 | IV 13 | IV 11 | IV 15 | IV 14 | IV 15 | IV 15 |
| GT-10048 | IV 14 | IV 10 | IV 15 | IV 15 | IV 15 | IV 10 | IV 15 | IV 9 | IV 15 | IV 7 | IV 7 | IV 7 | IV 14 | IV 10 | IV 15 | IV 15 | IV 15 | IV 15 |
| GT-10049 | IV 8 | IV 9 | IV 15 | IV 13 | IV 15 | IV 9 | IV 14 | IV 6 | IV 11 | IV 5 | IV 5 | IV 6 | IV 13 | IV 9 | IV 15 | IV 13 | IV 15 | IV 15 |
| GT-10050 | IV 15 | IV 7 | IV 15 | IV 12 | IV 15 | IV 9 | IV 15 | IV 7 | IV 15 | IV 5 | IV 14 | IV 5 | IV 15 | IV 8 | IV 15 | IV 8 | IV 15 | IV 11 |
| GT-10051 | IV 13 | IV 7 | IV 15 | IV 13 | IV 15 | IV 9 | IV 15 | IV 6 | IV 14 | IV 6 | IV 14 | IV 5 | IV 14 | IV 9 | IV 15 | IV 10 | IV 15 | IV 13 |
| GT-10052 | IV 14 | IV 10 | IV 15 | IV 15 | IV 15 | IV 11 | IV 15 | IV 9 | IV 15 | IV 8 | IV 10 | IV 9 | IV 14 | IV 11 | IV 15 | IV 11 | IV 15 | IV 15 |
| GT-10054 | IV 9 | IV 8 | IV 15 | IV 14 | IV 15 | IV 10 | IV 15 | IV 8 | IV 11 | IV 8 | IV 9 | IV 8 | IV 14 | IV 10 | IV 15 | IV 10 | IV 15 | IV 13 |
| GT-06951 | IV 13 | IV 10 | IV 14 | IV 13 | IV 15 | IV 14 | IV 15 | IV 14 | IV 14 | IV 13 | IV 10 | IV 9 | IV 14 | IV 14 | - | IV 13 | IV 14 | IV 12 |
| GT-09549 | IV 13 | IV 5 | IV 15 | IV 10 | IV 15 | IV 10 | IV 15 | IV 11 | IV 14 | IV 6 | IV 9 | IV 5 | IV 14 | IV 11 | - | IV 8 | IV 14 | IV 7 |
| GT-09548 | IV 13 | IV 6 | IV 14 | IV 13 | IV 15 | IV 14 | IV 15 | IV 13 | IV 14 | IV 11 | IV 9 | IV 7 | IV 14 | IV 13 | - | IV 12 | IV 13 | IV 10 |
| GT-09603 | IV 13 | IV 8 | IV 14 | IV 12 | IV 15 | IV 12 | IV 14 | IV 10 | IV 13 | IV 8 | IV 5 | IV 7 | IV 14 | IV 11 | - | IV 11 | IV 13 | IV 10 |

| | | | | | | | | | | | | | | | | | | |
|---|---|---|---|---|---|---|---|---|---|---|---|---|---|---|---|---|---|---|
| Note: in table IV, the symbols IV0, IV1, IV2, IV3, IV4, IV5, IV6, IV7, IV8, IV9, IV10, IV11, IV12, IV13, IV14, and IV15 are defined as follows: -320% ≤ IV0 < -200%, -200% ≤ IV1 < -100%, -100% ≤ IV2 < -50%, - 50% ≤ IV3 < -20%, -20% ≤ IV4 < 0%, 0% < IV5 ≤ 10%, 10% < IV6 ≤ 20%, 20% < IV7 ≤ 30%, 30% < IV8 ≤ 40%, 40% < IV9 ≤ 50%, 50% < IV10 ≤ 60%, 60% < IV11 ≤ 70%, 70% < IV12 ≤ 80%, 80% < IV13 ≤ 90%, 90% < IV14 < 100%, 100% ≤ IV15 ≤ 110%. The symbol "-" indicates "not tested". | | | | | | | | | | | | | | | | | | |

The experimental results demonstrate that the compounds of the present disclosure (including the compounds in Tables 1 and 2, and the Example compounds) exhibit excellent modulatory capacity in regulating the production levels of cytokines associated with inflammatory diseases and autoimmune diseases, with inhibitory effects significantly more pronounced than those of the positive control drugs lenalidomide and emavusertib. The positive control drugs lenalidomide and emavusertib elevate IL-2 or IL-10 levels, thereby activating immune responses; in contrast, the compounds of the present disclosure suppress IL-2 and IL-10 or show no significant stimulatory effect, which confers greater advantages in the treatment of immune system disorders. The compounds of the present disclosure (including the compounds in Tables 1 and 2, and the Example compounds) hold broad promise as potential therapeutic agents for the treatment of various inflammatory diseases and autoimmune diseases, such as rheumatoid arthritis, ankylosing spondylitis, psoriasis and psoriatic arthritis, systemic lupus erythematosus, atopic dermatitis, and hidradenitis suppurativa, among others.

### VII. Efficacy experiment of the compounds of the present disclosure on Psoriasis-like skin lesions induced by Imiquimod in mice

The compounds of the present disclosure (test compounds, including the compounds in Tables 1, 2, and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |

| | | | | | | |
|---|---|---|---|---|---|---|
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

### Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by CO₂ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-α were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean + SD. Statistical analysis between two groups was performed using SPSS, with p < 0.05 considered statistically significant.

The experimental results demonstrated that the compounds of the present disclosure exhibited ameliorative effects on psoriasis-like skin lesions on the back of mice induced by imiquimod and can be used for the treatment of psoriasis.

### VIII. Efficacy Experiment of the compounds of the present disclosure on the CIA Model in Rats Induced by Type II Collagen

The compounds of the present disclosure (test compounds, including the compounds in Tables 1, 2, and Examples) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:
10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |

| | | | | | | |
|---|---|---|---|---|---|---|
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

### Scoring

Arthritis Index (AI): The foot volume was measured twice a week, and the swelling of the four toes was scored simultaneously. Each foot was scored on a scale from 0 to 4, with a maximum score of 16 for each rat.

### Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by CO₂ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

### Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.
Scoring and Counting Method: 0, 1, 2, 3, and 4 levels, where "0" indicates no observation; "1" is mild; "2" is moderate; "3" is severe; and "4" is extremely severe.

Experimental data were presented as Mean + SEM. Data between two groups were analyzed using Excel-T test, with p<0.05 considered statistically significant. Scoring data were analyzed using the non-parametric Mann-Whitney U test in SPSS, with p<0.05 considered statistically significant.

The experimental results demonstrated that the compounds of the present disclosure regulated the serum inflammatory cytokines levels in the bovine type II collagen-induced rat model of collagen-induced arthritis (CIA), significantly ameliorated limb swelling in the rats, and thus can be applicable for the treatment of arthritis.

The foregoing has shown and described the basic principles, main features, and advantages of the present invention. It should be understood by those skilled in the art that the present invention is not limited to the above-described embodiments, and that various changes and modifications may be made thereto without departing from the spirit and scope of the present invention, and such changes and modifications all fall within the scope of the invention as claimed. The scope of protection sought by the present invention is defined by the appended claims and their equivalents.

## Claims

1. A compound of Formula (I)
PBM-LIN-ULM (I)
or salts, enantiomers, stereoisomers, solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 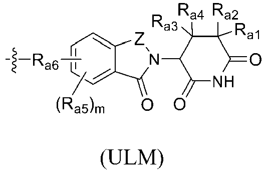
wherein Z represents C(O) or CH₂;
Rₐ₁, Rₐ₂, Rₐ₃, and Rₐ₄ are identical or different and each independently represents hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
m represents an integer of 0, 1, 2, or 3;
Rₐ₆ represents: 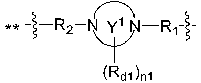
wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20;
R₁ represents N(R_{w}), where R_{w} represents hydrogen or C₁₋₃ alkyl, and R₂ represents a bond; or R₂ represents CH₂, C(O), **-NHC(O)-, or **-N(CH₃)C(O)-; or R₂ represents a structure of the following formula: 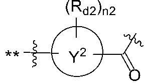 wherein ring Y² represents cycloalkylene, or heterocyclylene containing at least one nitrogen atom, and (R_{d2})ₙ₂ indicates that ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n2 represents an integer of 0-20;
or
R₁ represents a bond, and R₂ represents N(R_{w}), where R_{w} represents hydrogen or C₁₋₃ alkyl; or R₂ represents **C(O)N(R_{w}), where R_{w} represents hydrogen or C₁₋₃ alkyl; or R₂ represents **-CH=N-; or
R ₐ₆ represents: 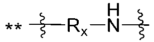 wherein Rₓ represents NH or **-CH=N-; or
R ₐ₆ represents: 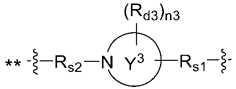
wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20; and
wherein Rₛ₁ represents 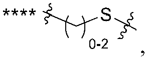 symbol **** indicates the point of attachment to ring Y³; and Rₛ₂ represents N(R_{w}) or **C(O)N(R_{w}), where R_{w} represents hydrogen or C₁₋₃ alkyl;
symbol ** in each of the above formulae indicates the point of attachment to LIN; and
LIN represents a bond, or a structure of the following formula: 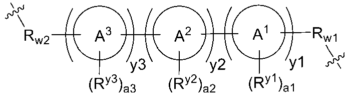
wherein R_{w2} is linked to PBM, and R_{w1} is linked to Rₐ₆;
R_{w2} represents a bond, C(O), C(O)NH, or NHC(O);
each ring A¹ is identical or different and each independently represents heterocyclylene, cycloalkylene, arylene, or heteroarylene, y1 represents an integer of 0, 1, 2, or 3, and (R^{y1})ₐ₁ indicates that each ring A¹ is independently optionally substituted with a1 R^{y1} substituents, wherein each R^{y1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl or C₂₋₆ alkenyl, and a1 represents an integer of 0-10;
each ring A² is identical or different and each independently represents heterocyclylene, cycloalkylene, arylene, or heteroarylene, y2 represents an integer of 0, 1, 2, or 3, and (R^{y2})ₐ₂ indicates that each ring A² is independently optionally substituted with a2 R^{y2} substituents, wherein each R^{y2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a2 represents an integer of 0-10;
each ring A³ is identical or different and each independently represents heterocyclylene, cycloalkylene, arylene, or heteroarylene, y3 represents an integer of 0, 1, 2, or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a3 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof.

2. The compound of Formula (I) or salts, enantiomers, stereoisomers, solvates, isotopically enriched analogs, prodrugs, or polymorphs thereof as claimed in claim 1, wherein the PBM represents a small molecule ligand binding to the following protein: epidermal growth factor receptor (EGFR), Cyclin-dependent kinase 4/6 (CDK4/6), breakpoint cluster region (BCR)-Abelson leukemia virus (ABL) (BCR-ABL) tyrosine kinase, Androgen receptor (AR), or Interleukin-1 receptor-associated kinase 1/2/3/4 (IRAK1/2/3/4).

3. The compound of Formula (I) or salts, enantiomers, stereoisomers, solvates, isotopically enriched analogs, prodrugs, or polymorphs thereof as claimed in claim 1, wherein said PBM comprises a structure selected from the following formula: 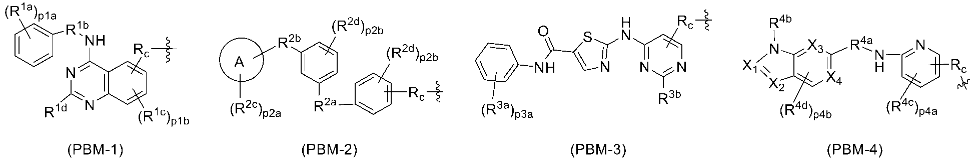 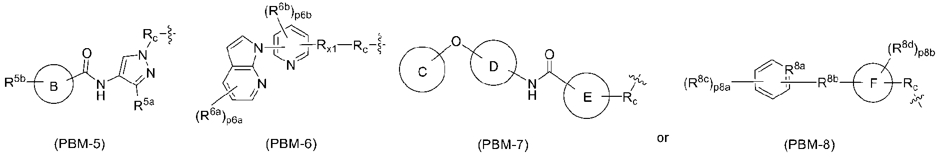
wherein (R^{1a})ₚ₁ₐ in Formula (PBM-1) indicates that the benzene ring to which it is attached is optionally substituted with p1a R^{1a} groups, wherein each R^{1a} is identical or different and each independently represents deuterium, halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
p1a represents an integer of 0, 1, 2, 3, 4, or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen;
(R^{1c})_{p1b} indicates that the quinazoline ring in Formula (PBM-1) is optionally substituted with p1b R^{1c} groups, wherein each R^{1c} is identical or different and each independently represents deuterium, halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R^{1e}, where R^{1e} represents C₁₋₁₀ alkyl, deuterated C₁₋₁₀ alkyl, or C₂₋₁₀ alkenyl, for example, R^{1c} represents methyl, methoxy, 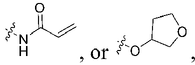 such as 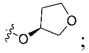
p1b represents an integer of 1, 2, or 3;
R^{1d} represents hydrogen, deuterium, or C₁₋₃ alkyl;
R^{2a} in Formula (PBM-2) represents -NHC(O)- or -C(O)NH-;
R^{2b} represents -NH-, -N(C₁₋₆ alkyl)-, or ethynylene;
ring A in Formula (PBM-2) represents a 5- to 20-membered monocyclic, bicyclic, or polycyclic heteroaryl group containing 1 to 4 heteroatoms selected from N, O, and S, and (R^{2c})ₚ₂ₐ indicates that the ring A is optionally substituted with p2a R^{2c}, wherein each R^{2c} is identical or different and each independently represents optionally substituted 5- to 15-membered heteroaryl, deuterium, halogen, hydroxy, cyano, amino, NO₂, C₁₋₆ alkyl, halogenated C₁₋₆ alkyl, deuterated C₁₋₆ alkyl, C₁₋₆ alkoxy, or halogenated C₁₋₆ alkoxy;
p2a represents an integer of 0, 1, 2, or 3;
each (R^{2d})_{p2b} in Formula (PBM-2) independently indicates that the benzene ring to which it is attached is independently optionally substituted with p2b R^{2d}, wherein each R^{2d} is identical or different and each independently represents deuterium, halogen, hydroxy, cyano, amino, NO₂, C₁₋₆ alkoxy, halogenated C₁₋₆ alkoxy, C₁₋₆ alkyl, deuterated C₁₋₆ alkyl or halogenated C₁₋₆ alkyl;
each p2b is identical or different and each independently represents an integer of 0, 1, 2, 3, or 4;
each (R^{3a})ₚ₃ₐ in Formula (PBM-3) independently indicates that the benzene ring to which it is attached is independently optionally substituted with p3a R^{3a}, wherein each R^{3a} is identical or different and each independently represents deuterium, halogen, hydroxy, cyano, amino, NO₂, C₁₋₆ alkoxy, halogenated C₁₋₆ alkoxy, C₁₋₆ alkyl, deuterated C₁₋₆ alkyl, or halogenated C₁₋₆ alkyl;
p3a represents an integer of 1, 2, 3, 4, or 5;
R^{3b} represents halogen, C₁₋₆ alkyl, deuterated C₁₋₆ alkyl, or halogenated C₁₋₆ alkyl;
X₁ in Formula (PBM-4) represents CR^{4e} or a fragment 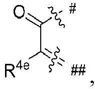 wherein symbol # indicates the point of attachment to N atom adjacent to X₁, symbol ## indicates the point of attachment to X₂, and each R^{4e} independently represents deuterium, hydrogen, halogen, C₁₋₆ alkyl, deuterated C₁₋₆ alkyl, halogenated C₁₋₆ alkyl, -C(O)-C₁₋₆ alkyl, -C(O)-deuterated C₁₋₆ alkyl, or -C(O)NR^{4f}R^{4g}, where R^{4f} and R^{4g} are identical or different and each independently represents hydrogen, C₁₋₆ alkyl, or deuterated C₁₋₆ alkyl, and R^{4f} and R^{4g} are not both hydrogen at the same time;
X₂ in Formula (PBM-4) represents N or CR^{4h}, wherein R^{4h} represents hydrogen, deuterium, halogen, C₁₋₆ alkyl, deuterated C₁₋₆ alkyl, or halogenated C₁₋₆ alkyl;
X₃ and X₄ each independently represent CH or N;
R^{4a} represents a bond, or optionally substituted heteroarylene (e.g., heteroarylene substituted with halogen or deuterium);
R^{4b} represents hydrogen, C₁₋₆ alkyl, halogenated C₁₋₆ alkyl, deuterated C₁₋₆ alkyl, or optionally substituted C₃₋₁₂ cycloalkyl;
(R^{4c})ₚ₄ₐ in Formula (PBM-4) indicates that the pyridine ring to which it is attached is optionally substituted with p4a R^{4c}, wherein each R^{4c} is identical or different and each independently represents deuterium, halogen, C₁₋₆ alkyl, deuterated C₁₋₆ alkyl, halogenated C₁₋₆ alkyl, C₁₋₆ alkoxy, or halogenated C₁₋₆ alkoxy, and p4a represents an integer of 0, 1, 2, or 3;
(R^{4d})_{p4b} in Formula (PBM-4) indicates that the nitrogen-containing bicyclic heteroaryl ring to which it is attached is optionally substituted with p4b R^{4d}, wherein each R^{4d} is identical or different and each independently represents deuterium, halogen, C₁₋₆ alkyl, deuterated C₁₋₆ alkyl, halogenated C₁₋₆ alkyl, C₁₋₆ alkoxy, or halogenated C₁₋₆ alkoxy, and p4b represents an integer of 0 or 1;
ring B in Formula (PBM-5) represents a 5- to 20-membered monocyclic, bicyclic, or polycyclic heteroarylene group containing 1 to 4 heteroatoms selected from N, O, and S;
R^{5b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{5a} represents hydrogen, halogen, C₁₋₆ alkyl, deuterated C₁₋₆ alkyl, halogenated C₁₋₆ alkyl, C₁₋₆ alkoxy, or halogenated C₁₋₆ alkoxy;
Rₓ₁ in Formula (PBM-6) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{6a})ₚ₆ₐ indicates that the 1H-pyrrolo[2,3-b]pyridine ring to which it is attached is optionally substituted with p6a R^{6a}, wherein each R^{6a} independently represents cyano, halogen, hydroxy, amino, nitro, C₁₋₆ alkyl, deuterated C₁₋₆ alkyl, halogenated C₁₋₆ alkyl, C₁₋₆ alkoxy, or halogenated C₁₋₆ alkoxy, and p6a represents an integer of 0, 1, 2, 3, 4, or 5;
(R^{6b})_{p6b} indicates that the pyridine ring to which it is attached is optionally substituted with p6b R^{6b}, wherein each R^{6b} independently represents cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy, or NR^{6c}R^{6d}, where R^{6c} and R^{6d} are identical or different and each independently represents hydrogen, C₁₋₆ alkyl, deuterated C₁₋₆ alkyl, halogenated C₁₋₆ alkyl, optionally substituted C₃₋₁₅ cycloalkyl, or optionally substituted 4- to 15-membered heterocyclyl, and p6b represents an integer of 0, 1, 2, or 3;
ring C in Formula (PBM-7) represents C₆₋₁₀ aryl or a 5- to 20-membered heteroaryl (e.g., a 5- to 20-membered heteroaryl comprising one or two 5- to 7-membered rings and 1 to 4 heteroatoms selected from N, O, and S), wherein said ring C is optionally substituted with one or more R^{7a}, and each R^{7a} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkenyl, or C₂₋₆ alkynyl;
ring D in Formula (PBM-7) represents C₃₋₁₅ cycloalkyl or 4- to 20-membered heterocyclyl (e.g., a 4- to 20-membered heterocyclyl comprising 1 to 4 heteroatoms selected from N, O, and S), wherein said ring D is optionally substituted with one or more R^{7b}, and each R^{7b} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, or halogenated C₁₋₆ alkoxy;
ring E in Formula (PBM-7) represents a 5- to 20-membered heteroaryl (e.g., a 5- to 20-membered monocyclic or bicyclic heteroaryl comprising 1 to 4 heteroatoms selected from N, O, and S), wherein said ring E is optionally substituted with one or more R^{7c}, and each R^{7c} is identical or different and each independently represents deuterium, halogen, hydroxy, cyano, amino, nitro, C₁₋₆ alkyl, deuterated C₁₋₆ alkyl, halogenated C₁₋₆ alkyl, C₁₋₆ alkoxy, or halogenated C₁₋₆ alkoxy;
R^{8a} in Formula (PBM-8) represents N or CH;
R^{8b} represents NHC(O), C(O)NH, NHS(O)₂, or S(O)₂NH;
(R^{8c})ₚ₈ₐ indicates that the 6-membered ring containing R^{8a} to which it is attached is substituted with p8a R^{8c}, wherein each R^{8c} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p8a represents an integer of 0, 1, 2, 3, or 4;
ring F represents 5- to 15-membered heteroarylene, and (R^{8d})_{p8b} indicates that the ring F to which it is attached is optionally substituted with p8b R^{8d}, and each R^{8d} is identical or different and each independently represents halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy, or optionally substituted C₃₋₁₅ cycloalkyl, and p8b represents an integer of 0, 1, 2, or 3; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -R_{f}-C(O)O-*, -R_{f}-OC(O)-*, -C(O)O-*, -OC(O)-*, -R_{f}-, -R_{f}-N(R_{d})-*, -O-R_{f}-N(R_{d})-*, 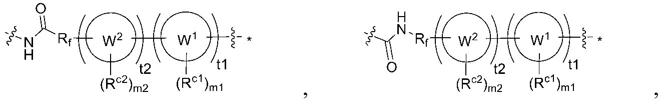 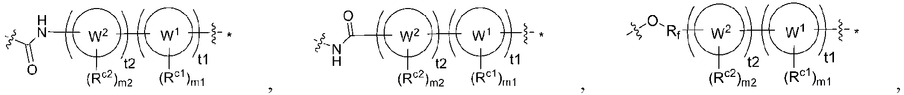 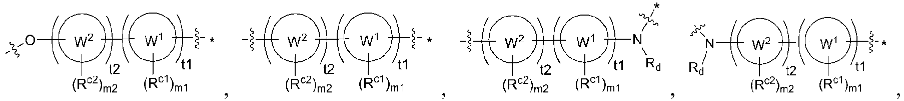 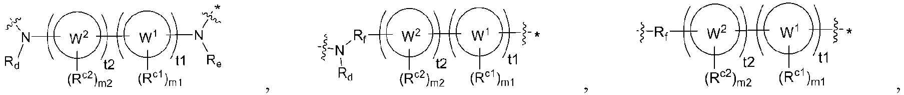 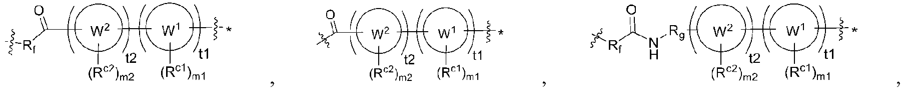 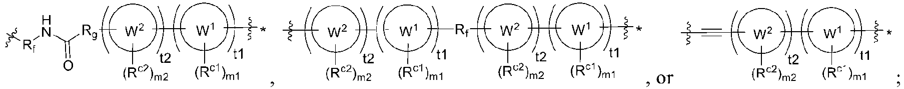
wherein each ring W¹ is identical or different and each independently represents nitrogen-containing heterocyclylene, cycloalkylene, arylene, or heteroarylene, t1 represents an integer of 0, 1, or 2, and (R^{c1})ₘ₁ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, R^{c4} represents halogen, R^{c5}R^{c6}N-, hydroxy, carboxyl, ethynyl, or vinyl, wherein R^{c5} and R^{c6} are identical or different and each independently represents hydrogen or C₁₋₃ alkyl, and m1 represents an integer of 0-20;
each ring W² is identical or different and each independently represents nitrogen-containing heterocyclylene, cycloalkylene, arylene, or heteroarylene, t2 represents an integer of 0 or 1, and (R^{c2})ₘ₂ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} represents optionally substituted C₁₋₆ alkylene or optionally substituted C₂₋₆ alkenylene, R^{c8} represents halogen, R^{c9}R^{c10}N-, hydroxy, carboxyl, ethynyl, or vinyl, wherein R^{c9} and R^{c10} are identical or different and each independently represents hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene; and symbol * indicates the point of attachment to LIN.

4. The compound of Formula (I) or salts, enantiomers, stereoisomers, solvates, isotopically enriched analogs, prodrugs, or polymorphs thereof as claimed in claim 3, wherein the PBM represents a structure of the following formula: 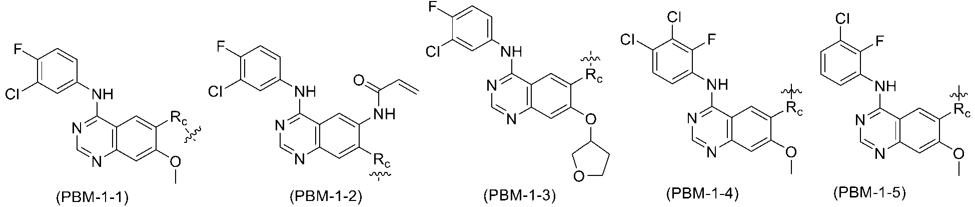 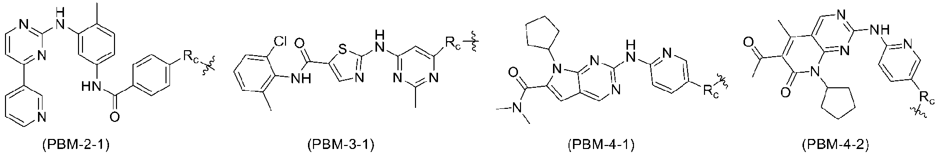 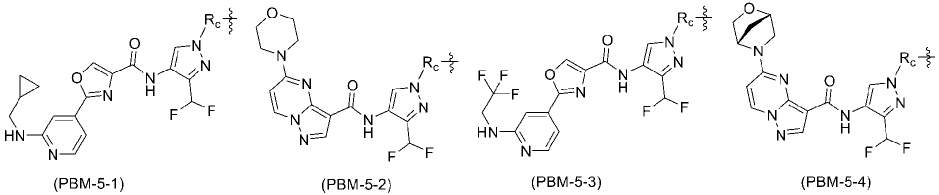 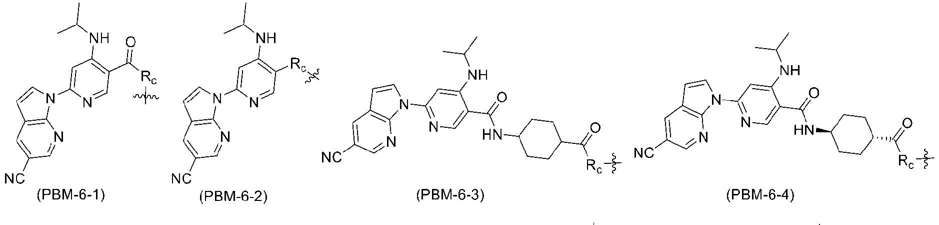 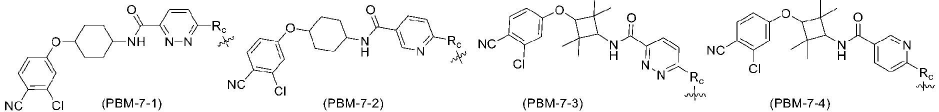 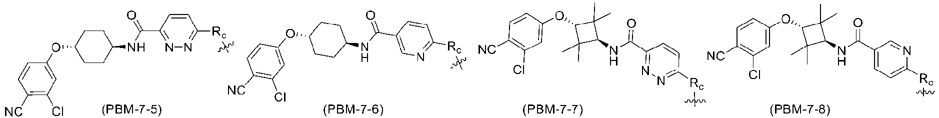 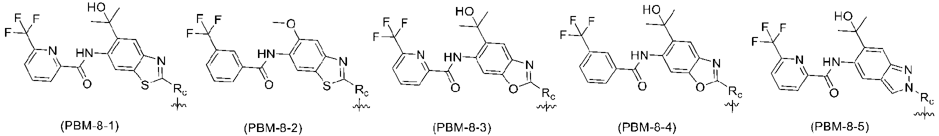 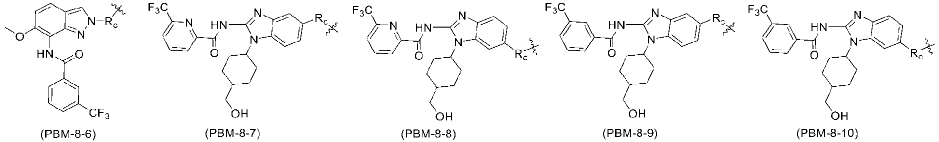 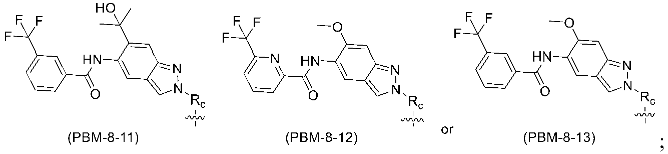 wherein R_{c} is as defined in claim 3.

5. The compound of Formula (I) or salts, enantiomers, stereoisomers, solvates, isotopically enriched analogs, prodrugs, or polymorphs thereof as claimed in claim 3, wherein
each R_{c} independently represents a bond, -O-, -NHC(O)-*, -C(O)NH-*, -C(O)O-*, -OC(O)-*, or -NH-, or
each R_{c} independently represents a structure of the following formula:
-N(R_{d})-, -N(R_{d})-R_{f}-N(Rₑ)-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 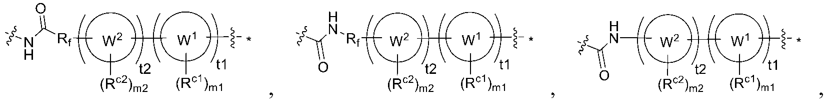 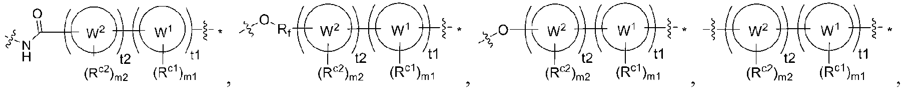 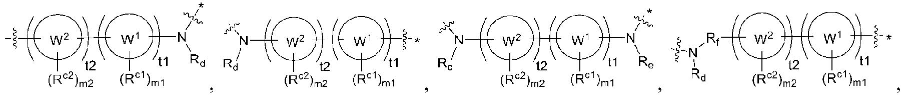 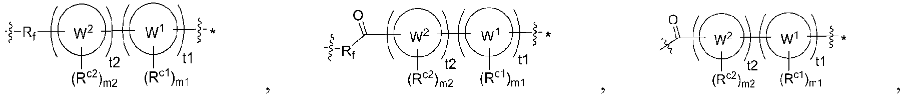 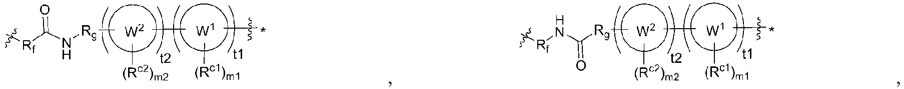 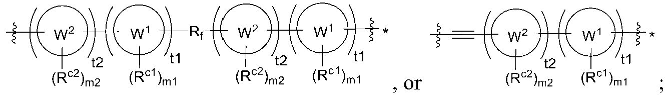
wherein each ring W¹ is identical or different and each independently represents 4- to 20-membered nitrogen-containing heterocyclylene, C₃₋₂₀ cycloalkylene, C₅₋₂₀ arylene, or 5- to 20-membered heteroarylene, t1 represents an integer of 0, 1, or 2, and (R^{c1})ₘ₁ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, R^{c4} represents halogen, R^{c5}R^{c6}N-, hydroxy, carboxyl, ethynyl, or vinyl, wherein R^{c5} and R^{c6} are identical or different and each independently represents hydrogen or C₁₋₃ alkyl, and m1 represents an integer of 0-20;
each ring W² is identical or different and each independently represents 4- to 20-membered nitrogen-containing heterocyclylene, C₃₋₂₀ cycloalkylene, C₅₋₂₀ arylene, or 5- to 20-membered heteroarylene, t2 represents an integer of 0 or 1, and (R^{c2})ₘ₂ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} represents optionally substituted C₁₋₆ alkylene, or optionally substituted C₂₋₆ alkenylene, R^{c8} represents halogen, R^{c9}R^{c10}N-, hydroxy, carboxyl, ethynyl, or vinyl, wherein R^{c9} and R^{c10} are identical or different and each independently represents hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents optionally selected from the group consisting of: deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and
symbol * indicates the point of attachment to LIN.

6. The compound of Formula (I) or salts, enantiomers, stereoisomers, solvates, isotopically enriched analogs, prodrugs, or polymorphs thereof as claimed in any one of claims 3-5, wherein
each ring W¹ is identical or different and each independently represents 4- to 15-membered nitrogen-containing heterocyclylene, C₃₋₁₅ cycloalkylene, 6- to 15-membered arylene, or 5- to 15-membered heteroarylene, such as
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxo-8-azaspiro[4.5]decanylene, 3-methyl-2-oxo-8-azaspiro[4.5]decanylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 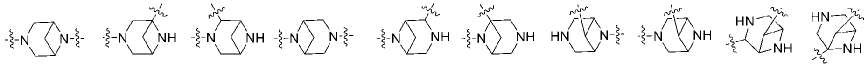 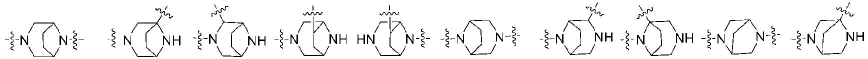 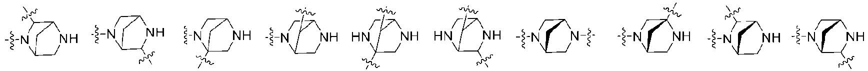 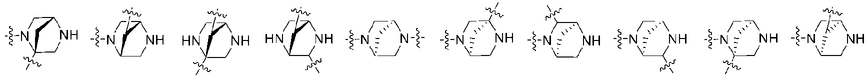 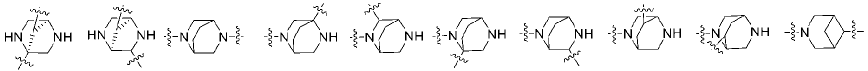 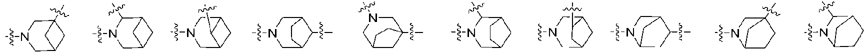 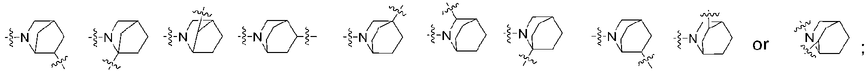
wherein each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, R^{c4} represents halogen, R^{c5}R^{c6}N-, hydroxy, carboxyl, ethynyl, or vinyl, wherein R^{c5} and R^{c6} are identical or different and each independently represents hydrogen or C₁₋₃ alkyl, and said C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy, and m1 represents an integer of 0-20; and/or
each ring W² is identical or different and each independently represents 4- to 15-membered nitrogen-containing heterocyclylene, C₃₋₁₅ cycloalkylene, 6- to 15-membered arylene, or 5- to 15-membered heteroarylene, such as,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxo-8-azaspiro[4.5]decanylene, 3-methyl-2-oxo-8-azaspiro[4.5]decanylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 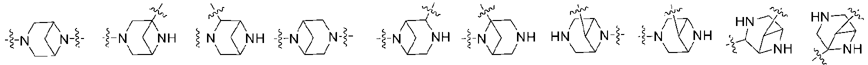 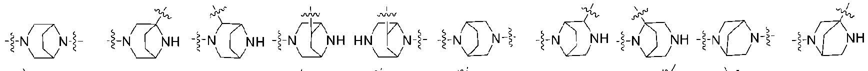 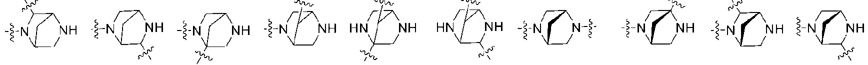 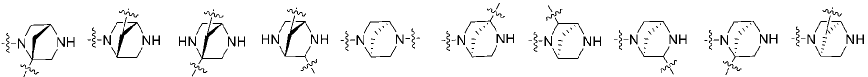 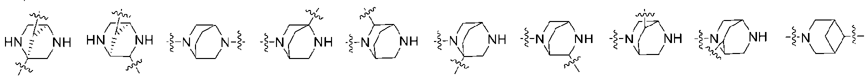 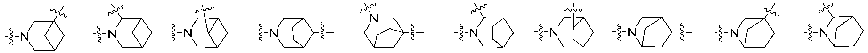 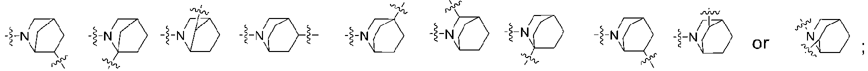
wherein each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represents hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy, and m2 represents an integer of 0-20.

7. The compound of Formula (I) or salts, enantiomers, stereoisomers, solvates, isotopically enriched analogs, prodrugs, or polymorphs thereof as claimed in any one of claims 3-5, wherein the 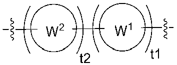 moiety of R_{c} in each general formula represents the following groups: 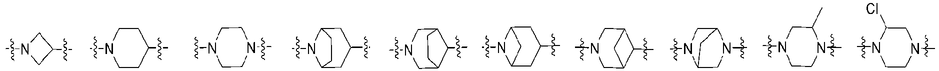 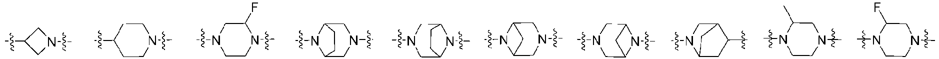 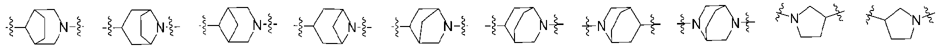 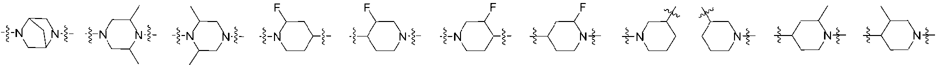 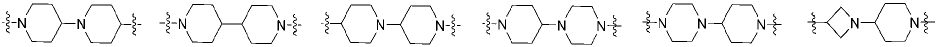 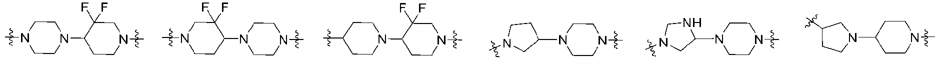 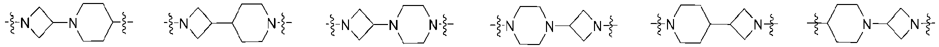 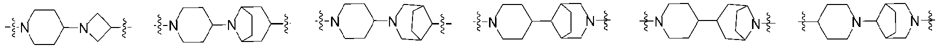 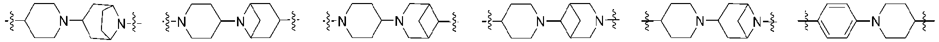 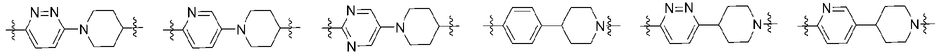 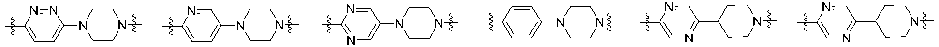 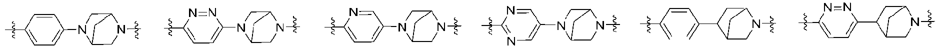 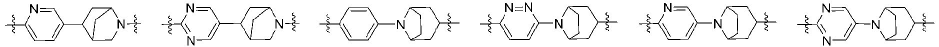 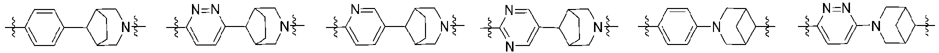 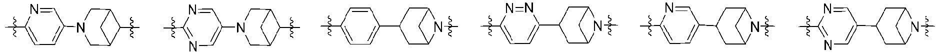 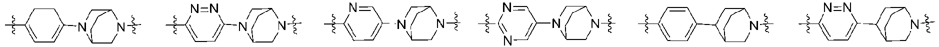 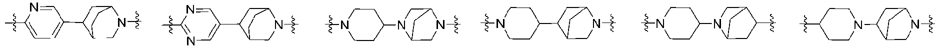 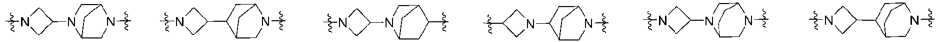 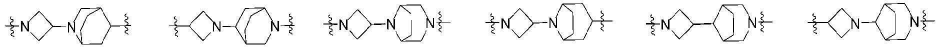 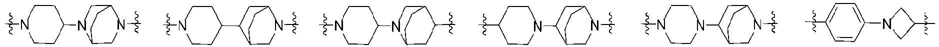 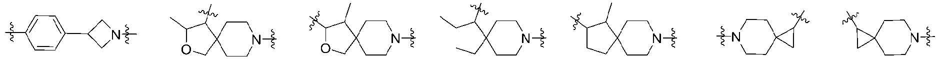 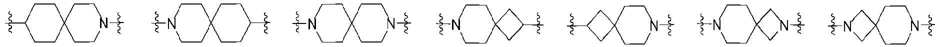 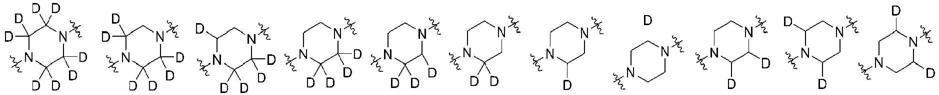 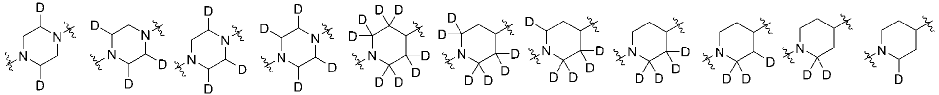 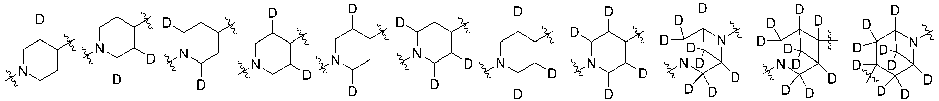 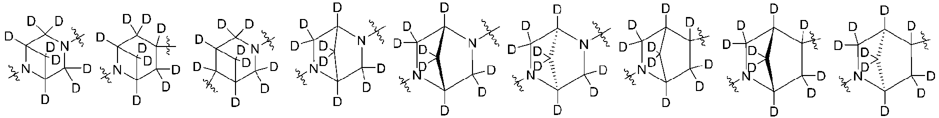 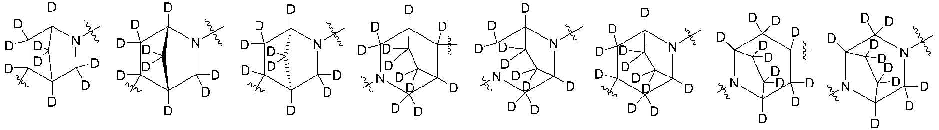 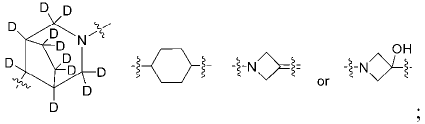 wherein the groups are each independently optionally substituted with one or more substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, R^{c12}-R^{c11}, and any combination thereof, wherein R^{c11} represents optionally substituted C₁₋₆ alkylene or optionally substituted C₂₋₆ alkenylene, R^{c12} represents halogen, R^{c13}R^{c14}N-, hydroxy, carboxyl, ethynyl, or vinyl, wherein R^{c13} and R^{c14} are identical or different and each independently represents hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy.

8. The compound of Formula (I) or salts, enantiomers, stereoisomers, solvates, isotopically enriched analogs, prodrugs, or polymorphs thereof as claimed in any one of claims 3-5, wherein the R_{c} represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -NH-CH₂-*, -N(CH₃)-CH₂-*, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 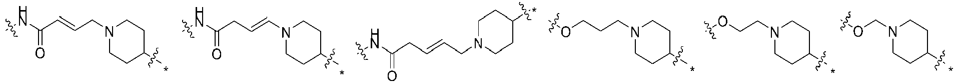 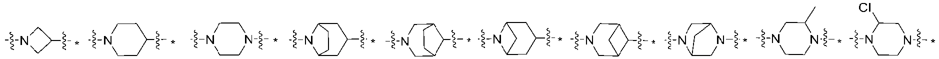 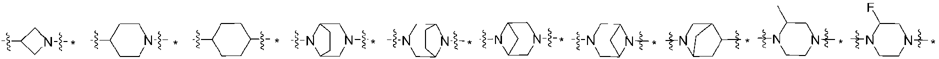 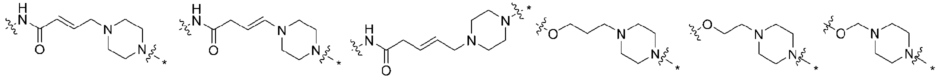 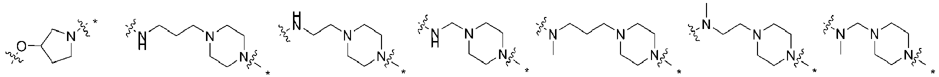 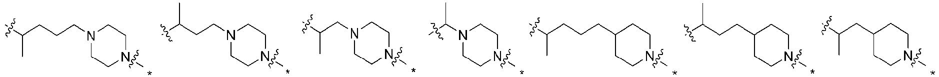 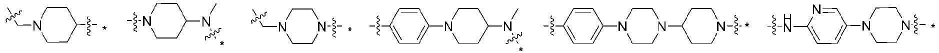 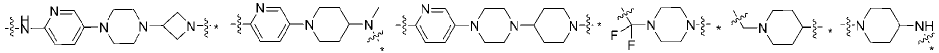 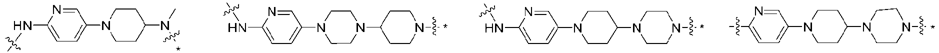 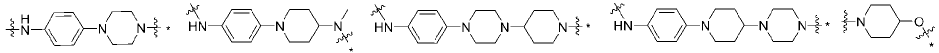 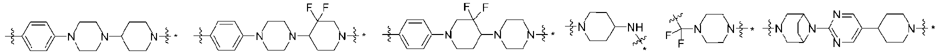 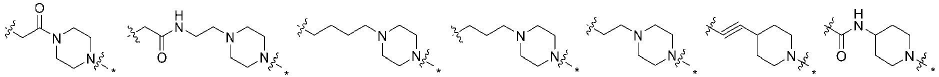 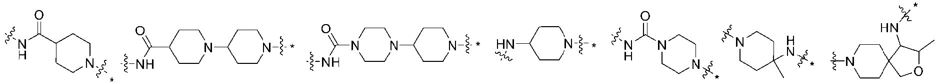 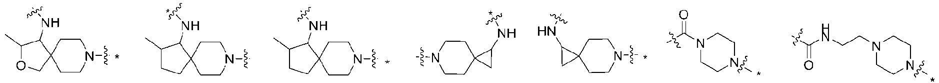 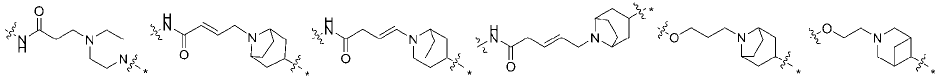 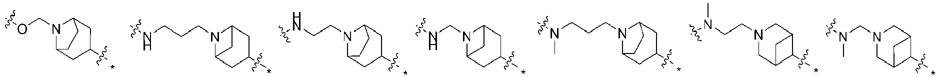 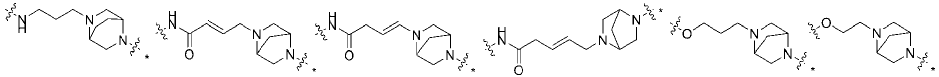 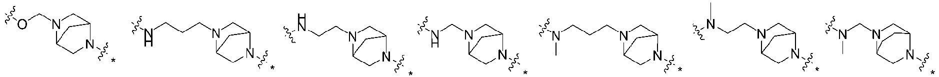 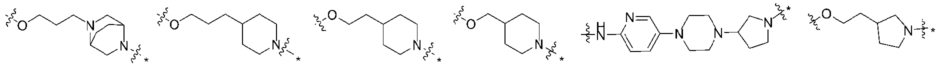 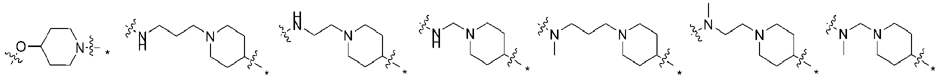 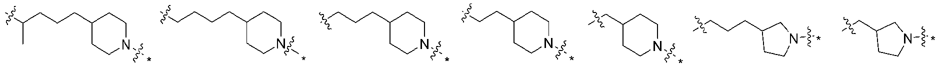 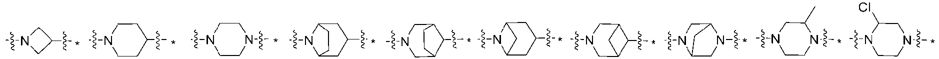 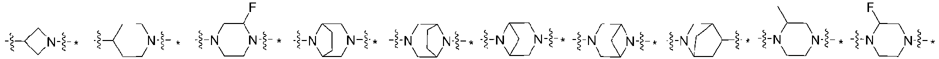 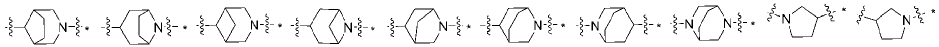 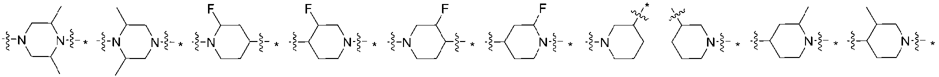 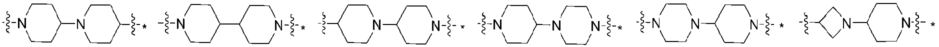 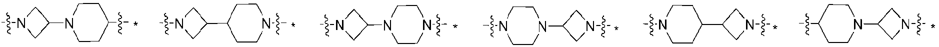 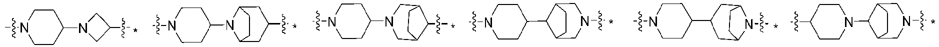 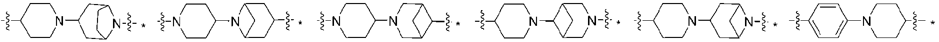 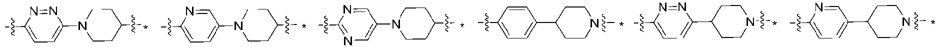 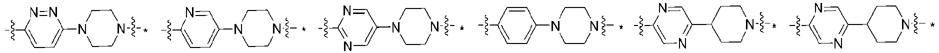 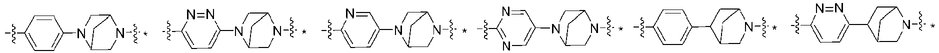 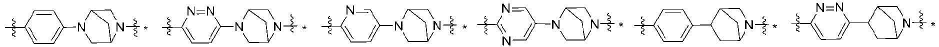 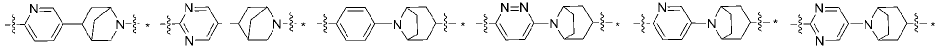 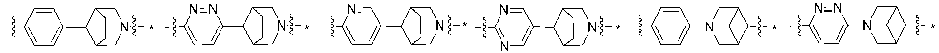 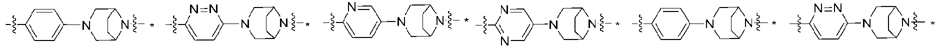 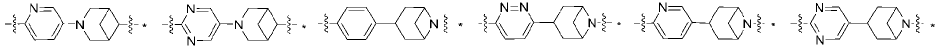 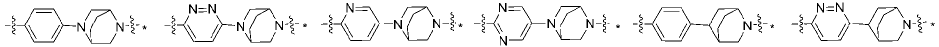 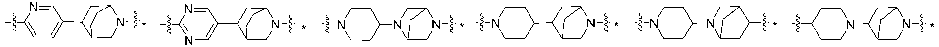 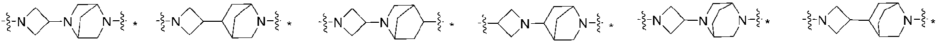 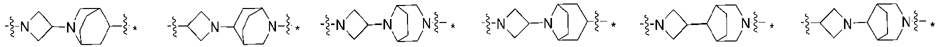 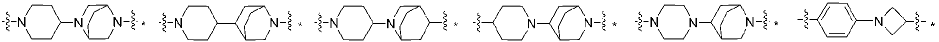 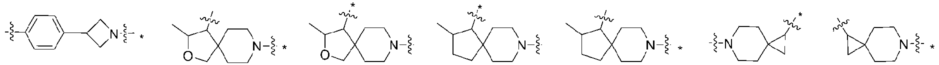 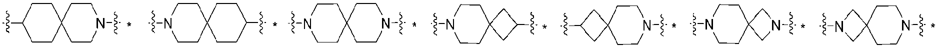 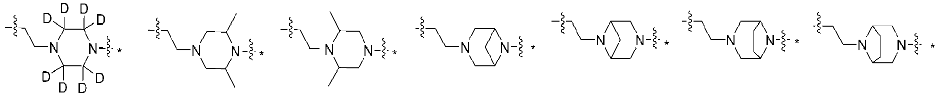 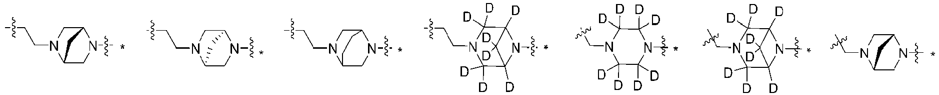 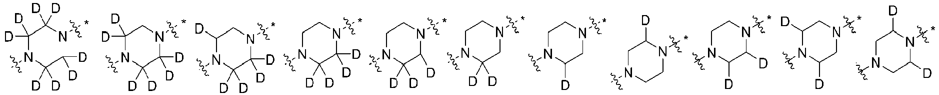 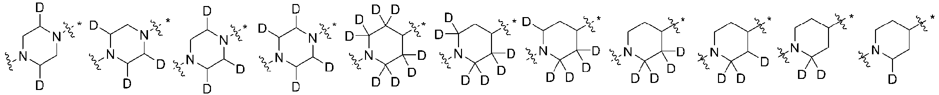 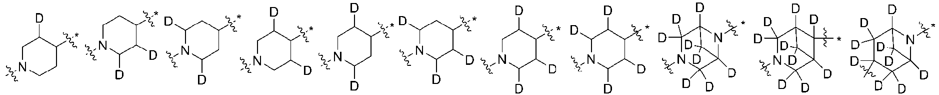 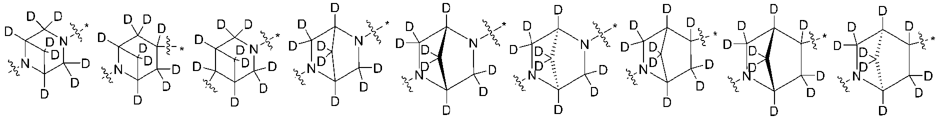 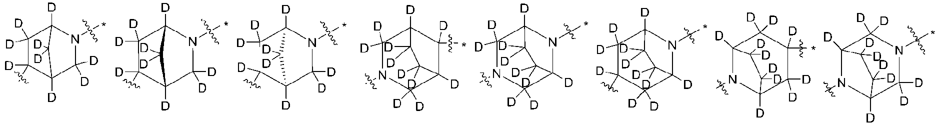 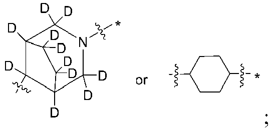 wherein the groups are each independently optionally substituted with one or more substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, C₂₋₆ alkynyl, C₂₋₆ alkenyl, optionally deuterated C₃₋₆ cycloalkyl, R^{c12}-R^{c11}-, and any combination thereof, wherein R^{c11} represents optionally substituted C₁₋₆ alkylene or optionally substituted C₂₋₆ alkenylene, R^{c12} represents halogen, R^{c13}R^{c14}N-, hydroxy, carboxyl, ethynyl, or vinyl, wherein R^{c13} and R^{c14} are identical or different and each independently represents hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and symbol * indicates the point of attachment to LIN.

9. The compound of Formula (I) or salts, enantiomers, stereoisomers, solvates, isotopically enriched analogs, prodrugs, or polymorphs thereof as claimed in any one of claims 1-5, wherein the PBM represents a structure of the following Formula (PBM-1-1-1), Formula (PBM-1-1-2), Formula (PBM-2-1-1), Formula (PBM-3-1-1), Formula (PBM-4-1-1), Formula (PBM-4-2-1), Formula (PBM-5-2-1), Formula (PBM-5-4-1), Formula (PBM-6-4-1), Formula (PBM-7-1-1), Formula (PBM-7-4-1), Formula (PBM-8-12-1), Formula (PBM-8-12-2), or Formula (PBM-8-13-1):

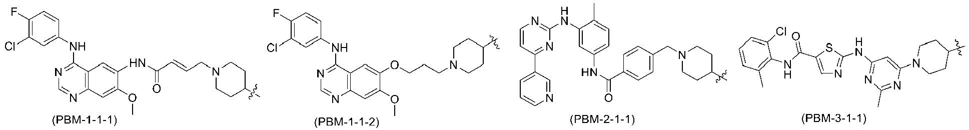

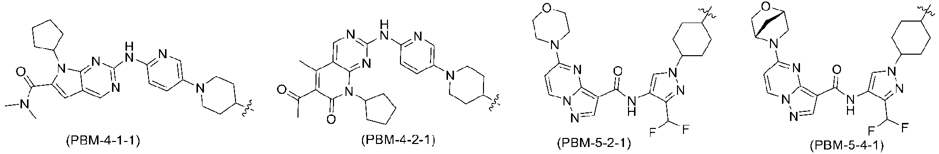

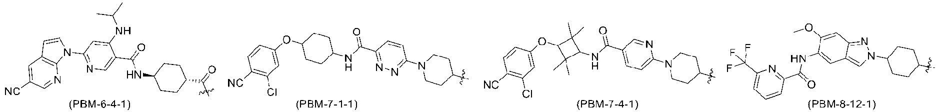

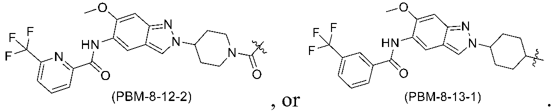

10. The compound of Formula (I) or salts, enantiomers, stereoisomers, solvates, isotopically enriched analogs, prodrugs, or polymorphs thereof as claimed in any one of claims 1-9, wherein the ULM represents a structure of the following Formula (ULM-1-1), Formula (ULM-1-2), Formula (ULM-1-3), or Formula (ULM-1-4): 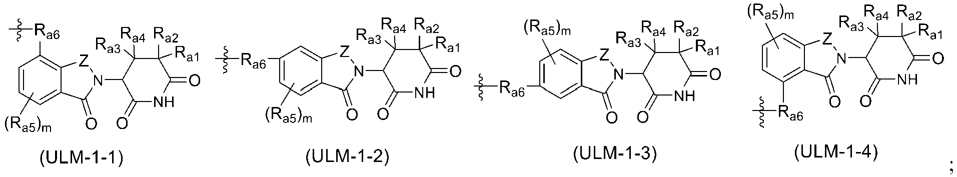 wherein Z, Rₐ₁, Rₐ₂, Rₐ₃, Rₐ₄, (Rₐ₅)ₘ, and Rₐ₆ are as defined in claim 1.

11. The compound of Formula (I) or salts, enantiomers, stereoisomers, solvates, isotopically enriched analogs, prodrugs, or polymorphs thereof as claimed in any one of claims 1-9, wherein the ULM represents a structure of the following Formula (ULM-2-1), Formula (ULM-2-2), Formula (ULM-2-3), or Formula (ULM-2-4): 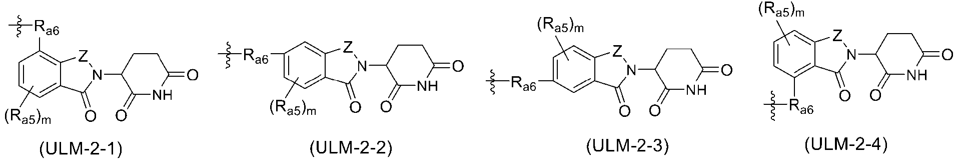 wherein Z, (Rₐ₅)ₘ, and Rₐ₆ are as defined in claim 1.

12. The compound of Formula (I) or salts, enantiomers, stereoisomers, solvates, isotopically enriched analogs, prodrugs, or polymorphs thereof as claimed in any one of claims 1-11, wherein
ring Y¹ represents 4- to 20-membered heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; or
ring Y² represents C₃₋₂₀ cycloalkylene, or 4- to 20-membered heterocyclylene containing at least one nitrogen atom, and (R_{d2})ₙ₂ indicates that ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n2 represents an integer of 0-20; or
ring Y³ represents 4- to 20-membered heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20.

13. The compound of Formula (I) or salts, enantiomers, stereoisomers, solvates, isotopically enriched analogs, prodrugs, or polymorphs thereof as claimed in any one of claims 1-12, wherein
(i) ring Y¹ represents 4- to 20-membered heterocyclylene containing 2 to 4 nitrogen atoms; e.g., ring Y¹ represents:
diazetidinylene, imidazolidylene, pyrazolidylene, piperazinylene, 1,2,3,4-tetrahydropyrazinylene, hexahydropyrimidinylene, hexahydropyridazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, diazaspiro[2.3]hexylene, diazaspiro [3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydropyrrolo[3,4-c]pyrrolylene, or decahydronaphthyridinylene, or 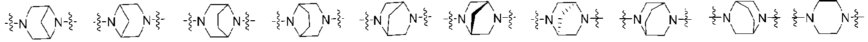 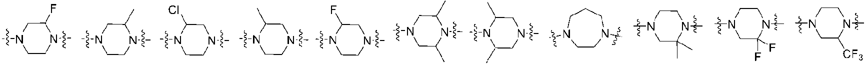 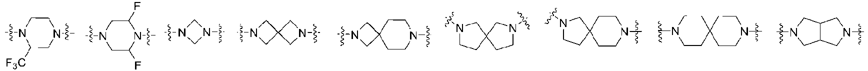 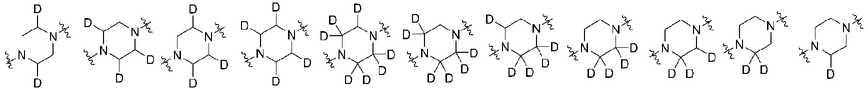 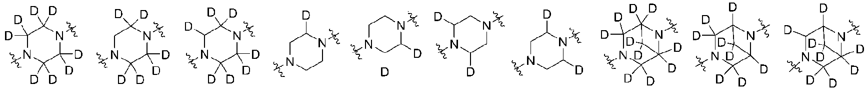 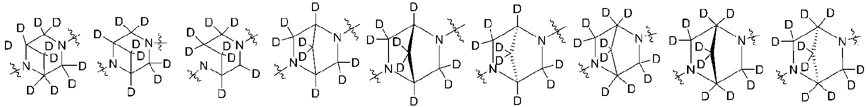 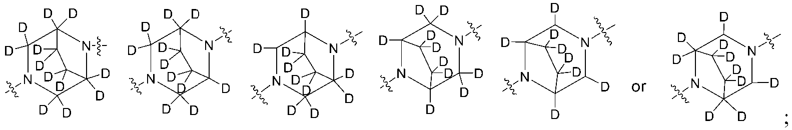
ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy;
n1 represents an integer of 0-20; or
(ii) ring Y² represents C₃₋₂₀ cycloalkylene, or 4- to 20-membered heterocyclylene containing 1 to 4 nitrogen atoms; e.g., ring Y² represents:
cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, C₅₋₁₅ bridged cycloalkylene (e.g., adamantanylene, noradamantanylene, and norbornylene), azetidinylene, diazetidinylene, pyrrolidinylene, imidazolidylene, pyrazolidylene, piperidinylene, tetrahydropyridylene, piperazinylene, 1,2,3,4-tetrahydropyrazinylene, hexahydropyrimidinylene, hexahydropyridazinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, azaspiro[2.3]hexylene, azaspiro[3.3]heptanylene, azaspiro[2.4]heptanylene, azaspiro[3.4]octanylene, azaspiro[3.5]nonanylene, azaspiro[4.4]nonanylene, azaspiro[4.5]decanylene, azaspiro[5.5]undecanylene, diazaspiro[2.3]hexylene, diazaspiro [3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydrocyclopenta[c]pyrrolylene, octahydropyrrolo[3,4-c]pyrrolylene, or decahydronaphthyridinylene, or 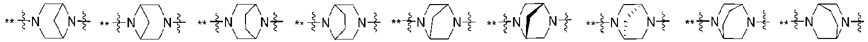 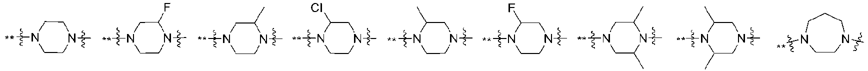 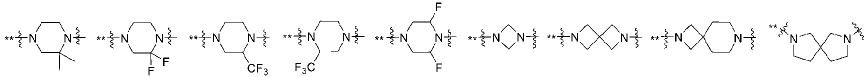 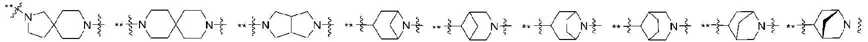 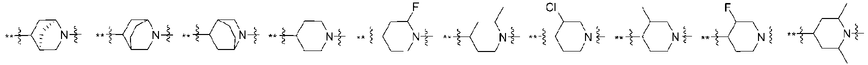 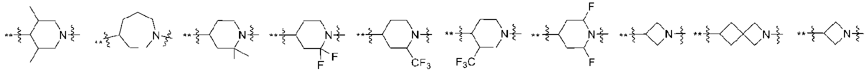 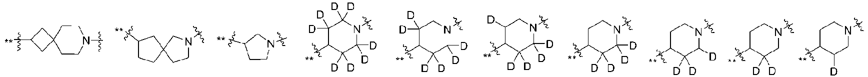 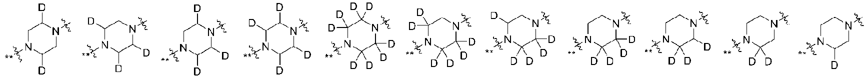 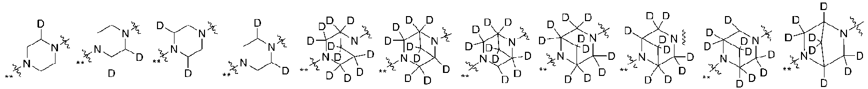 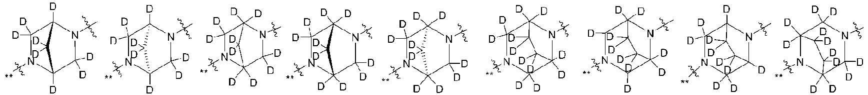 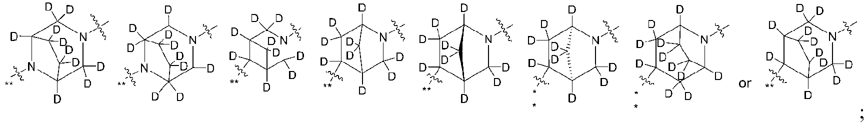
ring Y² is optionally substituted with n2 R_{d2} groups, wherein each R_{d2} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n2 represents an integer of 0-20; or
(iii) ring Y³ represents 4- to 20-membered heterocyclylene containing one nitrogen atom; e.g., ring Y³ represents:
azetidinylene, pyrrolidinylene, piperidinylene, tetrahydropyridylene, azepanylene, azacyclooctylene, azabicyclo[3.1.1]heptanylene, azabicyclo[2.2.1]heptanylene, azabicyclo[3.2.1]octanylene, azabicyclo[2.2.2]octanylene, azaspiro[2.3]hexylene, azaspiro[3.3]heptanylene, azaspiro[2.4]heptanylene, azaspiro[3.4]octanylene, azaspiro[3.5]nonanylene, azaspiro[4.4]nonanylene, azaspiro[4.5]decanylene, azaspiro[5.5]undecanylene, octahydrocyclopenta[c]pyrrolylene, or octahydropyrrolo[3,4-c]pyrrolylene, or 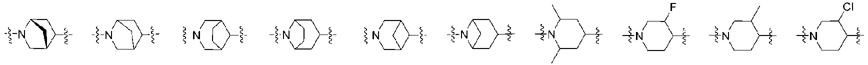 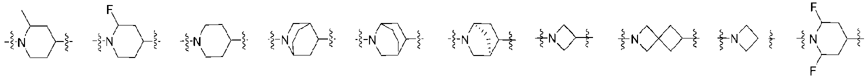 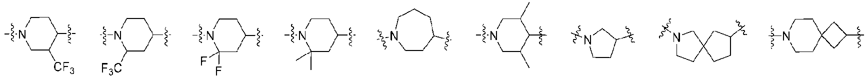 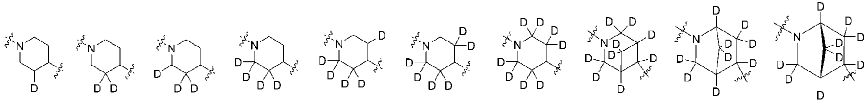 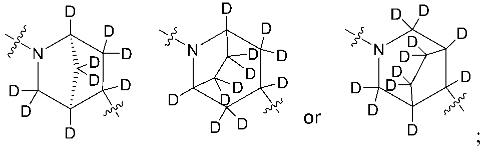
the ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy; and
n3 represents an integer of 0-20.

14. The compound of Formula (I) or salts, enantiomers, stereoisomers, solvates, isotopically enriched analogs, prodrugs, or polymorphs thereof as claimed in any one of claims 1-13, wherein
(i) each ring A¹ is identical or different and each independently represents 4- to 20-membered heterocyclylene, C₃₋₂₀ cycloalkylene, C₅₋₂₀ arylene, or 5- to 20-membered heteroarylene, e.g., each ring A¹ independently represents:
azetidinylene, diazetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, tetrahydropyridylene, piperazinylene, 1,2,3,4-tetrahydropyrazinylene, hexahydropyrimidinylene, hexahydropyridazinylene, morpholinylene, thiomorpholinylene, azacycloheptanylene, azacyclooctylene, dioxacyclohexylene, azepanylene, azacyclooctylene, diazacycloheptanylene, diazacyclooctylene, azabicyclo[3.1.1]heptanylene (e.g., 6-azabicyclo[3.1.1]heptanylene), azabicyclo[2.2.1]heptanylene, azabicyclo[3.2.1]octanylene (e.g., 3-azabicyclo[3.2.1]octanylene), azabicyclo[2.2.2]octanylene, diazabicyclo[3.1.1]heptanylene (e.g., 3,6-diazabicyclo[3.1.1]heptanylene), diazabicyclo[2.2.1]heptanylene (e.g., 2,5-diazabicyclo[2.2.1]heptanylene), diazabicyclo[3.2.1]octanylene (e.g., 3,8-diazabicyclo[3.2.1]octanylene), diazabicyclo[2.2.2]octanylene (e.g., 2,5-diazabicyclo[2.2.2]octanylene), 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 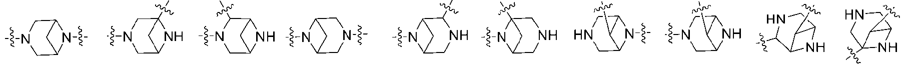 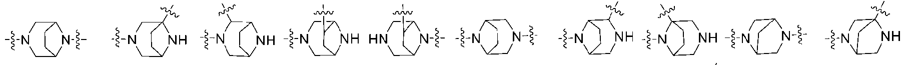 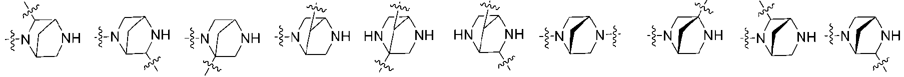 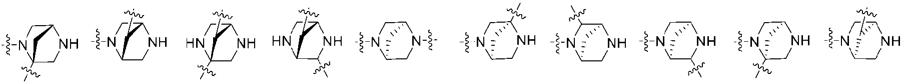 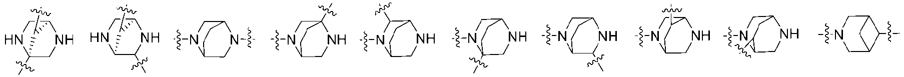 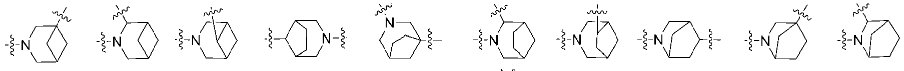 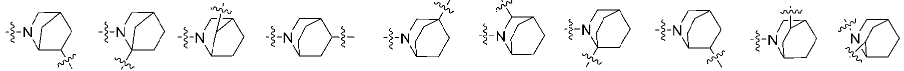 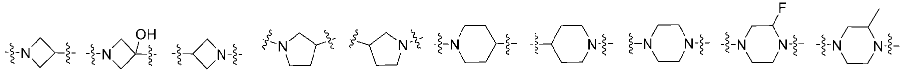 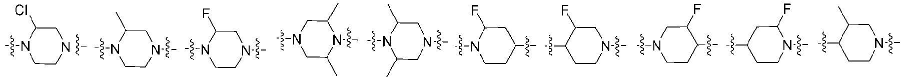 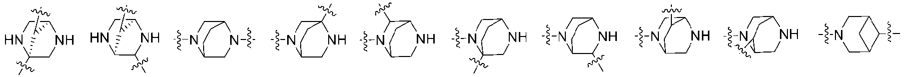 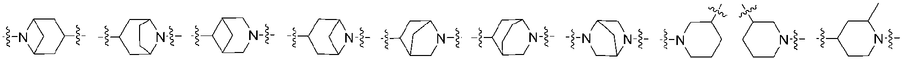 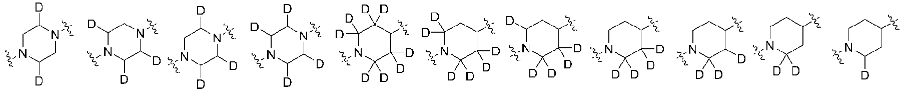 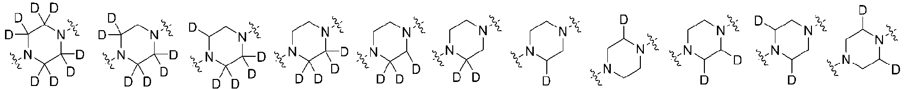 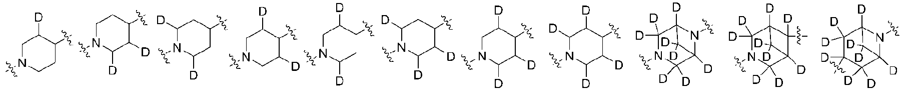 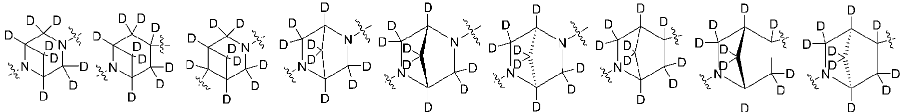 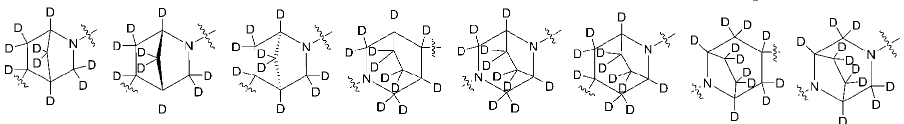 or 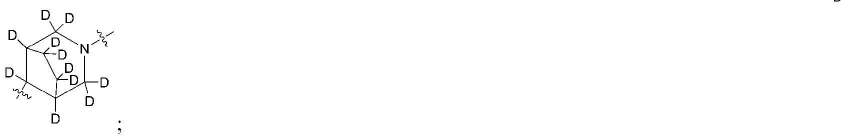 y1 represents an integer of 0, 1, 2, or 3, and (R^{y1})ₐ₁ indicates that each ring A¹ is independently optionally substituted with a1 R^{y1} groups, wherein each R^{y1} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a1 represents an integer of 0-10; and/or
(ii) each ring A² is identical or different and each independently represents 4- to 20-membered heterocyclylene, C₃₋₂₀ cycloalkylene, C₅₋₂₀ arylene, or 5- to 20-membered heteroarylene; e.g., each ring A² independently represents:
azetidinylene, diazetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, tetrahydropyridylene, piperazinylene, 1,2,3,4-tetrahydropyrazinylene, hexahydropyrimidinylene, hexahydropyridazinylene, morpholinylene, thiomorpholinylene, azepanylene, azacyclooctylene, dioxacyclohexylene, azepanylene, azacyclooctylene, diazacycloheptanylene, diazacyclooctylene, azabicyclo[3.1.1]heptanylene (e.g., 6-azabicyclo[3.1.1]heptanylene), azabicyclo[2.2.1]heptanylene, azabicyclo[3.2.1]octanylene (e.g., 3-azabicyclo[3.2.1]octanylene), azabicyclo[2.2.2]octanylene, diazabicyclo[3.1.1]heptanylene (e.g., 3,6-diazabicyclo[3.1.1]heptanylene), diazabicyclo[2.2.1]heptanylene (e.g., 2,5-diazabicyclo[2.2.1]heptanylene), diazabicyclo[3.2.1]octanylene (e.g., 3,8-diazabicyclo[3.2.1]octanylene), diazabicyclo[2.2.2]octanylene (e.g., 2,5-diazabicyclo[2.2.2]octanylene), 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 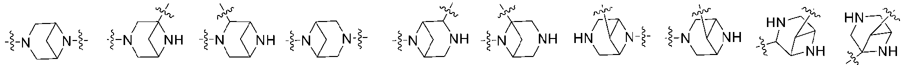 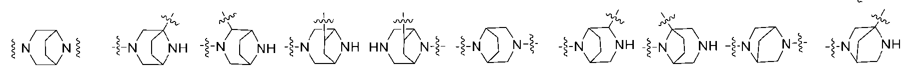 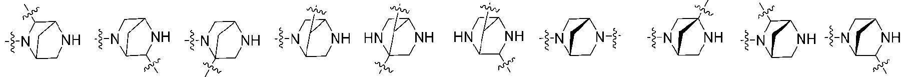 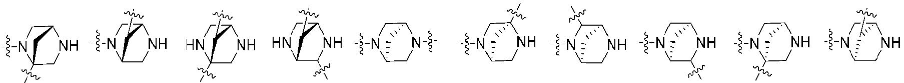 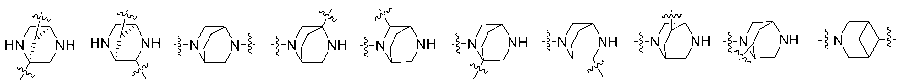 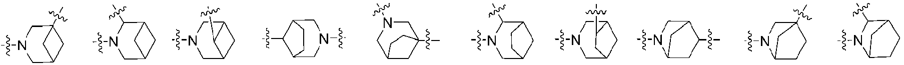 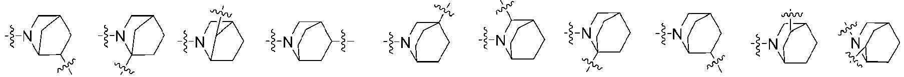 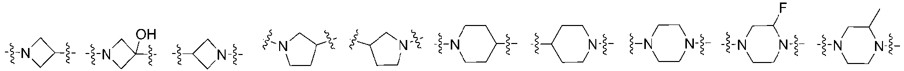 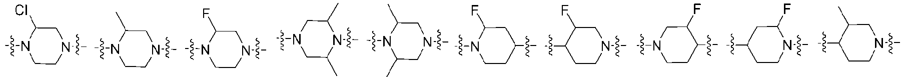 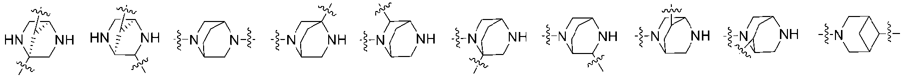 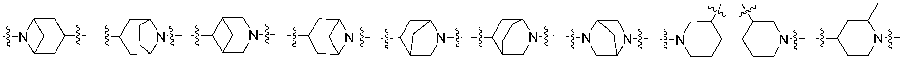 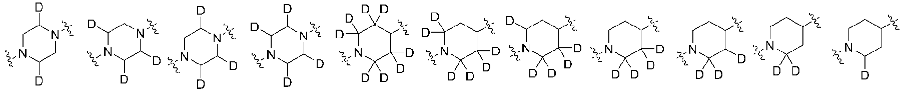 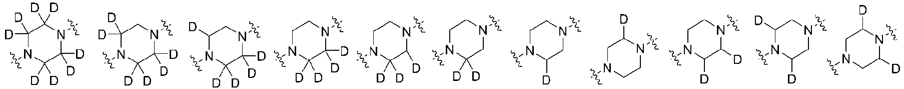 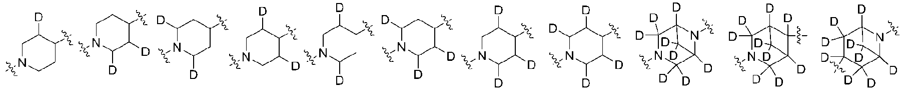 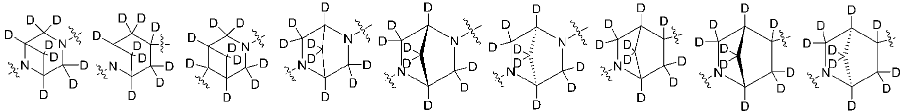 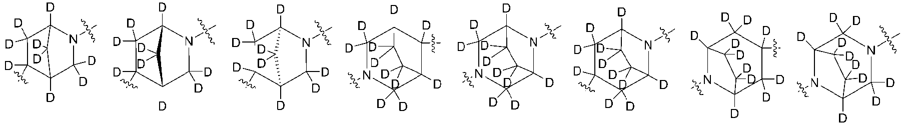 or 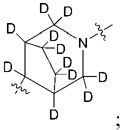 y2 represents an integer of 0, 1, 2, or 3, and (R^{y2})ₐ₂ indicates that each ring A² is independently optionally substituted with a2 R^{y2} groups, wherein each R^{y2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a2 represents an integer of 0-10; and/or
(iii) each ring A³ is identical or different and each independently represents 4- to 20-membered heterocyclylene, C₃₋₂₀ cycloalkylene, C₅₋₂₀ arylene, or 5- to 20-membered heteroarylene; e.g., each ring A³ independently represents:
azetidinylene, diazetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, tetrahydropyridylene, piperazinylene, 1,2,3,4-tetrahydropyrazinylene, hexahydropyrimidinylene, hexahydropyridazinylene, morpholinylene, thiomorpholinylene, azepanylene, azacyclooctylene, dioxacyclohexylene, azepanylene, azacyclooctylene, diazacycloheptanylene, diazacyclooctylene, azabicyclo[3.1.1]heptanylene (e.g., 6-azabicyclo[3.1.1]heptanylene), azabicyclo[2.2.1]heptanylene, azabicyclo[3.2.1]octanylene (e.g., 3-azabicyclo[3.2.1]octanylene), azabicyclo[2.2.2]octanylene, diazabicyclo[3.1.1]heptanylene (e.g., 3,6-diazabicyclo[3.1.1]heptanylene), diazabicyclo[2.2.1]heptanylene (e.g., 2,5-diazabicyclo[2.2.1]heptanylene), diazabicyclo[3.2.1]octanylene (e.g., 3,8-diazabicyclo[3.2.1]octanylene), diazabicyclo[2.2.2]octanylene (e.g., 2,5-diazabicyclo[2.2.2]octanylene), 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 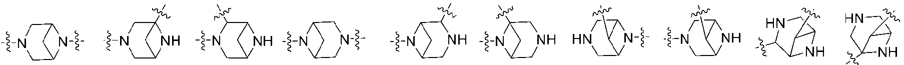 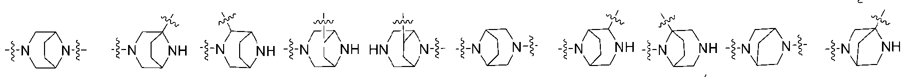 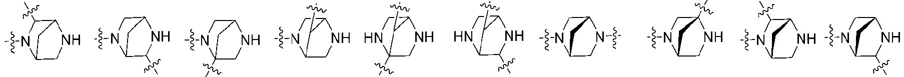 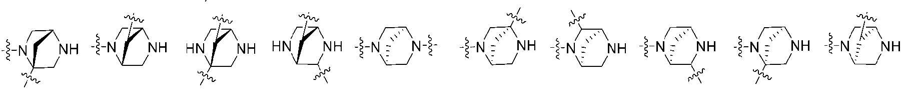 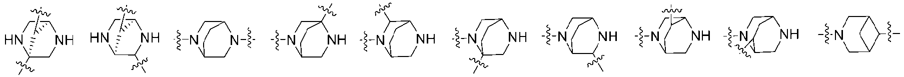 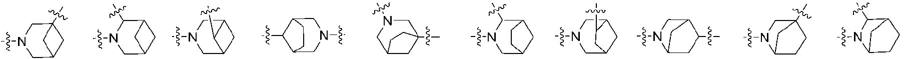 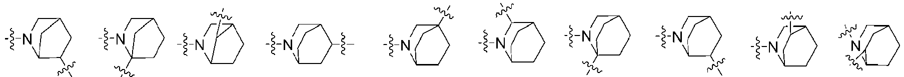 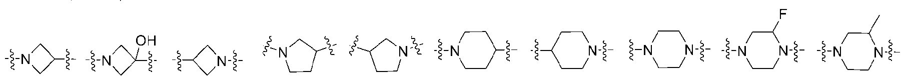 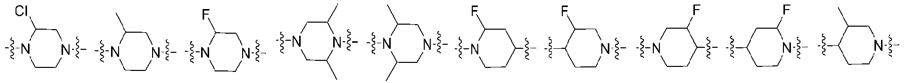 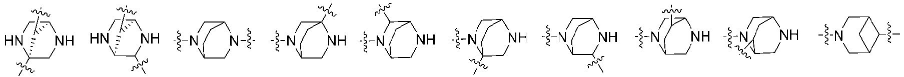 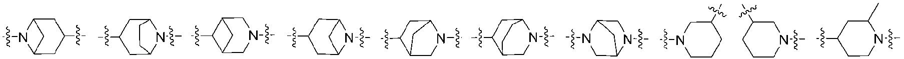 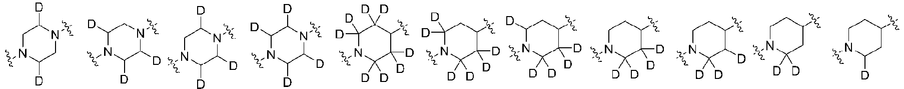 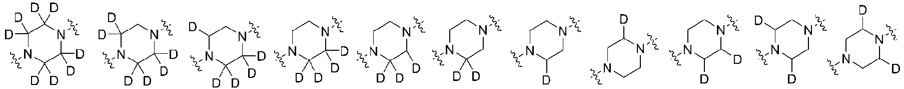 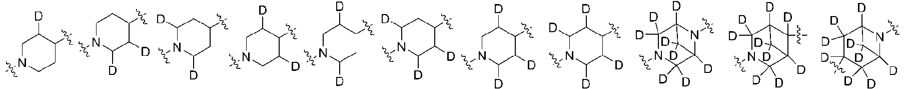 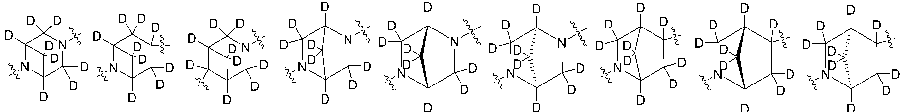 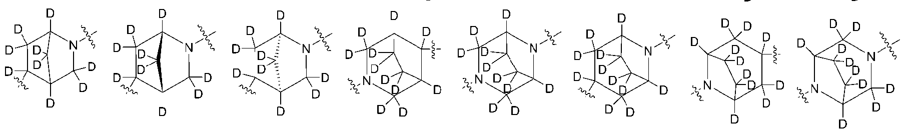 or 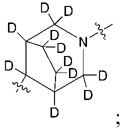 y3 represents an integer of 0, 1, 2, or 3, and (R^{y3})ₐ₃ indicates that each ring A³ is independently optionally substituted with a3 R^{y3} groups, wherein each R^{y3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, or C₂₋₆ alkenyl, and a3 represents an integer of 0-10; and/or
(iv) R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and wherein one or more hydrogens of said linear or branched C₁₋₂₀ alkylene are optionally replaced by a substituent selected from the group consisting of deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

15. The compound of Formula (I) or salts, enantiomers, stereoisomers, solvates, isotopically enriched analogs, prodrugs, or polymorphs thereof as claimed in any one of claims 1-14, wherein the 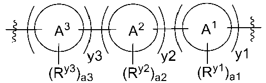 moiety in the general formula of LIN represents: 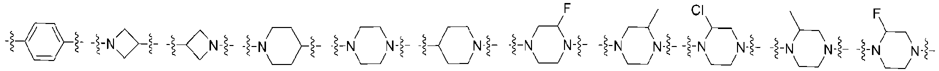 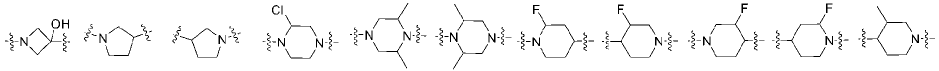 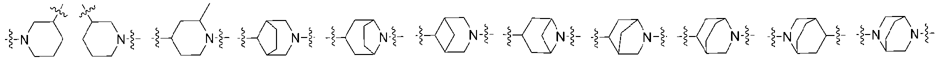 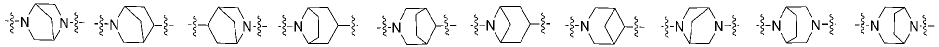 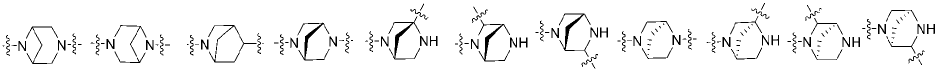 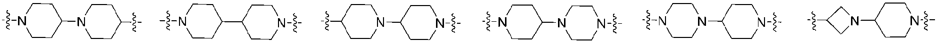 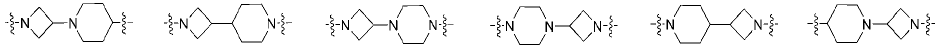 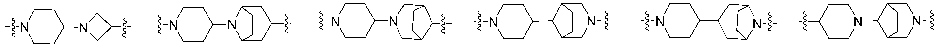 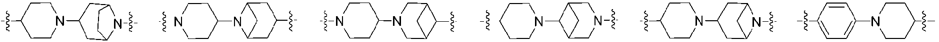 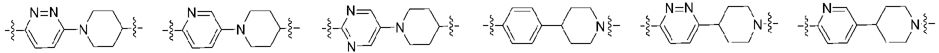 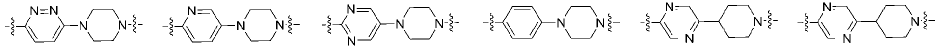 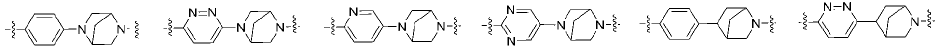 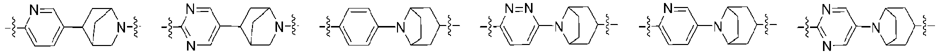 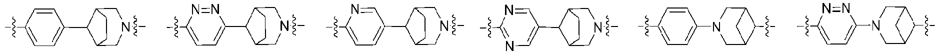 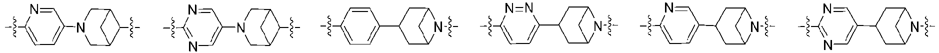 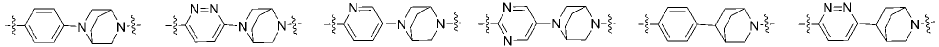 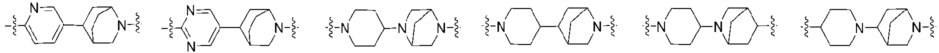 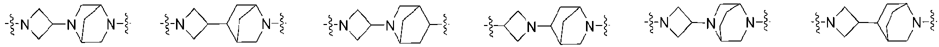 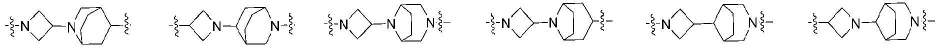 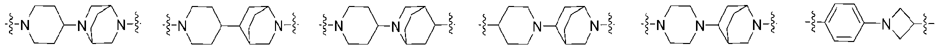 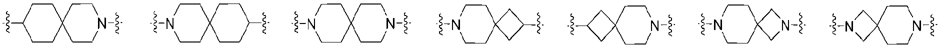 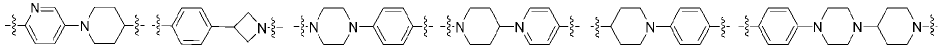 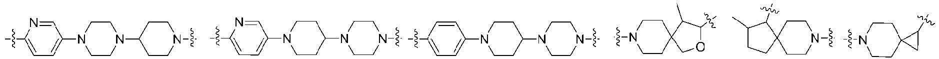 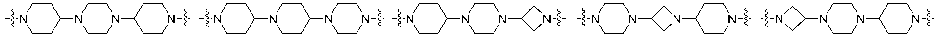 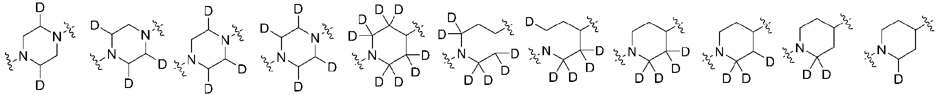 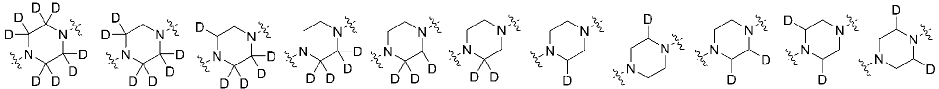 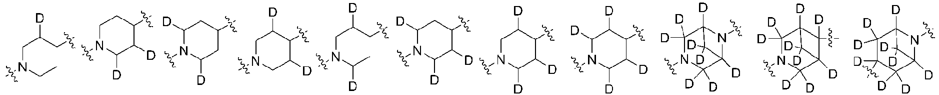 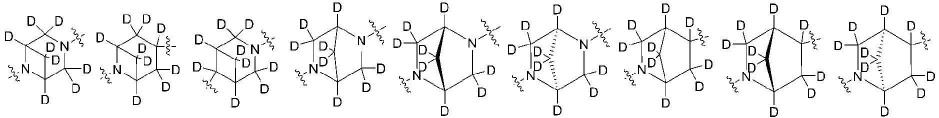 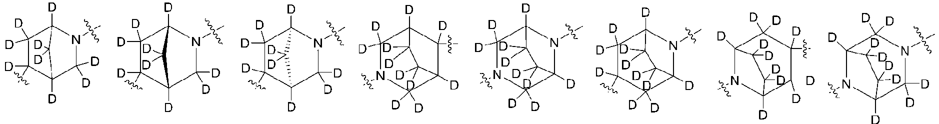 or 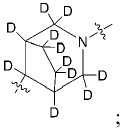 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, and C₂₋₆ alkenyl; and/or
R_{w1} represents a bond, or
R_{w1} represents:
-C₁₋₁₅ alkylene-;
-(C(Rₐ₈)(Rₐ₉))ₙ₄-(Rₐ(C(Rₐ₁₀)(Rₐ₁₁))ₙ₅)ₘ₃-;
-(C(Rₐ₈)(Rₐ₉))ₙ₄-(Rₐ(C(Rₐ₁₀)(Rₐ₁₁))ₙ₅)ₘ₃-(Rₐ(C(Rₐ₁₂)(Rₐ₁₃))ₙ₆)ₘ₄-;
-(C(Rₐ₈)(Rₐ₉))ₙ₄-(Rₐ(C(Rₐ₁₀)(Rₐ₁₁))ₙ₅)ₘ₃-(Rₐ(C(Rₐ₁₂)(Rₐ₁₃)ₙ₆)ₘ₄-(Rₐ(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅Rₐ)ₘ₃-;
-(C(Rₐ₈)(Rₐ₉))ₙ₄-((C(Rₐ₁₀)(Rₐ₁₁))ₙ₅Rₐ)ₘ₃-((C(Rₐ₁₂)(Rₐ₁₃)ₙ₆Rₐ)ₘ₄-;
-(C(Rₐ₈)(Rₐ₉))ₙ₄-((C(Rₐ₁₀)(Rₐ₁₁))ₙ₅Rₐ)ₘ₃-((C(Rₐ₁₂)(Rₐ₁₃))ₙ₆Rₐ)ₘ₄-((C(Rₐ₁₄)(Rₐ₁₅))ₙ₇Rₐ)ₘ₅-;
-(C(Rₐ₈)(Rₐ₉))ₙ₄-(R_{b}(C(Rₐ₁₀)(Rₐ₁₁))ₙ₅)ₘ₃-;
-(C(Rₐ₈)(Rₐ₉))ₙ₄-(R_{b}(C(Rₐ₁₀)(Rₐ₁₁))ₙ₅)ₘ₃-(R_{b}(C(Rₐ₁₂)(Rₐ₁₃))ₙ₆)ₘ₄-;
-(C(Rₐ₈)(Rₐ₉))ₙ₄-(R_{b}(C(Rₐ₁₀)(Rₐ₁₁))ₙ₅)ₘ₃-(R_{b}(C(Rₐ₁₂)(Rₐ₁₃))ₙ₆)ₘ₄-(R_{b}(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅R_{b})ₘ₃-;
-(C(Rₐ₈)(Rₐ₉))ₙ₄-((C(Rₐ₁₀)(Rₐ₁₁))ₙ₅R_{b})ₘ₃-((C(Rₐ₁₂)(Rₐ₁₃))ₙ₆R_{b})ₘ₄-;
-(c(Rₐ₈)(Rₐ₉))ₙ₄-((C(Rₐ₁₀)(Rₐ₁₁))ₙ₅R_{b})ₘ₃-((C(Rₐ₁₂)(Rₐ₁₃))ₙ₆R_{b})ₘ₄-((C(Rₐ₁₄)(Rₐ₁₅))ₙ₇R_{b})ₘ₅-;
-(C(Rₐ₈)(Rₐ₉))ₙ₄-(Rₐ-R_{b}-(C(Rₐ₁₀)(Rₐ₁₁))ₙ₅)ₘ₃-;
-(C(Rₐ₈)(Rₐ₉))ₙ₄-(Rₐ(C(Rₐ₁₀)(Rₐ₁₁))ₙ₅)ₘ₃-(R_{b}(C(Rₐ₁₂)(Rₐ₁₃))ₙ₆)ₘ₄-;
-(C(Rₐ₈)(Rₐ₉))ₙ₄-(R_{b}-Rₐ-(C(Rₐ₁₀)(Rₐ₁₁))ₙ₅)ₘ₃-;
-(C(Rₐ₈)(Rₐ₉))ₙ₄-(R_{b}(C(Rₐ₁₀)(Rₐ₁₁))ₙ₅)ₘ₃-(Rₐ(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁))ₙ₅-Rₐ-R_{b})ₘ₃-;
-(C(Rₐ₈)(Rₐ₉))ₙ₄-((C(Rₐ₁₀)(Rₐ₁₁))ₙ₅Rₐ)ₘ₃-((C(Rₐ₁₂)(Rₐ₁₃))ₙ₆R_{b})ₘ₄₋;
-(C(Rₐ₈)(Rₐ₉))ₙ₄-((C(Rₐ₁₀)(Rₐ₁₁))ₙ₅-R_{b}-Rₐ)ₘ₃-; or
-(C(Rₐ₈)(Rₐ₉))ₙ₄-((C(Rₐ₁₀)(Rₐ₁₁))ₙ₅R_{b})ₘ₃-((C(Rₐ₁₂)(Rₐ₁₃))ₙ₆Rₐ)ₘ₄-;
wherein each Rₐ is independently selected from the group consisting of O, C(O), OC(O), S, S(O), S(O)₂, S(O):N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxyl, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n4, n5, n6, n7, m3, m4, and m5 each independently represent an integer of 1, 2, 3, 4, 5, 6, 7, 8, 9, or 10; and
any one or more hydrogens in the main carbon chain skeleton of said C₁₋₁₅ alkylene are optionally replaced by a substituent selected from the group consisting of: deuterium, hydroxyl, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

16. The compound of Formula (I) or salts, enantiomers, stereoisomers, solvates, isotopically enriched analogs, prodrugs, or polymorphs thereof as claimed in any one of claims 1-15, wherein the R_{w1} represents a structure of the following formula:
-(C(Rₐ₈)(Rₐ₉))ₙ₄-(O(C(Rₐ₁₀)(Rₐ₁₁))ₙ₅)ₘ₃-;
-(C(Rₐ₈)(Rₐ₉))ₙ₄-(O(C(Rₐ₁₀)(Rₐ₁₁))ₙ₅)ₘ₃-(O(C(Rₐ₁₂)(Rₐ₁₃))ₙ₆)ₘ₄-;
-(C(Rₐ₈)(Rₐ₉))ₙ₄-(O(C(Rₐ₁₀)(Rₐ₁₁))ₙ₅)ₘ₃-(O(C(Rₐ₁₂)(Rₐ₁₃))ₙ₆)ₘ₄- (O(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅O)ₘ₃-;
-(C(Rₐ₈)(Rₐ₉))ₙ₄-((C(Rₐ₁₀)(Rₐ₁₁))ₙ₅O)ₘ₃-((C(Rₐ₁₂)(Rₐ₁₃))ₙ₆O)ₘ₄-;
-(C(Rₐ₈)(Rₐ₉))ₙ₄-((C(Rₐ₁₀)(Rₐ₁₁))ₙ₅O)ₘ₃-((C(Rₐ₁₂)(Rₐ₁₃))ₙ₆O)ₘ₄- ((C(Rₐ₁₄)(Rₐ₁₅))ₙ₇O)ₘ₅-;
-(C(Rₐ₈)(Rₐ₉))ₙ₄-(N(Rₐ₇)(C(Rₐ₁₀)(Rₐ₁₁))ₙ₅)ₘ₃-;
-(C(Rₐ₈)(Rₐ₉))ₙ₄-(N(Rₐ₇)(C(Rₐ₁₀)(Rₐ₁₁))ₙ₅)ₘ₃-(N(Rₐ₇)(C(Rₐ₁₂)(Rₐ₁₃))ₙ₆)ₘ₄-;
-(C(Rₐ₈)(Rₐ₉))ₙ₄-(N(Rₐ₇)(C(Rₐ₁₀)(Rₐ₁₁))ₙ₅)ₘ₃-(N(Rₐ₇)(C(Rₐ₁₂)(Rₐ₁₃))ₙ₆)ₘ₄-(N(Rₐ₇)(C(Rₐ₁₄)(Rₐ₁₅))ₙ₇)ₘ₅-;
-(C(Rₐ₈)(Rₐ₉))ₙ₄-((C(Rₐ₁₀)(Rₐ₁₁))ₙ₅N(Rₐ₇))ₘ₃-;
-(C(Rₐ₈)(Rₐ₉))ₙ₄-((C(Rₐ₁₀)(Rₐ₁₁))ₙ₅N(Rₐ₇))ₘ₃-((C(Rₐ₁₂)(Rₐ₁₃))ₙ₆N(Rₐ₇))ₘ₄-;
-(C(Rₐ₈)(Rₐ₉))ₙ₄-((C(Rₐ₁₀)(Rₐ₁₁))ₙ₅N(Rₐ₇))ₘ₃-((C(Rₐ₁₂)(Rₐ₁₃))ₙ₆N(Rₐ₇))ₘ₄-((C(Rₐ₁₄)(Rₐ₁₅))ₙ₇N(Rₐ₇))ₘ₅-;
-(C(Rₐ₈)(Rₐ₉))ₙ₄-(C(O)N(Rₐ₇)-(C(Rₐ₁₀)(Rₐ₁₁))ₙ₅)ₘ₃-;
-(C(Rₐ₈)(Rₐ₉))ₙ₄-(C(O)N(Rₐ₇)-(C(Rₐ₁₀)(Rₐ₁₁))ₙ₅)ₘ₃-(C(O)N(Rₐ₇)-(C(Rₐ₁₂)(Rₐ₁₃))ₙ₆)ₘ₄-;
-(C(Rₐ₈)(Rₐ₉))ₙ₄-(C(O)N(Rₐ₇)-(C(Rₐ₁₀)(Rₐ₁₁))ₙ₅)ₘ₃-(C(O)N(Rₐ₇)-(C(Rₐ₁₂)(Rₐ₁₃))ₙ₆)ₘ₄-(C(O)N(Rₐ₇)-(C(Rₐ₁₄)(Rₐ₁₅)ₙ₇)ₘ₅-;
-(C(Rₐ₈)(Rₐ₉))ₙ₄-((C(Rₐ₁₀)(Rₐ₁₁))ₙ₅-C(O)N(Rₐ₇))ₘ₃-;
-(C(Rₐ₈)(Rₐ₉))ₙ₄-((C(Rₐ₁₀)(Rₐ₁₁))ₙ₅-C(O)N(Rₐ₇))ₘ₃-((C(Rₐ₁₂)(Rₐ₁₃))ₙ₆-C(O)N(Rₐ₇))ₘ₄-;
-(C(Rₐ₈)(Rₐ₉))ₙ₄-((C(Rₐ₁₀)(Rₐ₁₁))ₙ₅-C(O)N(Rₐ₇))ₘ₃-((C(Rₐ₁₂)(Rₐ₁₃))ₙ₆-C(O)N(Rₐ₇))ₘ₄-((C(Rₐ₁₄)(Rₐ₁₅))ₙ₇-C(O)N(Rₐ₇))ₘ₅-;
-(C(Rₐ₈)(Rₐ₉))ₙ₄-(C(O)N(Rₐ₇)-(C(Rₐ₁₀)(Rₐ₁₁))ₙ₅)ₘ₃-(O-(C(Rₐ₁₂)(Rₐ₁₃))ₙ₆)ₘ₄-;
-(C(Rₐ₈)(Rₐ₉))ₙ₄-C(O)N(Rₐ₇)-(C(Rₐ₁₀)(Rₐ₁₁))ₙ₅-(O(C(Rₐ₁₂)(Rₐ₁₃))ₙ₆)ₘ₃-;
-(C(Rₐ₈)(Rₐ₉))ₙ₄-((C(Rₐ₁₀)(Rₐ₁₁))ₙ₅-C(O)N(Rₐ₇))ₘ₃-((C(Rₐ₁₂)(Rₐ₁₃))ₙ₆O)ₘ₄-;
-(C(Rₐ₈)(Rₐ₉))ₙ₄-(C(Rₐ₁₀)(Rₐ₁₁))ₙ₅-C(O)N(Rₐ₇)-((C(Rₐ₁₂)(Rₐ₁₃))ₙ₆O)ₘ₃-;
-(C(Rₐ₈)(Rₐ₉))ₙ₄-(N(Rₐ₇)C(O)-(C(Rₐ₁₀)(Rₐ₁₁))ₙ₅)ₘ₃-;
-(C(Rₐ₈)(Rₐ₉))ₙ₄-(N(Rₐ₇)C(O)-(C(Rₐ₁₀)(Rₐ₁₁))ₙ₅)ₘ₃-(O-(C(Rₐ₁₂)(Rₐ₁₃)))ₙ₆)ₘ₄-;
*-*(C(Rₐ₈)(Rₐ₉))ₙ₄-(N(Rₐ₇)C(O)-(C(Rₐ₁₀)(Rₐ₁₁))ₙ₅)ₘ₃-(O-(C(Rₐ₁₂)(Rₐ₁₃))ₙ₆)ₘ₄-(O-(C(Rₐ₁₄)(Rₐ₁₅))ₙ₇)ₘ₅-;
-(C(Rₐ₈)(Rₐ₉))ₙ₄-N(Rₐ₇)C(O)-(C(Rₐ₁₀)(Rₐ₁₁))ₙ₅-(O(C(Rₐ₁₂)(Rₐ₁₃))ₙ₆)ₘ₃-;
-(C(Rₐ₈)(Rₐ₉))ₙ₄-N(Rₐ₇)C(O)N(Rₐ₇)-(C(Rₐ₁₀)(Rₐ₁₁))ₙ₅-;
-(C(Rₐ₈)(Rₐ₉))ₙ₄-((C(Rₐ₁₀)(Rₐ₁₁))ₙ₅-N(Rₐ₇)C(O))ₘ₃-;
-(c(Rₐ₈)(Rₐ₉))ₙ₄-((C(Rₐ₁₀)(Rₐ₁₁))ₙ₅-N(Rₐ₇)C(O))ₘ₃-((C(Rₐ₁₂)(Rₐ₁₃)))ₙ₆-O)ₘ₄-;
-(c(Rₐ₈)(Rₐ₉))ₙ₄-((C(Rₐ₁₀)(Rₐ₁₁))ₙ₅-N(Rₐ₇)C(O))ₘ₃-((C(Rₐ₁₂)(Rₐ₁₃))ₙ₆O)ₘ₄-((C(Rₐ₁₄)(Rₐ₁₅))ₙ₇O)ₘ₅-;
-(C(Rₐ₈)(Rₐ₉))ₙ₄- (C(Rₐ₁₀)(Rₐ₁₁))ₙ₅-N(Rₐ₇)C(O)-((C(Rₐ₁₂)(Rₐ₁₃))ₙ₆O)ₘ₃-;
-(C(Rₐ₈)(Rₐ₉))ₙ₄-( arylene-(C(Rₐ₁₀)(Rₐ₁₁))ₙ₅)ₘ₃-;
-(C(Rₐ₈)(Rₐ₉))ₙ₄-( arylene-(C(Rₐ₁₀)(Rₐ₁₁))ₙ₅)ₘ₃- arylene-(C(Rₐ₁₂)(Rₐ₁₃))ₙ₆-;
-(C(Rₐ₈)(Rₐ₉))ₙ₄-((C(Rₐ₁₀)(Rₐ₁₁))ₙ₅- arylene)ₘ₃-;
-(C(Rₐ₈)(Rₐ₉))ₙ₄-((C(Rₐ₁₀)(Rₐ₁₁))ₙ₅- arylene)ₘ₃-(C(Rₐ₁₂)(Rₐ₁₃))ₙ₆- arylene-;
-(C(Rₐ₈)(Rₐ₉))ₙ₄-(heterocyclylene-(C(Rₐ₁₀)(Rₐ₁₁))ₙ₅)ₘ₃-;
-(C(Rₐ₈)(Rₐ₉))ₙ₄-(heterocyclylene-(C(Rₐ₁₀)(Rₐ₁₁))ₙ₅)ₘ₃-(heterocyclylene-(C(Rₐ₁₂)(Rₐ₁₃))ₙ₆)ₘ₄-;
-(C(Rₐ₈)(Rₐ₉))ₙ₄-((C(Rₐ₁₀)(Rₐ₁₁))ₙ₅-heterocyclylene)ₘ₃-;
-(C(Rₐ₈)(Rₐ₉))ₙ₄-((C(Rₐ₁₀)(Rₐ₁₁))ₙ₅-heterocyclylene)ₘ₃-((C(Rₐ₁₂)(Rₐ₁₃))ₙ₆-heterocyclylene)ₘ₄-;
-(C(Rₐ₈)(Rₐ₉))ₙ₄-(heteroarylene-(C(Rₐ₁₀)(Rₐ₁₁))ₙ₅)ₘ₃-;
-(C(Rₐ₈)(Rₐ₉))ₙ₄-(heteroarylene-(C(Rₐ₁₀)(Rₐ₁₁))ₙ₅)ₘ₃-(heteroarylene-(C(Rₐ₁₂)(Rₐ₁₃))ₙ₆)ₘ₄-;
~(C(Rₐ₈)(Rₐ₉))ₙ₄-((C(Rₐ₁₀)(Rₐ₁₁))ₙ₅-heteroarylene)ₘ₃-;
-(C(Rₐ₈)(Rₐ₉))ₙ₄-((C(Rₐ₁₀)(Rₐ₁₁))ₙ₅-heteroarylene)ₘ₃-((C(Rₐ₁₂)(Rₐ₁₃))ₙ₆-heteroarylene)ₘ₄-;
-(C(Rₐ₈)(Rₐ₉))ₙ₄-(cycloalkylene-(C(Rₐ₁₀)(Rₐ₁₁))ₙ₅)ₘ₃-;
-(C(Rₐ₈)(Rₐ₉))ₙ₄-(cycloalkylene-(C(Rₐ₁₀)(Rₐ₁₁))ₙ₅)ₘ₃-(cycloalkylene-(C(Rₐ₁₂)(Rₐ₁₃₎)ₙ₆)ₘ₄-;
-(C(Rₐ₈)(Rₐ₉))ₙ₄-((C(Rₐ₁₀)(Rₐ₁₁))ₙ₅-cycloalkylene)ₘ₃-; or
-(C(Rₐ₈)(Rₐ₉))ₙ₄-((C(Rₐ₁₀)(Rₐ₁₁))ₙ₅-cycloalkylene)ₘ₃-((C(Rₐ₁₂)(Rₐ₁₃))ₙ₆-cycloalkylene)ₘ₄-;
wherein each Rₐ₇ independently represents H or C₁₋₃ alkyl;
the cycloalkylene (e.g., C₃₋₂₀ cycloalkylene), arylene (e.g., C₅₋₂₀ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, or any combination thereof; and
n4, n5, n6, n7, m3, m4, and m5 each independently represent an integer of 1, 2, 3, 4, 5, 6, 7, 8, 9, or 10.

17. The compound of Formula (I) or salts, enantiomers, stereoisomers, solvates, isotopically enriched analogs, prodrugs, or polymorphs thereof as claimed in any one of claims 1-16, wherein R_{w1} represents:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, - (CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, - (CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, - (CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, - (CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, - (CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, - (CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, - (CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, - CH(CH₃)-O-(CH₂)₅-, -CH(CH₃)-O-(CH₂)₆-, -CH(CH₃)-O-(CH₂)₇-, -CH(CH₃)-O-(CH₂)₈-, -CH(CH₃)-O-(CH₂)₉-, -CH(CH₃)-O-(CH₂)₁₀-, -(O(CH₂)₂)₂-, -(O(CH₂)₂)₃-, -(O(CH₂)₂)₄-, -(O(CH₂)₂)₅-, -(O(CH₂)₂)₆-, -(O(CH₂)₂)₇-, - CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, - CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, - (CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -N(Rₐ₇)-(CH₂)₇-, -N(Rₐ₇)-(CH₂)₈-, -N(Rₐ₇)-(CH₂)₉-, - N(Rₐ₇)-(CH₂)₁₀-, -(CH₂)₂-N(Rₐ₇)-(CH₂)₁-, -(CH₂)₂-N(Rₐ₇)-(CH₂)₂-, -(CH2)2-N(Rₐ₇)-(CH₂)₃-, -(CH₂)₂-N(Rₐ₇)-(CH2)4-, -(CH2)2-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, - (CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, - (CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, - C(O)NH(CH₂)₂-, -C(O)NH(CH₂)₃-, -C(O)NH(CH₂)₄-, -C(O)NH(CH₂)₅-, -CH₂C(O)NHCH₂-, - (CH₂)₂C(O)NH(CH₂)₂-, -(CH₂)₂C(O)NH(CH₂)₃-, -(CH₂)₂C(O)NH(CH₂)₄-, -(CH₂)₂C(O)NH(CH₂)₅-, - (CH₂)₃C(O)NH(CH₂)₃-, -(CH₂)₃C(O)NH(CH₂)₄-, -(CH₂)₄C(O)NH(CH₂)₄-, -(CH₂)₅C(O)NH(CH₂)₅-, - (CH₂)₆C(O)NH(CH₂)₇-, -(CH₂)₆C(O)NH(CH₂)₆-, -(CH₂)₇C(O)NH(CH₂)₇-, -(CH₂)₂C(O)NH(CH₂)₂-O-(CH₂)₂-, - NHC(O)CH₂-, -NHC(O)(CH₂)₂-, -NHC(O)(CH₂)₃-, -NHC(O)(CH₂)₄-, -NHC(O)(CH₂)₅-, -CH₂NHC(O)CH₂-, - (CH₂)₂NHC(O)(CH₂)₂-, -(CH₂)₂NHC(O)(CH₂)₃-, -(CH₂)₂NHC(O)(CH₂)₄-, -(CH₂)₂NHC(O)(CH₂)₅-, - (CH₂)₃NHC(O)(CH₂)₃-, -(CH₂)₃NHC(O)(CH₂)₄-, -(CH₂)₄NHC(O)(CH₂)₄-, -(CH₂)₅NHC(O)(CH₂)₅-, - (CH₂)₆NHC(O)(CH₂)₇-, -(CH₂)₆NHC(O)(CH₂)₆-, -(CH₂)₇NHC(O)(CH₂)₇-, -(CH₂)₄NHC(O)(CH₂)₈-, - (CH₂)₂NHC(O)(CH₂)₂-O-(CH₂)₂-, -(CH₂)₄NHC(O)CH₂-, -phenylene-CH₂-, -phenylene-(CH₂)₂-, -phenylene-(CH₂)₃-, -phenylene-(CH₂)₄-, -phenylene-(CH₂)₅-, -phenylene-(CH₂)₆-, -phenylene-(CH₂)₇-, -phenylene-(CH₂)₈-, -CH₂-phenylene-CH₂-, -CH₂-phenylene-(CH₂)₂-, -CH₂-phenylene-(CH₂)₃-, -CH₂-phenylene-(CH₂)₄-, - CH₂-phenylene-(CH₂)₅-, -CH₂-phenylene-(CH₂)₆-, -CH₂-phenylene-(CH₂)₇-, -CH₂-phenylene-(CH₂)₈-, - (CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, - (CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, - (CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, - (CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, - (CH₂)₇-phenylene-(CH₂)₂-, -(CH₂)₇-phenylene-(CH₂)₃-, -(CH₂)₇-phenylene-(CH₂)₄-**,** -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, -(CH₂)₈-phenylene-(CH₂)₆-, -(CH₂)₈-phenylene-(CH₂)₇-, -N(Rₐ₇)-CH₂-phenylene-CH₂-, -N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, - N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, - CH₂-N(Rₐ₇)-CH₂-phenylene-(CH₂)₃-, **-**CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, - (CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, -(CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, - (CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -piperidinylene-CH₂-, -piperidinylene-(CH₂)₂-, - piperidinylene-(CH₂)₃-, -piperidinylene-(CH₂)₄-, -piperidinylene-(CH₂)₅-, -piperidinylene-(CH₂)₆-, - piperidinylene-(CH₂)₇-, -piperidinylene-(CH₂)₈-, -CH₂-piperidinylene-CH₂-, -CH₂-piperidinylene-(CH₂)₂-, - CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, - (CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, - (CH₂)₈-piperidinylene-(CH₂)₇-, -N(Rₐ₇)-CH₂-piperidinylene-CH₂-, -N(Rₐ₇)-CH₂-piperidinylene-(CH₂)₂-, - N(Rₐ₇)-CH₂-piperidinylene-(CH₂)₃-, -N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, -N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, - CH₂-N(Rₐ₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-**,** -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, - piperazinylene-CH₂-, -piperazinylene-(CH₂)₂-, -piperazinylene-(CH₂)₃-, -piperazinylene-(CH₂)₄-, - piperazinylene-(CH₂)₅-, -piperazinylene-(CH₂)₆-, -piperazinylene-(CH₂)₇-, -piperazinylene-(CH₂)₈-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxyl, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

18. The compound of Formula (I) or salts, enantiomers, stereoisomers, solvates, isotopically enriched analogs, prodrugs, or polymorphs thereof as claimed in any one of claims 1-17, wherein
LIN represents: a bond, C(O), C(O)NH, NHC(O), -CH₂-, -(CH₂)₂-, -(CH₂)₃-, -(CH₂)₄-, -(CH₂)₅-, -(CH₂)₆-, - (CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, - (CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, - (CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, - (CH₂)₁-O-(CH₂)₆-, -(CH₂)₁-O-(CH₂)₇-, -(CH₂)₁-O-(CH₂)₈-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, - (CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, - (CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, - (CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, - CH(CH₃)-O-(CH₂)₆-, -CH(CH₃)-O-(CH₂)₇-, -CH(CH₃)-O-(CH₂)₈-, -(O(CH₂)₂)₂-, -(O(CH₂)₂)₃-, -(O(CH₂)₂)₄-, - (O(CH₂)₂)₅-, -(O(CH₂)₂)₆-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -(CH₂)₂-(O(CH₂)₂)₂-, -(CH₂)₂-(O(CH₂)₂)₃-, - (CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, - (CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, - (CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -CH₂-O-(CH₂)₂-O-(CH₂)₃-, - CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, (CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, - N(Rₐ₇)-(CH₂)₅-, -N(Rₐ₇)-(CH₂)₆-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, - (CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, - (CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -C(O)NHCH₂-, -C(O)NH(CH₂)₂-, -C(O)NH(CH₂)₃-, -C(O)NH(CH₂)₄-, -C(O)NH(CH₂)₅-, - CH₂C(O)NHCH₂-, -(CH₂)₂C(O)NH(CH₂)₂-, -(CH₂)₂C(O)NH(CH₂)₃-, -(CH₂)₂C(O)NH(CH₂)₄-, - (CH₂)₂C(O)NH(CH₂)₅-, -(CH₂)₃C(O)NH(CH₂)₃-, -(CH₂)₃C(O)NH(CH₂)₄-, -(CH₂)₄C(O)NH(CH₂)₄-, - (CH₂)₅C(O)NH(CH₂)₅-, -(CH₂)₂C(O)NH(CH₂)₂-O-(CH₂)₂-, -NHC(O)CH₂-, -NHC(O)(CH₂)₂-, - NHC(O)(CH₂)₃-, -NHC(O)(CH₂)₄-, -NHC(O)(CH₂)₅-, -CH₂NHC(O)CH₂-, -(CH₂)₂NHC(O)(CH₂)₂-, - (CH₂)₂NHC(O)(CH₂)₃-, -(CH₂)₂NHC(O)(CH₂)₄-, -(CH₂)₂NHC(O)(CH₂)₅-, -(CH₂)₃NHC(O)(CH₂)₃-, - (CH₂)₃NHC(O)(CH₂)₄-, -(CH₂)₄NHC(O)(CH₂)₄-, -(CH₂)₅NHC(O)(CH₂)₅-, -(CH₂)₂NHC(O)(CH₂)₂-O-(CH₂)₂-, - (CH₂)₄NHC(O)CH₂-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, - (CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, - (CH₂)₃-phenylene-(CH₂)₅-, -(CH₂)₄-phenylene-CH₂-, -(CH₂)₄-phenylene-(CH₂)₂-, -(CH₂)₄-phenylene-(CH₂)₃-, - (CH₂)₄-phenylene-(CH₂)₄-, -(CH₂)₄-phenylene-(CH₂)₅-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, - (CH₂)₂-piperazinylene-(CH₂)₅-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -( CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, or -(CH₂)₈-piperazinylene-(CH₂)₅-; or
LIN represents: 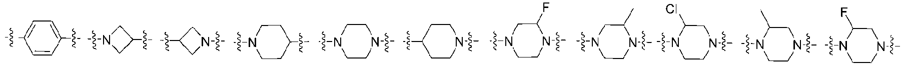 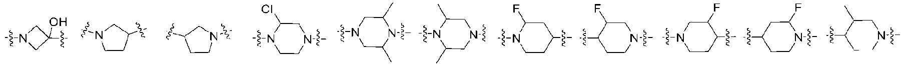 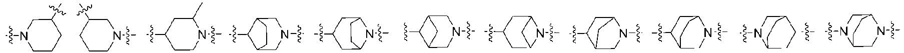 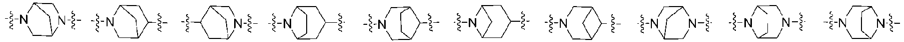 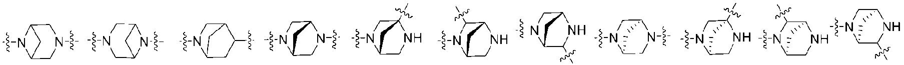 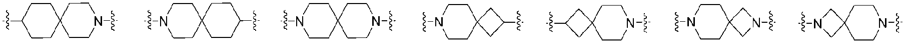 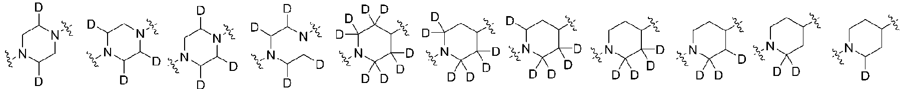 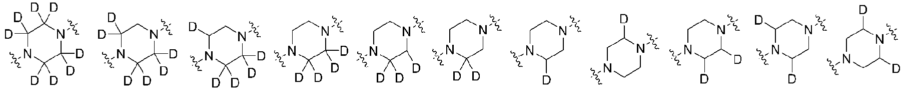 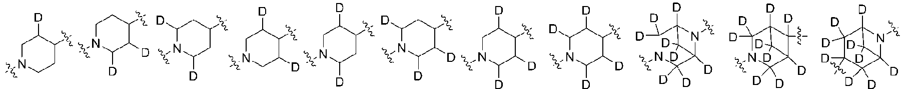 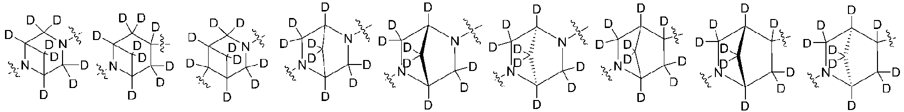 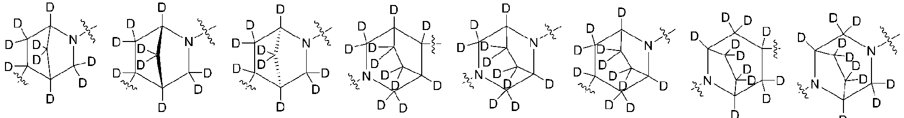 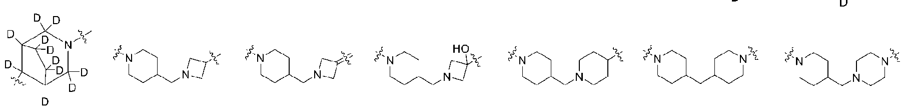 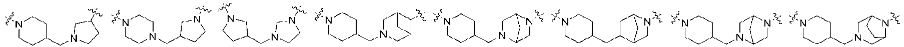 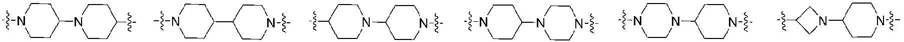 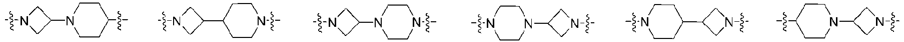 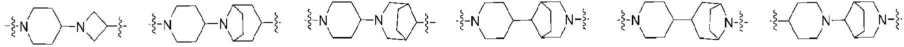 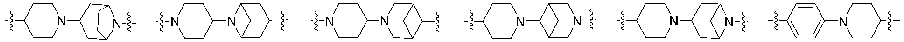 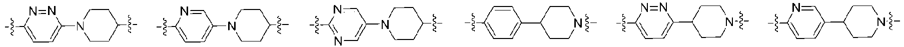 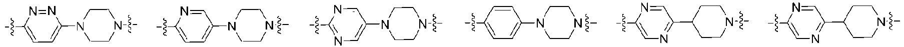 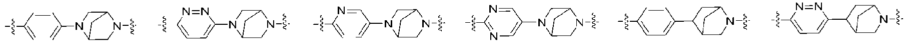 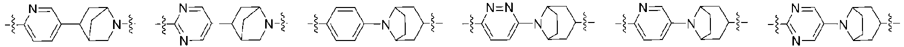 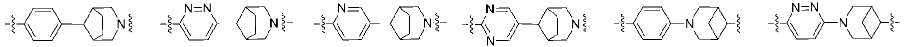 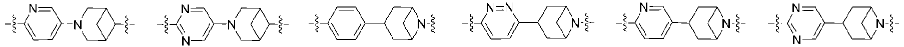 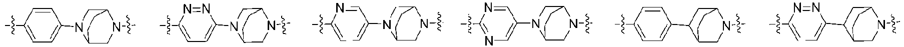 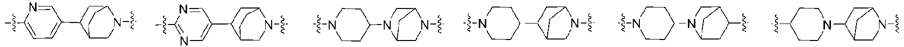 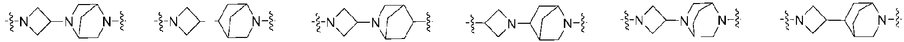 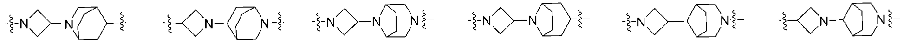 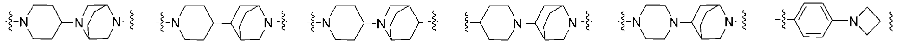 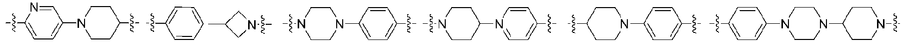 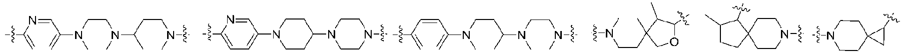 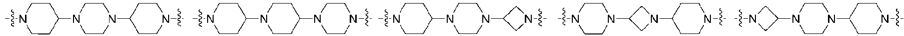 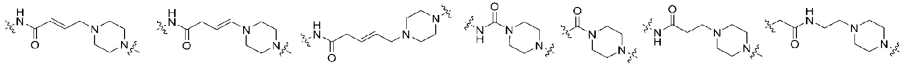 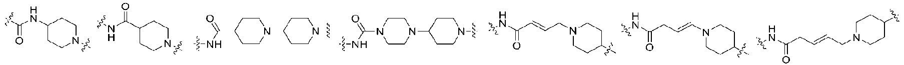 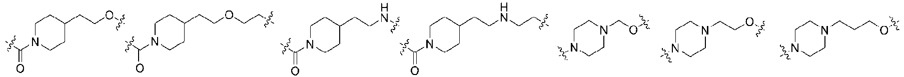 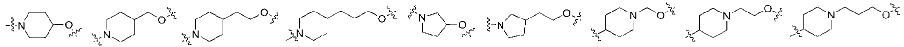 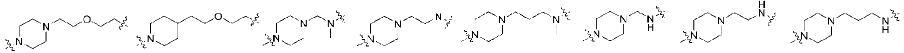 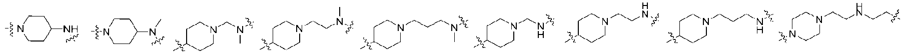 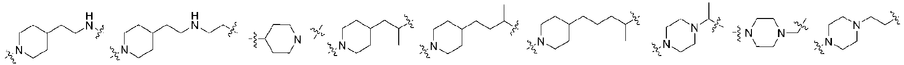 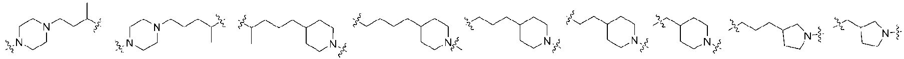 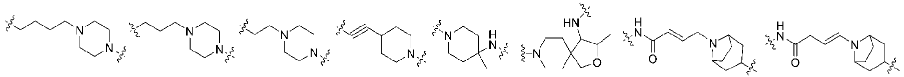 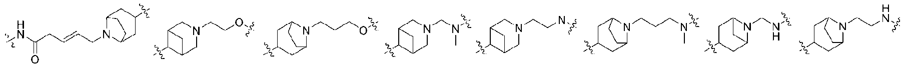 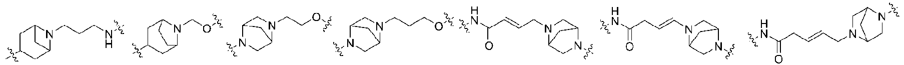 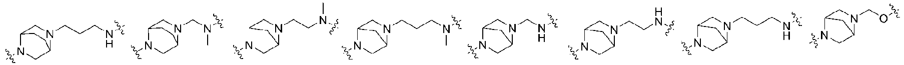 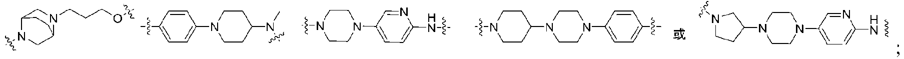 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, carboxyl, C₂₋₆ alkynyl, and C₂₋₆ alkenyl.

19. The compound of Formula (I) or salts, enantiomers, stereoisomers, solvates, isotopically enriched analogs, prodrugs, or polymorphs thereof as claimed in any one of claims 1-18, which is also represented by Formula (I-1), Formula (I-2), Formula (I-3), Formula (I-4), Formula (I-5), Formula (I-6), Formula (I-7), or Formula (I-8): 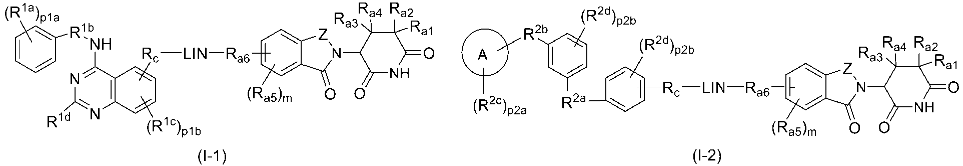 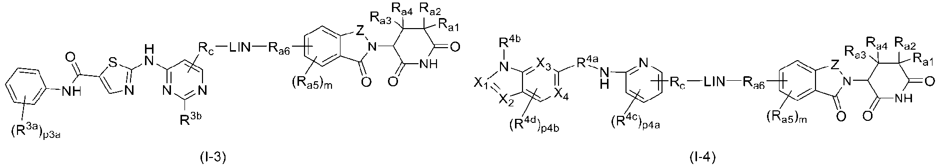 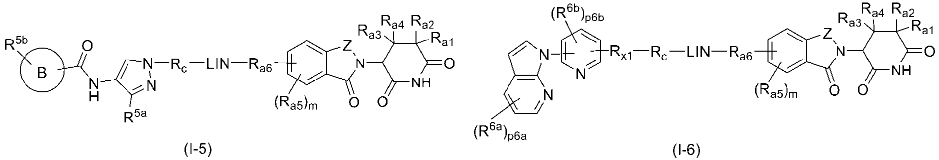 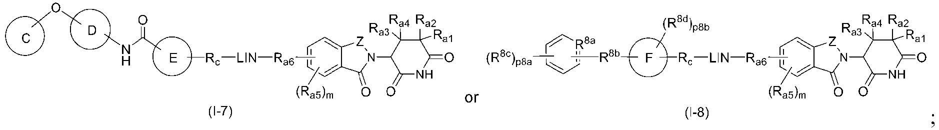 wherein Z, Rₐ₁, Rₐ₂, Rₐ₃, Rₐ₄, (Rₐ₅)ₘ, Rₐ₆, LIN, R_{c}, (R^{1a})ₚ₁ₐ, (R^{1c})_{p1b}, R^{1d}, ring A, R^{2a}, (R^{2c})ₚ₂ₐ, (R^{2d})_{p2b}, (R^{3a})ₚ₃ₐ, R^{3b}, X₁, X₂, X₃, X₄, R^{4a}, R^{4b}, (R^{4c})ₚ₄ₐ, (R^{4d})_{p4b}, ring B, R^{5a}, R^{5b}, (R^{6a})ₚ₆ₐ, (R^{6b})_{p6b}, Rₓ₁, ring C, ring D, ring E, R^{8a}, R^{8b}, (R^{8c})ₚ₈ₐ, and (R^{8d})_{p8b} are as defined in claim 3.

20. The compound of Formula (I) or salts, enantiomers, stereoisomers, solvates, isotopically enriched analogs, prodrugs, or polymorphs thereof as claimed in claim 1, which is selected from the compounds in Table 1.

21. The compound of Formula (I) or salts, enantiomers, stereoisomers, solvates, or polymorphs thereof as claimed in any one of claims 1-20, which is hydrohalides (including hydrochlorides, hydrobromides), sulfates, maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate of the compound of Formula (I).

22. A compound of Formula (II) 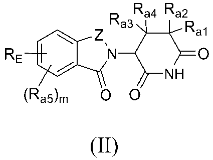 or salts, enantiomers, stereoisomers, solvates, isotopically enriched analogs, prodrugs, or polymorphs thereof,
wherein Z represents C(O) or CH₂;
Rₐ₁, Rₐ₂, Rₐ₃, and Rₐ₄ are identical or different and each independently represents hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
m represents an integer of 0, 1, 2, or 3;
R_{E} represents: 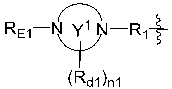
wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl, and R_{E1} represents hydrogen; or
R₁ represents a bond, and R_{E1} represents NH₂; or
R_{E} represents: 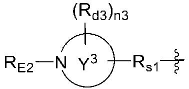
wherein ring Y³ represents heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20; and
wherein Rₛ₁ represents 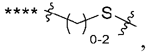 symbol **** indicates the point of attachment to ring Y³; and R_{E2} represents NH₂;
provided that the following compounds are excluded:
3-(1-oxo-5-(piperazin-1-ylamino)isoindolin-2-yl)piperidine-2,6-dione.

23. The compound of Formula (II) or salts, enantiomers, stereoisomers, solvates, isotopically enriched analogs, prodrugs, or polymorphs thereof as claimed in claim 22, wherein the compound of Formula (II) is also represented by Formula (II-1), Formula (II-2), Formula (II-3), or Formula (II-4): 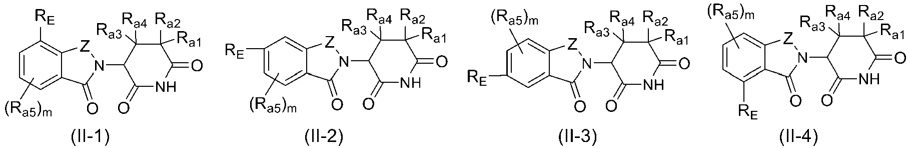 wherein Z, Rₐ₁, Rₐ₂, Rₐ₃, Rₐ₄, (Rₐ₅)ₘ, and R_{E} are as defined in claim 21.

24. The compound of Formula (II) or salts, enantiomers, stereoisomers, solvates, isotopically enriched analogs, prodrugs, or polymorphs thereof as claimed in claim 22 or 23, wherein
(i) when Z represents CH₂, m represents an integer of 0, R_{w} represents hydrogen, and ring Y¹ represents piperazinyl, ring Y¹ is substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 1-20; or
(ii) ring Y¹ represents 4- to 20-membered heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; or
(iii) ring Y³ represents 4- to 20-membered heterocyclylene containing one nitrogen atom, and (R_{d3})ₙ₃ indicates that ring Y³ is optionally substituted with n3 R_{d3} groups, wherein each R_{d3} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n3 represents an integer of 0-20.

25. The compound of Formula (II) or salts, enantiomers, stereoisomers, solvates, isotopically enriched analogs, prodrugs, or polymorphs thereof as claimed in any one of claims 22-24, wherein ring Y¹ represents 4- to 20-membered heterocyclylene containing 2 to 4 nitrogen atoms, e.g., ring Y¹ represents:
diazetidinylene, imidazolidylene, pyrazolidylene, piperazinylene, 1,2,3,4-tetrahydropyrazinylene, hexahydropyrimidinylene, hexahydropyridazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, diazaspiro[2.3]hexylene, diazaspiro[3.3]heptanylene, diazaspiro[2.4]heptanylene, diazaspiro[3.4]octanylene, diazaspiro[3.5]nonanylene, diazaspiro[4.4]nonanylene, diazaspiro[4.5]decanylene, diazaspiro[5.5]undecanylene, octahydropyrrolo[3,4-c]pyrrolylene, or decahydronaphthyridinylene, or 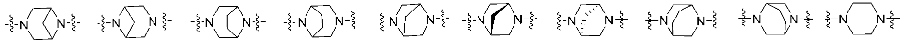 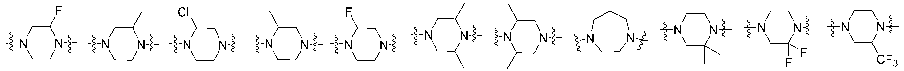 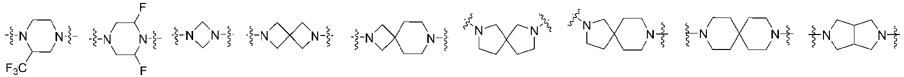 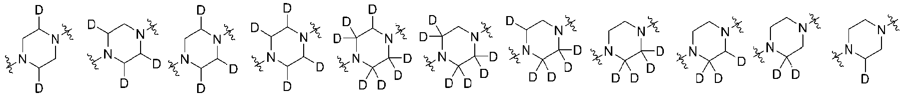 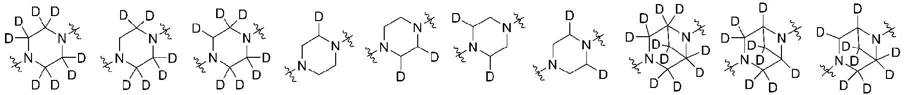 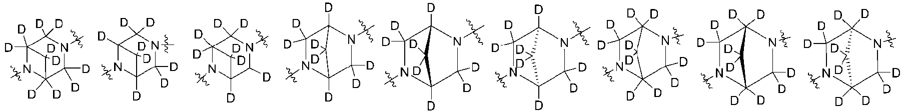 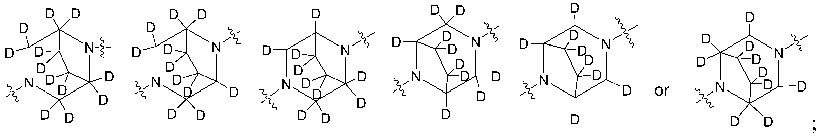
ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20.

26. The compound of Formula (II) or salts, enantiomers, stereoisomers, solvates, isotopically enriched analogs, prodrugs, or polymorphs thereof as claimed in claim 22, which is selected from the compounds in Table 2.

27. The compound of Formula (II) or salts, enantiomers, stereoisomers, solvates, or polymorphs thereof as claimed in any one of claims 22-26, which is hydrohalides (including hydrochlorides, hydrobromides), sulfates, maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate of the compound of Formula (II).

28. A pharmaceutical composition, comprising:
the compound of Formula (I) or pharmaceutically acceptable salts, enantiomers, stereoisomers, solvates, isotopically enriched analogs, prodrugs, or polymorphs thereof as claimed in any one of claims 1-21, and at least one pharmaceutically acceptable carrier; or
the compound of Formula (II) or pharmaceutically acceptable salts, enantiomers, stereoisomers, solvates, isotopically enriched analogs, prodrugs, or polymorphs thereof as claimed in any one of claims 22-27, and at least one pharmaceutically acceptable carrier;
and optionally comprising at least one additional therapeutic agent, e.g., an anticancer agent.

29. A medicine kit or reagent kit comprising:
the compound of Formula (I) or a pharmaceutically acceptable salt thereof as claimed in any one of claims 1-21; or
the compound of Formula (II) or a pharmaceutically acceptable salt thereof as claimed in any one of claims 22-27; or
the pharmaceutical composition as claimed in claim 28.

30. The compound of Formula (I) or salts, enantiomers, stereoisomers, solvates, isotopically enriched analogs, prodrugs, or polymorphs thereof as claimed in any one of claims 1-21, for use in the prevention and/or treatment of a disease or disorder selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

31. The compound of Formula (I) or salts, enantiomers, stereoisomers, solvates, isotopically enriched analogs, prodrugs, or polymorphs thereof as claimed in claim 30, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, inflammatory bowel disease (including Crohn's disease and ulcerative colitis); keratoconjunctivitis sicca; auto inflammatory diseases, including gout etc.; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

32. The compound of Formula (II) or salts, enantiomers, stereoisomers, solvates, isotopically enriched analogs, prodrugs, or polymorphs thereof as claimed in any one of claims 22-27, for use in the prevention and/or treatment of a disease or disorder associated with cereblon protein.

33. The compound of Formula (II) or salts, enantiomers, stereoisomers, solvates, isotopically enriched analogs, prodrugs, or polymorphs thereof as claimed in claim 32, wherein the disease or disorder associated with cereblon protein is selected from the group consisting of: tumors, cancers, and autoimmune diseases, e.g., myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); and autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, and inflammatory bowel disease (including Crohn's disease and ulcerative colitis).

34. Use of the compound of Formula (I) or salts, enantiomers, stereoisomers, solvates, isotopically enriched analogs, prodrugs, or polymorphs thereof as claimed in any one of claims 1-21 for the manufacture of a medicament for the prevention and/or treatment of a disease or disorder selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

35. A method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof as claimed in any one of claims 1-21, wherein the disease or disorder is selected from the group consisting of: tumors, infectious diseases, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

36. The use as claimed in claim 34, or the method as claimed in claim 35, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, inflammatory bowel disease (including Crohn's disease and ulcerative colitis); keratoconjunctivitis sicca; auto inflammatory diseases, including gout etc.; inflammatory diseases, including pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

37. Use of the compound of Formula (II) or salts, enantiomers, stereoisomers, solvates, isotopically enriched analogs, prodrugs, or polymorphs thereof as claimed in any one of claims 22-27, or 3-(1-oxo-5-(piperazin-1-ylamino)isoindolin-2-yl)piperidine-2,6-dione or salts, enantiomers, stereoisomers, solvates, isotopically enriched analogs, prodrugs, or polymorphs thereof for the manufacture of a medicament for the prevention and/or treatment of a disease or disorder associated with cereblon protein.

38. A method for treating or preventing a disease or disorder associated with cereblon protein in a subject, comprising administering to the subject a therapeutically effective amount of the compound of Formula (II) or salts, enantiomers, stereoisomers, solvates, isotopically enriched analogs, prodrugs, or polymorphs thereof as claimed in any one of claims 22-27, or the pharmaceutical composition as claimed in claim 30 or 31, 3-(1-oxo-5-(piperazin-1-ylamino)isoindolin-2-yl)piperidine-2,6-dione or salts, enantiomers, stereoisomers, solvates, isotopically enriched analogs, prodrugs, or polymorphs thereof.

39. The use as claimed in claim 37, or the method as claimed in claim 38, wherein the disease or disorder associated with cereblon protein is selected from the group consisting of: tumors, cancers, and autoimmune diseases.

40. The use as claimed in claim 37, or the method as claimed in claim 38, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); and autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type 1 diabetes mellitus, urticaria, and inflammatory bowel disease (including Crohn's disease and ulcerative colitis).

41. Use of the compound of Formula (II) or salts, enantiomers, stereoisomers, solvates, isotopically enriched analogs, prodrugs, or polymorphs thereof as claimed in any one of claims 22-27, or 3-(1-oxo-5-(piperazin-1-ylamino)isoindolin-2-yl)piperidine-2,6-dione or salts, enantiomers, stereoisomers, solvates, isotopically enriched analogs, prodrugs, or polymorphs thereof for the manufacture of a CRBN binder.

42. Use of the compound of Formula (II) or salts, enantiomers, stereoisomers, solvates, isotopically enriched analogs, prodrugs, or polymorphs thereof as claimed in any one of claims 22-27, or 3-(1-oxo-5-(piperazin-1-ylamino)isoindolin-2-yl)piperidine-2,6-dione or salts, enantiomers, stereoisomers, solvates, isotopically enriched analogs, prodrugs, or polymorphs thereof for the preparation of the compound of Formula (I) or salts, enantiomers, stereoisomers, solvates, isotopically enriched analogs, prodrugs, or polymorphs thereof as claimed in claim 1.

43. A method for preparing the compound of Formula (I) as claimed in claim 1, comprising using a compound of Formula (M1) and a compound of Formula (M2) as starting materials to obtain the compound of Formula (I): 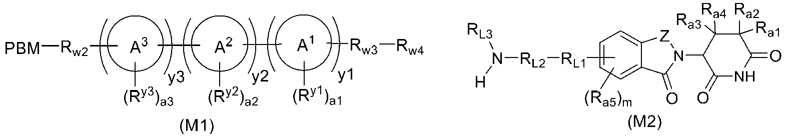 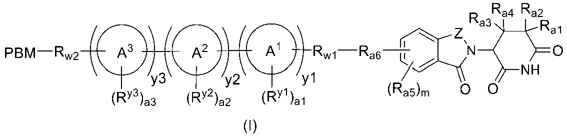
wherein PBM, Rₐ₁, Rₐ₂, Rₐ₃, Rₐ₄, (Rₐ₅)ₘ, Z, R_{w2}, ring A¹, ring A², ring A³, y1, y2, y3, (R^{y1})ₐ₁, (R^{y2})ₐ₂, and (R^{y3})ₐ₃ are as defined in claim 1; and
R_{w3} represents a bond or an optionally substituted linear or branched C₁₋₁₉ alkylene, wherein any one or more methylene groups in the main carbon chain skeleton of the linear or branched C₁₋₁₉ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₁₉ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and wherein one or more hydrogens of said linear or branched C₁₋₁₉ alkylene are optionally replaced by a substituent selected from the group consisting of deuterium, hydroxyl, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
R_{w4} represents CHO, COOH, or 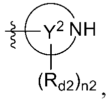 wherein ring Y² and (R_{d2})ₙ₂ are as defined in claim 1;
wherein (1) when R_{w4} represents CHO or COOH,
(a1) R_{L1} represents N(R_{w}), and R_{L2} and R_{L3}, together with the nitrogen atom to which they are attached, form 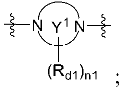 or
(a2) R_{L1} and R_{L2} together form 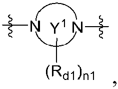 and R_{L3} represents H or C₁₋₃ alkyl; or
(a3) R_{L1} represents NH, R_{L2} represents a bond, and R_{L3} represents H; or
(a4) R_{L1} represents 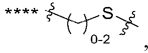 wherein the symbol **** indicates the point of attachment to R_{L2}, R_{L2} represents 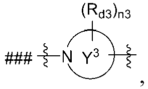 wherein the symbol ### indicates the point of attachment to N, and ring Y³ and (R_{d3})ₙ₃ are as defined in claim 1, and R_{L3} represents H or C₁₋₃ alkyl; or
wherein (2) when R_{w4} represents 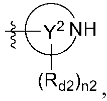 R_{L1} represents N(R_{w}), and R_{L2} and R_{L3}, together with the nitrogen atom to which they are attached, form 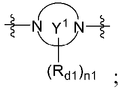 and
wherein each of the above R_{w}, ring Y¹, and (R_{d1})ₙ₁ is as defined in claim 1.

44. The method as claimed in claim 43, wherein
(1) when R_{w4} represents CHO, the compound of Formula (M1) and the compound of Formula (M2) are subjected to a reductive amination reaction to give the compound of Formula (I), wherein the moiety 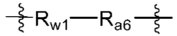 in the compound of Formula (I) correspondingly represents a structure represented by the following general formula: 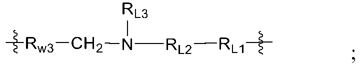
(2) when R_{w4} represents COOH, the compound of Formula (M1) and the compound of Formula (M2) are subjected to an amide condensation reaction to give the compound of Formula (I), wherein the moiety 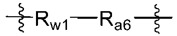 in the compound of Formula (I) correspondingly represents a structure represented by the following general formula: 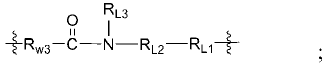 or
(3) when R_{w4} represents 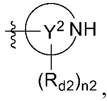 the compound of Formula (M1) and the compound of Formula (M2) are subjected to a urea-forming reaction to give the compound of Formula (I), wherein the moiety 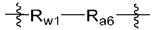 in the compound of Formula (I) correspondingly represents a structure represented by the following general formula:

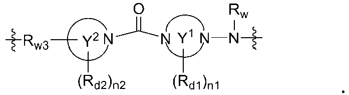

45. A method for preparing the compound of Formula (II) as claimed in claim 22, comprising using a compound of Formula (M3) and a compound of Formula (M4) as starting materials to obtain the compound of Formula (II): 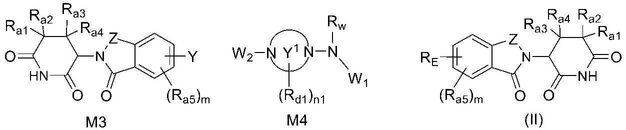
wherein Rₐ₁, Rₐ₂, Rₐ₃, Rₐ₄, (Rₐ₅)ₘ, Z, ring Y¹, R_{w}, and (R_{d1})ₙ₁ are as defined in claim 22;
Y represents F or Br;
W₁ represents H, and W₂ represents an amino protecting group; or
W₁ represents an amino protecting group, and W₂ represents H; and
R_{E} represents: 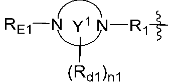
wherein ring Y¹ represents heterocyclylene containing at least two nitrogen atoms, and (R_{d1})ₙ₁ indicates that ring Y¹ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl, and R_{E1} represents hydrogen; or
R₁ represents a bond, and R_{E1} represents NH₂.
